# EUROPEAN PATENT SPECIFICATION

(11) **EP 3 390 661 B1**
(45) Date of publication and mention of the grant of the patent: **04.05.2022**
(21) Application number: 16822642.1
(22) Date of filing: 16.12.2016
(51) Int. Cl.: C12Q 1/68

(54) **USE OF ANTISENSE LONG NON-CODING RNAS FOR THE DIAGNOSIS OF PROSTATE CANCER**
VERWENDUNG VON LANGER NICHT-CODIERENDER ANTISENSE-RNA ZUR DIAGNOSE VON PROSTATAKREBS
UTILISATION D'ARN ANTISENS LONGS NON-CODANTS POUR LE DIAGNOSTIC DU CANCER DE LA PROSTATE

(30) Priority: 17.12.2015 EP 15307036
(43) Date of publication of application: 24.10.2018
(73) Proprietor: Institut Curie, 75248 Paris Cedex 05 (FR); Centre National de la Recherche Scientifique, 75016 Paris (FR); Sorbonne Université, 75006 Paris 6 (FR); Institut National de la Santé et de la Recherche Médicale, 75013 Paris (FR); Assistance Publique - Hôpitaux de Paris, 75004 Paris (FR); Universite Paris 12 - Val De Marne, 94000 Creteil (FR)
(72) Inventor: MORILLON, Antonin, 75005 Paris (FR); LONDONO-VALLEJO, Arturo, 75015 Paris (FR); ALLORY, Yves, 75011 Paris (FR); SACI, Zohra, Montréal, Québec H1T3S1 (CA)
(74) Representative: Cabinet Becker et Associés
(86) International application number: PCT/EP2016/081348
(87) International publication number: WO 2017/103047

(56) References cited:
- WO-A1-2014/160120
- WO-A2-2005/001092
- WO-A2-2012/068383
- WO-A2-2014/165818
- HO LYE LIN ET AL: "Androgen regulation of multidrug resistance-associated protein 4 (MRP4/ABCC4) in prostate cancer", PROSTATE, WILEY-LISS, NEW YORK, NY, US, vol. 68, no. 13, 1 September 2008 (2008-09-01), pages 1421-1429, XP008101316, ISSN: 0270-4137, DOI: 10.1002/PROS.20809 [retrieved on 2008-07-09]
- WEI ZHANG ET AL: "A novel urinary long non-coding RNA transcript improves diagnostic accuracy in patients undergoing prostate biopsy", PROSTATE., vol. 75, no. 6, 18 January 2015 (2015-01-18), pages 653-661, XP055278026, US ISSN: 0270-4137, DOI: 10.1002/pros.22949
- LIU YUAN ET AL: "Small molecule therapeutics targeting F-box proteins in cancer", SEMINARS IN CANCER BIOLOGY, SAUNDERS SCIENTIFIC PUBLICATIONS, PHILADELPHIA, PA, US, vol. 36, 30 September 2015 (2015-09-30), pages 105-119, XP029422099, ISSN: 1044-579X, DOI: 10.1016/J.SEMCANCER.2015.09.014
- JIN DAQING ET AL: "Prostaglandin signalling regulates ciliogenesis by modulating intraflagellar transport.", NATURE CELL BIOLOGY, vol. 16, no. 9, 31 August 2014 (2014-08-31), XP002758510, ISSN: 1476-4679, DOI: 10.1038/ncb3029
- GANG LUO ET AL: "Long Non-Coding RNA MEG3 Inhibits Cell Proliferation and Induces Apoptosis in Prostate Cancer", CELLULAR PHYSIOLOGY AND BIOCHEMISTRY., vol. 37, no. 6, 27 November 2015 (2015-11-27), pages 2209-2220, XP055277990, CH ISSN: 1015-8987, DOI: 10.1159/000438577
- H. WANG ET AL: "The mitotic regulator Hec1 is a critical modulator of prostate cancer through the long non-coding RNA BX647187 in vitro", BIOSCIENCE REPORTS, vol. 35, no. 6, 26 November 2015 (2015-11-26), pages e00273-e00273, XP055278017, US ISSN: 0144-8463, DOI: 10.1042/BSR20150003
- XIN YU ET AL: "Long non-coding RNA growth arrest-specific transcriptï¿ 5 in tumor biology (Review)", ONCOLOGY LETTERS, vol. 10, 1 January 2015 (2015-01-01), pages 1953-1958, XP055278023, GR ISSN: 1792-1074, DOI: 10.3892/ol.2015.3553
- JOHN R. PRENSNER ET AL: "The Long Non-Coding RNA PCAT-1 Promotes Prostate Cancer Cell Proliferation through cMyc", NEOPLASIA, vol. 16, no. 11, 1 November 2014 (2014-11-01), pages 900-908, XP055278036, US ISSN: 1476-5586, DOI: 10.1016/j.neo.2014.09.001
- Suyoun Chung ET AL: "Association of a novel long non-coding RNA in 8q24 with prostate cancer susceptibility", CANCER SCIENCE, vol. 102, no. 1, 1 January 2011 (2011-01-01), pages 245-252, XP055736122, JP ISSN: 1347-9032, DOI: 10.1111/j.1349-7006.2010.01737.x

## Description

### Field of the Invention

The present invention relates to the field of medicine, in particular of oncology. It provides new diagnostic markers in prostate cancer.

### Background of the Invention

Over the decade, cancer of the prostate has become the most commonly diagnosed malignancy among men and the second leading cause of male cancer deaths in the western population, following lung cancer.

Early detection and treatment of prostate cancer before it has spread from the prostate gland reduces the mortality of the disease. This realization has prompted increasing efforts for early diagnosis and treatment. Indeed, the American Cancer Society recommend that male population at large undergo annual screening for prostate cancer beginning at age 50. The recommended age for screening is lowered to 40 for men giving a family history of prostate cancer or other risk factors.

Early screening and detection could also translate into a reduction in the healthcare burden, as early treatment can be less radical, more successful and therefore provided at a lower cost per treated patient. The key to accomplish this goal is to provide better differential diagnostic tools.

Nowadays, screening for prostate cancer involves mainly palpation of the prostate by digital rectal examination and assay of plasma levels of prostate specific antigen (PSA). PSA is a serine protease produced by the prostatic epithelium that is normally secreted in the seminal fluid to liquefy it. Disruption of the anatomy integrity of the prostate gland can compromise the cellular barriers that normally restrict PSA to within the duct system of the prostate, allowing it to disperse into blood or urine. A number of conditions can result in this leakage of PSA, including inflammation of the prostate, urinary retention, prostatic infection, benign prostatic hyperplasia, and prostate cancer. It is therefore not surprising that screening of serum PSA as an indicator of prostate cancer is not absolutely predictive.

This low level of specificity results in additional more invasive and costly diagnostic procedures. Indeed, the normal procedure for a subject positive to PSA and palpation is to proceed to a biopsy which consists in 10 to 15 extractions of prostatic tissue. Among the subjects tested, only 45% appear to really have a cancer and 10 % develop a prostate infection after the biopsy. Biopsies, when unnecessary, are also very traumatic for the patients. The psychological impact of being diagnosed as positive until proven as a false positive should not be understated either. Moreover, even a biopsy is not always 100% certain, and a second biopsy procedure is often required.

High-throughput RNA sequencing has produced catalogues of long non-coding RNAs (lncRNAs) with now 58648 IncRNAs annotated (Iyer MK et al, Nat Genet, 2015, 47, pp. 199-208). LncRNA are RNA of at least 200 nucleotides long, cell type/tissue specific and poorly conserved during evolution. Among different classes of lncRNAs, natural antisense transcripts are the less described with only 4200 annotated antisense lncRNAs (Derrien T et al, Genome Res, 2012, 22, pp. 1775-1789), certainly due to their low expression. In addition, most genome-wide studies were focused on lncRNAs without addressing their strand-specificity explaining a lack of systematic characterization of antisense transcriptome.

LncRNAs have been shown to exhibit oncogenic or tumor suppressive functions in cancer biology. Being highly tissue specific and deregulated during tumorigenesis, an increasing number of lncRNAs were also proposed as biomarkers for diagnostic, prognostic and/or monitoring purposes. For example, WO 2014/160120 describes PDLIM5 as a marker correlated to prostate cancer, WO2014/165818, WO2005/001092 and Ho LL et al., (The Prostate, 2008, Wiley-Liss, NY, vol.68 no.13, pages 1421-1429) describe ABCC4 as a prostate cancer marker, and Zhang et al, (Prostate 2015, vol. 75 no.6 pages 653-661) describes PCA3 as a biomarker for the detection of prostate cancer. So far, only one lncRNA, PCA3, received the FDA approval for decisions to perform a biopsy in a prostate cancer (Groskopf J et al, Clinical chemistry, 2006, 52, pp. 1089-1095). Although addition of the PCA3 score globally improves diagnostic accuracy, it remains inaccurate in some tumours, especially among old patients and patients with advanced cancer, especially cancer relapse.

In view of the fact that advanced prostate cancer remains a life threatening disease reaching a very significant proportion of the male population and the number of unnecessary biopsies, there is a strong need to provide the most specific, selective, and rapid prostate cancer detection methods and kits. Thus, further investigations are still needed to identify new biomarkers and to evaluate their clinical performances. The present invention seeks to meet these and other needs.

### Summary of the Invention

The scope of the present invention is defined by the claims and any information that does not fall within the claims is provided for information only.

The inventors have discovered three antisense long non coding RNAs that present more robustness and better diagnostic precision of prostate cancer than the actual diagnostic PCA3 marker.

Accordingly, in a first aspect, the present disclosure concerns the use of at least one antisense long non-coding RNA (antisense lncRNA) or a fragment thereof of at least 50 nucleotides as a diagnosis marker for prostate cancer, wherein said at least one antisense IncRNA is selected from the group consisting of asFBXL7, asABCC4 and asPDLIM5.

Preferably, at least three antisense lncRNAs are used in combination and said combination comprises asFBXL7, asABCC4 and asPDLIM5.

The present disclosure also concerns, in a second aspect, an *in vitro* method for prostate cancer diagnosis of a subject, wherein the method comprises the step of determining the amount of at least one antisense IncRNA or a fragment thereof of at least 50 nucleotides in a biological sample from said subject, said at least one antisense IncRNA being selected from the group consisting of asFBXL7, asABCC4 and asPDLIM5, and wherein an increased amount of the at least one antisense IncRNA is indicative of prostate cancer.

Preferably, the amounts of at least three antisense lncRNAs are determined and said at least three antisense lncRNAs comprise asFBXL7, asABCC4 and asPDLIM5.

Optionally, the amounts of additional prostate cancer markers or fragments thereof are determined, preferably antisense lncRNAs prostate cancer markers or fragments thereof, more preferably the PCA3 antisense IncRNA or a fragment thereof.

Preferably, the sample is a body fluid, more preferably a urine sample.

Preferably, the antisense IncRNA amount is determined by amplification or by hybridization, more preferably by quantitative RT-PCR or by the Nanostring method, even more preferably by the Nanostring method.

The subject is a mammal, preferably a human. In particular, the subject is a man, preferably an adult man, more preferably a man of at least 50 years old.

The present disclosure also concerns, in a third aspect, a kit for the diagnosis of prostate cancer in a subject, wherein the kit comprises (i) probes and/or primers capable to specifically hybridize to asFBXL7; and/or (ii) probes and/or primers capable to specifically hybridize to asABCC4; and/or (iii) probes and/or primers capable to specifically hybridize to asPDLIM5; and, optionally, a leaflet providing guidelines to use such a kit.

Optionally, the kit further comprises probes and/or primers for the detection of additional prostate cancer markers, preferably probes and/or primers for the detection of antisense lncRNAs prostate cancer markers, more preferably probes and/or primers for the detection of the PCA3 antisense lncRNA.

Preferably, for each antisense lncRNA, the kit comprises two probes:
- A capture-probe which comprises a nucleotide sequence hybridizing a first part of the antisense IncRNA and a molecule able to bind a solid support; and
- A reporter-probe which comprises a nucleotide sequence hybridizing a second part of the antisense IncRNA and a detectable label.

The present disclosure also concerns, in a fourth aspect, the use of a kit as described above in the diagnosis of prostate cancer in a subject.

Preferably, the subject is an animal, more preferably a mammal, even more preferably a human.

In a most preferred aspect, the subject is an adult man of at least 50 years old.

### Brief Description of the Drawings

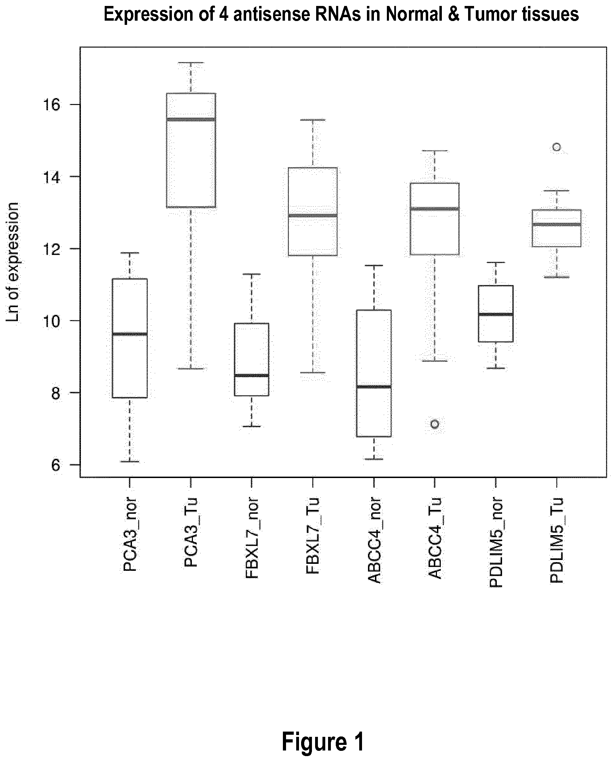

**Figure 1****:** Novel antisense lncRNAs are overexpressed in prostate cancer. PCA3, a known antisense lncRNA, and three new antisense lncRNAs (asFBXL7, asABCC4 and asPDLIM5) are highly expressed in a set of tumors (16 samples) compared to normal tissues (8 samples), measured by RNA-seq.
   Boxplots of the expression of the three new antisense lncRNAs are compared to PCA3. Normal tissues are referenced with the suffix "nor" (e.g. PCA3_nor), prostate cancer tissues are referenced with the suffix "Tu" (e.g. PCA3_Tu). The mean is indicated by a horizontal bar in each box plot. Dispersion of expression is represented by vertical dash lines. The three novel antisense IncRNA have much less dispersion than PCA3.

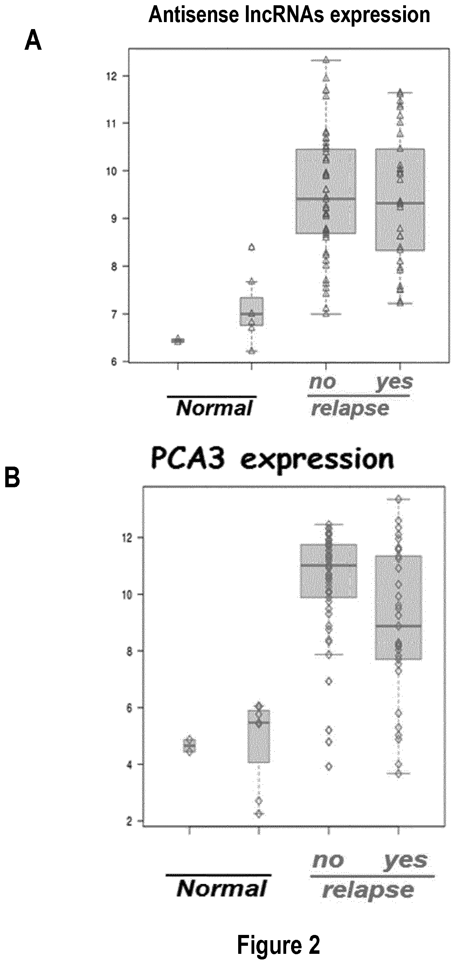

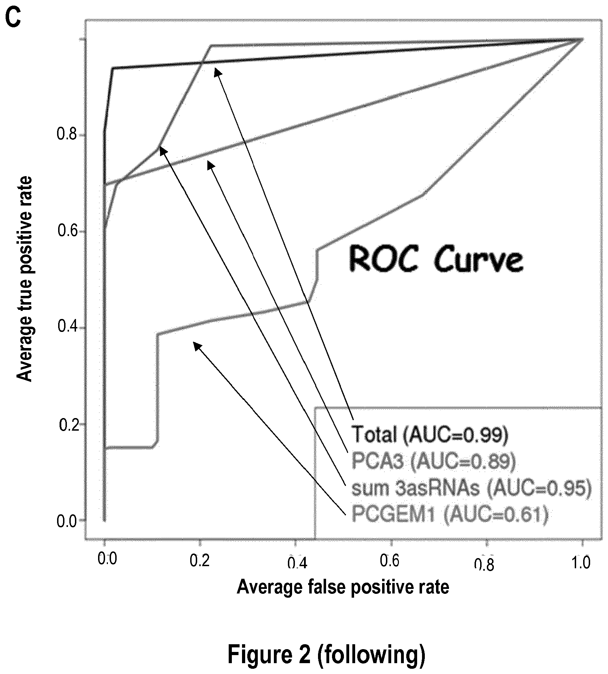

**Figure 2****:** Antisense lncRNAs are a statistically significant novel signature for detection of prostate tumors. (A) The 3 antisense IncRNA are highly expressed in tumors samples from patients with or without relapse. Nanostring measured RNA levels in 89 cohort of patients using 3 probes against asFBXL7, asABCC4 and asPDLIM5 normalized on references mRNA expression. Signals are expressed in relative expression scale for each patient (triangle). The mean is indicated by a horizontal bar in each box plot. Normal tissues are from healthy patient (first box) or from healthy region of prostate with cancer. No and yes represent patient without or with relapse after 10 years. (B) PCA3 is highly expressed in tumor samples but with less dispersion and higher levels in non-relapse patients. Same as Figure 2a but with PCA3 dedicated probes. (C) ROC curve for detection of relapse prostate tumors within a cohort of 89 patients using PCGEM3 lncRNA, *PCA3* alone, sum of novel 3 antisense lncRNAs (asFBXL7/asABCC4/asPDIML5) or total of all the 4 transcripts (without PCGEM3) measured by NanoString. AUC (Area Under the Curve) values indicate the specificity of each set to detect high-risk tumors. Value higher than 0.90 are statistically relevant.

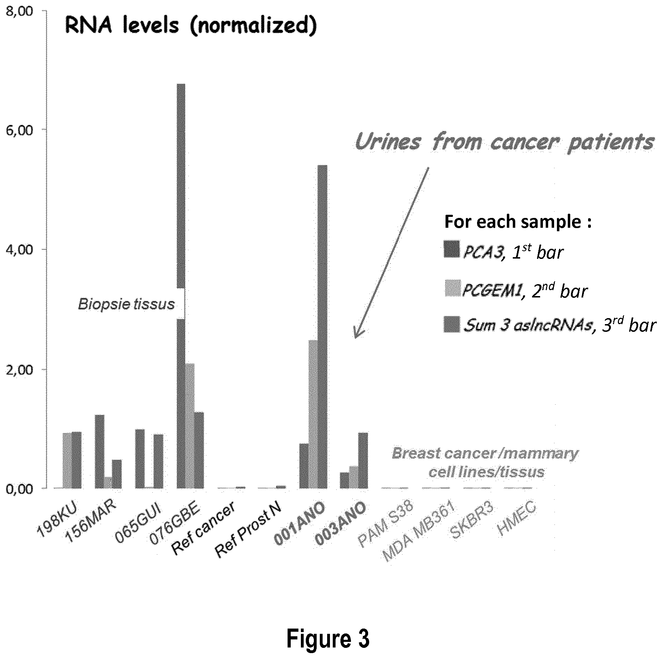

**Figure 3****:** The 3 antisense IncRNA are detectable in urines of cancer patients. Nanostring measured RNA levels in samples extracted from prostate biopsies tissues (198KU, 156MAR, 065GUI and 076GBE), commercial samples mixing different cancers (Ref cancer, a mix of Adenocarcinoma, mammary gland, Hepatoblastoma, liver Adenocarcinoma, cervix Embryonal carcinoma, testis Gliobastoma, brain Melanoma Liposarcoma, Histiocytic lymphoma, macrophage, histocyte, Lymphoblastic leukemia, T lymphoblast, Plasmacytoma myeloma, B lymphocyte),normal prostate RNAs (Ref ProstN), urines of 2 prostate cancer patients (001ANO and 003ANO), or breast cancer cell lines or tissues (PAMS38, MDA MB361, SKBR3 and HMEC) patients using a PCA3 probe (first bar of each sample results), a PCGEM1 probe (second bar of each sample results), or 3 probes against asFBXL7, asABCC4 and asPDLIM5 (third bar of each sample results). Results are normalized on references mRNA expression.

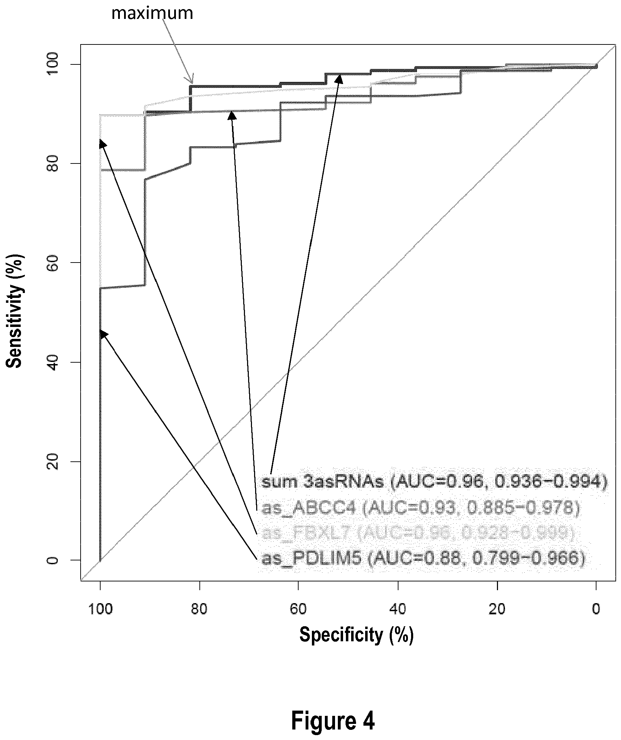

**Figure 4****:** Prediction of high sensitivity and specificity of the 3 antisense lncRNA. ROC curves for prediction of sensitivity and specificity of prostate cancer diagnosis within a cohort of 186 patients using as FBXL7 lncRNA, asABCC4 lncRNA, asPDIML5 lncRNA, and a sum of the three antisense lncRNAs (asFBXL7/asABCC4/asPDIML5) measured by NanoString. AUC (Area Under the Curve) values indicate the specificity and sensitivity of prostate cancer diagnosis of each set. Value higher than 0.85 are statistically relevant.

### Detailed description of the Invention

Recent data have uncovered a great number of long non-coding RNAs (IncRNAs). However, until now, antisense lncRNAs have been poorly studied despite their importance in disease and tissue specificity. By performing RNA-sequencing analyses, the inventors have discovered three novel antisense transcripts that are highly specific of prostate cancers and can be used as diagnostic tools for cancerous lesions. These new antisense lncRNAs appear to present more robustness and better diagnostic precision than the actual diagnostic PCA3 marker, recently approved by FDA.

The actual diagnosis procedure, i.e. prostate palpation and PSA assay followed by biopsy, leads to 55% of misadapted biopsies (the subjects tested doesn't have a cancer). With the inclusion of the test of these three new antisense IncRNA in the procedure, the percentages of misadapted biopsy fall under 10%. This new diagnosis test is highly specific (more than 90% of the biopsies are positives), rapid (results are obtained the day of the urine collection), and cost effective (unnecessary biopsies are avoided).

### Definitions

As used herein, the term "antisense lncRNAs" refers to a subset of long non-coding RNAs being natural antisense transcripts.

Long non-coding RNAs are non-protein coding, actively transcribed single-stranded RNAs. Mainly nuclear, IncRNA transcripts are subjected to processing in a manner analogous to mRNAs: they are capped, polyadenylated and frequently spliced. They are also cell type/tissue specific and poorly conserved during evolution. Their length, more than 200 nucleotides, distinguishes lncRNAs from small regulatory RNAs such as microRNAs (miRNAs), short interfering RNAs (siRNAs), Piwi-interacting RNAs (piRNAs), small nucleolar RNAs (snoRNAs), and other short RNAs.

As used herein, the terms "antisense IncRNA", "(as)lncRNA", and "Natural Antisense Transcripts (NATs)" are used interchangeably and have the same meaning. An antisense IncRNA is a IncRNA that is a natural antisense transcript of a given gene, which means that the sequence of an antisense IncRNA is complementary to the pre-mRNA sequence of said given gene. Therefore, the sequence of the antisense IncRNA cannot be complementary to artificial sequences, such as morpholinos, that are antisense to said given gene.

The term "pre-antisense IncRNA", as used herein, refers to the nascent antisense lncRNA, prior to its maturation (capping, polyadenylation and splicing).

As used herein, the term "splicing" refers to a modification of a pre-RNA transcript in which introns are removed and exons are joined. "Alternative splicing" refers to a particular splicing process that can create a range of unique RNA splicing products from the same pre-RNA. Alternative splicing can occur in many ways, exons can be extended or skipped, or introns can be retained.

The term "cancer" or "tumor", as used herein, refers to the presence of cells possessing characteristics typical of cancer-causing cells, such as uncontrolled proliferation, and/or immortality, and/or metastatic potential, and/or rapid growth and/or proliferation rate, and/or certain characteristic morphological features. This term refers to any type of malignancy (primary or metastases) in any type of subject. In particular, the term encompasses prostate cancer at any stage of progression.

As used herein, the term "diagnosis" refers to the determination as to whether a subject is likely to be affected of a prostate cancer. The skilled artisan often makes a diagnosis on the basis of one or more diagnosis markers, the presence, absence, or amount of which is indicative of the presence or absence of the prostate cancer. By "diagnosis" is also intended to refer to the providing of information useful for diagnosis.

As used herein, the term "diagnosis markers" and "biomarkers" are interchangeable and refer to biological parameters that aid the diagnosis of prostate cancer. It is a measurable indicator of the presence of this disease. This term refers particularly to "tumor diagnosis markers". Tumor diagnosis markers are substances that are produced by cancer or by other cells of the body in response to cancer. Most tumor diagnosis markers are produced by normal cells in the absence of cancer as well; however, they are produced at much higher levels in cancerous conditions. These substances can be found in the blood, urine, stool, tumor tissue, or other tissues or bodily fluids of some patients with cancer.

As used herein, the term "treatment", "treat" or "treating" refers to any act intended to ameliorate the health status of patients such as therapy, prevention, prophylaxis and retardation of the disease.

As used herein, the term "effective amount" refers to a quantity of a pharmaceutical composition which prevents, removes or reduces the deleterious effects of the prostate cancer.

As used herein, the term "nucleic acid molecule" or "nucleic acid" refer to an oligonucleotide, nucleotide or polynucleotide. A nucleic acid molecule may include deoxyribonucleotides, ribonucleotides, modified nucleotides or nucleotide analogs in any combination.

As used herein, the term "nucleotide" refers to a chemical moiety having a sugar (modified, unmodified, or an analog thereof), a nucleotide base (modified, unmodified, or an analog thereof), and a phosphate group (modified, unmodified, or an analog thereof). Nucleotides include deoxyribonucleotides, ribonucleotides, and modified nucleotide analogs including, for example, locked nucleic acids ("LNAs"), peptide nucleic acids ("PNAs"), L-nucleotides, ethylene-bridged nucleic acids ("ENAs"), arabinoside, and nucleotide analogs (including abasic nucleotides).

As used herein, the term "sequence identity" or "identity" refers to an exact nucleotide to nucleotide correspondence of two polynucleotides. Percent of identity can be determined by a direct comparison of the sequence information between two molecules by aligning the sequences, counting the exact number of matches between the two aligned sequences, dividing by the length of the shorter sequence, and multiplying the result by 100.

As used herein, the term "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (e.g., A to T, A to U, C to G). 100% complementary refers to the situation in which each nucleotide unit of one polynucleotide strand or region can hydrogen bond with each nucleotide unit of a second polynucleotide strand or region. Less than perfect complementarity refers to the situation in which some, but not all, nucleotide units of two strands or two regions can hydrogen bond with each other and can be expressed as a percentage.

The term "hybridization", as used herein, refers to "nucleic acid hybridization". Nucleic acid hybridization depends on a principle that two single-stranded nucleic acid molecules that have complementary base sequences will form a thermodynamically favored double-stranded structure if they are mixed under the proper conditions. The double-stranded structure will be formed between two complementary single-stranded nucleic acids even if one is immobilized.

As used herein, the term "amplification" refers to the amplification of a sequence of a nucleic acid. It's a method for generating large amounts of a target sequence. In general, one or more amplification primers are annealed to a nucleic acid sequence. Using appropriate enzymes, sequences found adjacent to, or in between the primers are amplified.

The term "probe", as used herein, means a strand of DNA or RNA of variable length (about 20-1000 bases long) which can be labelled. The probe is used in DNA or RNA samples to detect the presence of nucleotide sequences (the DNA or RNA target) that are complementary to the sequence in the probe.

The term "primer", as used herein, means a strand of short DNA sequence that serves as a starting point for DNA synthesis. The polymerase starts polymerization at the 3'-end of the primer, creating a complementary sequence to the opposite strand. "PCR primers" are chemically synthesized oligonucleotides, with a length between 10 and 30 bases long, preferably about 20 bases long.

As used herein, the term "complementary DNA" (cDNA) refers to recombinant nucleic acid molecules synthetized by reverse transcription of a RNA molecule, for example an antisense lncRNA.

As used herein, the term "hybridizing conditions" is intended to mean those conditions of time, temperature, and pH, and the necessary amounts and concentrations of reactants and reagents, sufficient to allow at least a portion of complementary sequences to anneal with each other. As it is well known in the art, the time, temperature, and pH conditions required to accomplish hybridization depend on the size of the oligonucleotide probe or primer to be hybridized, the degree of complementarity between the oligonucleotide probe or primer and the target, the nucleotide type (e.g., RNA, or DNA) of the oligonucleotide probe or primer and the target, and the presence of other materials in the hybridization reaction mixture. The actual conditions necessary for each hybridization step are well known in the art or can be determined without undue experimentation. General parameters for specific (i.e., stringent) hybridization conditions for nucleic acids are described in Sambrook, et al., Molecular Cloning: A Laboratory Manual (3rd Edition, 2001). One of skills in the art will in particular appreciate that as the oligonucleotides become shorter, it may become necessary to adjust their length to achieve a relatively uniform melting temperature for satisfactory hybridization results.

The terms "quantity," "amount," and "level" are used interchangeably herein and may refer to an absolute quantification of a molecule in a sample, or to a relative quantification of a molecule in a sample, i.e., relative to another value such as relative to a reference value as taught herein, or to a range of values for the biomarker. These values or ranges can be obtained from a single patient or from a group of patients.

In a first aspect, the disclosure relates to the use of at least one antisense IncRNA or a fragment thereof as a diagnosis marker for prostate cancer, wherein said at least one antisense IncRNA is selected from the group consisting of asFBXL7, asABCC4 and asPDLIM5, and wherein said fragment has a length of at least 50 nucleotides.

In a second aspect, the present disclosure also concerns an *in-vitro* method for prostate cancer diagnosis of a subject, wherein the method comprises the step of determining the amount of at least one antisense IncRNA or a fragment thereof in a biological sample from said subject, said at least one antisense IncRNA being selected from the group consisting of asFBXL7, asABCC4 and asPDLIM5, wherein said fragment has a length of at least 50 nucleotides, and wherein an increased amount of the at least one antisense IncRNA is indicative of prostate cancer.

### Antisense IncRNA

As used herein, the term "asFBXL7" encompasses the pre-antisense IncRNA having the nucleotide sequence of SEQ ID No.1, the different antisense lncRNAs obtainable by splicing and alternative splicing of the pre-antisense IncRNA and the RNAs having a nucleotide sequence identity to the pre-antisense IncRNA or to any of its splicing products of at least 90%, preferably of at least 95%, more preferably of at least 99%. The antisense IncRNA asFBXL7 is transcribed from a non-coding and non-annotated gene present on chromosome 5 from coordinate 15902312 to 15824308. Its sequence is complementary to the pre-mRNA of the FBXL7 (F-box and leucine-rich repeat protein 7) gene (Gene ID: 23194) but shorter than the sense gene.

As used herein, the term "asABCC4" encompasses the pre-antisense IncRNA having the nucleotide sequence of SEQ ID No.2, the different antisense lncRNAs obtainable by splicing or alternative splicing of the pre-antisense IncRNA and the RNAs having a nucleotide sequence identity to the pre-antisense IncRNA or to any of its splicing products of at least 90%, preferably of at least 95%, more preferably of at least 99%. In particular, two splicing isoforms are disclosed in SEQ ID No.28 and SEQ ID No.29. The antisense IncRNA asABCC4 is transcribed from a non-coding and non-annotated gene present on chromosome 13 from coordinate 95925901 to 95948506. Its sequence is complementary but shorter to the pre-mRNA of the ABCC4 (ATP-binding cassette, sub-family C (CFTR/MRP), member 4) gene (Gene ID: 10257).

As used herein, the term "asPDLIM5" encompasses the pre-antisense IncRNA having the nucleotide sequence of SEQ ID No.3, the different antisense lncRNAs obtainable by splicing or alternative splicing of the pre-antisense IncRNA and the RNAs having a nucleotide sequence identity to the pre-antisense IncRNA or to any of its splicing products of at least 90%, preferably of at least 95%, more preferably of at least 99%. In particular, a splicing isoform disclosed in SEQ ID No.30. The antisense IncRNA asPDLIM5 is transcribed from a non-coding and non-annotated gene present on chromosome 4 from coordinate 95545972 to 95507306. Its sequence is complementary but shorter to the pre-mRNA of the PDLIM5 (PDZ and LIM domain 5) gene (Gene ID: 10611).

In a preferred aspect, the above mentioned use and method relate to a combination of at least two antisense lncRNA, said combination comprising two RNAs selected from the group consisting of asFBXL7, asABCC4 and asPDLIM5. For instance, the combination can be selected from the group consisting of asFBXL7 and asABCC4, asFBXL7 and asPDLIM5, and asABCC4 and asPDLIM5.

In a more preferred aspect, the above mentioned use and method relate to a combination of at least three antisense lncRNA, said combination comprising asFBXL7, asABCC4 and asPDLIM5.

The above mentioned combination may further comprise additional antisense lncRNA, preferably antisense IncRNA which are diagnosis marker for prostate cancer, more preferably PCA3 (Gene ID: 50652).

In a particular aspect, the above mentioned use or method relate to a combination of no more than ten antisense lncRNAs or fragments thereof, preferably no more than 5 lncRNA.

The above mentioned use and method refer to an antisense IncRNA or a fragment thereof. As used herein, the term "fragment" refers to a portion of an antisense IncRNA constituted of consecutive nucleotides. Preferably, said fragment has a length of at least 50 nucleotides, more preferably of at least 60, 70, 80, 90, 100, 150, 200, 250, 500, 750, 1000 nucleotides. In a most preferred aspect, said fragment has a length of at least 100 nucleotides. Preferably, the fragment is located in an exonic sequence of a pre-antisense IncRNA and, more preferably in a sequence combining at least two exons.

### Subject

As used herein, the terms "subject", "individual" or "patient" are interchangeable and refer to an animal, preferably to a mammal, even more preferably to a human. However, the term "subject" can also refer to non-human animals, in particular mammals such as dogs, cats, horses, cows, pigs, sheep and non-human primates, among others, that are in need of diagnostic.

In a preferred aspect of the above mentioned method, the subject is a man, preferably an adult man, more preferably a man of at least 50 years old, even more preferably a man of at least 60 years old.

In a particular aspect of the above mentioned method, the subject has a family history of prostate cancer or other risk factors. In this case, the subject can be a man of at least 40 years old.

In another particular aspect of the above mentioned method, the subject has relapse from a previous prostate cancer.

In a preferred aspect of the above mentioned method, the subject is positive to a digital-rectal examination and/or to a prostate specific antigen (PSA) test.

In a preferred aspect of the above mentioned method, the subject is periodically submitted to a prostate cancer diagnosis, preferably once a year, alternatively every two years or every three years.

In a particular aspect of the above mentioned method, when the subject has a family history of prostate cancer or other risk factors, the subject is submitted to a prostate cancer diagnosis twice a year.

### Diagnosis method

The above mentioned method comprises a step of determining the amount of at least one antisense IncRNA or a fragment thereof in a biological sample from the subject.

Prior to this step, the method may further comprise a step of obtaining or providing a sample from the subject.

The method may also comprise a step of preparing or extracting the nucleic acids, preferably the ribonucleic acids, from the sample. The amount of at least one antisense IncRNA may then be quantified in the preparation of RNAs extracted from the sample.

### Sample

The term "biological sample", as used herein, means any sample containing antisense lncRNAs derived from the subject. Examples of such biological samples include fluids such as blood, plasma, urine, seminal fluid samples or mixed urine and seminal fluid (first urine sample following ejaculation) as well as biopsies, organs, tissues or cell samples, in particular a prostate sample. Preferably, the biological sample is a blood or a urine sample. More preferably, the biological sample is a urine sample.

In one aspect of the above mentioned method, the sample, preferably a urine sample, is obtained after an attentive digital-rectal examination (DRE) and/or a prostate specific antigen (PSA) test, preferably a urine sample is obtained if the PSA test and/or the digital-rectal examination were indicative of prostate cancer.

In a particular aspect of the above mentioned method, the urine sample is obtained just after a digital-rectal examination. Indeed, the digital-rectal examination increases the amount of prostatic cells and prostatic cell fragments in the urine sample, thereby facilitating the detection of the antisense lncRNAs.

Of course, it should be understood that the present method can also be used on a sample obtained without being preceded by a digital-rectal examination and/or PSA test.

A urine preservative can be added to the sample, preferably a Norgen tubes preservative (ref. 18113 Norgen bioteck corporation), allowing the conservation of the sample at room temperature for 2 years.

### Extraction

Several methods and kits are available for the person skilled in the art to extract the nucleic acids, and more particularly the ribonucleic acids, contained in the sample. For example, extraction may rely on lytic enzymes or chemical solutions or can be done with nucleic-acid-binding resins following the manufacturer's instructions.

In one aspect of the above mentioned method, the cells, cell fragments and exosomes present in the sample, preferably a urine sample, are collected, preferably by centrifugation. A total nucleic acid extraction is then carried out. Non-limiting example are a phenol/chloroform or Trizol extraction methods. Total nucleic acid extraction may also be carried out using a solid phase band method on silica beads. Of course, it should be understood that numerous nucleic acid extraction methods exist and thus, that other methods can be used in accordance with the present disclosure.

As used herein, the term "exosome" refers to any kind of cell-derived vesicles present in biological fluids, including blood and urine. This term encompasses "microvesicles", "epididimosomes", "argosomes", "exosome-like vesicles", "microparticles", "promininosomes", "prostasomes", "dexosomes", "texosomes", "dex", "tex", "archeosomes" "oncosomes", "liposomes" and "micelles".

Preferably, after total nucleic acid extraction, DNA is degraded so as to conserve only the RNA molecules. In particular, a deoxyribonuclease may be used to degrade DNA.

A variety of nucleic acids quantification techniques, well known by the skilled person, can be used to determine the amount of at least one antisense IncRNA from a biological sample, and in particular from the RNAs extracted from said sample. These techniques can be adapted in accordance with the type of sample, the sensitivity of the quantification desired, the amount of nucleic acid in the sample, and the like.

In particular, measurement of the amount of at least one antisense IncRNA can be direct or indirect. Indeed, the amount of an antisense lncRNAs can be directly quantified, preferably by hybridization, more preferably by hybridization of a labeled specific probe, still more preferably by hybridization of a fluorescent labeled specific probe immobilized directly or indirectly on a solid support, even more preferably by the Nanostring method.

Alternatively, the amount of an antisense IncRNA can be determined indirectly, especially after its conversion to cDNA, preferably by amplification, especially a quantitative amplification, more preferably by an amplification method coupled to real-time detection of the amplified products, even more preferably by quantitative RT-PCR.

Other methods of amplification include ligase chain reaction (LCR), transcription-mediated amplification (TMA), strand displacement amplification (SDA), nucleic acid sequence based amplification (NASBA), and microarray analysis. These methods are well known by the skilled person.

In a most preferred aspect, the amount of at least one antisense IncRNA is determined by the nanostring method.

### Labeled probes

The method for determining the amount of at least one antisense IncRNA may involve probes, in particular labeled probes.

The term "label", as used herein, refers to any atom or molecule that can be used to provide a quantifiable signal and that can be attached to a nucleic acid via a covalent bond or noncovalent interaction (e.g., through ionic or hydrogen bonding, or via immobilization, adsorption, or the like).

The probes of the present disclosure can be labeled by standard labeling techniques such as with a radiolabel, enzyme label, fluorescent label, biotin-avidin label, chemiluminescent label, and the like. After hybridization, the probes can be visualized using known methods. In particular, labels generally provide signals detectable by fluorescence, chemiluminescence, radioactivity, colorimetry, mass spectrometry, X-ray diffraction or absorption, magnetism, enzymatic activity, or the like.

Preferably, the detectable label may be a luminescent label. For example, fluorescent labels, bioluminescent labels, chemiluminescent labels, and colorimetric labels may be used in the practice of the disclosure, more preferably a fluorescent label.

The terms "fluorescent label", "fluorophore", "fluorogenic dye", "fluorescent dye" as used herein are interchangeable and designate a functional group attached to a nucleic acid that will absorb energy of a specific wavelength and re-emit energy at a different, but equally specific, wavelength.

Fluorescent labels that can be used in the context of this disclosure include, but are not limited to, fluorescein, a phosphor, a rhodamine, or a polymethine dye derivative. Additionally, commercially available fluorescent labels including, but not limited to, fluorescent phosphoramidites such as FluorePrime (Amersham Pharmacia, Piscataway, N.J.), Fluoredite (Miilipore, Bedford, Mass.), FAM (ABI, Foster City, Calif.), and Cy3 or Cy5 (Amersham Pharmacia, Piscataway, N.J.) can be used. The fluorescent label can be made of a combination of fluorescent labels.

In one aspect of the above described method, the probe is immobilized on a solid support. Examples of such solid supports include, but are not limited to, plastics such as polycarbonate, complex carbohydrates such as agarose and sepharose, and acrylic resins, such as polyacrylamide and latex beads. Techniques for coupling nucleic acid probes to such solid supports are well known in the art.

### Quantitative RT-PCR

In one aspect of the above mentioned method, the amount of at least one antisense IncRNA is quantified using an RNA reverse-transcription and amplification method. In such an aspect, the RNA amplification method is coupled to a quantitative amplification such as real-time detection of the amplified products using fluorescence specific probes. Preferably, the amplification method is PCR. More preferably, the PCR is a quantitative PCR or a related method enabling detection in real-time of the amplified products.

Among the amplification methods, quantitative reverse transcription PCR (quantitative RT-PCR) is preferred.

As used herein, the terms "quantitative RT-PCR", "qRT-PCR", "Real time RT-PCR" and "quantitative Real time RT-PCR" are equivalent and can be used interchangeably. Likewise, the terms "quantitative PCR", "qPCR", "Real time PCR" and "quantitative real time PCR" are equivalent and can be used interchangeably.

Any of a variety of published quantitative RT-PCR protocols can be used (and modified as needed) for use in the present method. Suitable quantitative RT-PCR procedures include but are not limited to those presented in U.S. Pat. No. 5,618,703 and in U.S. Patent Application No. 2005/0048542-

In a preferred aspect of the above mentioned method, the quantitative RT-PCR includes two main steps, the reverse transcription (RT) of RNA in cDNA and the quantitative PCR (Polymerase Chain Reaction) amplification of the cDNA.

Quantitative RT-PCR can be performed by an uncoupled or by a coupled procedure. In an uncoupled quantitative RT-PCR, the reverse transcription is performed independently from the quantitative PCR amplification, in separate reactions. Whereas, in a coupled quantitative RT-PCR, the reverse transcription and the quantitative PCR amplification are performed in a single reaction tube using a common reaction mixture including both the reverse transcriptase and the DNA polymerase. The method of the disclosure encompasses all versions of quantitative RT-PCR.

The term "reaction mixture" or "master mix" or "master mixture" refers to an aqueous solution of constituents in a quantitative RT-PCR reaction that can be constant across different reactions. In case of a coupled procedure, an exemplary "quantitative RT-PCR reaction mixture" includes buffer, a mixture of deoxyribonucleoside triphosphates, reverse transcriptase, RT and PCR primers, probes, and DNA polymerase. In case of uncoupled procedure, two reaction mixtures are needed, a "RT reaction mixture" including, for example, buffer, a mixture of deoxyribonucleoside triphosphates, reverse transcriptase and RT primers, and a "quantitative PCR" or "PCR reaction mixture" including, for example, buffer, a mixture of deoxyribonucleoside triphosphates, PCR primers, probes, and DNA polymerase.

The RNA or cDNA templates have to be added to theses reaction mixtures.

### Reverse transcription

The reverse transcription includes three basic steps: (1) denaturation of RNA, (2) hybridizing of the RT primers, (3) synthesis of cDNA.

The term "RT primers", as used herein, mean oligonucleotide primers that anneal to RNA and allow reverse transcriptase to elongate it into cDNA. Two different types of RT primers may be used, non-specific oligonucleotide RT primers or sequence specific oligonuclotide RT primers.

Using non-specific oligonucleotide RT primers such as Oligo dT (specific of polyA tail of RNAs) or random primers (for example hexamers) allows the reverse transcription of the most of the RNA present in the sample.

Using sequence specific RT primers (primers complementary to a sequence of the target RNA) allows the reverse-transcription of only the RNAs of interest. In particular, a sequence specific RT primer can be a primer which has a nucleic acid sequence complementary to the antisense IncRNA target but not to the corresponding mRNA, allowing the specific reverse transcription of the antisense lncRNA. However, to conduct a reverse transcription with a sequence specific RT primer, a multiplexing (use of several primers targeting several distinct RNA molecules) or multiple RT reactions are needed. In the latter case a greater amount of RNA is needed.

Preferably, the reverse transcription is non-specific, more preferably the RT primers used are random primers.

Reverse transcriptase enzymatic activity provides a cDNA transcript from an RNA template. There are many reverse transcriptases that are commercially available (e.g., Moloney Murine Leukemia Virus (M-MuLV) Reverse Transcriptase from New England Biolabs, Inc., Beverly, Mass.; HIV reverse transcriptase from Ambion, Inc., Austin, Tex.). The methods of the disclosure are not limited to any particular enzyme, although some enzymes may be preferable under specific conditions. The two most commonly used reverse transcriptases are avilo myeloblastosis virus reverse transcriptase (AMV-RT) and Moloney murine leukemia virus reverse transcriptase (MLV-RT).

Prior to the quantitative amplification by PCR, in case of an uncoupled procedure:
- the reverse transcription may further comprises a step of RNA degradation, for example by addition of an RNA nuclease to the reaction mixture; and/or
- the reverse transcription may further comprise a step of inactivating the reverse transcriptase.

### Quantitative PCR

Quantitative PCR allows quantification of reaction products for each sample per cycle. Commonly used instrumentation and software products perform the quantification calculations automatically. The PCR process generally consists in the repetition of a sequence of temperature changes (or cycle) conducted by a thermal cycler. Usually, 25 to 50 cycles are needed.

These cycles normally consist of three stages: the first (denaturation), at around 95 °C, allows the separation of the nucleic acids double chain; the second (alignment), at a temperature of around 50-60 °C, allows the binding of the PCR primers with the DNA template; the third (elongation), at between 68 - 72 °C, facilitates the polymerization carried out by the DNA polymerase. Due to the small size of the fragments amplified in quantitative PCR, the last step is usually omitted as the enzyme is able to increase their number during the change between the alignment stage and the denaturing stage. Preferably, a step of fluorescence measurement may be added. The temperatures and the timings used for each cycle depend on a wide variety of parameters, such as the DNA polymerase used, the concentration of divalent ions and deoxyribonucleotides in the reaction mixture and the binding temperature of the primers.

The recent advancement in PCR instrumentation technology (e.g., Cepheid's Smart Cycler^{®} II), allows the simultaneous detection and quantification of different fluorescent signals in different channels (PCR multiplex) in real-time. In addition, the latest generation of thermal cyclers are designed to maximize fluorescent dye excitation providing a more accurate means of detecting fluorescence. Thus, multiple amplification products can be assessed in the same reaction mixture and quantified more accurately. For example, the amounts of at least three antisense IncRNA as defined above can be simultaneously determined by quantitative RT-PCR.

The term "PCR primer" or "primer pair", as used herein, are equivalent and mean a pair of oligonucleotide primers that are complementary to the sequences within a target cDNA sequence in a PCR. The primer pair consists of a forward primer and a reverse primer which nucleic acid sequences are complementary to a minus (reverse) and a plus (forward) strand of the double stranded cDNA fragment of interest, respectively.

Primers of a primer pair should have similar hybridizing conditions and in particular similar melting temperatures since annealing in a PCR occurs for both simultaneously. A primer with a Tm (melting temperature) significantly higher than the reaction's annealing temperature may mishybridize and extend at an incorrect location along the DNA sequence, while a primer with a Tm significantly lower than the annealing temperature may fail to anneal and will not extend at all. Primer sequences also need to be chosen to uniquely select for a region of DNA, avoiding the possibility of mishybridization to a similar sequence nearby.

It is not necessary that every nucleotide of the PCR primers anneal to the template to allow the amplification of the cDNA. For example, a non-complementary nucleotide fragment may be attached to the 5' end of the PCR primers with the remainder of the primer sequence being complementary to the cDNA. Alternatively, non-complementary bases can be interspersed into the PCR primer, provided that the primer sequence has sufficient complementarity with the cDNA. Thus, the aspects of the disclosure contemplate variants of the PCR primers described herein.

In one particular aspect of the above mentioned method, the primers span the 3' region of a first exon and the 5' region of a second exon, so as such primers can only amplify sequences which the first and second exon have been spliced into a contiguous position (i.e. by removing an intervening intronic sequence). Knowing the sequences of the exon boundaries, as well as those of the different exons, the primers which can be designed and used in the context of the present disclosure can be readily determined by a person of ordinary skill in the art to which the present disclosure pertains.

In a preferred aspect of the above mentioned method, a primer pair consists in primers that are complementary to either exon or intron sequences of the pre-antisense lncRNA, but are obligatory out of exons of the corresponding pre-mRNA. Such primers cannot hybridize to the cDNA reversed transcript from said mRNA or any sequence deriving from it. Preferably, the primers are complementary to exon sequences of the pre-antisense IncRNA and are obligatory out of exons of the corresponding pre-mRNA.

This primer pair allows the specific amplification of the cDNA reversed transcript from the antisense lncRNA.

Many DNA polymerases suitable for quantitative PCR are commercially available (e.g., Taq and T7 DNA polymerases from New England Biolabs, Inc.; Pfu DNA polymerase from Promega, Inc., Madison, Wis.). The method of the disclosure is not limited to any particular enzyme, although some enzymes may be preferable under specific conditions.

The term "probe", in the context of the quantitative RT-PCR, refers to an oligonucleotide that hybridizes to a target sequence situated between the annealing sites of the two primers of the primer pair. The probe includes a detectable label, e.g., a fluorophore (Texas-Red^{®}, Fluorescein isothiocyanate, etc.) that can be covalently attached directly to the probe oligonucleotide, e.g., located at the probe's 5' end or at the probe's 3' end. The probe may also include a quencher. A probe includes about 8 nucleotides, about 10 nucleotides, about 15 nucleotides, about 20 nucleotides, about 30 nucleotides, about 40 nucleotides, or about 50 nucleotides. In some aspects, a probe includes from about 8 nucleotides to about 15 nucleotides.

The term "quenching", as used herein, refers to a decrease in fluorescence of a fluorescent detectable label caused by energy transfer associated with a quencher moiety, regardless of the mechanism. Suitable quencher moiety is for example Black Hole Quencher^{™} (Biosearch Technologies, Novato, Calif.) and Iowa Black (Integrated DNA Technologies, Coralville, Iowa).

Four different probe systems are in current use for quantitative PCR-Molecular Beacons (Sigma-Genosys, Inc., The Woodlands, Tex.), Scorpions^{®} (DxS Ltd., Manchester, UK), SYBR^{®} Green (Molecular Probes, Eugene, Oreg.), and TaqMan^{®} (Applied Biosystems, Foster City, Calif.). These four systems employ fluorescent labels that the instrumentation detects and the software interprets levels of fluorescence.

SYBR^{®} Green is a fluorescent dye that only strongly fluoresces when bound to double stranded DNA. SYBR^{®} green assay is a quick method, particularly useful for detection.

Molecular Beacons, Scorpions^{®}, and TaqMan^{®} utilize Förster Resonance Energy Transfer (FRET) by coupling a fluorescent label with a quencher moiety. A fluorescent label is covalently bound to the 5' end of an oligonucleotide probe, while the 3' end has a quencher moiety attached. These oligonucleotide probes are site specific to hybridize to the amplified product. Preferably, the oligonucleotide probes are designed to hybridize to a central region of the amplified product.

For TaqMan^{®} assays, the 5'→3' exonuclease activity of the DNA polymerase cleaves the probe during the elongation cycle. Due to the cleavage of the probe, the quencher moiety is no longer coupled to the fluorescence label and cannot quench fluorescence. One molecule of reporter fluorescent dye is liberated for each new molecule synthesized, and detection of the unquenched reporter fluorescent dye provides the basis for quantitative interpretation of the data. Fluorescence thus represents replicating DNA.

Similarly, Molecular Beacons utilizes an oligonucleotide probe with a fluorescent label attached to the 5' end and a quencher moiety attached to the 3' end. When free in solution, the Molecular Beacons oligonucleotide probe forms a hairpin structure. In the hairpin structure, the quencher moiety is able to quench fluorescence due to FRET. However, during PCR, the oligonucleotide probe unfolds and hybridizes to its complementary DNA, and the quencher is no longer close enough to the fluorescent label to quench fluorescence. Thus, fluorescence reports the hybridization between an oligonucleotide probe and its specific target cDNA.

Scorpions^{®} also utilize an oligonucleotide probe with a fluorescent label attached to the 5' end and a quencher moiety attached to the 3' end in a hairpin structure free in solution. However, the Scorpions^{®} oligonucleotide probe also serves as a primer. The Scorpions^{®} oligonucleotide probe/primer extends from the hairpin loop structure and hybridizes to the target. The Scorpions^{®} oligonucleotide probe/primer fluoresces, and the DNA polymerase extends the target DNA from the primer. Thus, the probe detects the extension product, which is its own primer-unimolecular rearrangement. Thereby, the fluorescence reports the extension and thus copy number of reaction product.

In a preferred aspect, the present method employs a SYBR^{®} Green fluorescent dye. Alternatively, the present method employs TaqMan^{®}-style probes, i.e. dual-labeled probes aimed to fluoresce upon 5'→3' exonuclease activity.

### Analysis of the quantitative RT-PCR results

RT-PCR can be performed using commercially available equipment, such as, for example, ABI PRISM 7700 sequence detection system. (Perkin-Elmer-Applied Biosystems, Foster City, Calif., USA), or Lightcycler (Roche Molecular Biochemicals, Mannheim, Germany). The system consists of a thermocycler, laser, charge-coupled device (CCD), camera and computer. The system includes software for running the instrument and for analyzing the data (determining the quantity of amplification product based upon the fluorescence data).

To minimize errors and the effect of sample-to-sample variations, quantitative RT-PCR is usually performed using an internal reference. The ideal internal reference is expressed at a constant level among different samples, and is unaffected by the experimental treatment. RNAs most frequently used to normalize patterns of gene expression are mRNAs for the housekeeping genes glyceraldehyde-3-phosphate-dehydrogenase (GAPDH) and beta-actin.

A standard curve can be generated from a cDNA of known concentration. The standard curve can then be used to determine absolute or relative cDNA levels.

The comparative cycle threshold (Ct) method, also known as the 2-ΔΔCt method, allows to quantify cDNA levels. Fluorescence values are recorded during every cycle and represent the amount of product amplified to that point in the amplification reaction. The point when the fluorescent signal is first recorded as statistically significant is the threshold cycle (Ct).

The Ct method compares a test reaction with a control or calibrator sample. The Ct values of both the control/calibrator sample and the test sample are normalized. In an aspect of the disclosure, the Ct values may be normalized to an arbitrary cutoff (e.g. 20-22).

The Ct method can also be described by the formula ΔΔCt=ΔCttest sample-ΔCt reference sample. The amplification efficiencies of the test sample and the reference sample must be about the same for the formula to operate. Amplification efficiencies can be determined by a comparison of the samples with template dilution. The amplification efficiency is about the same when a plot of cDNA dilution versus ΔCt approximates zero.

In one aspect of the above mentioned method, the amount of at least one antisense IncRNA is determined by quantitative RT-PCR, wherein said quantitative RT-PCR comprises the steps of:
- reverse transcription of the RNAs present in the sample into cDNAs; and
- amplification of the cDNA reverse transcript from the at least one antisense IncRNA or a part of said cDNA by quantitative PCR, wherein said part has a length of at least 50 nucleotides.

Preferably, said reverse transcription comprises a step of contacting said RNAs with RT primers, more preferably random primers.

Preferably, said amplification comprises a step of contacting said cDNA with at least one primer pair, more preferably said amplification further comprises the step of contacting said cDNA with at least one labeled probe. The labeled probe of the disclosure is complementary to either exon or intron sequences of the pre-antisense lncRNA, but is obligatory out of exons of the corresponding sense pre-mRNA. More preferably, the labeled probe is complementary to exon sequences of the pre-antisense IncRNA and is obligatory out of exons of the corresponding pre-mRNA. Thus, the labeled probe of the disclosure is able to label the pre-antisense IncRNA but not the corresponding sense pre-mRNA.

Preferably, the at least one primer pair consists in primers that are complementary to either exon or intron sequences of the pre-antisense lncRNA, but are obligatory out of exons of the corresponding sense pre-mRNA. More preferably, the primers are complementary to exon sequences of the pre-antisense IncRNA and are obligatory out of exons of the corresponding pre-mRNA. Thus, primer pairs of the disclosure are able to amplify the pre-antisense IncRNA but not the corresponding sense pre-mRNA.

Even more preferably, said at least one primer pair is selected from the group consisting of:
- the primers of SEQ ID No.4 and SEQ ID No.5, wherein said primers are specific of the cDNA reverse transcript from asABCC4; and
- the primers of SEQ ID No.6 and SEQ ID No.7, wherein said primers are specific of the cDNA reverse transcript from asABCC4; and
- the primers of SEQ ID No.8 and SEQ ID No.9, wherein said primers are specific of the cDNA reverse transcript from asPDLIM5; and
- the primers of SEQ ID No.10 and SEQ ID No.1 1, wherein said primers are specific of the cDNA reverse transcript from asPDLIM5; and
- the primers of SEQ ID No.12 and SEQ ID No.13, wherein said primers are specific of the cDNA reverse transcript from asPDLIM5; and
- the primers of SEQ ID No.14 and SEQ ID No.15, wherein said primers are specific of the cDNA reverse transcript from asPDLIM5; and
- the primers of SEQ ID No.16 and SEQ ID No.17, wherein said primers are specific of the cDNA reverse transcript from asFXL7; and
- the primers of SEQ ID No.18 and SEQ ID No.19, wherein said primers are specific of the cDNA reverse transcript from asFXL7; and
- the primers of SEQ ID No.20 and SEQ ID No.21, wherein said primers are specific of the cDNA reverse transcript from asFXL7.

Preferably, said amplification further comprises a step of contacting said cDNAs with a SYBR^{®} Green fluorescent dye, alternatively said amplification further comprises a step of contacting said cDNAs with at least a dual labeled probe, i.e. a probe with a fluorescent label and a quencher moiety, said at least one dual labeled probe being complementary to a sequence of cDNA localized between the two primers of the primer pair. More preferably, the dual labeled probe fluoresce upon 5'→3' exonuclease activity.

Preferably, when said at least one labeled probe comprises more than one probe, each probe has a different label, i.e. fluoresces at a different wavelength.

Alternatively, a combination of two different primer pairs specific of the same antisense IncRNA are used to amplify the cDNA by quantitative PCR. Preferably, the two primer pairs of this combination are used in separate PCR mixtures, alternatively they can be used in the same PCR mixture. For instance, this combination of two primer pairs can be selected from the group consisting of: the primer pair of SEQ ID No.4 and SEQ ID No.5 and the primer pair of SEQ ID No.6 and SEQ ID No.7, the primer pair of SEQ ID No.8 and SEQ ID No.9 and the primer pair of SEQ ID No.10 and SEQ ID No.11, the primer pair of SEQ ID No.8 and SEQ ID No.9 and the primer pair of SEQ ID No.12 and SEQ ID No.13; the primer pair of SEQ ID No.8 and SEQ ID No.9 and the primer pair of SEQ ID No.14 and SEQ ID No.15, the primer pair of SEQ ID No.10 and SEQ ID No.11 and the primer pair of SEQ ID No.12 and SEQ ID No.13, the primer pair of SEQ ID No.10 and SEQ ID No.11 and the primer pair of SEQ ID No.14 and SEQ ID No.15, the primer pair of SEQ ID No.12 and SEQ ID No.13 and the primer pair of SEQ ID No.14 and SEQ ID No.15, the primer pair of SEQ ID No.16 and SEQ ID No.17 and the primer pair of SEQ ID No.18 and SEQ ID No.19, the primer pair of SEQ ID No.15 and SEQ ID No.16 and the primer pair of SEQ ID No.20 and SEQ ID No.21, and the primer pair of SEQ ID No.18 and SEQ ID No.19 and the primer pair of SEQ ID No.20 and SEQ ID No.21.

In a particular aspect of the above mentioned method, the amount of at least two antisense IncRNA is determined by quantitative RT-PCR, wherein said quantitative RT-PCR comprises the steps of:
- reverse transcription of the RNAs present in the sample into cDNAs.
- amplification of the cDNA reverse transcripts from the at least two antisense lncRNAs or a part of said cDNAs by quantitative PCR, wherein said part has a length of at least 50 nucleotides and wherein said amplification comprises the step of contacting said cDNAs with at least two primer pairs.

Preferably, said amplification further comprises the step of contacting said cDNAs with at least two labeled probes, more preferably dual labeled probes, even more preferably dual labeled probe that fluoresce upon 5'→3' exonuclease activity, wherein each of said at least two dual labeled probes being complementary to a sequence of a cDNA localized between the two primers of one of the at least two primer pairs.

Preferably, said combination of at least two primer pairs are used in the same PCR mixture. Alternatively they can be used in different PCR mixture. For instance, this combination of at least two primer pairs can be selected from the group consisting of: the primer pair of SEQ ID No.4 and SEQ ID No.5 and the primer pair of SEQ ID No.8 and SEQ ID No.9, the primer pair of SEQ ID No.4 and SEQ ID No.5 and the primer pair of SEQ ID No.10 and SEQ ID No.11, the primer pair of SEQ ID No.4 and SEQ ID No.5 and the primer pair of SEQ ID No.12 and SEQ ID No.13, the primer pair of SEQ ID No.4 and SEQ ID No.5 and the primer pair of SEQ ID No.14 and SEQ ID No.15, the primer pair of SEQ ID No.4 and SEQ ID No.5 and the primer pair of SEQ ID No.16 and SEQ ID No.17, the primer pair of SEQ ID No.4 and SEQ ID No.5 and the primer pair of SEQ ID No.18 and SEQ ID No.19, the primer pair of SEQ ID No.4 and SEQ ID No.5 and the primer pair of SEQ ID No.20 and SEQ ID No.21, the primer pair of SEQ ID No.6 and SEQ ID No.7 and the primer pair of SEQ ID No.8 and SEQ ID No.9, the primer pair of SEQ ID No.6 and SEQ ID No.7 and the primer pair of SEQ ID No.10 and SEQ ID No.11, the primer pair of SEQ ID No.6 and SEQ ID No.7 and the primer pair of SEQ ID No.12 and SEQ ID No.13, the primer pair of SEQ ID No.6 and SEQ ID No.7 and the primer pair of SEQ ID No.14 and SEQ ID No.15, the primer pair of SEQ ID No.6 and SEQ ID No.7 and the primer pair of SEQ ID No.16 and SEQ ID No.17, the primer pair of SEQ ID No.6 and SEQ ID No.7 and the primer pair of SEQ ID No.18 and SEQ ID No.19, the primer pair of SEQ ID No.6 and SEQ ID No.7 and the primer pair of SEQ ID No.20 and SEQ ID No.21, the primer pair of SEQ ID No.8 and SEQ ID No.9 and the primer pair of SEQ ID No.16 and SEQ ID No.17, the primer pair of SEQ ID No.8 and SEQ ID No.9 and the primer pair of SEQ ID No.18 and SEQ ID No.19, the primer pair of SEQ ID No.8 and SEQ ID No.9 and the primer pair of SEQ ID No.20 and SEQ ID No.21, the primer pair of SEQ ID No.10 and SEQ ID No.11 and the primer pair of SEQ ID No.16 and SEQ ID No.17, the primer pair of SEQ ID No.10 and SEQ ID No.11 and the primer pair of SEQ ID No.18 and SEQ ID No.19, the primer pair of SEQ ID No.10 and SEQ ID No.11 and the primer pair of SEQ ID No.20 and SEQ ID No.21, the primer pair of SEQ ID No.12 and SEQ ID No.13 and the primer pair of SEQ ID No.16 and SEQ ID No.17, the primer pair of SEQ ID No.12 and SEQ ID No.13 and the primer pair of SEQ ID No.18 and SEQ ID No.19,the primer pair of SEQ ID No.12 and SEQ ID No.13 and the primer pair of SEQ ID No.20 and SEQ ID No.21, the primer pair of SEQ ID No.14 and SEQ ID No.15 and the primer pair of SEQ ID No.16 and SEQ ID No.17, the primer pair of SEQ ID No.14 and SEQ ID No. 15 and the primer pair of SEQ ID No. 18 and SEQ ID No.19,and the primer pair of SEQ ID No.14 and SEQ ID No.15 and the primer pair of SEQ ID No.20 and SEQ ID No.21.

Preferably, a combination of four different primer pairs specific of two different antisense lncRNAs are used to amplify the cDNAs by quantitative PCR. For instance, this combination of at least four primer pairs can be selected from the group consisting of: the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.10 and SEQ ID No.11, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.12 and SEQ ID No.13, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.14 and SEQ ID No.15, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No. 12 and SEQ ID No. 1, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.14 and SEQ ID No.15, the primer pair of SEQ ID No.4 and SEQ ID No.5 and the primer pair of SEQ ID No.6 and SEQ ID No.7 and the primer pair of SEQ ID No.12 and SEQ ID No.13 and the primer pair of SEQ ID No. 14 and SEQ ID No.15, the primer pair of SEQ ID No.4 and SEQ ID No.5 and the primer pair of SEQ ID No.6 and SEQ ID No.7 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.16 and SEQ ID No.7 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.18 and SEQ ID No.19 and the primer pair of SEQ ID No.20 and SEQ ID No.21, the primer pair of SEQ ID No.8 and SEQ ID No.9 and the primer pair of SEQ ID No.10 and SEQ ID No.11 and the primer pair of SEQ ID No.16 and SEQ ID No.17 and the primer pair of SEQ ID No.18 and SEQ ID No.19,the primer pair of SEQ ID No.8 and SEQ ID No.9 and the primer pair of SEQ ID No.10 and SEQ ID No.11 and the primer pair of SEQ ID No.16 and SEQ ID No.17 and the primer pair of SEQ ID No.20 and SEQ ID No.21, the primer pair of SEQ ID No.8 and SEQ ID No.9 and the primer pair of SEQ ID No.10 and SEQ ID No.11 and the primer pair of SEQ ID No.18 and SEQ ID No.19 and the primer pair of SEQ ID No.20 and SEQ ID No.21, the primer pair of SEQ ID No.8 and SEQ ID No.9 and the primer pair of SEQ ID No.12 and SEQ ID No.13 and the primer pair of SEQ ID No.16 and SEQ ID No.17 and the primer pair of SEQ ID No. 18 and SEQ ID No.19,the primer pair of SEQ ID No.8 and SEQ ID No.9 and the primer pair of SEQ ID No.12 and SEQ ID No.13 and the primer pair of SEQ ID No.16 and SEQ ID No.17 and the primer pair of SEQ ID No.20 and SEQ ID No.21, the primer pair of SEQ ID No.8 and SEQ ID No.9 and the primer pair of SEQ ID No.12 and SEQ ID No.13 and the primer pair of SEQ ID No.18 and SEQ ID No.19 and the primer pair of SEQ ID No.20 and SEQ ID No.21, the primer pair of SEQ ID No.8 and SEQ ID No.9 and the primer pair of SEQ ID No.14 and SEQ ID No.15 and the primer pair of SEQ ID No.16 and SEQ ID No.17 and the primer pair of SEQ ID No.18 and SEQ ID No.19, the primer pair of SEQ ID No.8 and SEQ ID No.9 and the primer pair of SEQ ID No.14 and SEQ ID No.15 and the primer pair of SEQ ID No.16 and SEQ ID No.17 and the primer pair of SEQ ID No.20 and SEQ ID No.21, the primer pair of SEQ ID No.8 and SEQ ID No.9 and the primer pair of SEQ ID No.14 and SEQ ID No.15 and the primer pair of SEQ ID No.18 and SEQ ID No.19 and the primer pair of SEQ ID No.20 and SEQ ID No.21, the primer pair of SEQ ID No.10 and SEQ ID No.11 and the primer pair of SEQ ID No.12 and SEQ ID No.13 and the primer pair of SEQ ID No.16 and SEQ ID No.17 and the primer pair of SEQ ID No.18 and SEQ ID No.19,the primer pair of SEQ ID No.10 and SEQ ID No.11 and the primer pair of SEQ ID No.12 and SEQ ID No.13 and the primer pair of SEQ ID No.16 and SEQ ID No.17 and the primer pair of SEQ ID No.20 and SEQ ID No.21, the primer pair of SEQ ID No.10 and SEQ ID No.11 and the primer pair of SEQ ID No.12 and SEQ ID No.13 and the primer pair of SEQ ID No.18 and SEQ ID No.19 and the primer pair of SEQ ID No.20 and SEQ ID No.21, the primer pair of SEQ ID No.10 and SEQ ID No.11 and the primer pair of SEQ ID No.14 and SEQ ID No.15 and the primer pair of SEQ ID No.16 and SEQ ID No.17 and the primer pair of SEQ ID No.18 and SEQ ID No.19,the primer pair of SEQ ID No.10 and SEQ ID No.11 and the primer pair of SEQ ID No.14 and SEQ ID No.15 and the primer pair of SEQ ID No.16 and SEQ ID No.17 and the primer pair of SEQ ID No.20 and SEQ ID No.21, the primer pair of SEQ ID No.10 and SEQ ID No.11 and the primer pair of SEQ ID No.14 and SEQ ID No.15 and the primer pair of SEQ ID No.18 and SEQ ID No.19 and the primer pair of SEQ ID No.20 and SEQ ID No.21, the primer pair of SEQ ID No.12 and SEQ ID No.13 and the primer pair of SEQ ID No.14 and SEQ ID No.15 and the primer pair of SEQ ID No.16 and SEQ ID No.17 and the primer pair of SEQ ID No.18 and SEQ ID No.19, the primer pair of SEQ ID No.12 and SEQ ID No.13 and the primer pair of SEQ ID No.14 and SEQ ID No.15 and the primer pair of SEQ ID No.16 and SEQ ID No.17 and the primer pair of SEQ ID No.20 and SEQ ID No.21, and the primer pair of SEQ ID No.12 and SEQ ID No.13 and the primer pair of SEQ ID No.14 and SEQ ID No.15 and the primer pair of SEQ ID No.18 and SEQ ID No.19 and the primer pair of SEQ ID No.20 and SEQ ID No.21.

In a preferred aspect of the above mentioned method, the amount of at least three antisense lncRNAs is determined by quantitative RT-PCR, wherein said quantitative RT-PCR comprises the steps of:
- reverse transcription of the RNAs present in the sample into cDNAs.
- amplification of the cDNA reverse transcripts from the at least three antisense lncRNAs or a part of said cDNAs by quantitative PCR, wherein said part has a length of at least 50 nucleotides and wherein said amplification comprises the step of contacting said cDNAs with at least three primer pairs.

Preferably, said combination of at least three primer pairs are used in the same PCR mixture, alternatively they can be used in different PCR mixture. For instance, this combination of at least three primer pairs can be selected from the group consisting of: the primer pair of SEQ ID No.4 and SEQ ID No.5 and the primer pair of SEQ ID No.8 and SEQ ID No.9 and the primer pair of SEQ ID No.16 and SEQ ID No.17, the primer pair of SEQ ID No.4 and SEQ ID No.5 and the primer pair of SEQ ID No.8 and SEQ ID No.9 and the primer pair of SEQ ID No.18 and SEQ ID No.19, the primer pair of SEQ ID No.4 and SEQ ID No.5 and the primer pair of SEQ ID No.8 and SEQ ID No.9 and the primer pair of SEQ ID No.20 and SEQ ID No.21, the primer pair of SEQ ID No.4 and SEQ ID No.5 and the primer pair of SEQ ID No.10 and SEQ ID No.11 and the primer pair of SEQ ID No.16 and SEQ ID No.17, the primer pair of SEQ ID No.4 and SEQ ID No.5 and the primer pair of SEQ ID No.10 and SEQ ID No.11 and the primer pair of SEQ ID No.18 and SEQ ID No.19, the primer pair of SEQ ID No.4 and SEQ ID No.5 and the primer pair of SEQ ID No.10 and SEQ ID No.11 and the primer pair of SEQ ID No.20 and SEQ ID No.21, the primer pair of SEQ ID No.4 and SEQ ID No.5 and the primer pair of SEQ ID No.12 and SEQ ID No.13 and the primer pair of SEQ ID No.16 and SEQ ID No.17, the primer pair of SEQ ID No.4 and SEQ ID No.5 and the primer pair of SEQ ID No.12 and SEQ ID No.13 and the primer pair of SEQ ID No.18 and SEQ ID No.19, the primer pair of SEQ ID No.4 and SEQ ID No.5 and the primer pair of SEQ ID No.11 and SEQ ID No.12 and the primer pair of SEQ ID No.20 and SEQ ID No.21, the primer pair of SEQ ID No.4 and SEQ ID No.5 and the primer pair of SEQ ID No.14 and SEQ ID No.15 and the primer pair of SEQ ID No.16 and SEQ ID No.17, the primer pair of SEQ ID No.4 and SEQ ID No.5 and the primer pair of SEQ ID No.14 and SEQ ID No.15 and the primer pair of SEQ ID No.18 and SEQ ID No.19, the primer pair of SEQ ID No.4 and SEQ ID No.5 and the primer pair of SEQ ID No.14 and SEQ ID No.15 and the primer pair of SEQ ID No.20 and SEQ ID No.21, the primer pair of SEQ ID No.6 and SEQ ID No.7 and the primer pair of SEQ ID No.8 and SEQ ID No.9 and the primer pair of SEQ ID No.16 and SEQ ID No.17, the primer pair of SEQ ID No.6 and SEQ ID No.7 and the primer pair of SEQ ID No.8 and SEQ ID No.9 and the primer pair of SEQ ID No.18 and SEQ ID No.19, the primer pair of SEQ ID No.6 and SEQ ID No.7 and the primer pair of SEQ ID No.8 and SEQ ID No.9 and the primer pair of SEQ ID No.20 and SEQ ID No.21, the primer pair of SEQ ID No.6 and SEQ ID No.7 and the primer pair of SEQ ID No.10 and SEQ ID No.11 and the primer pair of SEQ ID No.16 and SEQ ID No.17, the primer pair of SEQ ID No.6 and SEQ ID No.7 and the primer pair of SEQ ID No.10 and SEQ ID No.11 and the primer pair of SEQ ID No.18 and SEQ ID No.19, the primer pair of SEQ ID No.6 and SEQ ID No.7 and the primer pair of SEQ ID No.10 and SEQ ID No.11 and the primer pair of SEQ ID No.20 and SEQ ID No.21, the primer pair of SEQ ID No.6 and SEQ ID No.7 and the primer pair of SEQ ID No.12 and SEQ ID No.13 and the primer pair of SEQ ID No.16 and SEQ ID No.17, the primer pair of SEQ ID No.6 and SEQ ID No.7 and the primer pair of SEQ ID No.12 and SEQ ID No.13 and the primer pair of SEQ ID No.18 and SEQ ID No.19, the primer pair of SEQ ID No.6 and SEQ ID No.7 and the primer pair of SEQ ID No.11 and SEQ ID No.12 and the primer pair of SEQ ID No.20 and SEQ ID No.21, the primer pair of SEQ ID No.6 and SEQ ID No.7 and the primer pair of SEQ ID No.14 and SEQ ID No.15 and the primer pair of SEQ ID No.16 and SEQ ID No.17, the primer pair of SEQ ID No.6 and SEQ ID No.7 and the primer pair of SEQ ID No.14 and SEQ ID No.15 and the primer pair of SEQ ID No.18 and SEQ ID No.19,and the primer pair of SEQ ID No.6 and SEQ ID No.7 and the primer pair of SEQ ID No.14 and SEQ ID No.15 and the primer pair of SEQ ID No.20 and SEQ ID No.21.

Preferably, said amplification further comprises the step of contacting said cDNAs with at least three labeled probe, more preferably dual labeled probes, even more preferably dual labeled probe that fluoresce upon 5'→3' exonuclease activity, wherein each of said at least three dual labeled probes being complementary to a sequence of cDNA localized between the two primers of one of the at least three primer pairs.

Preferably, a combination of six different primer pairs specific of three different antisense lncRNAs are used to amplify the cDNAs by quantitative PCR. For instance, this combination of at least six primer pairs can be selected from the group consisting of: the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.10 and SEQ ID No.11 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.10 and SEQ ID No.11 and the primer pair of SEQ ID No.16 and SEQ ID No.17 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.10 and SEQ ID No.11 and the primer pair of SEQ ID No.18 and SEQ ID No.19 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.12 and SEQ ID No.13 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.12 and SEQ ID No.13 and the primer pair of SEQ ID No.16 and SEQ ID No.17 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.12 and SEQ ID No.13 and the primer pair of SEQ ID No.18 and SEQ ID No.19 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.14 and SEQ ID No.15 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.14 and SEQ ID No.15 and the primer pair of SEQ ID No.16 and SEQ ID No.17 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.14 and SEQ ID No.15 and the primer pair of SEQ ID No.18 and SEQ ID No.19 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.12 and SEQ ID No.13 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.12 and SEQ ID No.13 and the primer pair of SEQ ID No.16 and SEQ ID No.17 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.12 and SEQ ID No.13 and the primer pair of SEQ ID No.18 and SEQ ID No.19 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.14 and SEQ ID No.15 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.14 and SEQ ID No.15 and the primer pair of SEQ ID No.16 and SEQ ID No.17 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.14 and SEQ ID No.15 and the primer pair of SEQ ID No.18 and SEQ ID No.19 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.12 and SEQ ID No.13 and the primer pair of SEQ ID No.14 and SEQ ID No.15 and the primer pair of SEQ ID No.16 and SEQ ID No.17 and the primer pair of SEQ ID No.18 and SEQ ID No.19,the primer pair of SEQ ID No.4 and SEQ ID No.5 and the primer pair of SEQ ID No.6 and SEQ ID No.7 and the primer pair of SEQ ID No.12 and SEQ ID No.13 and the primer pair of SEQ ID No.14 and SEQ ID No.15 and the primer pair of SEQ ID No.16 and SEQ ID No.17 and the primer pair of SEQ ID No.20 and SEQ ID No.21, and the primer pair of SEQ ID No.4 and SEQ ID No.5 and the primer pair of SEQ ID No.6 and SEQ ID No.7 and the primer pair of SEQ ID No.12 and SEQ ID No.13 and the primer pair of SEQ ID No.14 and SEQ ID No.15 and the primer pair of SEQ ID No.18 and SEQ ID No.19 and the primer pair of SEQ ID No.20 and SEQ ID No.21.

The above mentioned method may further comprise additional primer pairs, preferably primer pairs targeting other antisense IncRNA which are diagnosis marker for prostate cancer, more preferably primer pairs for PCA3 quantitative amplification.

### Hybridization method

In one aspect of the above mentioned method, the amount of at least one antisense IncRNA is determined using an RNA hybridization method. In such an aspect, the RNA hybridization method is coupled to the detection of the hybridized RNA using a probe, preferably a labeled specific probe. Even more preferably, the specific probe is labeled by a fluorophore.

When the amounts of several antisense lncRNAs are determined simultaneously, each probe is linked to a different label.

The complex probe/antisense IncRNA may be bound on a solid support, allowing to wash the reaction of the unbound probes.

After extraction of the RNA from the sample, the determination of the amount of at least one antisense IncRNA by an RNA hybridization method comprises the following steps:
- Contacting at least one labeled specific probe with the extracted RNAs in conditions suitable for hybridization of said probe with its antisense IncRNA target; and
- Quantification of the signal emitted by the probe.

Optionally, the RNAs are denatured prior to the contacting step, thereby removing the RNA secondary structure.

In a preferred aspect, the at least one antisense IncRNA is immobilized on a solid support. Optionally, after immobilization of the at least one antisense IncRNA and the step of contacting, the method described above comprises a washing step, thereby removing the unbound probes.

The antisense lncRNAs may be directly immobilized on a solid support prior to be contacted with the probes. In such an aspect, the amount of at least one antisense IncRNA is preferably determined by Northern blot.

Alternatively, the antisense lncRNAs may be indirectly immobilized on a solid support through its hybridization to its specific probe. Particularly, the sample may be contacted with probes already immobilized on a solid support. Preferably, the complex antisense IncRNA/probe is immobilized on a solid support after hybridization.

Preferably, the probe is linked to a molecule that helps the probe to bind a solid support. More preferably said molecule is biotin, thereby the probe and its complementary antisense IncRNA are immobilized on the solid support.

Examples of solid supports suitable for the disclosure include, but are not limited to, plastics such as polycarbonate, complex carbohydrates such as agarose and sepharose, and acrylic resins such as polyacrylamide and latex beads. Techniques for coupling nucleic acid and/or nucleic acid probes to such solid supports are well known in the art.

In another particular aspect, the amount of at least one antisense IncRNA is determined by a LNA (Lock nucleic acid) method (see for example the techniques developed by Exiqon).

Preferably, the amount of at least one antisense IncRNA is determined by the Nanostring method.

### Nanostring method

The nanostring method is a hybridization method that allows to quantify RNA without requiring linear (array) nor exponential (PCR) amplification. It is a very sensitive method, since only 10 ng of RNA are needed to perform it, allowing analysis of quantity limited biological samples, such as urine. The restricted number of sample manipulation steps together with the absence of enzymatic reaction allows precise and physiologically correct quantifications. This method is also extremely flexible since it can be applied to various types of samples.

The Nanostring method necessitates the use of a pair of probes specifically designed for each antisense IncRNA target.

The first probe, called the capture-probe, specifically hybridizes the antisense IncRNA target and binds it to a solid support, preferably a counting stand. Preferably, the capture probe is linked to a molecule that allows the probe to bind the solid support, more preferably said molecule is biotin, thereby immobilizing the antisense IncRNA of interest onto the counting stand.

The second probe, called the reporter-probe, specifically hybridizes the antisense IncRNA of interest and is linked to a label that allows the detection and quantification of the antisens lncRNA. Preferably, this label is a fluorescent label. More preferably the label is made of a combination of fluorochromes. Even more preferably the label is made of a combination of 6 fluorochromes chosen among 4 fluorochromes of different colors, defining a code specific to each target antisense lncRNA. This color code confers to the technique a very high sensitivity and enables the analysis of quantity-limited biological samples. When several reporter probes are used simultaneously to determine the amounts of several antisense lncRNA, each reporter probe is linked to a different label, preferably a different combination of 6 fluorochromes chosen among 4 fluorochromes of different colors.

Preferably, the fluorescence is analyzed by an nCounter, an optical system that is capable to identify the color codes.

Preferably, the determination of the amount of at least one antisense IncRNA by the Nanostring method comprises the following steps:
- Contacting the sample with the capture and the reporter probes, in conditions suitable for hybridization with its antisense IncRNA target; and
- Elimination of the excess probes.
- Alignment and immobilization of the probes/target complexes into the solid support.
- Collection of the signal by the Nanostring device.

More preferably, the determination of the amount of at least one antisense IncRNA by the Nanostring method comprises the following steps:
- Contacting the sample with the capture and the reporter probes, in conditions suitable for hybridization with its antisense IncRNA target; and
- Elimination of the excess probes is achieved on a Nanostring Prep Station following an high sensitivity mode containing a dual purification process. The Nanostring prep station is robotic liquid handling system dedicated to purification of molecules coupled to probes and specially to capture probes and reporter probes. As capture probes and reporter probes contain also a specific tail (generic and specific sequence to the capture probe, idem for the reporter probes), two types of dedicated magnetic beads supplied by Nanostring will be used in this main step. First, magnetic bead coupled with sequences complementary to tails of capture probes are used to purify capture probes hybridized or not to targets of interest. After washes, purified molecules are mixed with another magnetic beads that are coupled to sequences complementary to reporter probes tails. This step selects molecules bound to the set of capture probes and reporter probes. Washes are applied to by the Nanostring Prep station to ensure correct purification of hybrids.
- Next, dually purified molecules are injected in a solid device: a 12 slot Nanostring Cartridge. This device contains 12 slots meaning that 12 samples can be analyzed in the meantime. Per slot, purified and injected molecules are immobilized thanks to a streptavidin coating. A magnetic fields is also applied to align the probes/target complexes into the solid device,.
- Collection of the signal, in particular by the Digital Analyzer on the sample Cartridges. Color codes on the surface of the cartridge are counted and tabulated for each target molecule.

Optionally, the ARNs are denatured prior to the contacting step, thereby removing the ARN secondary structure.

The Nanostring probes hybridize to their antisense IncRNA target with a part of their nucleotide sequence which is of a length of between about 40 and about 60 nucleotides, preferably of a length of about 50 nucleotides. The capture probe may further comprise a sequence that allows to link it to biotin. The reporter probe may further comprise a sequence that allows to link it to a six fluorochromes code. The color code is specific to each lot of production done by Nanostring. To deconvoluate signals and assign color code to targets, Nanostring provides a correspondence file specific to each kit.

Preferably, the sequences of the Nanostring probes of a pair of probes target different parts of their antisense IncRNA and these parts do not overlap.

Optionally, the sequences of the Nanostring probes of a pair of probes may be chosen so as they are not distant of more than 5 nucleotides on their antisense IncRNA target, preferably they are not distant of more than 10, 15, 20, 30, 40, 50, 100 nucleotides. In a most preferred aspect, the Nanostring probes of a pair of probes are directly consecutive.

In one aspect of the above mentioned method, the amount of at least one antisense IncRNA is determined by the Nanostring method, wherein said Nanostring method comprises the step of contacting the sample with at least one capture-probe and one reporter probe, wherein said capture probe is able to specifically hybridize the at least one antisense IncRNA and is preferably linked to a molecule that helps to immobilize said at least one antisense IncRNA onto a solid support, more preferably said molecule is biotin, and wherein said reporter-probe is able to specifically hybridize the at least one antisense IncRNA and is linked to a fluorescent label, preferably a combination of fluorophores, more preferably a combination of 6 fluorochromes chosen among 4 fluorochromes of different colors defining a code specific to said at least one antisense lncRNA.

Preferably, said at least one capture-probe and one reporter probe are selected from the group consisting of:
- the probes of SEQ ID No.22 and SEQ ID No.23, wherein said probes are complementary to the antisense IncRNA of SEQ ID No.1; and
- the probes of SEQ ID No.24 and SEQ ID No.25, wherein said probes are complementary to the antisense IncRNA of SEQ ID No.2; and
- the probes of SEQ ID No.26 and SEQ ID No.27, wherein said probes are complementary to the antisense IncRNA of SEQ ID No.3;

In a particular aspect of the above mentioned method, the amount of at least two antisense lncRNAs are determined by the Nanostring method, wherein said Nanostring method comprises the step of contacting the sample with a combination of at least two pairs of probes, said combination comprising two of the pairs of probes listed above. For instance, the combination can be selected from the group consisting of the pair of probes of SEQ ID No.22 and SEQ ID No.23 and the pair of probes consisted of SEQ ID No.24 and SEQ ID No.25, or the pair of probes of SEQ ID No.22 and SEQ ID No.23 and the pair of probes consisted of SEQ ID No.26 and SEQ ID No.27, or the pair of probes of SEQ ID No.24 and SEQ ID No.25 and the pair of probes consisted of SEQ ID No.26 and SEQ ID No.27.

In a preferred aspect of the above mentioned method, the amount of at least three antisense lncRNAs are determined by the Nanostring method, wherein said Nanostring method comprises the step of contacting the sample with a combination of at least three pairs of probes, said combination comprising the three pairs of probes listed above.

The above mentioned combination may further comprise additional pairs of probes, preferably pairs of probes targeting other antisense lncRNAs which are diagnosis markers for prostate cancer, more preferably pairs of probes for PCA3 quantification by the Nanostring method.

The amount of an antisense IncRNA can also be determined from a biological sample by serial analysis of gene expression (SAGE), immunoassay, mass spectrometry, and any sequencing-based methods known in the art.

### Comparison to a control reference value and treatment

In an aspect of the above mentioned methods, the method further comprises the step of comparing the amount of antisense IncRNA in a biological sample to a reference amount. In particular, the reference amount can be the amount of the same antisense IncRNA in a normal sample, i.e. a sample from a subject that did not have a prostate cancer. The normal sample may be obtained from the subject affected with the prostate cancer before the beginning of the disease or from another subject, preferably a normal or healthy subject, i.e. a subject who does not suffer from a cancer, especially a prostate cancer. The reference amount can be an average of the amounts obtained with different normal samples from different subjects.

The amounts of antisense lncRNAs obtained with subjects may also be normalized by using the amounts obtained with other RNAs which are known to have stable expression.

In a further aspect of the above mentioned methods, the method further comprises the step of determining whether the amount of an antisense IncRNA is dysregulated, on the basis of the comparison between the amount in the biological sample and the reference amount, a higher amount of the antisense IncRNA being indicative of a higher susceptibility to have or to develop a prostate cancer.

The amount of an antisense IncRNA in a biological sample is considered as significantly different (i.e. dysregulated) compared to the reference amount, if, optionally after normalization, differences are in the order of 2-fold higher than the reference amount or more. Preferably, the amount of an antisense IncRNA in a biological sample is considered as dysregulated if the amount is at least 2.5-fold higher, or 3, 3.5, 4, 4.5 or 5-fold higher than the reference amount.

In a preferred aspect, the present disclosure provides a method for detecting prostate cancer in a subject from a sample comprising the steps of: (i) determining the amount of at least one antisense IncRNA or a fragment thereof in a biological sample from the subject; (ii) comparing the amount in the sample to a reference amount derived from the amount of said antisense IncRNA in samples obtained from subjects who haven't a prostate cancer; and (iii) identifying the subject as having a prostate cancer or as having an increased risk to develop a prostate cancer when the amount of said antisense IncRNA in the sample is greater than the reference amount and/or identifying the subject as not having a prostate cancer or as not having an increased risk to develop a prostate cancer when the amount of said antisense IncRNA in the sample is equal or less than the reference amount.

Optionally, the method may further comprise performing a biopsy of the prostate of the subject to confirm that the subject has a prostate cancer when the amount of said antisense IncRNA in the sample is greater than the reference amount. Alternatively, the method may further comprise observing a biopsy of the prostate of the subject to confirm that the subject has a prostate cancer when the amount of said antisense IncRNA in the sample is greater than the reference amount.

In yet another aspect, the present method further comprises a step of treating the patient diagnosed as having a prostate cancer.

The treatment of the patient diagnosed as having a prostate cancer may comprise the administration to the patient of an effective amount of a therapeutic agent and/or the prostate resection.

The therapeutic agent may be selected from the group consisting of radiotherapeutic agents, hormonal therapy agents, chemotherapy agents, immunotherapy agents and monoclonal antibody therapy agents, preferably hormonal therapy agents.

It is understood that the administered dose of the therapeutic agent may be adapted by those skilled in the art according to the patient, the pathology, the mode of administration, etc. The dosage and regimen depends in particular on the stage and severity of the prostate cancer, the weight and general state of health of the patient and the judgment of the prescribing physician.

In some aspects, the treatment includes one or more of open prostatectomy, minimally invasive laparoscopic robotic surgery, intensity modulated radiation therapy (IMRT), proton therapy, brachytherapy, cryotherapy, molecular-targeted therapy, vaccine therapy and gene therapy, hormone therapy, active surveillance, or a combination thereof.

In a particular aspect, the present disclosure provides a method to assess the efficiency of a prostate cancer treatment in a subject having a prostate cancer comprising the steps of: (i) administering a prostate cancer treatment, i.e. an effective amount of a therapeutic agent, to the subject, (ii) determining the amount of at least one antisense IncRNA or a fragment thereof in a biological sample from the subject, (iii) comparing the amount in the sample to a reference amount derived from the amount of said antisense IncRNA in samples obtained from subjects that do not have a prostate cancer, and (iv) identifying the method as efficient or the subject as having recovered from prostate cancer when the amount of said antisense IncRNA in the sample is equal or less than the reference amount.

Optionally or alternatively, the amount at least one antisense IncRNA determined in a biological sample obtained after administration of an effective amount of a therapeutic agent may further be compare to the amount of said antisense IncRNA in a sample from the same patient obtained before the treatment, a significant decrease in the amount of said antisense IncRNA being indicative of the efficiency of said prostate cancer treatment.

### Additional markers

In another aspect of the above mentioned method, the method further comprises the determination of the amount of additional prostate cancer markers or fragments thereof, preferably antisense lncRNAs which are prostate cancer markers, more preferably the PCA3 antisense lncRNA.

Non-limiting other examples of prostate cancer markers include PSA and other kallikrein family members, GSTP1 (Glutathione S-Transferase Pi-1), AMACR (AlphaMethyl-Acyl-CoA Racemase), ERG (ETS-Related Gene), gene fusions involving ETS (Erythroblast Transformation-Specific)-related genes, and PCGEM1 (prostate-specific transcript 1 (non-protein coding)).

The determination of the amounts of antisense lncRNAs of the disclosure and other prostate cancer markers can be carried out in the same or in different reaction mixtures, simultaneously or not.

Different prostate cancer scores can also be calculated to contribute to the diagnostic of the subject: the prostate specific antigen (PSA) Score, the Myriad Prolaris Assay (MPA) Score, the Oncotype DX Genomic Prostate Score (GPS), the Cancer of the Prostate Risk Assessment (CAPRA) Score, and the Gleason Score.

### Kit and use of a kit

The disclosure also concerns a kit for the diagnosis of prostate cancer in a subject, wherein the kit comprises probes and/or primers capable to specifically hybridize to at least one antisense IncRNA selected from the group consisting of asFBXL7, asABCC4, and asPDLIM5.

Preferably, the kit comprises probes and/or primers capable to specifically hybridize to at least two antisense lncRNA, said at least two antisense IncRNA comprising two of the antisense IncRNA described above. For instance, the kit may comprise probes and/or primers capable to specifically hybridize to asFBXL7 and asABCC4, or asFBXL7 and asPDLIM5, or asABCC4, and asPDLIM5.

Even more preferably, the kit comprises probes and/or primers capable to specifically hybridize to at least three antisense lncRNA, said at least three antisense IncRNA comprising three of the antisense IncRNA described above.

Optionally, the above mentioned kit further comprises a leaflet providing guidelines to use such a kit.

In an aspect of the above mentioned kit, the kit further comprises probes and/or primers for the detection of additional prostate cancer markers, preferably probes and/or primers for the detection of antisense lncRNAs prostate cancer markers, more preferably probes and/or primers for the detection of the PCA3 antisense lncRNA.

Probes and/or primers of the kit, as used herein, are as defined above in any aspect.

Preferably, for each antisense lncRNA, the kit comprises two probes, preferably Nanostring probes consisting of:
- A capture-probe which comprises a nucleotide sequence hybridizing a first part of the antisense IncRNA and a molecule able to bind a solid support; and
- A reporter-probe which comprises a nucleotide sequence hybridizing a second part of the antisense IncRNA and a detectable label.

Alternatively, for each antisense lncRNA, the kit comprises two probes, preferably Nanostring probes consisting of:
- A capture-probe which hybridizes the antisense IncRNA and links a molecule that allows to immobilize the antisense IncRNA onto a solid support; and
- A reporter-probe which hybridizes the antisense IncRNA and links a label that allows the detection of the immobilized antisense lncRNA.

In a particular aspect, the kit comprises at least one pair of probes selected from the group consisting of:
- the probes of SEQ ID No.22 and SEQ ID No.23, wherein said probes are complementary to the antisense IncRNA of SEQ ID No.1; and
- the probes of SEQ ID No.24 and SEQ ID No.25, wherein said probes are complementary to the antisense IncRNA of SEQ ID No.2; and
- the probes of SEQ ID No.26 and SEQ ID No.27, wherein said probes are complementary to the antisense IncRNA of SEQ ID No.3;

Preferably, the kit comprises at least two pairs of probes, said at least two pairs of probes comprising two pairs of probes selected from the group consisting of the pair of probes of SEQ ID No.22 and SEQ ID No.23 and the pair of probes consisted of SEQ ID No.24 and SEQ ID No.25, or the pair of probes of SEQ ID No.22 and SEQ ID No.23 and the pair of probes consisted of SEQ ID No.26 and SEQ ID No.27, or the pair of probes of SEQ ID No.24 and SEQ ID No.25 and the pair of probes consisted of SEQ ID No.26 and SEQ ID No.27.

More preferably, the kit comprise at least the three pairs of probes, said at least the three pairs of probes comprising described above.

In another particular aspect, the kit comprises at least one pair of probes selected from the group consisting of:
- the probes of SEQ ID No.22 and SEQ ID No.23, wherein said probes are complementary to the antisense IncRNA of SEQ ID No.1, and wherein the probe of SEQ ID No.22 further comprises an immobilizing agent and the probe of SEQ ID No. 23 further comprises a label; and
- the probes of SEQ ID No.24 and SEQ ID No.25, wherein said probes are complementary to the antisense IncRNA of SEQ ID No.2, and wherein the probe of SEQ ID No.24 further comprises an immobilizing agent and the probe of SEQ ID No. 25 further comprises a label; and
- the probes of SEQ ID No.26 and SEQ ID No.27, wherein said probes are complementary to the antisense IncRNA of SEQ ID No.3, and wherein the probe of SEQ ID No.26 further comprises an immobilizing agent and the probe of SEQ ID No. 27 further comprises a label.

Preferably, the immobilizing agent comprises biotin.

Preferably, the label is a fluorescent label, more preferably a combination of several fluorophores, even more preferably a combination of six fluorophores selected from four different fluorophores.

Preferably, the kit comprises at least two pairs of probes selected from the pairs of probes above mentioned. More preferably, the kit comprises at least the three pairs of probes above mentioned.

When several pairs of probes are included in the kit, the label is different for each of the probes presenting a label.

Alternatively, for each antisense lncRNA, the kit comprises:
- at least one primer pair;
- optionally, RT primers, preferably random primers, alternatively a RT primer complementary to said at least one antisense lncRNA.
- optionally, a labeled probe.

Preferably, the labeled probe, is a fluorescent probe, more preferably a dual labeled probe which is complementary to a sequence of the target sequence localized between the two primers, even more preferably, the dual label probe is aimed to fluoresce upon 5'→3' exonuclease activity.

Preferably, the at least one primer pair consists in primers that are complementary to either exon or intron sequences of the pre-antisense lncRNA, and are obligatory out of exons of the corresponding pre-mRNA. More preferably, the primers are complementary to exon sequences of the pre-antisense lncRNA, and are obligatory out of exons of the corresponding pre-mRNA.

In a particular aspect, the kit comprises at least one primer pair selected from the group consisting of:
- the primers of SEQ ID No.4 and SEQ ID No.5, wherein said primers are specific of the cDNA reverse transcript from asABCC4; and
- the primers of SEQ ID No.6 and SEQ ID No.7, wherein said primers are specific of the cDNA reverse transcript from asABCC4; and
- the primers of SEQ ID No.8 and SEQ ID No.9, wherein said primers are specific of the cDNA reverse transcript from asPDLIM5; and
- the primers of SEQ ID No.10 and SEQ ID No.11, wherein said primers are specific of the cDNA reverse transcript from asPDLIM5; and
- the primers of SEQ ID No.12 and SEQ ID No.13, wherein said primers are specific of the cDNA reverse transcript from asPDLIM5; and
- the primers of SEQ ID No.14 and SEQ ID No.15, wherein said primers are specific of the cDNA reverse transcript from asPDLIM5; and
- the primers of SEQ ID No.16 and SEQ ID No.17, wherein said primers are specific of the cDNA reverse transcript from asFXL7; and
- the primers of SEQ ID No.18 and SEQ ID No.19, wherein said primers are specific of the cDNA reverse transcript from asFXL7; and
- the primers of SEQ ID No.20 and SEQ ID No.21, wherein said primers are specific of the cDNA reverse transcript from asFXL7.

Preferably, the kit comprises a combination of two different primer pairs specific of the same antisense lncRNA. For instance, this combination of two primer pairs can be selected from the group consisting of: the primer pair of SEQ ID No.4 and SEQ ID No.5 and the primer pair of SEQ ID No.6 and SEQ ID No.7, the primer pair of SEQ ID No.8 and SEQ ID No.9 and the primer pair of SEQ ID No.10 and SEQ ID No.11, the primer pair of SEQ ID No.8 and SEQ ID No.9 and the primer pair of SEQ ID No.12 and SEQ ID No.13; the primer pair of SEQ ID No.8 and SEQ ID No.9 and the primer pair of SEQ ID No.14 and SEQ ID No.15, the primer pair of SEQ ID No.10 and SEQ ID No.11 and the primer pair of SEQ ID No.12 and SEQ ID No.13, the primer pair of SEQ ID No.10 and SEQ ID No.11 and the primer pair of SEQ ID No.14 and SEQ ID No.15, the primer pair of SEQ ID No.12 and SEQ ID No.13 and the primer pair of SEQ ID No.14 and SEQ ID No.15, the primer pair of SEQ ID No.16 and SEQ ID No.17 and the primer pair of SEQ ID No.18 and SEQ ID No.19, the primer pair of SEQ ID No.15 and SEQ ID No.16 and the primer pair of SEQ ID No.20 and SEQ ID No.21, and the primer pair of SEQ ID No.18 and SEQ ID No.19 and the primer pair of SEQ ID No.20 and SEQ ID No.21.

In a preferred aspect of the above mentioned kit, the kit comprises at least two primer pairs targeting two different antisense IncRNA and selected from the group consisting of: the primer pair of SEQ ID No.4 and SEQ ID No.5 and the primer pair of SEQ ID No.8 and SEQ ID No.9, the primer pair of SEQ ID No.4 and SEQ ID No.5 and the primer pair of SEQ ID No.10 and SEQ ID No.11, the primer pair of SEQ ID No.4 and SEQ ID No.5 and the primer pair of SEQ ID No.12 and SEQ ID No.13, the primer pair of SEQ ID No.4 and SEQ ID No.5 and the primer pair of SEQ ID No.14 and SEQ ID No.15, the primer pair of SEQ ID No.4 and SEQ ID No.5 and the primer pair of SEQ ID No.16 and SEQ ID No.17, the primer pair of SEQ ID No.4 and SEQ ID No.5 and the primer pair of SEQ ID No.18 and SEQ ID No.19,the primer pair of SEQ ID No.4 and SEQ ID No.5 and the primer pair of SEQ ID No.20 and SEQ ID No.21, the primer pair of SEQ ID No.6 and SEQ ID No.7 and the primer pair of SEQ ID No.8 and SEQ ID No.9, the primer pair of SEQ ID No.6 and SEQ ID No.7 and the primer pair of SEQ ID No.10 and SEQ ID No.11, the primer pair of SEQ ID No.6 and SEQ ID No.7 and the primer pair of SEQ ID No.12 and SEQ ID No.13, the primer pair of SEQ ID No.6 and SEQ ID No.7 and the primer pair of SEQ ID No.14 and SEQ ID No.15, the primer pair of SEQ ID No.6 and SEQ ID No.7 and the primer pair of SEQ ID No.16 and SEQ ID No.17, the primer pair of SEQ ID No.6 and SEQ ID No.7 and the primer pair of SEQ ID No.18 and SEQ ID No.19, the primer pair of SEQ ID No.6 and SEQ ID No.7 and the primer pair of SEQ ID No.20 and SEQ ID No.21, the primer pair of SEQ ID No.8 and SEQ ID No.9 and the primer pair of SEQ ID No.16 and SEQ ID No.17, the primer pair of SEQ ID No.8 and SEQ ID No.9 and the primer pair of SEQ ID No.18 and SEQ ID No.19, the primer pair of SEQ ID No.8 and SEQ ID No.9 and the primer pair of SEQ ID No.20 and SEQ ID No.21, the primer pair of SEQ ID No.10 and SEQ ID No.11 and the primer pair of SEQ ID No.16 and SEQ ID No.17, the primer pair of SEQ ID No.10 and SEQ ID No.11 and the primer pair of SEQ ID No.18 and SEQ ID No.19, the primer pair of SEQ ID No.10 and SEQ ID No.11 and the primer pair of SEQ ID No.20 and SEQ ID No.21, the primer pair of SEQ ID No.12 and SEQ ID No.13 and the primer pair of SEQ ID No.16 and SEQ ID No.17, the primer pair of SEQ ID No.12 and SEQ ID No.13 and the primer pair of SEQ ID No.18 and SEQ ID No.19, the primer pair of SEQ ID No.12 and SEQ ID No.13 and the primer pair of SEQ ID No.20 and SEQ ID No.21, the primer pair of SEQ ID No.14 and SEQ ID No.15 and the primer pair of SEQ ID No.16 and SEQ ID No.17, the primer pair of SEQ ID No.14 and SEQ ID No.15 and the primer pair of SEQ ID No.18 and SEQ ID No.19, and the primer pair of SEQ ID No.14 and SEQ ID No.15 and the primer pair of SEQ ID No.20 and SEQ ID No.21.

Preferably, the kit comprises a combination of four different primer pairs specific of two different antisense lncRNAs and selected from the group consisting of: the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.10 and SEQ ID No.11, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.12 and SEQ ID No.13, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.14 and SEQ ID No.15, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.12 and SEQ ID No.1, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.14 and SEQ ID No.15, the primer pair of SEQ ID No.4 and SEQ ID No.5 and the primer pair of SEQ ID No.6 and SEQ ID No.7 and the primer pair of SEQ ID No.12 and SEQ ID No.13 and the primer pair of SEQ ID No.14 and SEQ ID No.15, the primer pair of SEQ ID No.4 and SEQ ID No.5 and the primer pair of SEQ ID No.6 and SEQ ID No.7 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No. 16 and SEQ ID No.7 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.18 and SEQ ID No.19 and the primer pair of SEQ ID No.20 and SEQ ID No.21, the primer pair of SEQ ID No.8 and SEQ ID No.9 and the primer pair of SEQ ID No.10 and SEQ ID No.11 and the primer pair of SEQ ID No.16 and SEQ ID No.17 and the primer pair of SEQ ID No.18 and SEQ ID No.19,the primer pair of SEQ ID No.8 and SEQ ID No.9 and the primer pair of SEQ ID No.10 and SEQ ID No.11 and the primer pair of SEQ ID No.16 and SEQ ID No.17 and the primer pair of SEQ ID No.20 and SEQ ID No.21, the primer pair of SEQ ID No.8 and SEQ ID No.9 and the primer pair of SEQ ID No.10 and SEQ ID No.11 and the primer pair of SEQ ID No.18 and SEQ ID No.19 and the primer pair of SEQ ID No.20 and SEQ ID No.21, the primer pair of SEQ ID No.8 and SEQ ID No.9 and the primer pair of SEQ ID No.12 and SEQ ID No.13 and the primer pair of SEQ ID No.16 and SEQ ID No.17 and the primer pair of SEQ ID No.18 and SEQ ID No.19,the primer pair of SEQ ID No.8 and SEQ ID No.9 and the primer pair of SEQ ID No.12 and SEQ ID No.13 and the primer pair of SEQ ID No.16 and SEQ ID No.17 and the primer pair of SEQ ID No.20 and SEQ ID No.21, the primer pair of SEQ ID No.8 and SEQ ID No.9 and the primer pair of SEQ ID No.12 and SEQ ID No.13 and the primer pair of SEQ ID No.18 and SEQ ID No.19 and the primer pair of SEQ ID No.20 and SEQ ID No.21, the primer pair of SEQ ID No.8 and SEQ ID No.9 and the primer pair of SEQ ID No.14 and SEQ ID No.15 and the primer pair of SEQ ID No.16 and SEQ ID No.17 and the primer pair of SEQ ID No.18 and SEQ ID No.19, the primer pair of SEQ ID No.8 and SEQ ID No.9 and the primer pair of SEQ ID No.14 and SEQ ID No.15 and the primer pair of SEQ ID No.16 and SEQ ID No.17 and the primer pair of SEQ ID No.20 and SEQ ID No.21, the primer pair of SEQ ID No.8 and SEQ ID No.9 and the primer pair of SEQ ID No.14 and SEQ ID No.15 and the primer pair of SEQ ID No.18 and SEQ ID No.19 and the primer pair of SEQ ID No.20 and SEQ ID No.21, the primer pair of SEQ ID No.10 and SEQ ID No.11 and the primer pair of SEQ ID No.12 and SEQ ID No.13 and the primer pair of SEQ ID No.16 and SEQ ID No.17 and the primer pair of SEQ ID No.18 and SEQ ID No.19, the primer pair of SEQ ID No.10 and SEQ ID No.11 and the primer pair of SEQ ID No.12 and SEQ ID No.13 and the primer pair of SEQ ID No.16 and SEQ ID No.17 and the primer pair of SEQ ID No.20 and SEQ ID No.21, the primer pair of SEQ ID No.10 and SEQ ID No.11 and the primer pair of SEQ ID No.12 and SEQ ID No.13 and the primer pair of SEQ ID No.18 and SEQ ID No.19 and the primer pair of SEQ ID No.20 and SEQ ID No.21, the primer pair of SEQ ID No.10 and SEQ ID No.11 and the primer pair of SEQ ID No.14 and SEQ ID No.15 and the primer pair of SEQ ID No.16 and SEQ ID No.17 and the primer pair of SEQ ID No.18 and SEQ ID No.19, the primer pair of SEQ ID No.10 and SEQ ID No.11 and the primer pair of SEQ ID No.14 and SEQ ID No.15 and the primer pair of SEQ ID No.16 and SEQ ID No.17 and the primer pair of SEQ ID No.20 and SEQ ID No.21, the primer pair of SEQ ID No.10 and SEQ ID No.11 and the primer pair of SEQ ID No.14 and SEQ ID No.15 and the primer pair of SEQ ID No.18 and SEQ ID No.19 and the primer pair of SEQ ID No.20 and SEQ ID No.21, the primer pair of SEQ ID No.12 and SEQ ID No.13 and the primer pair of SEQ ID No.14 and SEQ ID No.15 and the primer pair of SEQ ID No.16 and SEQ ID No.17 and the primer pair of SEQ ID No.18 and SEQ ID No.19, the primer pair of SEQ ID No.12 and SEQ ID No.13 and the primer pair of SEQ ID No.14 and SEQ ID No.15 and the primer pair of SEQ ID No.16 and SEQ ID No.17 and the primer pair of SEQ ID No.20 and SEQ ID No.21, and the primer pair of SEQ ID No.12 and SEQ ID No.13 and the primer pair of SEQ ID No.14 and SEQ ID No.15 and the primer pair of SEQ ID No.18 and SEQ ID No.19 and the primer pair of SEQ ID No.20 and SEQ ID No.21.

In an even more preferred aspect of the above mentioned kit, the kit comprises at least three primer pairs targeting three different antisense IncRNA and selected from the group consisting of: the primer pair of SEQ ID No.4 and SEQ ID No.5 and the primer pair of SEQ ID No.8 and SEQ ID No.9 and the primer pair of SEQ ID No. 16 and SEQ ID No.17, the primer pair of SEQ ID No.4 and SEQ ID No.5 and the primer pair of SEQ ID No.8 and SEQ ID No.9 and the primer pair of SEQ ID No.18 and SEQ ID No.19, the primer pair of SEQ ID No.4 and SEQ ID No.5 and the primer pair of SEQ ID No.8 and SEQ ID No.9 and the primer pair of SEQ ID No.20 and SEQ ID No.21, the primer pair of SEQ ID No.4 and SEQ ID No.5 and the primer pair of SEQ ID No.10 and SEQ ID No.11 and the primer pair of SEQ ID No. 16 and SEQ ID No.17, the primer pair of SEQ ID No.4 and SEQ ID No.5 and the primer pair of SEQ ID No.10 and SEQ ID No.11 and the primer pair of SEQ ID No.18 and SEQ ID No.19, the primer pair of SEQ ID No.4 and SEQ ID No.5 and the primer pair of SEQ ID No.10 and SEQ ID No.11 and the primer pair of SEQ ID No.20 and SEQ ID No.21, the primer pair of SEQ ID No.4 and SEQ ID No.5 and the primer pair of SEQ ID No.12 and SEQ ID No.13 and the primer pair of SEQ ID No.16 and SEQ ID No.17, the primer pair of SEQ ID No.4 and SEQ ID No.5 and the primer pair of SEQ ID No.12 and SEQ ID No.13 and the primer pair of SEQ ID No.18 and SEQ ID No.19, the primer pair of SEQ ID No.4 and SEQ ID No.5 and the primer pair of SEQ ID No.11 and SEQ ID No.12 and the primer pair of SEQ ID No.20 and SEQ ID No.21, the primer pair of SEQ ID No.4 and SEQ ID No.5 and the primer pair of SEQ ID No.14 and SEQ ID No.15 and the primer pair of SEQ ID No.16 and SEQ ID No.17, the primer pair of SEQ ID No.4 and SEQ ID No.5 and the primer pair of SEQ ID No.14 and SEQ ID No.15 and the primer pair of SEQ ID No.18 and SEQ ID No.19, the primer pair of SEQ ID No.4 and SEQ ID No.5 and the primer pair of SEQ ID No.14 and SEQ ID No.15 and the primer pair of SEQ ID No.20 and SEQ ID No.21, the primer pair of SEQ ID No.6 and SEQ ID No.7 and the primer pair of SEQ ID No.8 and SEQ ID No.9 and the primer pair of SEQ ID No.16 and SEQ ID No.17, the primer pair of SEQ ID No.6 and SEQ ID No.7 and the primer pair of SEQ ID No.8 and SEQ ID No.9 and the primer pair of SEQ ID No.18 and SEQ ID No.19, the primer pair of SEQ ID No.6 and SEQ ID No.7 and the primer pair of SEQ ID No.8 and SEQ ID No.9 and the primer pair of SEQ ID No.20 and SEQ ID No.21, the primer pair of SEQ ID No.6 and SEQ ID No.7 and the primer pair of SEQ ID No.10 and SEQ ID No.11 and the primer pair of SEQ ID No.16 and SEQ ID No.17, the primer pair of SEQ ID No.6 and SEQ ID No.7 and the primer pair of SEQ ID No.10 and SEQ ID No.11 and the primer pair of SEQ ID No.18 and SEQ ID No.19,the primer pair of SEQ ID No.6 and SEQ ID No.7 and the primer pair of SEQ ID No.10 and SEQ ID No.11 and the primer pair of SEQ ID No.20 and SEQ ID No.21, the primer pair of SEQ ID No.6 and SEQ ID No.7 and the primer pair of SEQ ID No.12 and SEQ ID No.13 and the primer pair of SEQ ID No.16 and SEQ ID No.17, the primer pair of SEQ ID No.6 and SEQ ID No.7 and the primer pair of SEQ ID No.12 and SEQ ID No.13 and the primer pair of SEQ ID No.18 and SEQ ID No.19, the primer pair of SEQ ID No.6 and SEQ ID No.7 and the primer pair of SEQ ID No.11 and SEQ ID No.12 and the primer pair of SEQ ID No.20 and SEQ ID No.21, the primer pair of SEQ ID No.6 and SEQ ID No.7 and the primer pair of SEQ ID No.14 and SEQ ID No.15 and the primer pair of SEQ ID No.16 and SEQ ID No.17, the primer pair of SEQ ID No.6 and SEQ ID No.7 and the primer pair of SEQ ID No.14 and SEQ ID No.15 and the primer pair of SEQ ID No.18 and SEQ ID No.19, and the primer pair of SEQ ID No.6 and SEQ ID No.7 and the primer pair of SEQ ID No.14 and SEQ ID No.15 and the primer pair of SEQ ID No.20 and SEQ ID No.21.

Preferably, the kit comprises a combination of six different primer pairs specific of three different antisense lncRNAs selected from the group consisting of: the primer pairs of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.10 and SEQ ID No.11 and the primer pair of SEQ ID No.16 and SEQ ID No.17 and the primer pair of SEQ ID No.18 and SEQ ID No.19,the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.10 and SEQ ID No.11 and the primer pair of SEQ ID No.16 and SEQ ID No.17 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.10 and SEQ ID No.11 and the primer pair of SEQ ID No.18 and SEQ ID No.19 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.12 and SEQ ID No.13 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.12 and SEQ ID No.13 and the primer pair of SEQ ID No.16 and SEQ ID No.17 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.12 and SEQ ID No.13 and the primer pair of SEQ ID No.18 and SEQ ID No.19 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.14 and SEQ ID No.15 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.14 and SEQ ID No.15 and the primer pair of SEQ ID No.16 and SEQ ID No.17 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.8 and SEQ ID No.9 and the primer pair of SEQ ID No.14 and SEQ ID No.15 and the primer pair of SEQ ID No.18 and SEQ ID No.19 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.12 and SEQ ID No.13 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.12 and SEQ ID No.13 and the primer pair of SEQ ID No.16 and SEQ ID No.17 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.12 and SEQ ID No.13 and the primer pair of SEQ ID No.18 and SEQ ID No.19 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.14 and SEQ ID No.15 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.14 and SEQ ID No.15 and the primer pair of SEQ ID No.16 and SEQ ID No.17 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.10 and SEQ ID No.11 and the primer pair of SEQ ID No.14 and SEQ ID No.15 and the primer pair of SEQ ID No.18 and SEQ ID No.19 and the primer pair of SEQ ID No.20 and SEQ ID No.21, the primer pair of SEQ ID No.4 and SEQ ID No.5 and the primer pair of SEQ ID No.6 and SEQ ID No.7 and the primer pair of SEQ ID No.12 and SEQ ID No.13 and the primer pair of SEQ ID No.14 and SEQ ID No.15 and the primer pair of SEQ ID No.16 and SEQ ID No.17 and the primer pair of SEQ ID No.18 and SEQ ID No.19, the primer pair of SEQ ID No.4 and SEQ ID No.5 and the primer pair of SEQ ID No.6 and SEQ ID No.7 and the primer pair of SEQ ID No.12 and SEQ ID No.13 and the primer pair of SEQ ID No.14 and SEQ ID No.15 and the primer pair of SEQ ID No.16 and SEQ ID No. 17 and the primer pair of SEQ ID No.20 and SEQ ID No.21, and the primer pair of SEQ ID No.4 and SEQ ID No.5 and the primer pair of SEQ ID No.6 and SEQ ID No.7 and the primer pair of SEQ ID No.12 and SEQ ID No.13 and the primer pair of SEQ ID No.14 and SEQ ID No.15 and the primer pair of SEQ ID No.18 and SEQ ID No.19 and the primer pair of SEQ ID No.20 and SEQ ID No.21.

The above mentioned combination may further comprise additional primer pairs, preferably primer pairs targeting other antisense IncRNA which are diagnosis marker for prostate cancer, more preferably primer pairs for PCA3 quantitative amplification.

The disclosure also concerns the use of a kit as described above in the diagnosis of prostate cancer in a subject.

Preferably, the subject is an animal, more preferably a mammal, even more preferably a human.

In a most preferred aspect, the subject is an adult man of at least 50 years old.

### Examples

### Material and Methods

### Prostate and breast tissues, RNA samples:

Prostate tissues were provided by the Mondor Hospital and breast tissues by Institut Curie. The histological status of each tumor was determined by respective clinical pathology services.

Total RNA was extracted from tissues or urines using Qiagen kit, quantified and quality controlled using a 2100 Bioanalyzer (Agilent). RNA samples with RNA Integrity Number (RIN) above 6 were depleted for ribosomal RNA and converted into cDNA library using a *TruSeq* Stranded *Total* Library Preparation *kit* (Illumina). cDNA libraries were normalized using an Illumina Duplex-specific Nuclease (DSN) protocol.

### RNA sequencing and data analysis:

Strand-specific cDNA library were sequenced in a paired-end mode on HiSeq^{™} 2500 (Illumina, NGS Curie platform). At least a 20X coverage per sample was considered as minimum of unique sequences for further data analysis. RNAseq data has been further analysed by in-house developed computational pipeline using a novel extended antisense annotation file to isolate all antisense IncRNA expressed to 0.8 RPKM.

### Signature of prostate tumor tissues:

Differential expression analysis between prostate normal and tumor samples was performed using the DESeq R package. Only antisense lncRNAs with an expression fold change >2 and adjusted P-value below 0.05 were retained as differentially expressed to constitute prostate tumor signature.

### Receiver Operating Curve (ROC) analysis:

ROC curves assessed the assignment of 89 patients as high-risk versus low or intermediate risk, based on PCGEM1 only, PCA3 only or the sum of 3 novel antisense IncRNA (asFBXL7, asABCC4 and asPDLIM5) or the sum of 4 lncRNAs (asFBXL7, asABCC4, asPDLIM5 and PCA3), using pROC R-package and log2(expression) scores from Nanostring data.

Reverse transcription and quantitative PCR: Reverse Transcription was performed on 100 ng of total RNA using gene-specific, strand-specific or random primers and the SuperScript II (Invitrogen) reverse transcriptase. cDNA for each transcript was quantified relative to the POLR2A housekeeping gene by the real-time PCR with LightCycler-time PCR with as quantified relative to Roche LightCycler480. Error bars represent standard deviations of three independent RT.

### NanoString nCounter Gene Expression Assay:

Total RNA isolated from 80 tumors and 9 normal prostate tissues was used for direct digital detection of 13 different RNAs: 3 housekeeping genes (RPL11, GAPDH and POLR2A), 4 mRNAs deregulated in our or published RNAseq datasets (HPN, OR51E2, PDLIM5, PCA3 and SLC14A1), 3 lncRNAs previously reported as deregulated in different cancers (HOTAIR, ANRIL and PCGEM1), and 3 antisense IncRNA (asFBXL7, asPDLIM5 and asABCC4). Each target gene of interest was detected using reporter and capture probes carrying 35- to 50-base target-specific sequences on Nanostring nCounter V2 (Curie Genomic platform).

### Results

Here, we investigated the presence of antisense lncRNAs by performing RNA-seq experiments within 8 normal and 16 tumor prostate tissues. Differential expression analysis revealed 157 antisense lncRNAs significantly overexpressed in tumors encompassing 48 previously annotated antisense lncRNA, and 109 novel transcripts. Interestingly, the 3 most up-regulated antisense IncRNA showed similar fold-changes as the classic prostate cancer marker *PCA3* albeit with lower dispersion, revealing a novel robust restricted signature for prostate cancer (Figure 1). Expression of these three antisense lncRNAs have been further validated by the quantitative RT-PCR (data not shown) and nanostring single molecule quantification, all of them being significantly (at least 5 fold) higher expressed in tumor tissues comparing to normal ones (Figure 2A). Interestingly, antisense IncRNA nanostring signals were constant in relapse and non-relapse tumor patients revealing robust detection accuracy. In contrast, the commercial PCA3 RNA level failed to maintain such robustness since the relapse patients show a dramatic decrease in PCA3 expression measurements (Figure 2B). Strikingly, a prediction analysis on a cohort of 89 patients from RNA extraction measured by Nanostring demonstrated that the "restricted signature" discriminates relapse patients more efficiently than *PCA3* alone (AUC 0.95 and 0.89, respectively; Figure 2C). In contrast, these novel markers had similar or lower AUC than *PCA3* for non-relapsing cases (data not shown), supporting their combined usage as markers for prognostic studies. Strikingly, the detection of prostate tumors is compatible with RNA extracted directly from urines after prostate massages (Figure 3), suggesting an easy, fast and cost effective way to detect prostate tumors at early stages without intrusive biopsy.

The inventors have discovered three new antisense IncRNA that allow discriminating prostate tumors from normal prostate tissues, but also prostate tumors from breast tumor and normal tissues. Interestingly, using a cohort of 89 patients' biopsies they showed comparable fold-changes of expression as PCA3 albeit with lower dispersion, revealing a novel robust restricted signature for prostate cancer. Strikingly, a prediction analysis on urines patients based on NanoString antisense lncRNAs measurements, demonstrated that the restricted signature discriminates prostate tumors more efficiently than PCA3.

The inventors also compared sensitivity and specificity prediction for the three new antisense IncRNA alone and in combination. ROC curves analysis show that each antisense IncRNA has a significant prediction level: area under the curve are of 0.88 for asPDLIM5, 0.96 for asFBXL7, and 0.93 for asABCC4. The sum of the three antisense IncRNA is characterized by an area under the curve of 0.96, indicating an excellent robustness of detection with a maximum of both specificity and sensitivity when compared to the 3 separately.

## Claims

1. Use of at least one antisense long non-coding RNA (antisense lncRNA) as a diagnosis marker for prostate cancer, wherein said at least one antisense IncRNA is selected from the group consisting of
- asFBXL7 or a RNA having a nucleotide sequence identity of at least 90 % with SEQ ID NO: 1,
- asABCC4 or a RNA having a nucleotide sequence identity of at least 90 % with SEQ ID NO: 2, 28 or 29,
- asPDLIM5 of SEQ ID NO: 3 or 30, or a RNA having a nucleotide sequence identity of at least 90 % with SEQ ID NO: 3 or 30,
- a combination of asFBXL7 or a RNA having a nucleotide sequence identity of at least 90 % with SEQ ID NO: 1 and asABCC4 or a RNA having a nucleotide sequence identity of at least 90 % with SEQ ID NO: 2, 28 or 29,
- a combination of asFBXL7 or a RNA having a nucleotide sequence identity of at least 90 % with SEQ ID NO: 1 and asPDLIM5 or a RNA having a nucleotide sequence identity of at least 90 % with SEQ ID NO: 3 or 30,
- a combination of asABCC4 or a RNA having a nucleotide sequence identity of at least 90 % with SEQ ID NO: 2, 28 or 29 and asPDLIM5 or a RNA having a nucleotide sequence identity of at least 90 % with SEQ ID NO: 3 or 30, and
- a combination of asFBXL7 or a RNA having a nucleotide sequence identity of at least 90 % with SEQ ID NO: 1, asABCC4 or a RNA having a nucleotide sequence identity of at least 90 % with SEQ ID NO: 2, 28 or 29, and asPDLIM5 or a RNA having a nucleotide sequence identity of at least 90 % with SEQ ID NO: 3 or 30.

2. The use according to claim 1, wherein at least three antisense lncRNAs are used in combination and wherein said combination comprises asFBXL7 or a RNA having a nucleotide sequence identity of at least 90 % with SEQ ID NO: 1, asABCC4 or a RNA having a nucleotide sequence identity of at least 90 % with SEQ ID NO: 2, 28 or 29 and asPDLIM5 or a RNA having a nucleotide sequence identity of at least 90 % with SEQ ID NO: 3 or 30.

3. An *in vitro* method for prostate cancer diagnosis of a subject, wherein the method comprises the step of determining the amount of at least one antisense IncRNA in a biological sample from said subject, said at least one antisense IncRNA being selected from the group consisting of
- asFBXL7 or a RNA having a nucleotide sequence identity of at least 90 % with SEQ ID NO: 1,
- asABCC4 or a RNA having a nucleotide sequence identity of at least 90 % with SEQ ID NO: 2, 28 or 29,
- asPDLIM5 of SEQ ID NO: 3 or 30, or a RNA having a nucleotide sequence identity of at least 90 % with SEQ ID NO: 3 or 30,
- a combination of asFBXL7 or a RNA having a nucleotide sequence identity of at least 90 % with SEQ ID NO: 1 and asABCC4 or a RNA having a nucleotide sequence identity of at least 90 % with SEQ ID NO: 2, 28 or 29,
- a combination of asFBXL7 or a RNA having a nucleotide sequence identity of at least 90 % with SEQ ID NO: 1 and asPDLIM5 or a RNA having a nucleotide sequence identity of at least 90 % with SEQ ID NO: 3 or 30,
- a combination of asABCC4 or a RNA having a nucleotide sequence identity of at least 90 % with SEQ ID NO: 2, 28 or 29 and asPDLIM5 or a RNA having a nucleotide sequence identity of at least 90 % with SEQ ID NO: 3 or 30, and
- a combination of asFBXL7 or a RNA having a nucleotide sequence identity of at least 90 % with SEQ ID NO: 1, asABCC4 or a RNA having a nucleotide sequence identity of at least 90 % with SEQ ID NO: 2, 28 or 29, and asPDLIM5 or a RNA having a nucleotide sequence identity of at least 90 % with SEQ ID NO: 3 or 30.
and wherein an increased amount of the at least one antisense IncRNA is indicative of prostate cancer.

4. The method according to claim 3, wherein the method comprises the determination of the amounts of at least three antisense lncRNAs and wherein said at least three antisense lncRNAs comprise asFBXL7 or a RNA having a nucleotide sequence identity of at least 90 % with SEQ ID NO: 1, asABCC4 or a RNA having a nucleotide sequence identity of at least 90 % with SEQ ID NO: 2, 28 or 29 and asPDLIM5 or a RNA having a nucleotide sequence identity of at least 90 % with SEQ ID NO: 3 or 30.

5. The method according to claim 3 or 4, wherein the method further comprises the determination of the amounts of additional prostate cancer markers or fragments thereof, preferably antisense lncRNAs prostate cancer markers or fragments thereof, more preferably the PCA3 antisense IncRNA or a fragment thereof.

6. The method according to claim 3-5, wherein said sample is a body fluid, preferably a urine sample.

7. The method according to claim 3-6, wherein the antisense IncRNA amount is determined by amplification or by hybridization, preferably by quantitative RT-PCR or by the Nanostring method, preferably by the Nanostring method.

8. The method according to claim 3-7, wherein the subject is a mammal, preferably a human.

9. The method according to claim 8, wherein the subject is a man, preferably an adult man, more preferably a man of at least 50 years old.

10. Use of a kit for the diagnosis of prostate cancer in a subject, wherein the kit comprises
(i) probes and/or primers capable to specifically hybridize to SEQ ID No. 1 ; or
(ii) probes and/or primers capable to specifically hybridize to SEQ ID No. 2; or
(i) probes and/or primers capable to specifically hybridize to SEQ ID No. 1 ; and (ii) probes and/or primers capable to specifically hybridize to SEQ ID No. 2; or
(i) probes and/or primers capable to specifically hybridize to SEQ ID No. 1 ; and (iii) probes and/or primers capable to specifically hybridize to SEQ ID No. 3; or
(ii) probes and/or primers capable to specifically hybridize to SEQ ID No. 2, and (iii) probes and/or primers capable to specifically hybridize to SEQ ID No. 3; or
(i) probes and/or primers capable to specifically hybridize to SEQ ID No. 1, (ii) probes and/or primers capable to specifically hybridize to SEQ ID No. 2, and (iii) probes and/or primers capable to specifically hybridize to SEQ ID No. 3;
and optionally, a leaflet providing guidelines to use such a kit.

11. Use of the kit according to claim 10, wherein the kit further comprises probes and/or primers for the detection of additional prostate cancer markers, preferably probes and/or primers for the detection of antisense lncRNAs prostate cancer markers, more preferably probes and/or primers for the detection of the PCA3 antisense lncRNA.

12. Use of the kit according to claim 10 or 11, wherein, for each antisense lncRNA, the kit comprises two probes:
- A capture-probe which comprises a nucleotide sequence hybridizing a first part of the antisense IncRNA and a molecule able to bind a solid support; and
- A reporter-probe which comprises a nucleotide sequence hybridizing a second part of the antisense IncRNA and a detectable label.

13. Use of a kit according to claims 10-12, wherein the subject is an animal, preferably a mammal, more preferably a human, even more preferably an adult man of at least 50 years old.

## Patentansprüche

1. - Verwendung mindestens einer Antisense langen nicht-kodierenden RNA (Antisense-lncRNA; antisense long non-coding RNA) als Diagnosemarker für Prostatakrebs, wobei die mindestens eine Antisense-lncRNA ausgewählt ist aus der Gruppe bestehend aus
- asFBXL7 oder einer RNA mit einer Nukleotidsequenzidentität von mindestens 90% mit SEQ ID NO: 1,
- asABCC4 oder einer RNA mit einer Nukleotidsequenzidentität von mindestens 90% mit SEQ ID NO: 2, 28 oder 29,
- asPDLIM5 der SEQ ID NO: 3 oder 30 oder einer RNA mit einer Nukleotidsequenzidentität von mindestens 90% mit SEQ ID NO: 3 oder 30,
- einer Kombination aus asFBXL7 oder einer RNA mit einer Nukleotidsequenzidentität von mindestens 90% mit SEQ ID NO: 1 und asABCC4 oder einer RNA mit einer Nukleotidsequenzidentität von mindestens 90% mit SEQ ID NO: 2, 28 oder 29,
- einer Kombination aus asFBXL7 oder einer RNA mit einer Nukleotidsequenzidentität von mindestens 90% mit SEQ ID NO: 1 und asPDLIM5 oder einer RNA mit einer Nukleotidsequenzidentität von mindestens 90% mit SEQ ID NO: 3 oder 30,
- einer Kombination aus asABCC4 oder einer RNA mit einer Nukleotidsequenzidentität von mindestens 90% mit SEQ ID NO: 2, 28 oder 29 und asPDLIM5 oder einer RNA mit einer Nukleotidsequenzidentität von mindestens 90% mit SEQ ID NO: 3 oder 30, und
- einer Kombination aus asFBXL7 oder einer RNA mit einer Nukleotidsequenzidentität von mindestens 90% mit SEQ ID NO: 1, asABCC4 oder einer RNA mit einer Nukleotidsequenzidentität von mindestens 90% mit SEQ ID NO: 2, 28 oder 29, und asPDLIM5 oder einer RNA mit einer Nukleotidsequenzidentität von mindestens 90% mit SEQ ID NO: 3 oder 30.

2. Verwendung nach Anspruch 1, wobei mindestens drei Antisense-lncRNAs in Kombination verwendet werden und wobei die Kombination asFBXL7 oder eine RNA mit einer Nukleotidsequenzidentität von mindestens 90% mit SEQ ID NO: 1, asABCC4 oder eine RNA mit einer Nukleotidsequenzidentität von mindestens 90% mit SEQ ID NO: 2, 28 oder 29 und asPDLIM5 oder eine RNA mit einer Nukleotidsequenzidentität von mindestens 90% mit SEQ ID NO: 3 oder 30 umfasst.

3. *In-vitro-*Verfahren zur Prostatakrebsdiagnose eines Subjekts, wobei das Verfahren den Schritt des Bestimmens der Menge von mindestens einer Antisense-lncRNA in einer biologischen Probe von diesem Subjekt umfasst, wobei die mindestens eine Antisense-lncRNA ausgewählt ist aus der Gruppe bestehend aus
- asFBXL7 oder einer RNA mit einer Nukleotidsequenzidentität von mindestens 90% mit SEQ ID NO: 1,
- asABCC4 oder einer RNA mit einer Nukleotidsequenzidentität von mindestens 90% mit SEQ ID NO: 2, 28 oder 29,
- asPDLIM5 der SEQ ID NO: 3 oder 30 oder einer RNA mit einer Nukleotidsequenzidentität von mindestens 90% mit SEQ ID NO: 3 oder 30,
- einer Kombination aus asFBXL7 oder einer RNA mit einer Nukleotidsequenzidentität von mindestens 90% mit SEQ ID NO: 1 und asABCC4 oder einer RNA mit einer Nukleotidsequenzidentität von mindestens 90% mit SEQ ID NO: 2, 28 oder 29,
- einer Kombination aus asFBXL7 oder einer RNA mit einer Nukleotidsequenzidentität von mindestens 90% mit SEQ ID NO: 1 und asPDLIM5 oder einer RNA mit einer Nukleotidsequenzidentität von mindestens 90% mit SEQ ID NO: 3 oder 30,
- einer Kombination aus asABCC4 oder einer RNA mit einer Nukleotidsequenzidentität von mindestens 90% mit SEQ ID NO: 2, 28 oder 29 und asPDLIM5 oder einer RNA mit einer Nukleotidsequenzidentität von mindestens 90% mit SEQ ID NO: 3 oder 30, und
- einer Kombination aus asFBXL7 oder einer RNA mit einer Nukleotidsequenzidentität von mindestens 90% mit SEQ ID NO: 1, asABCC4 oder einer RNA mit einer Nukleotidsequenzidentität von mindestens 90% mit SEQ ID NO: 2, 28 oder 29, und asPDLIM5 oder einer RNA mit einer Nukleotidsequenzidentität von mindestens 90 % mit SEQ ID NO: 3 oder 30;
und wobei eine erhöhte Menge der mindestens einen Antisense-lncRNA auf Prostatakrebs hinweist.

4. Verfahren nach Anspruch 3, wobei das Verfahren die Bestimmung der Mengen von mindestens drei Antisense-lncRNAs umfasst und wobei diese mindestens drei Antisense IncRNAs asFBXL7 oder eine RNA mit einer Nukleotidsequenzidentität von mindestens 90% mit SEQ ID NO: 1, asABCC4 oder eine RNA mit einer Nukleotidsequenzidentität von mindestens 90% mit SEQ ID NO: 2, 28 oder 29 und asPDLIM5 oder eine RNA mit einer Nukleotidsequenzidentität von mindestens 90% mit SEQ ID NO: 3 oder 30 umfasst.

5. Verfahren nach Anspruch 3 oder 4, wobei das Verfahren ferner die Bestimmung der Mengen zusätzlicher Prostatakrebsmarker oder Fragmente davon, vorzugsweise Antisense-lncRNAs Prostatakrebsmarker oder Fragmente davon, stärker bevorzugt der PCA3-Antisense-lncRNA oder eines Fragments davon, umfasst.

6. Verfahren nach Anspruch 3-5, wobei diese Probe eine Körperflüssigkeit, vorzugsweise eine Urinprobe ist.

7. Verfahren nach Anspruch 3-6, wobei die Antisense-lncRNA-Menge durch Amplifikation oder durch Hybridisierung bestimmt wird, vorzugsweise durch quantitative RT-PCR oder durch das Nanostring-Verfahren, vorzugsweise durch das Nanostring-Verfahren.

8. - Verfahren nach Anspruch 3-7, wobei das Subjekt ein Säugetier ist, vorzugsweise ein Mensch.

9. - Verfahren nach Anspruch 8, wobei das Subjekt ein Mann ist, vorzugsweise ein erwachsener Mann, stärker bevorzugt ein Mann von mindestens 50 Jahren.

10. - Verwendung eines Kits zur Diagnose von Prostatakrebs in einem Subjekt, wobei das Kit umfasst
(i) Sonden und/oder Primer, die in der Lage sind, spezifisch mit SEQ ID Nr. 1 zu hybridisieren; oder
(ii) Sonden und/oder Primer, die in der Lage sind, spezifisch mit SEQ ID Nr. 2 zu hybridisieren; oder
(i) Sonden und/oder Primer, die in der Lage sind, spezifisch mit SEQ ID Nr. 1 zu hybridisieren; und (ii) Sonden und/oder Primer, die in der Lage sind, spezifisch mit SEQ ID Nr. 2 zu hybridisieren; oder
(i) Sonden und/oder Primer, die in der Lage sind, spezifisch mit SEQ ID Nr. 1 zu hybridisieren; und (iii) Sonden und/oder Primer, die in der Lage sind, spezifisch mit SEQ ID Nr. 3 zu hybridisieren; oder
(ii) Sonden und/oder Primer, die in der Lage sind, spezifisch mit SEQ ID Nr. 2 zu hybridisieren, und (iii) Sonden und/oder Primer, die in der Lage sind, spezifisch mit SEQ ID Nr. 3 zu hybridisieren; oder
(i) Sonden und/oder Primer, die in der Lage sind, spezifisch mit SEQ ID Nr. 1 zu hybridisieren, (ii) Sonden und/oder Primer, die in der Lage sind, spezifisch mit SEQ ID Nr. 2 zu hybridisieren, und (iii) Sonden und/oder Primer, die in der Lage sind, spezifisch mit SEQ ID Nr. 3 zu hybridisieren;
und optional eine Broschüre mit Richtlinien zur Verwendung eines solchen Kits.

11. Verwendung des Kits nach Anspruch 10, wobei das Kit ferner Sonden und/oder Primer zum Nachweis von zusätzlichen Prostatakrebsmarkern, vorzugsweise Sonden und/oder Primer zum Nachweis von Antisense-lncRNAs-Prostatakrebsmarkern, besonders bevorzugt Sonden und/oder Primer für den Nachweis der PCA3-Antisense-lncRNA, umfasst.

12. Verwendung des Kits nach Anspruch 10 oder 11, wobei das Kit für jede Antisense-lncRNA zwei Sonden umfasst:
- eine Fängersonde, die eine Nukleotidsequenz umfasst, die mit einem ersten Teil der Antisense-lncRNA hybridisiert, und ein Molekül, das in der Lage ist, einen festen Träger zu binden; und
- eine Reportersonde, die eine Nukleotidsequenz umfasst, die mit einem zweiten Teil der Antisense-lncRNA hybridisiert, und ein nachweisbares Label.

13. Verwendung eines Kits nach den Ansprüchen 10-12, wobei das Subjekt ein Tier, vorzugsweise ein Säugetier, stärker bevorzugt ein Mensch, noch stärker bevorzugt ein erwachsener Mann von mindestens 50 Jahren ist.

## Revendications

1. Utilisation d'au moins un ARN non codant long antisens (ARNncl antisens) en tant que marqueur diagnostique d'un cancer de la prostate, dans laquelle ledit au moins un ARNncl est choisi dans le groupe constitué par
- asFBXL7 ou un ARN ayant une identité de séquence de nucléotides d'au moins 90 % avec la SEQ ID NO : 1,
- asABCC4 ou un ARN ayant une identité de séquence de nucléotides d'au moins 90 % avec la SEQ ID NO : 2, 28 ou 29,
- asPDLIM5 de la SEQ ID NO : 3 ou 30, ou un ARN ayant une identité de séquence de nucléotides d'au moins 90 % avec la SEQ ID NO : 3 ou 30,
- une combinaison d'asFBXL7 ou d'un ARN ayant une identité de séquence de nucléotides d'au moins 90 % avec la SEQ ID NO : 1 et d'asABCC4 ou d'un ARN ayant une identité de séquence de nucléotides d'au moins 90 % avec la SEQ ID NO : 2, 28 ou 29,
- une combinaison d'asFBXL7 ou d'un ARN ayant une identité de séquence de nucléotides d'au moins 90 % avec la SEQ ID NO : 1 et d'asPDLIM5 ou d'un ARN ayant une identité de séquence de nucléotides d'au moins 90 % avec la SEQ ID NO : 3 ou 30,
- une combinaison d'asABCC4 ou d'un ARN ayant une identité de séquence de nucléotides d'au moins 90 % avec la SEQ ID NO : 2, 28 ou 29 et d'asPDLIM5 ou d'un ARN ayant une identité de séquence de nucléotides d'au moins 90 % avec la SEQ ID NO : 3 ou 30, et
- une combinaison d'asFBXL7 ou d'un ARN ayant une identité de séquence de nucléotides d'au moins 90 % avec la SEQ ID NO : 1, d'asABCC4 ou d'un ARN ayant une identité de séquence de nucléotides d'au moins 90 % avec la SEQ ID NO : 2, 28 ou 29, et d'asPDLIM5 ou d'un ARN ayant une identité de séquence de nucléotides d'au moins 90 % avec la SEQ ID NO : 3 ou 30.

2. Utilisation selon la revendication 1, dans laquelle au moins trois ARNncl antisens sont utilisés en combinaison et dans laquelle ladite combinaison comprend asFBXL7 ou un ARN ayant une identité de séquence de nucléotides d'au moins 90 % avec la SEQ ID NO : 1, asABCC4 ou un ARN ayant une identité de séquence de nucléotides d'au moins 90 % avec la SEQ ID NO : 2, 28 ou 29 et asPDLIM5 ou un ARN ayant une identité de séquence de nucléotides d'au moins 90 % avec la SEQ ID NO : 3 ou 30.

3. Méthode *in vitro* pour le diagnostic d'un cancer de la prostate d'un sujet, laquelle méthode comprend une étape de détermination d'au moins un ARNncl antisens dans un échantillon biologique provenant dudit sujet, ledit au moins un ARNncl antisens étant choisi dans le groupe constitué par
- asFBXL7 ou un ARN ayant une identité de séquence de nucléotides d'au moins 90 % avec la SEQ ID NO : 1,
- asABCC4 ou un ARN ayant une identité de séquence de nucléotides d'au moins 90 % avec la SEQ ID NO : 2, 28 ou 29,
- asPDLIM5 de la SEQ ID NO : 3 ou 30, ou un ARN ayant une identité de séquence de nucléotides d'au moins 90 % avec la SEQ ID NO : 3 ou 30,
- une combinaison d'asFBXL7 ou d'un ARN ayant une identité de séquence de nucléotides d'au moins 90 % avec la SEQ ID NO : 1 et d'asABCC4 ou d'un ARN ayant une identité de séquence de nucléotides d'au moins 90 % avec la SEQ ID NO : 2, 28 ou 29,
- une combinaison d'asFBXL7 ou d'un ARN ayant une identité de séquence de nucléotides d'au moins 90 % avec la SEQ ID NO : 1 et d'asPDLIM5 ou d'un ARN ayant une identité de séquence de nucléotides d'au moins 90 % avec la SEQ ID NO : 3 ou 30,
- une combinaison d'asABCC4 ou d'un ARN ayant une identité de séquence de nucléotides d'au moins 90 % avec la SEQ ID NO : 2, 28 ou 29 et d'asPDLIM5 ou d'un ARN ayant une identité de séquence de nucléotides d'au moins 90 % avec la SEQ ID NO : 3 ou 30, et
- une combinaison d'asFBXL7 ou d'un ARN ayant une identité de séquence de nucléotides d'au moins 90 % avec la SEQ ID NO : 1, d'asABCC4 ou d'un ARN ayant une identité de séquence de nucléotides d'au moins 90 % avec la SEQ ID NO : 2, 28 ou 29, et d'asPDLIM5 ou d'un ARN ayant une identité de séquence de nucléotides d'au moins 90 % avec la SEQ ID NO : 3 ou 30,
et dans laquelle une quantité accrue de l'au moins un ARNncl antisens est indicative d'un cancer de la prostate.

4. Méthode selon la revendication 3, laquelle méthode comprend la détermination des quantités d'au moins trois ARNncl antisens, et dans laquelle lesdits au moins trois ARNncl antisens comprennent asFBXL7 ou un ARN ayant une identité de séquence de nucléotides d'au moins 90 % avec la SEQ ID NO : 1, asABCC4 ou un ARN ayant une identité de séquence de nucléotides d'au moins 90 % avec la SEQ ID NO : 2, 28 ou 29 et asPDLIM5 ou un ARN ayant une identité de séquence de nucléotides d'au moins 90 % avec la SEQ ID NO : 3 ou 30.

5. Méthode selon la revendication 3 ou 4, laquelle méthode comprend en outre la détermination des quantités de marqueurs additionnels de cancer de la prostate ou de fragments de ceux-ci, de préférence de marqueurs ARNncl antisens de cancer de la prostate ou de fragments de ceux-ci, de préférence encore de l'ARNncl antisens PCA3 ou d'un fragment de celui-ci.

6. Méthode selon les revendications 3 à 5, dans laquelle ledit échantillon est un fluide corporel, de préférence un échantillon d'urine.

7. Méthode selon les revendications 3 à 6, dans laquelle la quantité d'ARNncl antisens est déterminée par amplification ou par hybridation, de préférence par RT-PCR quantitative ou par la méthode Nanostring, de préférence par la méthode Nanostring.

8. Méthode selon les revendications 3 à 7, dans laquelle le sujet est un mammifère, de préférence un humain.

9. Méthode selon la revendication 8, dans laquelle le sujet est un homme, de préférence un homme adulte, de manière préférée un homme âgé d'au moins 50 ans.

10. Utilisation d'une trousse pour le diagnostic d'un cancer de la prostate chez un sujet, dans laquelle la trousse comprend
(i) des sondes et/ou amorces capables de s'hybrider spécifiquement à la SEQ ID NO : 1 ; ou
(ii) des sondes et/ou amorces capables de s'hybrider spécifiquement à la SEQ ID NO : 2 ; ou
(i) des sondes et/ou amorces capables de s'hybrider spécifiquement à la SEQ ID NO : 1 ; et (ii) des sondes et/ou amorces capables de s'hybrider spécifiquement à la SEQ ID NO : 2 ; ou
(i) des sondes et/ou amorces capables de s'hybrider spécifiquement à la SEQ ID NO : 1 ; et (iii) des sondes et/ou amorces capables de s'hybrider spécifiquement à la SEQ ID NO : 3 ; ou
(ii) des sondes et/ou amorces capables de s'hybrider spécifiquement à la SEQ ID NO : 2 ; et (iii) des sondes et/ou amorces capables de s'hybrider spécifiquement à la SEQ ID NO : 3 ; ou
(i) des sondes et/ou amorces capables de s'hybrider spécifiquement à la SEQ ID NO : 1, (ii) des sondes et/ou amorces capables de s'hybrider spécifiquement à la SEQ ID NO : 2, et (iii) des sondes et/ou amorces capables de s'hybrider spécifiquement à la SEQ ID NO : 3 ;
et éventuellement une notice présentant des directives pour utiliser une telle trousse.

11. Utilisation d'une trousse selon la revendication 10, dans laquelle la trousse comprend en outre des sondes et/ou amorces pour la détection de marqueurs additionnels de cancer de la prostate, de préférence des sondes et/ou amorces pour la détection de marqueurs ARNncl antisens de cancer de la prostate, de manière préférée des sondes et/ou amorces pour la détection de l'ARNncl antisens PCA3.

12. Utilisation d'une trousse selon la revendication 10 ou 11, dans laquelle, pour chaque ARNncl antisens, la trousse comprend deux sondes :
- une sonde de capture qui comprend une séquence de nucléotides hybridant une première partie de l'ARNncl antisens et une molécule capable de se lier à un support solide ; et
- une sonde rapporteuse qui comprend une séquence de nucléotides hybridant une deuxième partie de l'ARNncl antisens et un marqueur détectable.

13. Utilisation d'une trousse selon les revendications 10 à 12, dans laquelle le sujet est un animal, de préférence un mammifère, de manière préférée un humain, plus particulièrement un homme adulte âgé d'au moins 50 ans.


### SEQUENCE LISTING

<110> Institut Curie et al.
<120> USE OF ANTISENSE LONG NON-CODING RNAS FOR THE DIAGNOSIS OF PROSTATE CANCER
<130> B2155PC00
<160> 30
<170> PatentIn version 3.5
<210> 1
   <211> 78001
   <212> RNA
   <213> Homo sapiens
<400> 1
   augcuguggg ggaaggggga aaacucagcc auguggaagc aagacaacca auauaaaacc 60
   gucacugugu acuccccaua cagagagaag guuaagagca acaagauggg aggcagcugc 120
   auggaaccug ucccacugag gaaguaaaac agguaauggg guggcagcau gagaggagug 180
   gaguguuuau ggggccuauu auaugcaagu uuucuggcca uauaaucauc acaucacuuu 240
   ugcuagucug aacaaugcaa guuaaauguu gaaggaugca guuaaauguu gaaggaugca 300
   guuaaauuuc cuagguccuu gcauucugaa auugguauuu accugaaaaa uaaaccuaua 360
   caacauacag caagacacgg caaucauauc cagauuaaaa cucuucaaga augaaaaacc 420
   aaguauuaac acucaugaau gaguauuuag ugcacugugc uauguaugaa ucaaaaaucu 480
   uuuugaagug agagaaauua uccaggucuu cguccuacuc agaagggcaa uacuuuguga 540
   aaaauuuagc aguaagaucu aacuuuagcu ucaucccauu uuuguuuguu cuaaugucuu 600
   gugggucacu uagaaucuug uagggaagua aauuauggau uauuaauuua aaaacaugau 660
   aacaagggaa ggaacuuuug guccucccag cauucauuau uuagugcaau agacugaaug 720
   uguuuauauc cuugcaaaau ucauguguug aaaccugauu ccugaugugc ugauauuugc 780
   aagugggguc uuugggacgu gauuagguca ugugggcaga accuucauga auggaauuag 840
   ugcuuuuaug aaagggaccu uagagagcuc ucuugcccuu cuucuaccac gugaagacac 900
   agucagaaga cagucaucca ugaaucaaaa agcaggcccc caccaggcac caaucugcag 960
   aggcuugauu uuggaccccu cagccuccag cugugagaaa uacauuucug uuguuuauaa 1020
   gccaucuagu cugugguauu uuuguuacag cauccugaac agaauaagac auucagugaa 1080
   cuguaguggu ggaguauaca uuaauagcuu uuagaaauug ggguagccac ugaagaaugc 1140
   auuuuuagag uggccacuga agaaucuacu ucugauuuuu cuaauuuuuc cacauguguu 1200
   uuugaaguua agcagguucc uacuugauac uuuugaagaa uuuagguuuc aucaagaguc 1260
   cagagugcuc uaaagauucu uaauuucaau gaagcacuuu uuaugaguua ucuguuuagc 1320
   uuuucuucag auuguaaagu aaacuuguuc uaaagaaaac uggaagccug aaaauuaggc 1380
   caaggggcac augcucuucu aaauauuagc agaaguugga guagcaauga uuuucuuugu 1440
   ccuugaacca aaccguuuac auuucagaca guccauacua cuucaauucu uauaaucauc 1500
   auuuuuaguu uuauugauuu auuuguuauu uggagacauu uaugauguac uaugauguuu 1560
   gcucauaagg uucuaaaguu uauaccuuug auuuguuuuu auaugcugcc uugacuucua 1620
   aaggaaucca aaauaaaacg uuugcuggga auuauaaaga auuucauuga gaaaguaaaa 1680
   aagaaaaauc uaugcaaagg aagugauucu guuguuuaau gccaucaccu gucauucugu 1740
   cucuuaccau ggcuggcuug gcuaguggca ccacgaacag aucccuacag accccaacac 1800
   caagaaaagu agguuuuucu ucuuucuuug caguucuuug guauagauug auguuacuag 1860
   ggugguggaa ggccaucgug ugagaaaaga uuacacuuag uauugaggaa uguggugagg      1920
   augacuuccu uuuucuuacc cuaauaaugg acuuuuguuu uuagagaugc uguccuguaa 1980
   ccucagguuu aagaugagga uauuucugga uccaguguuu guacaguacu auuaauuauu 2040
   uauuucuucg uguaaaaaau aaauugauua uauucuugug agcuuagaca acagaaggcu 2100
   gucccaagua ggaccauucc cuauaugcug gaagaaaaaa aaaaaauuga gcugaccaag 2160
   cugagaaccu gaguuggauc auaguguggu uaccagguag acuuccccaa cagauauucc 2220
   aaaggccagu uaaucuugau acuaauucuc aguaccaggg acaaauuucu gcaucaaaga 2280
   cccugaggaa auacuuggcg acauuuucuu agguguguau cuuuaaaaag cauuaaagau      2340
   gaguuucaua ucuuugguca uuaccaccaa cauucuuaua auaagucugu ugaaagcucu 2400
   uucccuucuc aguuauuugc aggaaggucu uuaugggcuc cugaaauuuu uucuucucau 2460
   uaaugauuua gauuaugggu cuuuuuguuu cagauuuccc uuucaggccu cugaauaguu 2520
   ccugacuggg gagacugcau gacaucaccc uucugagcuu cucuaaggca uugauauggu 2580
   uguucuccag gucuuaagca cugcuggggc aaaacuaggg uuuuuugguu ugguuugguu 2640
   ucuugauuuu cuuaagucau ucagaccaaa gcuuauaaaa aaaucuugua aaacuuugcu 2700
   uuucaaaucc gcuuaucuca augauaacuc cuuuuucaua gaaauguuau cacaugcuau 2760
   cauuucaauc agcagaacac uugaaucauu acacuguuuc agaauuaaag uuaagaguua 2820
   aaaauauugu aaugauuggc caggcgcggu ggcucacgcc uguuaauccc agcacauugg 2880
   gaggccgagg caggcggauc accugagguu gggaguucaa gaccagccug accaacaugg 2940
   agaaaccccg ucucuacuaa aaauacaaaa uuagcugggu gugguugcac augccuguaa 3000
   ucccagcuac uugggaggau gaggcagaag aaucgcuuga acccaggagg uggagguugc 3060
   ggugagccga gauugcacca uugcacucca gccugggcaa caaaagcaaa acuccgucuc 3120
   aaaaaauaua uauauauauu uuguaaugau ugauagaaug uguguauaaa uauauauaua 3180
   augauugaua gaaugugugu auacauaucu cacacacauu uauauuauau acacauaauc 3240
   uugguuuuga aagcucugug acuuauucag uccuuucaaa aaggaggaaa aaaaaaaucc 3300
   uuguaauuug cuggccaaaa aauaaauacu uggcaaaggc ugagcaggag caaugaagug 3360
   acaucccaga ugaaaggcaa cagaaacuga ggaaauggcg cugcaaacgc augugguauu 3420
   gaauggguuc auaacacagc ucugagcugc cguuuuucua ugggcauaau ggauuagcug 3480
   agccgagaau acauugaguu uucuccucgu gucuauggga acuauuucag gugaagaacu 3540
   guauccugcg acacaucuuu uuagcuugag gcuaaaaucu aaauuuuacc auaaaagcac 3600
   uuuucacauu ucuaugccua aggacaauuu uguuggaauc aaugcacaaa ucuccuuuga 3660
   gaaugaugag ugcucaagac aaucucuuca uuucucucuu uuaaugcuga cugauaggca 3720
   ggauaucccu ucugcuuucu cccuuuuucu cucucacaca cauaaauguu augugucuau 3780
   caaucagucu cgugguuggc ugacauugca cuaccccuau auaucacuac cagguuuuug 3840
   gugaccauug cuuaggccuu uucgucaugu uuucaaguaa auuaacagaa uuuugaacgu 3900
   ggcuggggag aaggaggcuc agacauuuuc gucccaccag aaaugaguag cugauggaua 3960
   guugcuggcu gaacaaaugu uccagcucau gaagucccug gcuggggcug aguuuacuuu 4020
   cugaguaucu ucacucugcc uguaggugaa auccugguuu cucuuacuuu ggaugcaaua 4080
   gcuauacuaa acaaggcuga agacagaucu ucauagucau gaguuuggaa aacaugggau 4140
   uuguauugag gaggaggugg aagugaaugg gaaacucugg cuauauacau cacuaagucu 4200
   aucgcauuug caugcuggcu uugcaaugug ucucauuauu uguaggaguc acagugacau 4260
   uaugaaauau auugauagug auggccuccc aauggguuuc acaccuucua cucuuuucca 4320
   acucuagucc auucccauug gauuaauuuu uuaaaaacac agcugcuauc cauccacuga 4380
   ucccuuacgc aacaucuugc auugccugcu gaauuccaaa caaaguucuu gccuauccac 4440
   auaaggcagg ccuggcuggg gcuucaacac aauuuuucau ccucuucucc uauaccuuuc 4500
   ugacuucuag aaaaacuaga cuauucccua uuuccgggag cauagccuaa acucucccac 4560
   ccuucagaca cuacucaugu uguccccucu uccuucucau ccuugggaau uuauguuuuu 4620
   acuuuaaaau cuuuucacau cugacucaau ugguaucuuc uucauaaagu auuuccuggg 4680
   aucuucaaca ucugauguac accucacuua aacuauuaca gcauguucua uguuaauuaa 4740
   gauauuuguu ccucagccua cuaccagacu guaaacucca ugaucaugca ugcccugaug 4800
   auccuguuca aaacuauuuu ccaacaucua cagaagugcc ucacacuuaa gaggaaaaua 4860
   caauaaguau uuguugaaua aaugaguaag acgaacaaag gcguuagauu uaguuccuua 4920
   aaaucuuucu uucuaucucu ugcagcauca aggugaguga uugcauagaa gacuggauaa 4980
   auauuuguua aauuuagaag acccuucaaa auuuuguuga auuaggaaaa acuauguuua 5040
   ugaaaaaaaa auauauacgu auauauagag agagauagag ucucacucca ucacccaggc 5100
   uggagugcag uggcacaauc ucaguucacu gcaaccucug cuuccagguu caagcgauuc 5160
   uugugccuca cccuccagag cagcugggau uacaggcaca ugccaccacg ccuugcuaau 5220
   uuuuauauuu uuaguagaga ugggguuuug ccauguuggc caaacugauc ucgaacuccu 5280
   ggccucaagu gaucugccca ccucagccuc ccaaaaugcc gggauuacag gcaaggccca 5340
   ccaugccagg ccauccuauu acguauugua aaacaccuuu aaauaguuac cuuucuucag 5400
   acuuaaugcc uuaaguuggg caaaucuggg uuuuuuuuuu uucuucucau guucagauug 5460
   aguuauuaag gcauacacag aaauggaaau aguagcccug ggauaggacu gcugguuuuc 5520
   ugaugcuugu caauaacaac agaugccuga guaacccauc cccagauaau cuccauuuca 5580
   aaggggcaga ggaaggagga acuggcagga guuauucucc uaggccugug aucugggcuu 5640
   uccagcugga gaacuggugg cacaugccac uguugguccg ccagagccac aauauacaga 5700
   uuggcuuaaa gaccauuauc uucacacccg gugagauucu cccacacccu agcuggaacu 5760
   augagauagg ucguuuuaga ggcuuuuuuc aacauucuga uggcuuauuc auuugcauua 5820
   gguuucagaa augaggaaua gaugacagau caacaauuuu aguauccaug ccucucuacc 5880
   uaaccucuga cauuuccgac cuaaacaucu aaggucaaaa guagacgagg uggguggauc 5940
   acuugagguc aggaguucga gaccaggcug gccaacaugg ugaaacccca ucucuacuaa 6000
   aaauacaaaa aauuagccag gcaugguggc auaugccugu aaucccacau acuugggagg 6060
   cugaggcagg agaauugccu gaaccuggga ggcagcgguu gcagugagcu gagauccugc 6120
   cauugcacuc cagccugggc aacagauuga gacucugucu caaaaaaaaa aaaaaguaga 6180
   cucagucauu cagcugacuc ugagcucaug ucuuaaaucc uaauuuuaca gcuggccuug 6240
   gguuuaaaag uaugaaaaag gggccgggcc gguggcucac gccuguaauc ccagcacuuu 6300
   gggaggcuga ggaaggugga ucaugagguc aggagauuga gaccauccug gcuaacagag 6360
   ugaaaccccg ucucuacuaa aaauacaaaa aaauuagcca ggcguggugg cgggcaccug 6420
   uagucccagc uacuagggag gcugaggcag gagaauggcg ugaacccagg aggcagaguu 6480
   ugcagugagc cgaguuugcg ccacugcacu ccagagccug ggcaacagag cgagacucca 6540
   ucucaaaaaa aaaaaaaaaa aaaaaaaaaa guaugaaaaa gggcccuaag caucaaaaau 6600
   gggugaucau uuguaaggca acagaguuua aauaauugga gauguucacg uuguaauguu 6660
   ugcaaaugaa auauucacua agugcaauau aucuagcuua uagcaccuuc uuuacuuaug 6720
   auauuuuaau cucauauucu ucucuggaaa acuuuaaagg acuuuuaagg cuuucuauga 6780
   gagugccaau uaugcccugu uccaugugcu aagcaugaua cuugguuucc uugccuucaa 6840
   aauuaacuag auuaaaaaaa guuuaaauag cuuuauagaa ugccaauuuu agaaauaaua 6900
   caugcacaau gcuaaaaauu caggaaauaa uaucaaaaag aggaaaaaug auuaucuaua 6960
   aucccccuca cucaucggua aucauuuuaa cuuuuugaua uauaucuuuc caaacauagg 7020
   uagguaauug agguuuuugc cacuacuuuu aacagcaaaa accacaauua cuuuugugcc 7080
   aaccuaacag auuaacgagu agaauagaua ggacaaaggu auuuuagaau auugacaguu 7140
   uugaacuauu uuugaaacac uuucauuugg uaauuuguuu uauagacauu aaugagucaa 7200
   uaccugaaac aucacacaau gaaacccaaa uucuuaaaac cauuaaacug uuuggaaggc 7260
   uuauuuuuuc uucugugaag caacuugcua ucaccuuuua aaaaauuagc auucacucug 7320
   uccaauucua agaaaaaaaa accuuaaaaa aagauuuaaa uuucucucua ggccugaugg 7380
   cacagcuaga auugaguugg guguaguuuu ugggccaacu ccagauauuu ucuucuauga 7440
   auugaccuuu uuuugaggcu uaauaacagu aaaacucuac cucauaaaua uugugucaag 7500
   auuugacaua uuccauacau accuacuaaa aauguggcag aauauucucc uuagaaggaa 7560
   ucuuuuaggu aacucccaua gacaucagua aaaccucauc uaaacccuaa acagaaaacg 7620
   caauagaaaa uacaauuuca uauguaaagg ugauauguca cugcuaucaa ucaguaacuu 7680
   cuccauaguu cuuauaccug caugauaucu aucacugaaa uuuacuguuu gugcuuccau 7740
   caggauuugg uuagauaagu cucauauuuc aauaugcccu gaaauaaaaa uguuaucagc 7800
   uuugauuuuu guuauuagaa cauuauaagc uagcugugug uugcaauaac cagcuacaua 7860
   uuccagaagu ccauuugacc cuucuguuac ugggacugau auucuuuuuu guuuguuugu 7920
   uuguuuuguu uguuuuguuu uugagacaga guuuuacucu uguugcccag gcuggagcgc 7980
   aauggugcga ucucggcuca ccgcaaucuc ugccuccuga guucaagcga ggagcaagug 8040
   aguagcuggg acaacaggug ugccaccaug ccuggcuaau uuuguacuuu uaguagaaau 8100
   gggguuucuc uguuggucag gcuggucuug aacucccgac cgcccgccuu ggccucccaa 8160
   aguguuggga uuacaggcgu aagccaccgc gccuggcugg gacuaauauu cuaugaaggc 8220
   ugccagccug gggcuaagca ccuagcuuuu ccaaauguug acauguuuuc ucuauagccc 8280
   uaccugaugg acuggacuuc ugccuggauu ggaguuugau caugccucca uauggguguu 8340
   uaccaggcgu augcauugaa ccugaguuug ucucuucaau acaagguaaa acagagagaa 8400
   augauuuaau ucauccuaua gcauuauguc uucaaauaua aguucuaaua ugaaauauuu 8460
   gaccuaaauu auuuugauca caauuggaua acuccguuuc aaccagguga cuaauuuugg 8520
   cuugaaguug cauuugaaac auuaaaaaga auacccaaug ccauuauuuu uuaauuggac 8580
   cuuguagaca aaggcauauu caaaauuaaa aaaaaaagua cauucagauc augcuuuuau 8640
   gcaguaaguu aacugaaauu caauagguac uaugcuuacu auggucauuu cuuccaugua 8700
   ugcgauuauu aacauaaauu auucagguga uacguucauu ggccccagug gcacuaauuu 8760
   ucugugcaua aacucagaau ugauuuauaa caucccuaag uauuaugcuu auaucugauu 8820
   auuaaaauga auacaaauua acucuggcca uuucuuguuu cauaauuuca uagcauccuu 8880
   ugaaagaggc auugaaagag uuugccaauu guuauagaau aaacaguauc uguccguguc 8940
   uuacauccca uauuaguggg ggcaugaugg uuagugggag agacugauug gggucagacu 9000
   gauuuccacc cuguccugcc ucuccccagc ugagaggccu uguacauauu uaacucucua 9060
   agccuugauu ucuuuuuuuu uuuuuuuuga gacagagucu cacucuguca cccaggcugg 9120
   agugcagugg cgugaucucc gcucacugca agcucugccu cccagguucg cgccauucuc      9180
   cugucucagc cucccgagua gcugggacua caggugccca ccaccacgcc cagcuaauuu      9240
   uuguauuuuu aguagagacg ggguuucacc cuguuagcca ggauggucuc gaucuccuga 9300
   ccucgugauc cgccccccuc ggccucccaa agugcuggga uuacaggcgu gagccaccgc 9360
   gcccggccua agccuugauc ucuuaaucug uaaaaugagg aaaauaguuu ccaccucaca 9420
   gcauuguuau aaaaaugaaa cagaaaacaa augggcccag gugaaauacu uaaugaaggg 9480
   ucaggcacac ugcauucccu uucuccuucg cuugagcuga ggaacugucu gaauuccugc 9540
   cuugcacaua guaguugcuu aaucaauguc uuuaaaauga augaggauau gauauaauac 9600
   guuuucagau guaauaagua agcugcgaac ccaaagcaca guggaaucaa uuucaucaac 9660
   ccugcucaua accucugcug cuaccaccuu auuucaaacu accagucucc caucugaccu 9720
   uccgcuguga cuucugagcu ccugcagcca uugccguugu agaccccuga ggaucugucu 9780
   uccacacagc ugcauguccc gccucugcuc aaaacucucc agcaacaucu ucaacucacc 9840
   ucuuagcaua agagccacag ucuuuguaga acaugcaugg uuccacauca ucugccccua 9900
   ccguguguca accaccuggc uuucuacaug ccuuccucuu gcucacaggg acccccuugc 9960
   uguuccuaga augugccaug cacacuuccc ucaggaccua cccucugguu ggaguguugu 10020
   cuucaagaua ugggagggcu cacuccguuu guuccccugu cccccaggcu uucucugugc 10080
   ucugcgcuuu gugcugcagg aggcugaccu ccaugaacug cuucuccugg cucccuagug 10140
   cuguggcucc caggagcacu ggcaggaggc uggugagcag gaggaggcag ggagcacuga 10200
   ucucccuugc aaccuggggc uguucccucc ugccuggugc uuugggcagu ggcugaguuc 10260
   cuuuacuucu ggccccacug cagucaagca gacuuuuccu cuuggcuaca gcucuugcua 10320
   aguuccaaca ccgccacucu cucccucugc cccuucagga cugugugugg uaacagcuuc 10380
   cuacuguagc uaguccgugg guacugucua uaccugugua aauagcaccu uuauucaguu 10440
   cucgucaauu ucuccuuuga augugccauc uguuccugcu gggaucuaaa cugggcucag 10500
   aggucaccuc cagagggaag ccuuccagaa uaaccucuuu aaaauaauaa uccucucacu 10560
   cauuuaugau acuuuauccu ggcgguacuu accaccaucu gacauauaau auacucauuu 10620
   auauuugcug auugugucuc ccucuacuag acuguaagcu caaaggacau caucuuucuu 10680
   uuguuuccug augugucucc aguuccuauu acagagucua guuuacauaa auauguacuu 10740
   uaauaaaugu uuguugagug gauggauucu aaaauguaga aaauuacauu ggggauuuca 10800
   uuucuuucua acauuuuaaa ugguaagggc agugaacuaa uagacacaga aacuauucug 10860
   auccaugcag agcagaauuu aaggagagga ugggauauaa aaucauugaa aaccugacug 10920
   ucccuagguu aggcuuguag uaaaaguaac ugguacaucu cugacuugag caagaacuug 10980
   aagauauuua aacauucuac ucuccugaca uaucugagug ccuacaguag aauuagcaag 11040
   ugaugaauua aaugguuuca cuggaggccu uuuaaaagua cuccaucuca guauuucuca 11100
   cccugaguau cuaaauggua auagguuaug gcaccaacaa uaccacaaaa aagauaaauu 11160
   augcagagaa aacacuaagg auaauauggc caucauuaag cuagacucuc ucugaacaga 11220
   uaauagauuu ucaaguuuuu uuuucuaauu aucuagauua ucuuuuuaaa uauaucacua 11280
   uucaaguaau uuuauuuuau ccuaaauauc accacauuua ugaucagaaa uuaguuaaua 11340
   aacucuugau cuccuccuga auuuguuuau ggcguuuuga guuuacaugu uaaaagguag 11400
   ggcuaaucuc aagacuucaa ucacguagau cuucauuuuc agaugagccu uagaacagcg 11460
   guccccagcc uuuucggcac cagagacuag uuccauggaa gaccauuuuu ucacacaugc 11520
   agggaggggc agaggauggu uucaggauga aacuuuucca ucucagauca ucaggaauua 11580
   gagucucaua aagggcgugc aaccuagguc cguugcaugc gcaguucaca auagggcuug 11640
   cacuccugua agaaucgaaa gccagccggg cacggugacu cacaccugua aucccaguac 11700
   uuugggaggc ugaggugggu ggaucaccug aggucgagag uucaagagca gccugaccaa 11760
   cauggagaaa ccccgucugu acuaaaaaua caaaauuagc ugggcgucau gccuguaauc 11820
   ccagcuacuu gggaugcuga ggcaggagaa ucgcuugaac ccgggaggcg gagguugcag 11880
   ugagcagauu gugcugcgau ccagccuggg caacaagagc aaaacucugu cuuaaaaaaa 11940
   acaaaaaaac aaaaagaauc aaaugccccu gcugaucuga cagguggugg agcucaggug 12000
   guaaugcucg cucaccagcu gcucaccucc ugcugugugg cccaguuccu aacuggccac 12060
   agaccgguag uggucugugg cccaggggcu aggggaccuc ugccuuacag gauaccaaag 12120
   uugagcucug uuaauguucu gcucuucauu ugaaaugaga gaaugaaguc ugauaggccc 12180
   aagaacuuuu ucuuucauuc uaaagucagg acuaucagaa aucucccucc gguuaggaau 12240
   gguccaccuc cucccaucuc acuuuuuaag ucagccuggg uuauagcacc ugguuuuuuu 12300
   uguuugugua uuguuuguau cuuacaagug uuaguccugg uuuuuaguug ggaauagcug 12360
   uugaaugauc auaugucugu ggaagaaaag aauaaaugga cucuggagac agaaauguuc 12420
   aaugcacagu gccauaugcc ugcagucauu uacaaaugca ccuauucuca cacuuggcug 12480
   cauguugaaa ucaccugagg gaguuucaga acaacacuga uaccuggauc cuaucccuag 12540
   agaccaugau uuaauugaua ugggguacau cacaagcauu ggagguuuuc aaagcucuug 12600
   gguauucuaa uauggcagag aguuggugaa cugcugauuu aggccaucca aacaucacug 12660
   gcauacauuu gaugcagguu ucacugauuc uagucacaag uagcuucugc ugcuuuaacu 12720
   cuagagacac uacaguuccu cuugcauucu cccacuuugg uucaauagga aaaucucugc 12780
   cgcuuaguga uuuuccaaga aacaugagcu ucugccuuuc aaugaggaag auacucagaa 12840
   gucauguucg agcacuccgg uaagucacac agcaaggaau cuuaauccca gguagggaug 12900
   uuugggggug uaucagaaga auggguaauc acuucugagg agaaugaucu cucagccugu 12960
   gaaucuuuaa ccacugaagc ccagagagga guguuuucau uaacuugugg ccaggcauug 13020
   guaagaucug gccacauacc aguuuccuuc cuaguggauu auaccauucc uugucucuga 13080
   gagguuguga auguuccuuc uuuccccccg ccccacuuuu uucugcuugu uugucugcuu 13140
   ccuugacuau uuaugaugug gagagaccaa ggcccagaua uuucuacagg gacacagaaa 13200
   aagcaaacuc uuaggggaac cgcaguacug gcagugccaa ggauaauaca gcucuacuua 13260
   agcuagaaau cucucugcac agauaauaga uuuuugaaga uuuuuuuugg uucuucuaau 13320
   ugucuagauu aucucuuuaa auauaucacu auucaaguaa uuucaauuua uccaaaaugu 13380
   uuucaccaaa uuuauuauca ggauuuaguu aauaaacucu ugaucgaagg caaaagacca 13440
   auuuugccau uuuuuuguaa aguagaggga aauaugcaag aguggcagag ugggccguga 13500
   aagagaagug cagaagguua uuggucuuag accucauuuc cuuguuagau uuuuuucuaa 13560
   accugaccaa auggucuacu gaaguacuca caugguuuaa aaaaucacuu uacaaaaauu 13620
   aaaucaaagg ggaaaaagcu cgguggcuuc uggaaggcau uuguacuagc auuugaggug 13680
   guguuuauag cucauaccug uaaucccagc acuuugggag gcugaggucg gcagaucacu 13740
   ugaggucagg aguucgagac cagccuggcc aacacgguga aaucccauuu cuacuaaaaa 13800
   auacaaaaau cagccaggcg ugguggcaca caucugugau cccagcuacu caggaggcug 13860
   aggcgggaga aucguuugaa cccaggaggc agagguugca gugagccgag auugcgccac 13920
   ugcacuccag ccuaggugac agagcgaaac uccaucucaa aaauaaauaa auaaauaaau 13980
   aaauaaauaa auaaauaaau aaauaaagug guguuuacaa agggaucaua acaaucaguu 14040
   caucagaauu uggagggcaa aauugauucu uauugacaau uuccuggaug auagugcaau 14100
   augaauuagu uacuauaaaa gcacuuaucu ccuuuaauuu aauuugcugu cucagauugu 14160
   ccaucagggg auuuuugagg aaaaguguuc aaggauauuc ugaacuuuug guucuguuau 14220
   gaugucagac acaggagacc cagucaggug ucuggacucu ggaagagcuc ucuaugcuga 14280
   gagagagaac auggaagcua cuggcggagc aaagaaucca agaaauuuau uguuuuuuaa 14340
   cacuggcuuu uccuccccac ucuacuggaa acaacacaua uguauuuaaa agaguuucac 14400
   auauacuaau gcacagccaa gcuucuugag uguaccaaaa gauacugucu aguuuccaua 14460
   aaagggcugu auuaugccua gauaauacca agauugugga gugacaaccc ccaugaguag 14520
   cggaguuaau auuccaauac ucuacguguu uacugaggua cuuguuagcu cccugucuuu 14580
   agggauacac cuuaacaaag cuuagaagcg cagagggcca auauuuauac uuguuagaca 14640
   ugccuugaug guuuggagcc auuuccaaca acagcuguac aaaugguuga uguuauggug 14700
   cuuucucaga cuuaaauccc auuuggcaau uccuauguau gugcaaacac ugaaaauauu 14760
   uucauuuuug acauguaggg gaauucaauu ugggauuuaa auguuaaccc aaaagggauc 14820
   auccauucuc cugcuugccc uuccacauau guuccucccu cguucucccu cucagugaca 14880
   gugcugacug ccaugacuuc cccucaagaa auucccagag ccugcucuuu ugugcuguca 14940
   uaaacauaag gagugaaaca uuggcuggaa auguaaacuu ggaucgaguu uccaaaacac 15000
   aacagaacaa uugguggucu ugucaagucc aguccuuaaa uuggacaaaa ccauagccuu 15060
   gacuauucug uuguuuauaa gguucauccc auauauaaac gauuuuuuua aaauuauggg 15120
   agauacacau uuauaagugc agugaaucuu uguuuagaaa aagcaauuug ucugucuccu 15180
   gaauugguaa gcuuuuucuc uuuccucuuu ccagcccaaa guaaaacaaa gcacuguuuc 15240
   accuuguaau ugcgggugag ccacaaugaa cuugcucaau guaaagaaau cuucauggca 15300
   cuuuaaauca uuuuggggag aauauaguug uuagauaaga gguguugccu ugugagccac 15360
   gauucuugga aaaugucuga gacuuaggca cacacaugug cacaugcaaa cacuugggca 15420
   aacacauaug cagcuuccaa aguuuuguca gauuuuauua gaagaggauu uucuccuugg 15480
   cuccguguac acugcuugua cccugcuagu uaagggaagu ggaagauccu ugguuuauag 15540
   ggugaaaagc ccaaguggca ggauaaccau ccauaaacca gccucaggaa ggggccaagu 15600
   agggauaacu gaaauuagcc acuaacugau gauaaugggg auuaucuggg gguaacuuug 15660
   gggaugcacu aagcaacaua cauugggguu gcuuuuuaag gaaaaaacag uguacuucug 15720
   agaaaggaaa ugcuuuuugg agucaggaca aauuuaggcc ucucuaaucu aaaaaguccc 15780
   uuugccugau uuuacggcca uagugagaaa agagcaaggu aaauauacaa ggccacccac 15840
   agggacacag agccuuuccc cugccugugu ucucacugua aaaauaugca uggccacuca 15900
   cacacaugca uacacacaga cacacacaca ugcauucaug cacacacaca gguaugcaca 15960
   uauacauuug caugcacaca cacacacaca caccucaaaa uuauuucuug auacugcuuu 16020
   guagaaacag aaacaggaac acaccauauc caggcuacau uugguagaga acauuccagc 16080
   uuugugccuc aguugauuuc cuucucucuu ccucuugcuc ucuuuuggua auuuauucac 16140
   auauauauca aacaggagaa uaucauuuuu aaaaauguga cuaggcuggg cacaguggcu 16200
   caugccuaua aucccagcac uuugagaggc ugagucaggc agaucacuug agcugaggcg 16260
   uucaagacca gccugggcua cacggugaaa ccucgucucu acaaaaaaaa aaauaaaaua 16320
   aauuaaaaaa ucccugguua ggugguacac accuguauuc ccagcuacuc aggaggcuga 16380
   gguaggagga uuccuuaaac cugggaaauu gaggcugugg ugagccguga ucacaccacu 16440
   gccugccugg auggaagagu gagaccuguc ucaaaaaaau aaaaaaaaau gugacuaaca 16500
   auaauaugga uauuuaacuu gcccagugcg acuuauacau auggccacag auacagaacc 16560
   aggcauuaga gaauuaucug uauguuuuga acauuuuaca ggaaaauguc cuuggaguuu 16620
   caacauuucu uuggucuucc acauuucauu uuguccugau uaaagaggaa gccaguugcu 16680
   guuuguggua ggugcucccu gacgugggug ccaguggaau cucaucuucu ggaauuuaua 16740
   uacuugugua aucacauccc cuggagugug uggauuggau gcaguggugu gucucuaaua 16800
   aaugaaauuu ggaaaaagug guaagauguc acuuccaagg uuagauuaug aaagaugacu 16860
   acuuccuucu ugguuucauc cucucucucg cccucucacu ugcucacucu gaugaagcaa 16920
   gaugccgugc uggaaccugc ccuguggaga ggcauacaug gugaaaaacc agggaaggcc 16980
   ucugagcaac agcucuugag aaacuaaagc ccuaaaccca ucaacuugca aagaacugca 17040
   uccugccacc aacugcguga gugaggucag guggaucuuu cccuggucca gccuugagau 17100
   gacugcaguc ccagacaaca ccuugacugc ugccuuguga ugcagcuggc gccaagcagu 17160
   gccaauuccu ggcccucaaa aaccaugaga uaauagugcu guuguuguaa gucacccaag 17220
   uuugggguaa uuuguuaucu agcaguagau agccaauaca ucaaacuaga aacauucucc 17280
   aucauugauu ggaagacuuu ggguuuuuaa aaaggguaaa cacacauaua cacagcuuau 17340
   uauauuuccu uccacauuua uuuuggaggg gaaauguuuu acuucuuauc ccaccgcuuu 17400
   gcccuuucua aaagaaugug uuaggauuac ugugauucaa augguaaaaa uuuucccagu 17460
   gaaauaaaga cuuuauacca acuggauuuc uugcuuagaa agcuguguuu ucuccacuaa 17520
   uaucuugaau uugauauuuu caacuaucuc uauugaauuc acuuguaaau gaauugcguu 17580
   uuucccucua uuaugaacug aauguuugua ucucuucaaa auucauaugu ugaggcugua 17640
   auucccagug uggcuauauu uaaagaugag gccucuaagg aaguaauucu cguuaaauuu 17700
   ggccauaaga uucccugauc ccaucuuggc uaacacggug aaaccccauc ucuacuaaaa 17760
   auacaaaaaa uuagccgggc gugguagcag guaccuguag ucccagcuac ucgggaggcu 17820
   gaggcaggag aaugguguga acccgggagg uggagcuggc auugagccga gaucgcacca 17880
   cugcacucca gccugggaga cagagcgaga cuccaucuca aaaaaaaaga agaaagaaag 17940
   aaagaaagaa agaagauucc cugaucccau aguauuagug uccuccuaag aagaggaaac 18000
   aggggagcuc acucuuucuc uccuucucuc ccaguggcca ugugagcagg cagggagaug 18060
   guggcugccu agaagccaag agaaguggcc ucaagaugaa aucuaccuug cugguacuuu 18120
   gaucuuggac uugcagccuc cagaacugug agguaaauuu cuauuaagug ugugggauuu 18180
   uguuacagca gccuaaguug acuucuguac ccucucaguc ugguuuuguu ugaucacuuc 18240
   agcaacaaaa uaauggagaa gacaaaugca guuauuugga ggaaagauua aaacuuuccu 18300
   uauacaaaau uaaucccaua aguauuuauu aggcaguuau ccauguuuga aauuauacua 18360
   gacacuuuag gaaauaguuu gguauuuaca gauuaucucu acuuggaaug gucugguaaa 18420
   aauagguggg uuuuagaauu uaauuaguuu gaaugacuca gccacauauu cuuguccacu     18480
   accacccacu gaaaaaauug ucuccagaug uucaacaagg cugagugauc aauaaaacug 18540
   gcaauuugac cagcagugau cugggacuua agggaaugua ccuaugauca cauuuuggac 18600
   uuucugcccc aauauuucca acaaaaauga cgcauauuuc accaccugaa gaaugguaag 18660
   ugaauucgug gugcccaauu ugguguuaua aaacccauuc uuacaacacg aaaaaauugu 18720
   uaugcauaaa acuugugaug agaaaccaac augugggaaa ucuaguuugg ugaaucacag 18780
   uuuuucuaua cauggguuua gaaaccaccg aguaucucau agucuauuuu ucaauaagaa 18840
   uggggcaaaa ugguaguauu uuaucuucca gaaaaaacaa aaauuaaaga aaaacaaauc 18900
   aaaccuuaaa acuucucugu caaagauaaa uguacauucu ucugaauucc cugccccauc 18960
   acaaggcuuu uaggcagcau cuauaccaag auagcaauua ugccaacccu gaacauguag 19020
   guuaaacaua auccuuauau uuucuaccuu aacuuaaaug cuugaagggu acacaauaau 19080
   auaugaugau aaauuugaaa aggaugauuu uaauuauaau gaagaauuuu uaacaguugg 19140
   gaguuggcug ccauuuugaa agaugagaua auuauugaua augugucuga ggcuaagaca 19200
   gagcaagcug ggucccauuu uauugauuuu uuuuucuaaa augauuaaag ugauauauca 19260
   gaaaauacau uuaauuuaua aucccugcaa ugaaaaaaua cucuuauauu auuuucaaca 19320
   ugaagagcuc ugacauucga gcuguauuag gccuuaaaag aacuaucuca ucgggugauc 19380
   ccacacucau augauugcug cucugcaaac ucaaucauua gaccucaguu uaaucuuagg 19440
   guucuguuca aggagaauga acugugccca uaugguaugg uuuucuucca aaggagauug 19500
   agaagagauu gugguuucuu gcauuaaagg ccauuuggcu ucauaagacu uauuguugau 19560
   acucccuucc ucagagcaca gauguauuuc uguuucugac uagugacuuc ugucaggucu 19620
   gaaauguuag uuuuauagaa auccuggcug uguaagaaga auggggaagg uggauugauu 19680
   ccuagucauu gugaacagac auuccucuuc ucaugguacc acacuuugga caggucagcu 19740
   acccagagaa agcucuacaa ccccaacacc aaccuggacu aggucccaca agaaagccug 19800
   acaugucagg ugcugggaca cucaucucug gcuagucacu gagcccagca gcuccagaug 19860
   accuggcgcc accacuuccu gacagcuucu ucuaggaucu uacauucagu auauccggug 19920
   ugacuaguca gaagucacag aagccuacuc agaaguaaag cuagugauaa gaguuuaaau 19980
   cuguguuccu gcccaaaucu cauauuaaaa uguaaucccc auuguuggag guggggccug 20040
   guuggaggug auuggaucau gggggcagau uucucaugaa ugguuuagca ccauuccccu 20100
   uggugcuguc gucacgauag ugaaugaauu cucauuugac cugguccuuu aaaaguuugc 20160
   agcaccucuc ccaacacucc uccucuugcu ccagccaugu gaagugccug uccccucuuu 20220
   gccuuccauc augauuguaa guuuccugag gccuccccag aaggugagca gaugccagca 20280
   uuaugcuucc uauauagccu gaagaaccau gagccaauua gaccucuuuu cuuuauaaau 20340
   uacccaaucu cagguauuuc uuuauagcaa ugugagaaga gacuaguaca gcaagugagu 20400
   cuuacaaaug aauauguuug cccuuuaaag auaaucaacu uuuaagaacu gaauacuauu 20460
   aacuguguua gcucuuggcu gcucuuaggu ugggaauguc auacaaggua gggaaaguau 20520
   guuuuggugg guaagagaaa agggaacacu uucauacuau uggugggaau guaaacuaau 20580
   auaaccacua uggaaaaaaa guguggagau uccuuaaaga acuaaaagua gaucuaccau 20640
   uugauccagc aaucccacua cuagguaucu acucagugga aaagaaguca ugauaugaaa 20700
   aagauacuug cacaugcaua uuuauagcag cauaaucugc aauugcaaaa auauggaacc 20760
   agcccaaaug cccaucaauc aaggaaagga caaagaaaau gugguauaaa uauaccacaa 20820
   aauacuauuc agccauaaaa gaaacaaaau aauggcacuc ugcagcaacg uggaugguac 20880
   uggagccuau uauucuaagu gaaguaacuc aggaauggaa agccaaacau gguguauucu 20940
   cacucauaug ugggagcuaa gcuaugagga ugcaaaggca uaagaaugau acaaugaacu 21000
   uuggagacuu gagggaaagg uagggggugg ugagggauaa aagacuacac auuggguacu 21060
   gcuuggguga ugggugcacc aaaaucucag aaaucaccac uaaagaacuu auucauguaa 21120
   cccaucacca ccuguuccca aaaaacaaaa uaaaauaagu aaaauauuuu aaaaauuuaa 21180
   aaagagugaa auuucuggaa ucagauagcu ugggaaaguc cauuaaucuu ucugugucuc 21240
   aguuuucuua ucucuaaaac caaaauaaaa aacaguacua gaaucauaga auaauaaagc 21300
   uuaaaugauc auacuacaga gcuaggcuua aaugagcaua cuacagagca uacuacacuu 21360
   acuacaguaa guguucaaua uauuaguuuu uugcuauuga auaaaaauua caacuuuccu 21420
   uggcuuacag gaauugaauu uauuuuacua aguauauauu acuuuauaua uauauauaua 21480
   uaauauagua uauauauuau auauacuaag uagagugcuu cucaaacucu cugcauuaaa 21540
   aggccagguu uuuuguuugc aauccauugu agccugauag uuuugcaaau uauaaucaaa 21600
   acgaauuagu agaaaaauga aauaaaauga aacaaagaua uacaaaaccu aagcuuaaac 21660
   agcuguaaga agaguuucua aaugcuuccu cucaauuucu guacuucugu cuuugucacu 21720
   ggguugcaua guucccaggu gggcaccugu ucacagagua gcacuggaag aaaaaaagca 21780
   gcuguguuuc ugcuggacug uggugcacgu gcagcuucuc cuguguuggg gacuccccuc 21840
   cuccugaggu ggagcucagc ucugagggcu agugcuaaaa acgucuggug cuugcaaucu 21900
   cacuguucuu auagcuaugg ccagcucagg gcgguggacc uggacccauc agacacacuc 21960
   auacuuagaa ugcaagcuaa acaugagaag aagcaggaag aacacaggag caaguccagu 22020
   aaaugguaga ggcagcacca gcagcacccu cuacuuccag agguaauagu agccgugauu 22080
   uuagaagcau cauggaaggc agcugugaau gguccuucac cagaccagca uccugacaug 22140
   guuguggucu gcuguccuca agguggaguc ucucugguuu ucagauucuu cuggagggau 22200
   agugauugac ccaacacucu uuuaacaagu uuuuuuuuuc ugcuuaaauu agucgauaga 22260
   uuuguauugc uugaaaacuc agaaccagga uuaauucaua uggauuuaga aaagaaaacc 22320
   augaagauag gugauccugg cauuuggcau cucaucacag cuggggaucc uauagauguu 22380
   uagaaugcuu ugguauugau cucuguauua ucaucucaug uacucacucu agcucuacuc 22440
   cuuuuugguc uuguucacag uacaugcuaa caauacccuu caguuuagau cugaaaucga 22500
   gcugaagaug gaugaauuug auacuaccuu augacagguu uaagugucau gggcuuguag 22560
   gcaagccauc ugucacaagg augaaaggaa cugaucuucc cagaguuucc uuuuuuagcc 22620
   ugggggaauu uauaccaaga auucacucuu cugugauacu aagcaaagau uuuguucccu 22680
   aaagcugauu ucucucauag gaaacugcuc augugaaagc cuccugccau uuaaugggca 22740
   gcaaagguaa uguuuugcuu gguggcuaaa ggccucuuua accuccagag aggcuaagua 22800
   uggugcagac uccccuaggg acacguguuc uucauuccug guggagggca agcagaggcu 22860
   auccuggcgu cugguaggga ugcuuuagaa uuagaaagaa ggagauuaaa ccugaagauu 22920
   ucuuuucccu ucacaacuga gaaucagguu acugucuuua aggauuucuu cuggccguua 22980
   uaaaugcugg gcuuccaguu uaaucggcua gcauacuagc auauuuugca ugacuucugc 23040
   aaggagguaa aaggauaaaa aaagaugaac uucuagaagu gaaaauuaau gcacuagcuu 23100
   ucaucaucaa gacagaaaua auucuugaga aagaaaacaa gugaaauugu uacccagaaa 23160
   acuauccaag ucugguccca uuccuagagc aacugaugca acugaucuag auggaccuaa 23220
   acaucaagau uuuuaaaaag cuccccagcu uucugauacu aaugcaaaau gacagaacca 23280
   uugcuuucug guuucuggug gagacucugg guucugcuuu cccuucuuag gggagaugua 23340
   gccagaguuc aggucagcca agggcagcuu gaaaagugag aaggaggcua ggaaagaaua 23400
   uaugaagacc cuaagugcau uuaaucuccu ccaaaaugua guguauggac agaaaacuca 23460
   gccuggcugc gucuccauuu ucucuagcuu ccaucacauu cuuuaauuga cuguaugagc 23520
   ugugcuuuag uauggaugaa aggcagugug guuuaauaaa aagggucacc auuagaaagg 23580
   guuccagauu uucuguagga aauacaugug uccaaacauu agagaacucc ucaaggaguu 23640
   ggagauccag aaacuagauc auaauuugag aauuaauuau aaauaguuug aagauaaguu 23700
   ccaggcucuu ucuccugggc uaugaagauu aaaucgagag cccacacugg uccaucuuca 23760
   ugcagaguug cuggagaaca uggagagaga cuugggggga aagaagccau caguggguau 23820
   uugguuaaca gcucagaaag gcauuauuug uagggcauca cuuugaguuu auuguguguu 23880
   ucaguaugua uaaguaucag gaguuuauug acagaaauau ugacaucucc cuaaacagcu 23940
   uccauuauag cuacuguaga aguaacuuca guccucuaua aucccugacu ccccgcaacu 24000
   ucugcagugu ucuuuguuca aaaagaugug gcugaccuug aggguggcgu gcucugugaa 24060
   gugccugcca gaacaacuga aggauuuaca uagccuuuuc acacucacgu uguccucaua 24120
   gugcagcacu uuggggagcu cauuggucug gguuuugguc ugcauuugua gcuaagcuac 24180
   acucccagag ggcugaaacc cagcaccucg ccccuuuagg acuaggccug aaagucuggu 24240
   aaggcuagaa ggucaugcga gaaacuguau gaagagacac agaguaagga ugggcaugag 24300
   agaugaacac gaacaagugu ggaagaucag aauuuuuuca gaggguaacu gguuauaagu 24360
   gcuauguuuc ucuuuagcgg gaauauuaaa aauauaguaa uuucuuuagc aaacagguga 24420
   ucagacccgc aaaaauuuag aaagcauuau gaguuauuuu aaaaagagaa acucaguacc 24480
   acagauaggc uuggcaaugc agaaauaaca guagacuaaa aauuaauauu acuaauuuuc 24540
   ucuuugcuuu auuaauuucc ucuauuacag aaucugucuu uugcuaccua cagagagaug 24600
   gcuccacaua aucauuuuac auuauuuuau uauucauugu uauuuaccuu gccaagugca 24660
   uagaucuaca cagcaaaaaa uuagaaugac aaagauaggc uguuuguauc aucccaacug 24720
   caucucuuca gucucuaugg aauugaaaua cgauuaaaug aaucccucau gaaaauuaac 24780
   aggcaacaag aaggaaauua uuauuucugu ugccauuuca cauuagucua acuuuucuuu 24840
   gaauauugca uuauaaaguc aauagaaacu augcaaagug acugagcaag agcuuuccau 24900
   ucaaauucuc aaauaggcuc auucauuuuc auaacccaaa uauaaucaug gaauaaguag 24960
   gaggacuucu ggagacuuga aacaauuuuu acauguuacu gaagagaaaa cugcugcaaa 25020
   ugucaagagu ugcagauaug uugugaagcc agcgacagcu uuucaggaaa aaugggaugc 25080
   ugugugcugg ggguuugauu ugucuuuggu aguaacuagc uaugugauca gauacaagcc 25140
   agcugaccuu ucugaauuug gaauaaacgc ugauaucuag aaaaagcaaa uucacaguga 25200
   uguuaaauau uuaaaaucuc ugcucuuuga cauacaaaac uuuagaggaa ugaacccagg 25260
   uuuagaaugc cuuuucaguu cuucauucau caauucacaa aauacuuagu aaccaucucc 25320
   cacuaguagg gcucagugcc ucacacugau aaauuuguuu cauucugcaa acuaucaggc 25380
   aucauguagu gaaaauacuu ucuaaaaggc auuaccugac uuuaguuuuc uaauugcuua 25440
   uuuuaaagaa auugaaauaa gagcucccau aaguggaacu uagaugacaa auauaccuaa 25500
   cuauaacaca gaaagguuaa aaaauagaua cagguuaaua guuucuuacu auaagaagua 25560
   aagucaguuu cuuauuuuga uguccaugug caguaacugg uagaguuucu aaguuucacg 25620
   uuucuaauac ucaaaugaag agaaaccuuu gaacauggag cuuaaaagca augcccuuac 25680
   ugagugcaaa aucaacacau uagcuauaaa uuaaucaacu aaaaugucau auuuucaaac 25740
   uaacuuuaaa ugcaacauaa uaaguaucua guuuaaaaua augggaacac cuuuuuuuuu 25800
   uaauuauuau acuuuaaguu cugggacaca uguguagaac guucagguuu cuuacauaga 25860
   uauacaugug ccaugguggu uugcugcacc caucaacccg ucaucuacau uagguauuuc 25920
   uccuaaugcu aucccucccc uaacccccca cccucugaca ggcacccaug ugcgauguuc 25980
   ccuucccugu auccaugugu ucucauuguu caacucccac uuaugaguga gaacaugcgg 26040
   uauuggguuu ucuguucugu uaguuugcug agaaugaugg uuuccagcuu cauccauguc 26100
   ccugcaaagg acaugaacuc auucuguuuu auggcugcau auuauuccau gguguauauu 26160
   ugccacauuu ucuuuaucca gucuaucacu gaugggcauc uggguugguu ccaagucuuu 26220
   gauauuguga auagugcuuu aauaaacaua cgugugcaug ugucuuuaua guagaaugau 26280
   uuauaauccu uuggguauau acccaguaau gggauugcug ggucaaaugg uauuucuagu 26340
   ucuagauccu uaagaaauug ccacacuguc uuccacaaug guugaacuaa uuuacacucc 26400
   caccgucagu guaaaagcuu ucuuauuucu ccacauccuc uccaggaucu guuguuuccu 26460
   gacuuuuuaa ugaucaccau uuuaacuggu gugagauggu auuucauugu gguuuugauu 26520
   ugcauuucuc uaauggccag cgacgaugag cuuuuuuuca uguuugcugg ccguauaaau 26580
   gucuucuuuu gaaaaguguc uguucauaua cucugcccaa uuuuugaugg gguuuuuugu 26640
   uuuuuucuug uaaauuugcu uaaguuccuu guagauucug gguauuagcu cuuugucaga 26700
   uggauggauu gcaaaaauuu ucucccauuc uguagguugc cuguucacuc ugaugauagu 26760
   uucuuuuccu gugcagaagc uauuuaguuu aauuagaucc uauuugucaa uuuuggcuuu 26820
   uguugccauu gcuuuuggug uuuuagucau gaagucuuug cacaugcaau gguauugccu 26880
   agguuuucuu cuaggguuuu uaugguuuua gaucuuaugu uuacaucuuu aaucuaucug 26940
   gaguuaauuu uugcguaaga uguaaggaag ggguccagug ucaacuuucu gccuauggcu 27000
   agccaauuuu ccuagcacca uuuauuaaau agggaauccu uuccccacug cuuguuuuau 27060
   cagguuuguc aaagaucaga uguguguaga ugugugacgu uauuucugag gccucuguuc 27120
   uguuccauug gucuauauau cuguuuuggu accaguacua ugcuguuuug guuauuuuag 27180
   ccuuguagua uaguuugaag ucagguagcg ugaugccucc agcuuuguuc uuuuugcuua 27240
   ggauuuucuu ggcuauaugg gcucuuuuuu cauuccauau gaaauuuaaa guagguuuuu 27300
   cuaauucugu gaagaaaguc aaugguagcu ugauggggau agcauugaau cuauaaauua 27360
   cuuugggcag uauggccauu uucacaauau uuauuuuucc uauccaugag cauggaaugu 27420
   ucuuccauuu guuugugucc ucucugauuu ccuugagcag ugguuuguag uucuccuuga 27480
   agagguccuu cacaucccuu guaaguugua uuccuaggua uuuuauucuc uuuguagcaa 27540
   uugugaaugg gaguuugcuc augauuuggc uguuugucua uuauuggugu auaggaaugg 27600
   uugugauuuu ugcacauuga uuuuguaucc ugagacuuag cugaaguugc uuaucagcuu 27660
   aaggaguuuc ugggcugaga ugaugggguu cucuaaauau aaaaucaugu caucugcaaa 27720
   cagagauaaa uugacuucau cucuuccuau uugaauaugc uuuauuucuu ucucuugccu 27780
   gacugcccug accagaacuu ccaauacuau guugaauagg aguggugaga gagggcaucc 27840
   uuguccugug cugguuuuca aagggaaugc uuccagcuuu uguccauuca guauauuggc 27900
   ugugggucug ccauauacag cucuuauuau uuugagacau guuucaucaa uaccuaguuu 27960
   auuguguguu uuuagcauga agggcuguug aauuuuguca aaggcuuuuu cugcaucuuu 28020
   ugagauaauc augugguuuu ugucguuggu ucuguuuaug ugauggauua cguuuauuga 28080
   uuugcauaug uugaaccagc uuugcauccc agggaugaag cugacuugau ugugguggau 28140
   aagcuuuuua augugcuacu ggauuugguu ugccaguauu uuauugggua uuuucgcauu 28200
   gaucuucauc agggauauug gccugaaauu uucuuuuuuu gugugugucu cugccagguu 28260
   uuguuauuag gaugauuuug gccucauaaa aaaugaguua gggaggauuc ccucuuuuuc 28320
   uaucguuugg aauaguuuca gaaggaaugg uaccagcucc ucuuuguacc ucugguagaa 28380
   uucagcugug aauacaccug guccugggcc ucuuuugguu gguaggcuau uaauugcugc 28440
   cucaauuuca gaacuuguua uuggucuagu cagggauuug acuucuuccu gguuuagucu 28500
   ugggagggcg uaugugucca ggaauuuaug cacuucuucu agauuuucua guauauuugu 28560
   guagaggugu uuaugguauu cucugagagu acuuuguauu ucugcgggau cagugaugau 28620
   cuccccuuua ucauucuuuu auuguguaua uuugauucuu cucuauuuuc uucuuuauua 28680
   gucuggcuag uggucuaucu auuuugcuau ucuuuucaaa aaaccaccuc cuggauucau 28740
   ugauuuuuuu gaaggguuuu uuugugucuc uaucucccuc aguucugcuc uaaucuuugu 28800
   uauuucuugu cuucugcugg cuuuugaauu uguuugcucu ugcuucucua guucuuuuaa 28860
   uugugauguu agggugucaa uuuuagaucu uucccgcuuu cuccuggggg cauuuagugc 28920
   uauaaauuuc ccucuaaaca cugcuuuagc ugugucccac agauucuggu acauuguguc 28980
   uuuguucuca uugguuucaa acaacuuauu cauuuuugcc uuaagucauu caggagcagg 29040
   uuguucaguu uccauguagu ugugcaguuu ugagugaguu ucuuaauccu gaguucuaau 29100
   uugauugcac uguggacuga cagacucuuu cuuaugauuu ccauucuuuu gcauuugccg 29160
   aggaguguuu uacuuccaau uaugugguug auuuuagaau aagugcuaug uggugcugag 29220
   aagaauguau auucuguuga uuuggggugg agaguucucu agaugucuau uagguccacu 29280
   ugguccagag cugaguucaa guccugaaua uccuucuuaa uuuucugucu uguugauaug 29340
   ucuaauauug auaguggggu guuaaggucg cucacuauua uuguguggga aucuacaucu 29400
   cuauguaguc ucuaugaacu ugcuuuauga aucugggugc uccuguauug ggugcaaaua 29460
   uauuuaggau aguuaacccu ucuuguugca gugaucccuu uaccauuaug uaauguccuu 29520
   cugucuuuuu ugaucuuugu ugguuuaaag ucuguuuuau cagagacuag gauugcaacc 29580
   ccuguuuuuu uucuugcuuu acauuugcuu gguaaaucuu ccuccaucug cuuauuuuga 29640
   gccuaugugu gucuuugcac augagauggg ucuccugaau acagcacagc gaugggccua 29700
   gacucucuau ccaauuugcc agucugugcc uuuuaauugg ggaauuuagu ccauuuacau 29760
   uuaagguuaa uauuguuauu guuauuguua uuguuaugug ugaacuugau ccugucauua 29820
   ugaugcuagc uggcuaguug augcagcuuc uucauagugu ugauggucuu uacaaguugg 29880
   uauguuuuug caauggcugg uaccaguuuu uccuuucugu auuuagugcu uccugcucuu 29940
   guaaggcagg ucugguggug acaaaauccc ucaguauuuc cuugucugga aaggauuuua 30000
   uuucuccuuu gcuuaugaag cuguuuggcu ggauaugaaa uucuggguug aaaauucuuu 30060
   uaagaauguu gaauauuguc ccccacuguc uuguggcuug uaggguuucu gcagagagau 30120
   ccgcucuuag ucugcuggac uucccuuugu ggguaacccg accuuucucu cuggcugccc 30180
   uuaacauuuu uuccuucauu ucaacuuugg ggaaucugau gauugugugu cuuggaguug 30240
   cuauucuuga ggauuaucuu ugugguguuc ucuguauuuc auaaauuuga auguuugccu 30300
   aucuugcuau gguggauaag uucuccugaa uaauauccug aaguguguuu uccaacuugg 30360
   uuccauucuc cccaucacuu ucagguacau caaucaaacg uugguuuuuu cacauagucc 30420
   cauauuuccu ggaggcuuuu uccuuccuuu ucauucuuuu uucucuaauc uugucuucau 30480
   gcuuuaauga ucuucaaucu cugauauccu uucuuccacu ugaucaauuc agcuauugau 30540
   acuuguguau gcuccacgaa guucucgugc uguguuuuuc agcuccauca ggucauuuaa 30600
   guucuucugu aaacuaguua uucuaguuag caauuccucu caccuuuuau caagguucuu 30660
   agcuuccuug cauuggguua gaacaugcuc cuuuagcuca gaggaguuug uuauuaccca 30720
   ccuucugaag ccuacuucug ucaauucguc aaacucauuc uccauccacu uuuguucccu 30780
   ugcuggcaag gaguugugau cuuuuggagg agaagaggca uucuaguuuu uggaauuuuc 30840
   agccuuuuug ugcugguuuu uccucaucuu cguggauuua ucuaccuuug gucuuuggug 30900
   uuggugaccu uuggauggag cuuuugcauu gucauccuuu uuguugaugu ugaugcuauu 30960
   gcuuuuuguu uguuaguuuu ccuucuaaca gucugcaggu cugcuggagu uugcuggggg 31020
   uccacuccag acccuguuug ccuggguguc accagcagag gcugcagaac agcagauauu     31080
   gcugccugcu ccuuccugug gaagcuucau cccagaggga caccugccag augccagcaa 31140
   gagcucuccu guaugaggug uuugucagcu ccugcuggga gaugucuccc caucaggagg 31200
   cacguggguc agggacccac uugaggaggc agucuguccc uuagcagagc uugaacgcug 31260
   ugcugggaga ucugcugcuc ucuucagagc uggcaggaag gaacguguaa gucugcugaa 31320
   gcugugccca cagccgcccc uuccccuggg ugcuugccug agugagaugg gaguuuuauc 31380
   uauaagcccc ugaguggggc ugcugccuuu cuuucagaga ugcccugcuc agagaggagg 31440
   aaucuagaga ggcauucugg cuauaguggc ucuguggcac uguggugggc uccacccagu 31500
   ccaaacuuag ugggaacacu uuaauauaac uucuuuaauu uacaagugau auaacuacau 31560
   aauaaaaaug ugauagaaaa acuaauuuau agucaauaua cccagcaaaa ccagcaugaa 31620
   cauguuugag uaaucccccc accaucuuuu ucuuacuaaa uuucccucua cccuaacuga 31680
   cacuccaaaa aacccuuaau cuuuuccuuc cuuccaggug uaucuaaccu cauccucucu 31740
   acugccacag gagggacuuu ucugauccgu gcauuuguuu cuccauucuc ccucuucaaa 31800
   ugcuccagca gugccuugga gucuuuggga uaaguccaca cuccacagca uggucuuuaa 31860
   gccucuugau uuugucugug ccaccucacc aaccucugcu cuucauacau ugccacaccc 31920
   ccaucuccau gcaaccccug aaugccugcu uaugauuuua aacucaaccc uggcucacca 31980
   acuccaaaac aucaucccca ucccaccccc auggaucauu uagguuggua ggucccuucu 32040
   uuuccccccu caccauaccu ugacaauagc aacuaacuua uuauuguuau uauugacaga 32100
   gggucucacu cugucaccca uccuggagua caguggugca aucgcggauc acuauauccu 32160
   uaagcuccug gguuuaagug auccuuccac cucagccucc ugcguagcug auacuacagg 32220
   agugugccac cacauccagc uaauuuuugu auuuuuugua aagacaggau uucaucaugu 32280
   ugcccaggcu ggucuggaac uccuggacuc aagugaucug cacaucucag ccucccaaag     32340
   uauugggauu acagguguga gccaccaugg ccagcuuaau uauuauuuug augauucccc     32400
   aaaacaguau ugaugaaaac cgugagauua cauagcaaca auauguauuc ccaaaacacc 32460
   aauuuuacuc aguggcguag uuuuaaacac uucaugacaa aauauaugcu caaaauaugu 32520
   uuugaagauu gacuacuaau cugggcacug gggauacagc ggcgaacaaa acauauaaaa 32580
   aucucuauuu agggaucccu aaauccugcu uagggaucug cuauuuagag aaggauuugg 32640
   auaagauacc aaaacaaauu aguaaauucu auauuauauu ggaaagcauu aagagggaag 32700
   augacguaau uuauugucuc agucuggaca auucuugcgu gaaagaagug ccccugauaa 32760
   uuacaccagg gccacaugug ucggccggaa guacggcuga uaacaacauu aaagcagugu 32820
   uuguuaacgg ggacuguccc auuuagccug gggagcauga ugugguaggc agcuucugac 32880
   aaggcucccc caaaugccug cuuccuggua uucacacccu uguguaauca ccucuaacua 32940
   ugggcuagac cuagugacuu ccuucuaaca aagaaaauau aggcaaagug augggauauc 33000
   acuuccaaga uuaggcuaca aagucuguga cucccgucuu acuagcacuc ucucuugccu 33060
   ucuaggcuug uccauuuuaa ugagaagcuu auguggcaug gaacugaguu cacccccaac 33120
   cagcagcugg ugaggaaugg agcucuucgu ucaaugacuc auaaugagcu uugagacaac 33180
   caaagccccc uagcuaaccu gugccuggau uccuggccca caaagacagu gagagaguaa 33240
   guacguauug uuugaagucc cuuaguuuug gggugauuug uuacacagcg auaguuaacu 33300
   cuuauauaca auuacucuag ugguaaguac uacggggaaa aaaaugaagu agggaagaga 33360
   acuagaaugu gaugagguug ggggugaggg augaguugaa aucuuaagua gcaugaccaa 33420
   ggaaggacuu uaaggaggag guuccauuga auggaaacuu gacagaggug agauuguguc 33480
   auguagacau cugcggggag auccuuccag gcugaggggc auggauuugu aaugaggugg 33540
   aggugaaaau ucauaugugc caaccaucuu cugagugcuu uaccuauacc uucacaugcu 33600
   guccacacag aagccuaagc gacagguucu ggauucuauc cauguacaga auguggccac 33660
   ucagauuuag uuccccaaau cacacugcag uagcauugau gcuggguacu aaacacaguu 33720
   cuguugaguc caaguccaug ccggagaagg uaucuuaguu uccuuuugcu gcuguaauga 33780
   auuaucaugg acuuaguggc uuaacacaug caaauuuaua auuuuacagu uuuguggguc 33840
   agaauucugg cauggaucuc accaggcuaa gaucaaggca ucaggagagc ugcauucuuu 33900
   ucuggagguu cuagaggcaa acucauuuau cuucucauuc agacuguuga cagaauucca 33960
   cuguaggacu uggcuuugca gguuguaggu uccuguugcc uugcuggcuu gccagccagg 34020
   ggcugcuccc ugauucuaga ggacacccac auuccuuggc ccaugguccc uuuccuccau 34080
   uuuuuuuuuu uuuuuuuuuu ugagacggag ucuugcucug ucgccacgcu ggaguacagu 34140
   ggcgcaaucu cagcucaccg caaccuccgc cucccggguu caagugauuc uccugccuca 34200
   gccuccuaag uagcugagac uacaggugca caccaccaca cccggcuaau uuuuguauuu 34260
   uuaccagaga cgggguuuca ccauguuggc caggaugguc uugaucucuu gaccuuguga 34320
   uccgccugcc ucacccuccc acagugcugg gauuacaggc augagccacu gcacccagcc 34380
   cuuuuccucc auuuuuaaag ccagcaacag uggguagagu cccucuaaca cuuugaaucu 34440
   uuccugccuc uuuguugugg uuguguuucu ucugccuguc ucuuccacuu uaagcaccca 34500
   ggugaucaga cugagcccac uuaaguaauc aagauuaauc ucccucuauu aaguucagua 34560
   accuuaauuc aaucugcaaa auccuuuuug ccauuuaaua gaacucauuc acagauucca 34620
   gagauuggca uacagacacu uugggggcca uuauuguacu ucccagaggg gcauuuuggg 34680
   aaaacaauau cagaauuacu gaagauacaa ggauguucug uuggaguuga aacacaagua 34740
   aaccagucac ugcugugaca ggucuaugug auagauguua uuuaugggac ugacaccaac 34800
   uuccuuagac ugaaggacug ucaagacagg cuuucuguag gacguguguu ugaguuaagu 34860
   cuuaaggaua gucaggagaa gguggagaug auagguaguc uucuagagag aggaagccug 34920
   gagcaaaauc acuaaguuga ugaacagcau gcccaguuua aagaacugua ugcuaagaac 34980
   ugaaagccac cugcuuuagg gaagggaaga cugugaguga uuccaagucu gagagaacag 35040
   gggacuuuag ugcauaguga guacugaaac uuuauccuaa aggauaugga uuugaauuua 35100
   caaggucuuc uaauuaaaga gauuugaaua ucaggauuua uauguagagu ggaaaaaaag 35160
   cgcugcucua ugccucaccu ccaaauuuau uuccuaccau aauguaucuu ucuuaaguuu 35220
   cuaacuuaac ucucaaguua gacagggaca aaaguugguu cauucuaccc cauuccauau 35280
   aagugauggg cauuaauaga cuggguuaaa aaaauguggu acauauacac cauggaagac 35340
   uauacagcca uaaaaaggaa ugaaauugug ucuuuugcag ggauauggau gaagcuggaa 35400
   gccaucaucc ucaguaaacu aacacaguaa cagaaaacca aauaccgcau auucucacuu 35460
   auaaguggga guugaacaau gagaacacau ggacacagag ggggaacaac acacgccagg 35520
   ucuuguuggg gggugggggg ugaggggagg gaaauuaaag gaugggucaa uaggugcggc 35580
   aaaccaccau ggcacacaua ugccuaugua acaaaccugc acauucugca cauguauccc 35640
   auuuuuuuuu agaagaaaua aaaaaaaagu aaugggcauu agcuacagau aaaguuaaag 35700
   gaaaaaauac aggaacaauu ucucagaagu aucauuugaa aacuucugau caugaguggc 35760
   uagcuaucug cacauaugug uuauauuacu guauucacaa ccugugaaug uuuugguggu 35820
   uuucucugau aaugcacauu auauauuaga cauugauguc agugacuuaa cuaauucuag 35880
   caaugguuag gaaaacaucu ugguccagua acauuuuuca cucagcuaau aauuccacag 35940
   uugcaaggcu cauuccugug ucuacaugug acaaauaaca uguagaacug cagccauaau 36000
   ugaaaacaaa aucuaccucu guuucauucg cacaauuucu agggaaaagg agacuuuuuu 36060
   uuccaguaac aaaacuguca uauagauuuu auccuaggag aaaaaauaca aacaauuuua 36120
   uuucuguaag aauuauuugg cuaugguggg ccuucugguc uaaggggucc cauuguuguc 36180
   aucuuuaaag auaauaagaa uggguaggag aucuacucuu aacgaaaacu agagaauuac 36240
   auguucuggu gucagggagg gagcaguuac uuuauguggg cauuuucccu gaagcuguua 36300
   uuuaggggau uguuuucucu gaccuuugaa gcacuuuuaa uuuuuucaac auguaaagaa 36360
   aggccagcau uucaucucac aacaauuaug augaaaauag cucuaaguaa aagugaguug 36420
   aaaggaacug uccucaccac augguucuau uguauauugu uuucaagaag gcauuuuaag 36480
   gagagagaag ggaggaggga agaagaaggg gaaaaaaggg agacagaaau agaagugggg 36540
   guggggagag agagagagac ccagagacca agaacggagu aagcaaaaug cugagugaag 36600
   auguaaaggg auuaggagag agggugcuaa gagugagagg cuugucauca auucuuagcu 36660
   ucauccgugg gucugugaag ugugauggag aggcagcauu augcagugca ggaauuaagu 36720
   cuaaguuaua gaaguaucuu ugcuucuaga uccuccugac auauauaauc ucaauucaaa 36780
   ucuuucucuc uuuaaagccc ucuucgaaca uucagguaag cuccaauuug cuuuuaagcu 36840
   guuuucuccc ugugggacca ggaucuaauc agucucauua uggauagcua ugguacucau 36900
   ucaaccuccu ugcaaccuuu ccuuugggaa cagcccuucc uucagcucgu agcauuuaga 36960
   uggagcuguc agucagggcg uuacuccucu cccacagaag cuggacaaau gaugcuuacu 37020
   uacccaucag agaccuuuug ggggcuggaa uggauguuug gagccagaga ugguuucucu 37080
   ccuuaugaaa uuuugaguua uaaugaccac uuaaguuuaa aaugguaauc cuucccauca 37140
   uguaaagaga auccuucagc aaaugaaagc agcacugcca gaaaucagag ccaagucuug 37200
   gagggagaau caguuccuga gaucauucac agaaugucug aauuuagcca gccuaaagga 37260
   agacaauacc agaccuuuuu aucugcagca gccaauagac uccaucuuaa guugaggcua 37320
   guuugagcuu gcauucuuuu cccugcaaug gaaaaggccu uagauauaau aauauuucuu 37380
   uaauauuuuc uuaagaugga uuucaauaga aauuagaauc uacugaaccu gcugacuuau 37440
   ucuacugcaa cguaaaccau gugucuuggc uacauuucag cauuaacaaa ucugcacuac 37500
   uuuucugguu ccucccagua acuuuauucu acuuguucug ugacacuugu guuaagcgau 37560
   uucugccaag uuugguuacu cuucucugag agaccuaaca gauaaucuua uuuucaucca 37620
   uucuuagcac cucgcagucc ucaauaccca uccguaacuc uagucaauag accaucagug 37680
   auagggaacc accuacaacu cuagaauauc aaugacaggg cuucuggaaa uggugucuua 37740
   guccauuugg auugcuauaa aggaauaccu aggcugagua auucagaaug gaaaaaaaaa 37800
   guuugauuua caauucuaau gaccaccaag uacaagauua ggcacugaua gagaaccacc 37860
   uacaacucua gaauaucagu gacagggcuu cuagaaaugg ugucuuaguc cauuuggguu 37920
   gcuauaaagg aauaccuaga cuggguaauu cagaaugaaa aaaaaguuug uuugauuuac 37980
   aauucuaaug accaccaagu ucaagauuag gcagugauag ggaaccaccu acaacucuag 38040
   aauaucaaug acagggcuuc uagaaauggu gucuuagucc auuuggguug cuauaaagga 38100
   auaccuagac acucauguua agugauuucu gccaaguuug guuacucuuc ucugagagac 38160
   cugacagaua aucuuauuuu cauccauucu uagcacauca caguccucaa uacccauccg 38220
   uaacucuagu caauagacua ucagugauag ggaaccaccu acaacucuag aauugauuua 38280
   caauucuaau ggcuaccaag uucaagauua ggcaucuagu gagggccaac agcugcuucc 38340
   acucauggug gaaggcgagg aggacccugc augggcagag agcauacaaa gagaggaagc 38400
   augagagagu gaggagguac cagguucuuu uuaacaacca gcucucuugg ugaacuaaca 38460
   gggugagaac ucacucauca ccaaaagaga gcauuauucu auucaugagg gcucugucuc 38520
   caugacccaa auaccuccca cuaggcccca ccucccagga cugacacacu gaugaaauuu 38580
   caacaugaga uuuggugcug acaaaugaac cauauccaaa ccauaagaga cagcaagagg 38640
   aacacagaaa uggugccaga caguaaaagg aauuuuguca ccaaugugag gucacaucga 38700
   ccauucucca uggcucccau guagauauuu uaugacccua gcccacgugg aucacaucag 38760
   acugguaccu gucaaauucc ugccuugauu uaugugaaua uaacucuaua uucucuggca 38820
   aaagcacaug aaaaaagacu uuuucuucuu aaauaaaaau gggcccuuaa aaagaaaaug 38880
   augaccagaa agcacauguu ccaaguggaa aagguuaaca cugaaaaucu uuaauuuuag 38940
   ggauuuccuc aguucuucuc uuaagcuucu auuucucgcu uuccauccuu ggguuuuugc 39000
   uacuuauugc uaccugcacu agagagcaaa uaaaaugaaa ucuaauuuag aguggacguc 39060
   ugcuaccuca cagugcccuu ugggaaauuu uaguacaaga ggucacuguu uaaacacuuu 39120
   gcuauacauc augugugggg auugcaguca accgagauac cccugugcag uguacacaca 39180
   cccauacaua agaacaugcu cucaauacac acagaugugc acauguagua aaauguuuga 39240
   guuccccagg ggcuuucuua gaugggaaug agcagcccua cuggccucag guuuuggaug 39300
   uggcauagga uagggaguau gggacagaau cccuuuccuc cacgucagua aggacgaggc 39360
   uccaccccag cuggacucuc agggaguuga gguuccuuuc ugcugcacau cagcacuccc 39420
   ugcuucugcc ccaaaucagc uuugcccacc uucccacugc cacaucaucc agcugcuuca 39480
   guggcauguu ccuguucugc auggcagggg acccaacuca gcccaugaag ccacugaaau 39540
   acaggaaaga gcaucuucau ugaagccacu ggagcauggg aaagagcacc accagcuccc 39600
   ugcucgcuag cugugaaccu cagguacucu acagaucuuu gaguuguuuc cuuaauuugu 39660
   uaauugagaa uaaaaaugac ugccuaauaa gagucuugug aggauucagu acaauaauau 39720
   acauaaaaug cccgguuuuu guagauguuc aauaaagguc uguauuaguc uguuuuuaug 39780
   cuguugauaa ggacauagcc gagacagggc aauuuacaaa ugaaaggggu uuaauggauu 39840
   cacaguucca uguggcugug gaggccucac aaucauggug gaaggugaaa ggcacguuuc 39900
   acaugguggc agacaagaga agaaugagag ccaagugaaa ggcauuuccc cuuauaaaac 39960
   caucagaucu ugugagacuu auucacuacc acgagaacag uaugggggaa accgccccca 40020
   ugauucaguu aucuuccaca ggaucccucc cacaacauga gagaauuaug ggagcuacaa 40080
   uucaagauga gauuugggug gggacacagu caaaccacau uaaggucauu ucuccauucu 40140
   uuucuuucca uucucaaaga cagucagugu uaaaacucuu uccauaaguu uaauuuucca 40200
   ugucaccuua uaccacuaau uaugccuggc cuauaguaga uacuccauaa auaaauguuu 40260
   aauaaacaau uaaaugaagu cuggcugauc auauauuaaa auauguauuu guagaagauu 40320
   acugcaaccu agguccuuag acaaauggag uuggaauaug gcggauauaa cagggugagc 40380
   augugccacg uuuuucagaa ggagcccaac aaugugccac auugcaccag gugaccugaa 40440
   gagggcugcu gauuuucugu ggucaacacc ccacgacuuc aggacaccag cagggcuggu 40500
   uauguuaaua ggacuaugcu accagcucuc ucucuucugu ucauaucccu gcugcuauau 40560
   ucuagagaag ccuguguuuc uaucuuugcu uggccuuaaa accagagguu gggaaguaug 40620
   gguaauaagg ucugcuccau gcaguccucu augcucagug gcucugagaa ucaccaagua 40680
   caguguaguu ucccacugag ucaguauaga uguccuuugu uggguguccg ugcaacuguc 40740
   acuaccuuau gccccaucac ucugugccau agcaaagaug ccuuuugccu ucaaccacau 40800
   agguggcuga gcaagagagg gcuuuggcuu gacagaagau cucaagcauu ugcuuucugc 40860
   cuucuaguuu gagugguugu uguugcucuu uucaaagacu ucagauacag ggacaaugua 40920
   aaugucaguc auucugguaa uauccuugca uauguggucu gugaggcagc cauccuuggg 40980
   agcuugugcc ugugucugaa uuauaagaaa gugugaugac aauggcacuc cuguuucaag 41040
   uuuuagguau caguacugcc auggucacac cugggccuaa gaaaccaccu auuaucuugg 41100
   uaaggccaau uaagccacaa aacaugcagu guuaaagaau acacacuggg uacagaugua 41160
   ccacugugga augccaugua gcaaauauua ucuugaagca cauucuaggg uagcaaaaag 41220
   uguggugcag acagccuaga aaugagaauc agcagauaag aacugcgguu ccaccauaac 41280
   ugcuaaccag cuguuugacc uuggcaaaga uacuuaaccu cuuuggaccu uagcuucucu 41340
   aaguauauaa uuagacaucu ggucuagaca auuaguuucu aaaucuccug ucaacucuaa 41400
   uacucuauga uuauuucggc ccauagucua guaauuuucc aagacaaguc caauaauaag 41460
   aaccauauca ccccuaagua ucuaauuggu uaaagaagcc uuggauuugu ugacaacuac 41520
   gaaaaugagg aacuaaaaag ccagguuaag ggaaauuaau acacugauga ggaugaaugu 41580
   gagaagacaa caaagacugg aauaagugcu uuucaaggac uggcuccaag ggaccugaau 41640
   uugcauaagc uuauccgaaa cuaaaauaag cccaacuucc uaaagaauua aauauuaucu 41700
   gugcaacuag gacuuuucaa uacgaugugc aaggcuugga ucagaauucc uuauugguag 41760
   ccuggaugca cuggacuucu uugacaagag auacaaagaa uaaaauaaau caagcaggac 41820
   agccagaggg cauuucucaa uauuuauaaa cuaaaaauga agaguucugg aaauaauauu 41880
   gaaaauaaaa uaucugauuc aauuuucuca uacuuaauaa auuuagguaa cucuaggcug 41940
   ucagauagga gaauaugugu ccacauaaau agaaaaguac aaggaauaaa auuuuguuac 42000
   caauaaauug uuugaaaacu ggaauuccac guaugccuau auauuuuccc uagucuucuc 42060
   agcugaacuu ugauuuccua gaaguaugug cuuuuuauuu aauaguucua gauauaaaua 42120
   uucaauggau cuaaaaucuc uaugauguac aguccccuaa uauuuacaac ugucugcaac 42180
   uggauguuga ucugcauuuu cugauugugu uucuaacuac ccaugaagga augaguuuua 42240
   uugauaaaga ggauaaacuu augagucaag ccugaguuug auguuaacuc uaccauucac 42300
   aaauugugaa ccuuggucaa cguauucaaa uuuuuauauc cucagguucu uuaucuguaa 42360
   aaugggucag gguuucucag ccuuggcgcu auugauaucu ugaguggaga caugguuugu 42420
   cuguguccuc acccgaaucu caucuugaac uguuguuccc guaaucccua caugucgugg 42480
   gagggaccug augggaggua auugaauaau gggggcaggu cuuuccugug cuguucucau 42540
   uauaguaaau aagacuaaug agaucugauu guuuuuauaa aagggaguuc cucugcacac 42600
   acucucuugu cugccgccau gcgagacaug acuuugcucc uuuugccuuc ugccaugauu 42660
   gugagacuuc ccgagccacg uggaacugug agucaauuaa aacccuuucc uuuaucaguu 42720
   accuugucuu gcguaugucu uuauuaucag uguggaaugg acuaauacag gugagauaau 42780
   uuguccuuua cucaguagau gccaguugga uaccagcagu ugugacaacc aacaaugucu 42840
   cuagacauug uucuguguuc ccuguggggc aaaaucaccc uagguugagg aucucugaug 42900
   cuuaucucaa cugggguaau caugcuuauc uuauuguguc acuggaagga uggaauagaa     42960
   gauggauaaa aagcuucuac ugcagccugg gcccaguuca cuuucucucu uggcuccaau 43020
   ccugguggac acaguagggg cuccucaaaa gcuccaccca ucaccuccuu gaaucacugc 43080
   aucaucuacu ucacacuggg gcacagacaa uuggauggcu ucuuugaucu cugucuagca 43140
   cuugaucgua aguauuggau uugaaaaccu aacuuuaaau aaugugagga agaggaucca 43200
   agguauguuu uauuucaaca uauuaaaaau ggauaucaga uauaaaaaua cugguuaagu 43260
   uauauaagua gaauuguuuc cagaccauau uguguauuaa uuauauccua guucauagcc 43320
   auaaaacaau auuuuauuau acuacauuuc cgguggucac ugagcaucca ugaguauagu 43380
   aaguggaaaa augguaaaau cugcaaaaug guucuauauu agauuuuauu aaauaagaga 43440
   agcaacaacu uaaaucagac ucaaaaaauc aaaauaauuu ggcuccuuua aaaagcaaag 43500
   aaagaucaac aaaaaacuaa cauuuggccg agguagcuug cucccuugug gauucagagc 43560
   ugaaaguuac ugaagacaua ucaguacaaa ugugcacauu agauaauucc cacuaaagca 43620
   ucucuugcaa auaaaauuua uggacaucug acagcauuua uugccugucc uuuauaaaca 43680
   caguacuuaa caauaaaugc ccucaaagaa uucuccaguu cucagauccc accacagaag 43740
   gacuuaagcu ucaaaacuac uggcuuucuu cugaaaaccu uuuuuucauc caaugaauac 43800
   caaaagaacc ucuuuccuga auaaaauauu agcugaaugg uuuacuguag aauaaucccu 43860
   uaucuuagcu aucaaugcug augugucauu uaugguuauu uguuauauga uagauaaaau 43920
   guuugaauga uaaauccuaa auaaguuuug aauauguauu auauauaaua ugcauauguu 43980
   auauauauua uguaaaauau auauauacuu uuuccucccu gcuccaguuu uuuccaucuc 44040
   uaauaguugu aaggauaaaa uuugcaauaa uucuuuuaca guguuacaga auucuaaugu 44100
   gaauaagaaa aaaaggaaua agacuggaau accaagccau gaacuuggaa agaaauccaa 44160
   acuggcauac uggugaauac uacauuuucu ugaaccaaug ugccaacuug gaacaauucu 44220
   uuaacuuacc uguuucuauu uccucaacuu auaucuaucg guccucaaaa uagagacaug 44280
   acaaacauac acauucaaua auuauacaca uauacacaua uaccagaugc uguagcauuu 44340
   acaaugaagu cacccuuugc aaaucauuuu cacauauguc ugcuaguagu uaaaaagaua 44400
   ccuaacuuua uuggaggaaa aaauaaauca ucuauuucug cauaauuaag cuaagcucaa 44460
   auauguuuuc ugaucccaau cuaauuguau ugauuucuca cuuugccuaa gaccacaaga 44520
   aaacauucac uacuuuugcc accuuuaaaa ucuuugaucc ugaucuucua uguuugaugg 44580
   ugccuacggc ucucuuaugc ucaugacauu uuagugaacu ucacuaggug acuuuguugg 44640
   aauaccucuu ggaaacaacu uacuuuauag aaaauaagua aaacauagaa auauagcaug 44700
   caagugaaga gcauggacuc ugggaccaua uugccuaauu cuaauggugg cucagccucu 44760
   caggagaugu gcugauggga aacgucauuu aggcucucuc uguguuccuc agcuuccuua 44820
   ucugcaaucu ggggauagua auuguaccca acucacagag uugauaugag aauuaaauga 44880
   guuuaaaauu agaacagugc cuggaacucu gaaguguugg cuugcuauua uuauacuuua 44940
   cauaguuaau auuauaauuc agcugggcuu gaccccagua cuuucuacag guguaaggau 45000
   gagcaaaccc cuaccccucu caggcucagu guuuugauca guaaaauuga ggugggauga 45060
   augaucucua ucccugucau uugguuaggg uuuacugaca augccugcgg cagugccaug 45120
   ggaguauaaa uugguaagau guuauggaag gcaauguugc aguaugguuu auaauguaau 45180
   ggguucauac uuuuagucuu gggaauuugu aauguaggua auuuaucuua caaaaauacu 45240
   ugcauacaua cauaaagaua aauauauaug aauguuuauu aaaacauguu uaaaaaugac 45300
   cgcaauaugc auuuauuagu aacugguuaa cuaugacaga uaaugcuacu uuaauuuaua 45360
   gcuauucaaa aggaguuugg aaggagucau ggaaguaaac agccaagugg aaaaacacaa 45420
   guauguauau cauggucaca uuuugaaaag aaagaaaaaa aaggaaggaa ggaaggaagg 45480
   aaaagugaau gaaagcagua aaaaaauaca uguguaucuu agaauaugga cagaaaacag 45540
   auuuuaaaaa caugugccag acaauuaaca guaguuaauc uggaguggau uuauugaugu 45600
   cucuaucuuu uaugucaugu auuucuuuau ucucuaguuu uuuuuuuaau uuuacuuuca 45660
   guuugggggu acaugugcag guuuguuaua uagguaaacu uggguugugg guguuuguug 45720
   ugcagauuau uucaucaccc aggaauuaag ccuagcaccc auuauuuauu uuuccugauc 45780
   cucucccacc ucccaccuuc cacccucuga uaggccccag aaugugcuuu uccccugguu 45840
   uagcucugug uccccaccca aaucucaucu ugaauuguag cucccauaau uccuacgugu 45900
   ugugggaggg accagguggg aaauaauuug aaucaugggg ucgguuuccc caugauacug 45960
   uucuugugau agugaauaag ucucacaaga ucugaugguu uuaucagggg uuuccgcuuu 46020
   ugcaucuucu ucauuuucuc uugccaccac cauguaagaa gugccuuuca ccucccuguc 46080
   augauucuga gaucucccca gccacaugga aguauauguc caaugaaacc ucuuuuucuu 46140
   cccagucuca gguaugucuu uaucagcagu gugaaaaugg acuaauacau ccccucuauu 46200
   uguccauaag uaaauauuac uuugguaguu ugaaacaaaa agcuguuaag uuuuucuuuu 46260
   uaagaacaau cauagacuuu ggaaucagac aagcuugggu ucaaauugua ucuuuauagc 46320
   uuuaaagcag ugugaccaag uucaaacuau uuaaacucuc uguucuuauu uccuuaguug 46380
   uaaaauggau aauauggaac ucuccuuguu acauaaaaau uaguuuagaa aguacauuua 46440
   aauucacuau cacaaagccu ggcaaacugu aaauguucca uaaauguugg uucucuuccc 46500
   uccacucccu gcuaauuaca aaauaucugc uuaugaagcu guuuauaguu uaaaaauaga 46560
   aauauauuuc ucuacaaaga uuuuuauuua gcucuaggug ugacacaaau uuaacagauu 46620
   gaauuuguga uaacuuuuac cuuaaaugua ucauuuuucc uacgaucaga guuauuuuag 46680
   gaggaacaag aaaauauaug accuaagauc ugaaaaaaaa ugguuaacau uuagcacuau 46740
   uuuacaguag guccauccag aauaaaagua ugggugucca cugugucuca auccaucuau 46800
   aaaacaguaa agcaccacau agugaaguga gaguuauuuc uauauauaua uaugcagaaa 46860
   aacgucugaa aggguaaaag caccucagca ucaagugucu auuuuucaga uguaaaauua 46920
   caaguaaauu ucuucugagc uuuuuuauau uuuacacauu aacaaaauuu augucuuuug 46980
   aaauaaaaau gcacaaaauu uuguuuauac cuaaaauaug uagaguuaua caaugaugaa 47040
   aaauaaacca uggcccuuga uaaugauuuu acuuuaauuu ucuaugaaaa uuagcagggc 47100
   aaacuuucaa gcuaaguguu uucuuaauuu ggaugagguc aacuaaauuc ugcaaaggaa 47160
   guagagacaa uggauuuuga cuuguuucua auaugaggaa auacccuggu cuuaucauua 47220
   caacagugga uuguuacuuu uguuuuauug uaaacaauuc aaaaacacau uaaugaagaa 47280
   auugaugggu auuauuuagg gaauagcauc uguuuuuuuu gguuugacug agucuguuau 47340
   gaucgaauag uuuaguguau uugugagaag ggaauaacca ucaaaugcuc acucaaaaca 47400
   gaacuugacu aaaaaguuug gguaacuuaa ugcaauggga aauuguuaaa aaugggugaa 47460
   ggauguuugu cacucgcuuc agccaaaaug accuguuuca ggcugaaagc augagccugc 47520
   aacagguggg agagugcgug agaaaguaug aaguauuaua cucuuaauau guucaaauuc 47580
   aaaagcauuu ucaaauuaaa aaaggauuua aaaaucauac aguuuaauuu cuccuauuuc 47640
   uagaaaagca uuugaguuga acuugaacug guucuggaac ccagaucucc caucucaacc 47700
   uucuuccuuc uuuccuuucc acuaaagcau gcuaauguaa uuaacauuca gguaaaguca 47760
   cauggcacau aggcugaaga uuacccagac agacuucaga uccuacugaa agauagaaua 47820
   auuaauauga uggcuaacag gauguuuguc aaaauuauau uuuuggaauu ugauuuuuuu 47880
   ucaccuuuag gcugaccuau uaauuauagu caauuaccgu auguacacac uuaugaggaa 47940
   aaaacacuuc uuaguguugc auguccaaau gaacccacug ugcugaggcc cuauaugugu 48000
   cugauaaauu acuaacagau gguuguuuau uauauggauu ucacagcucc ucugccucuc 48060
   ucucuuauuu cugcugagua uucugguaac auaaccauuu cucucuaaag acagaaagau 48120
   gaaaucagag uaaggaaacu cccaaguggu ccacuuuauu cuacugaaua uuuuagucca 48180
   auggcuuggc agagaggacc uuuguagaug uugaugaaaa caaucuguua caucgcuguc 48240
   acuguucauu aagagacuug caaagaucaa auauccauau gucauucaag aagauagcca 48300
   auuauuauaa auguggaauu uagagcugug uacugaacaa auucaaacuc cacaaagcua 48360
   cugcacugau uuuucuaaaa ccaucaccau aaggaaaauu cauauuccug gucacaaaca 48420
   caaaacucuu uagaucuuac ccuugcccau agccuugacu ucaugcuuug ccccuucuug 48480
   ccuuguauua auacuuaaaa auccagcccu gugaauggcg uuucuucuca agucagagca 48540
   ugcauccacc ugccugccug gggcaugaag gacuuggacu ugaaggacag caugauaugc 48600
   uccucgacaa ggucuccucc caguuacucc gcauugaaca ucccuaucac aaggaugcuu 48660
   guccaggauu gagucucauu acuaaggucc caggaauaga uguuccccgc ucaccucucg 48720
   ccaccccugc cacuagcccc aaguaccauc ucucaacucg aggucucuac ccagccaugc 48780
   cuucuccgug guaggcuccu cagaucucuu cauucaacua guaaccccuu ucucauccca 48840
   uaaauucccc uucaagaugu caccuccuug aaaauacaga agcacagcca gagacugcuc 48900
   auggccccuc uaucucaaac cccacuucac ugaaucagcc cuggggugug ccggcuggcu 48960
   cuucuccaug cucuauggca cucagugcuu gcauguucaa cacauuugcu guguuccacu 49020
   guagcacagu gauucugagu guggacucug gaacaagugc cccugggcuc aaauucuggc 49080
   ucuucauuug ccagcaggau uacauaagac aagugacuug cuucaccuuc cuuauuugua 49140
   aacuagacau aaaaagcaug aucugccuca uagagaagag gauuaaauaa aucaauauau 49200
   cuaaagcuua caauagucag cccuccauga gugacagccc uccauaagug acuguauuug 49260
   ugcuucaccc uugaugucag gauuuaguuu ucccaggaaa gucuuacuua uuuguauaac 49320
   cauugccccu cacagagugc uuugccucua guaagcucca uauguacagu ggccaaacug 49380
   aacuaguaag aauaagggga auaaacucaa ggugagaaaa uccuagaacu ggaauuaucu 49440
   aaacacccag guuuaguucc aaauucauga agcaaagagg agaucuaugu aggcauauau 49500
   ggccauguuu uuuuuuuuuc caggggaaac uauuucagau gauucuaccc uugacuguaa 49560
   auguggucuu cuccaacagc agucaguaug uagcuucuga cuaauaccaa aagguuucau 49620
   gucuaugcac gguauucuau uauaguuuuc aacagaaagu acaaauuacc ggaugccuga 49680
   uaaauucauc cagaaacacu aagaaaaaca gaugcuauuu uccaaaccuu aucaaaugau 49740
   ucccuuucuc cucacucacc aggguaguuu ggggccaaau augauggaaa aguaauuugc 49800
   ugacuucuga ccacuugcac acuccagagg gaggggcugg ggcaguaagg cagauauauc 49860
   aauuucccac acucuacucu cuuccuucuc ugcgugguua aaaacaaaac aaaguauuug 49920
   auuuccucau uuuuucuacu aauggaagau gacagaucca augguugagc ucacaaauga 49980
   aaaucuuuau uuuguaugau cagaaucuuu cuguuuccau uguaaccaau ccaauaccag 50040
   agagauauuc caaaguuugg aaggauagug caagucagcu augaucuagc auguacugaa 50100
   ggacuuuuca ccaagauguc agcucccuac acacucagag acuuagcuag cuuugaccuu 50160
   gaagccauug acagggcaug caaaggagag auaauggguu guuccugcuu ggagguuugu 50220
   guucuagaua uaccuuuauu auauuuuaga cauucuucac uacuaaaaag uaaagugguu 50280
   accuuagggg gaggccacag gguagcuaca uauaggaaau auauauauua caaaaaaacu 50340
   agccaaggaa cuuugugugu gugugugugu gugugugugu gugugugugu auuaguuugc 50400
   agaaucuuuu uuuccauuug uuauuuauuu gcgugagaau agcaauacac aggcuuauau 50460
   uuauccucag cccucccuag cccucccaaa cuauauuaac uaccugacuc uuaaaaagaa 50520
   uugugaagga acuaaaauga uaaugcaaag agacauggaa aauaauauuu auuuaauuua 50580
   uauggauuua gccuugagaa uuacuuuaug cuaagagacu cagggugacu acaaccccca 50640
   cucacgggca gaacaagagg cuaaacaugu ccuuaugcug cagaguauau uucaaacuau 50700
   uagagugacc cauuauuuca uuaauuuaag uacauuucug guugcugaua aaaugaguuc 50760
   uagccuguug gcggacaaga agagaagaac aaggcuuuau uccuaugguu aggugccguu 50820
   gcgaccugag gcacaugcua uuguaugagu gauucugaca aagagaauau cuguuuucaa 50880
   cuugcacuuc auuuugcaca caggcuaaaa uaggaaaaga ggccauuuau gcaucacuuu 50940
   caaacacaaa guacuugaua gagaaaaaaa aauucucuga acagugguca gacucuccaa 51000
   auagacaagc ugaugccaaa uaaaaggagu gauugugaac ggaccuggca gcacguucuu 51060
   caggaaaugg ccgggugugu guauccuugc ugauuacaug uauaauagcu ccacaagcaa 51120
   ugcugucuau caagaaaggc ugcaucucag agucguguuu ucaaaaggaa agagaaugag 51180
   cacucagcug agccugucug gaaagaggca aagcggcucu aggagagggg aaggaguugg 51240
   gaguucuuac augcuggcga cagccaagcc gcaagagaug agcagggcac agagcacacg 51300
   uucaaaggaa aauccagugu ugacuuuuac cucuacagga uguuugaaaa aggauucagg 51360
   aaaaucaaaa cucaaaagca uagaucagcc uguggaaagu ccagcauguc uuuccacuuu 51420
   auagugaacu cucuggaaca aaaagugaca uucagauuuc aagucacagc uaggauaucu 51480
   gagccucaug gguaauggga acauguccgg acaacauugg gaaaugagug ggcgcugccc 51540
   ugcaagaacc gaaugggaua caaugcugga acacacuugc cacaaugccu agcaguaagu 51600
   ucuaugaaaa cauuaucauu cccaacauua auacuaauca uuauaacaaa caucuaaauu 51660
   uuaaaaggcc cuucuaccuu ugucacccug uaaaugacau ggauaaaguc auaguuuaug 51720
   uggcugugac accacaaaga cccagggcug auuuaccugg caauaccccc uuucucuacg 51780
   gaagcugugg uucaaaggga ccauagcuag aagcuacucu gcaagggagg cuaugagcuu 51840
   agcucuuaga gcccaacugg gaaagaauau uguacaauaa cgucuacaaa auuacuacuu 51900
   uucagccugg aaucacguuu gugaucuucu uuggaacuua ucaaugcaaa gcuucauaau 51960
   aacggaaugu uuuccuauug uugcuuggau ugccuuaauc ucuuaccaua acuuuuucau 52020
   cccccaacau uuaaaguuuu uguauacuaa aaaguaauag cuauuuauuu aauuuaaaau 52080
   auuuuaccag cuuugaaguu acagugugug gauaucuaag auaucaaucu aagcucuaau 52140
   cuauacaggc agaagugcug gugcuuguga aagcaggacc uggaaauaga cuggguucaa 52200
   guccuaaguc aacccccacu agcucagguc agauaaguug auuaaucacu ccaugcuuca 52260
   guuuucucau cuguauaaug ggaaaaauga gagugcuuca cucaugggag aauugaagga 52320
   guucuuaaua auuaucaagc ucuaaaaaca gacgaggcag aagcuaaaag auuuuaauga 52380
   acuucacauu uuuacaggua ugaauuuggg uaaacuguca uaauaguaug aaucauuuca 52440
   agaaguuaaa aacaaacaug aaaauuugca uuuugaauuu guagggaugu cuuaaucugu 52500
   uuccacucug cuaauaaaga cacaccugag acuggguaau uuauaaagaa aaagagguuu 52560
   aguggacuca caguuccaca uggcugggga ggccucacac ucauggcaga aggugaaaga 52620
   cacgucuuac augguggcag acaagaguga acuuguccaa ggaaacuucc ccuuauaaaa 52680
   ccaucagauc uugugagacu uauucacuac caugagaacg guauggggga aaccacuccc 52740
   acaauucaau uaucuccuau caagucccuc cuacaacagg ugguaguuau gggagcuaaa 52800
   auucaagaug agauuuggcu gggggcacag caaaccauau caaggaauuu guuucuaauu 52860
   cgcuaauucu auucauccau ucaccaaaug acuauuagcc ucuccuagag gaucucuuuc 52920
   agaugcaaug aagagaaaaa acaaacugau acuaaauugu aauaggcaga ucaagagagg 52980
   agucacacac ugcauucacu gcacaguugg cuuuaauuaa auuuggcuuu aaguuugcaa 53040
   auauuaaaau cacaguccaa aggauaaaag gaguagguga agccuauuaa gguuuuuaau 53100
   gcauggagga aucaccugca cagaccaugu aucaguggag uucaugaaac ugcacauuac 53160
   uagagcaauc auuuauacau uaagcgcuaa gaacacccaa agaaacauuc caucucagca 53220
   gcugccaucu uggauuuacu uuuccaaaag agacaauaau ccagacuaua gcauuugaaa 53280
   acauucacuc gggcuuuguu uccuuuuaaa cagucaaaaa uucagugcuc cugaaaaaua 53340
   uuguuugaag uaauugaggc cccuaaaguu ucugcagaaa aaacaggggc ucuuucuucc 53400
   uuuuucauga aauaaauuuu gggcucucag ccacaacaaa aucccacagu uacuuuaguu 53460
   ucauuagaaa gguuuacuuu ugacuuaaag uucucucuua guuaaaugau uccauuaaac 53520
   augcggccgg ggaugguggc ucacgcuugu aaucccagcu cuuugggaga cugaggcagg 53580
   cagaucacga ggucaggauu ucaagacuag ccuggcuaac guggugaaac cccaucucua 53640
   cuaaaaauac aaauauuagc ugggcguggu ggcaggugcu gguaauccca gcuacucggg 53700
   aggcugaggc aggagaaucg cuugaaccug ggagguggag guuucaguga gccgaaauca 53760
   cgccacugca cuccagccua ggugacagag cuagacuccg ucucaaaaaa acaaaaacaa 53820
   aaugcaugca ugcauauaca cucggaaaac uguuuaagcu ucaaagaaug uugugucugc 53880
   cauauugugu uuuuuuuuua agaaagguag aauuaaguaa uugagguagu ccugaugaca 53940
   caaagcauca gaaaagccuu ccaaauuauc acagaaagaa cagauuauua uaaauaauua 54000
   uucaaagaac auaauuguac auacaacaau guacacuuug auauuuuugu ccaauacauu 54060
   ucuagcagua caacugcugg auggaguauc ugccuguugc uguguaaaua auuaucauaa 54120
   acucaacggc uuuaaacaaa gcuauuuauu uucucacagu uuuugugggu caggaguauu 54180
   guuugggcac ucagcaaggg ccucacaaga cugcaaucag uguuagcugg gcuguguucc 54240
   ucuccagugc uaagcguccc cuuucaaacu uagguguugu uagaagaauu cagguccuuu 54300
   uggcaguaag acuauagccc cuguuuucuu gcugucugcc cacugcgggu ggcucucugc 54360
   uccaagaggc ucccauaacc ucucccacaa cauggcaguu uacuucuuaa aggucagcag 54420
   gaggauuuuu cucuuuaaca aggcccuagu cucucuucug agggcuuuca ccugauuagg 54480
   ucagguccuc cuuggauaau cucccuuuca auuaaaucaa aaucaacuga uuugagagcu 54540
   uucugauauc ugcaaggucc ccccaccuuu uccauauaau gaaaccuaau caagagagug 54600
   aaauucuuuc auaugcauag gucuugcuug cccuaaaagg agugaauuau auacaggaug 54660
   uacacagagg acagauauug ugggggcuga uaugguuugg cuugucccca cccaaaucuc 54720
   aucuugaauu guaguuccca uaauccccac gugucauggg agggacccag ugaaagguaa 54780
   ucaaaucaug ggggugguug cccucaugcu gcuguucuug ugauagugga ugaguucuca 54840
   ugagaucuga ugguuuuaua aggggcuuuc ccugcuuuug cucagcauuu cuugcugcug 54900
   ccaugugaau aaggaugugu uugcuucccc uuccaccaug acuguaaguu uccugaggcc 54960
   uccucagcca uguugaacug ugagucaauu aaaccucuuu ccuuuauaaa uuacccaguc 55020
   uuggguaugu cuguauuagu agcaugagaa uggauuaauu cagggaccau cugggaauuc 55080
   ugccuaccac acuggguaaa gcagugcuuc cuuuaaaacu cuggaucagu augcuaaauu 55140
   guccucuaaa gaggguggga uaauuaugag uucaccaaga guguacgauu ugcuauuuuc 55200
   ucauaccuca ucaauauuca agauuauugg uucuuuaauu uuccacccaa ucugguagaa 55260
   cauuuuuuuc uuauaauuuu uaauuucuau aucuuugaaa aaaagauuga gcauaauuuc 55320
   auguguucac aaaucuguga auuaacuuuu uacaucauuu gcuuucauuu uaaaugguca 55380
   uuuauuuugu uuuuacugau aaaagcuguc uguauauaac agaugacaac cuucucuuuu 55440
   uucaucuguu acaacuauuc ucucuucucu uucucucucu cccaagaaug uaacuugagc 55500
   ugugucucau uaaugccacu uuuaaagaaa aaucagauuu uaagcucaac cagucagaag 55560
   cagccaacaa ccuguauaac uagggacuuu gcaacaagau ggaccaaaua agacaacggu 55620
   auaauuguaa ccaaucaaau auuuucuugg cuuuccuuug cagguuauca uauaaaaguc 55680
   ccccuacccu ugcauucucu uggugaaacu ccagaacauc uucuggcuug gagcuaccug 55740
   auucauaaau ugcuguuugc ucaaagaaac ucuugaaaau uuuauugugc cucgguucac 55800
   auauuuaaga gauuuuguga cuuauauuuc uuucacucuu uuugccaugg agagauuuuu 55860
   auaacaaaua uauaauagug aaauaaaaac aauguaaacu ccauuaauuu guuaaaaaac 55920
   ugacauguau uaauacucuu caaacauuag aagaugagau gaauuuauaa gcuauggaua 55980
   uggaaagaag aacucuauua caaucaguag aaaaacaagu uauaaaaaag gauucauuca 56040
   uugugauacc cugcuugauc aaauguuugu uaucuacaua uuugaauaau uguguauuca 56100
   uuuaaaaaug uccagaaugg cacaugccaa gauauaucca cacaccauag uggauauauu 56160
   uggagaacug gacuaggcag auggcuuuga cuuuuuacuu uauucauuua uguauuuuua 56220
   uuuuuauuuu uugagacgga gucucgcucu guugcccagg cuggagugca guggugcaau 56280
   cucggcucau ugcaagcucc gccuccuggg guucacgcca uucuccugcc ucagccuccc 56340
   gaguagcugg gacuccaggc acccgccacc acgcccggcu aauuuuuugu auuuuuacua 56400
   gagacggggu uucaccgugu uagccaggau ggucucgauc uccugaccuc augauccacc 56460
   caccuuggcc ucccaaagug cugggauuac aggcaugagc caccacaccc agccucugua 56520
   uuuuuaaauu caauuuugua auaaauaauc uuugauuuua ucauuaaaaa gaaguuaagu 56580
   aaugauauaa uguuuaaucu acuuuuaaua ugaaacaugg cauuuugcug uaucuuaauu 56640
   auuugaauau uaucauuuua auauuuaaca gauauuuauu uagcaauaug agcuccuucc 56700
   aauuauuuau acuagauggg uuaguccuaa uuuucucagc uuguaagagg agguaaaagu 56760
   auguuacuug gcuagggguc agggguuggu uaguauguaa aggggaacua uaaccuugac 56820
   acuuaaaauc cuaucagaac aucuauuuua caugucaccc aucaauggga acuuauugaa 56880
   ugcauucaag uuguauuugg cugguaugug cagaaagaca aacuagaaac agucaauuca 56940
   ccgcaucaga agaguacaca gaaauaacca uuaccagcau aaggaaauug uccaaaugug 57000
   ugcuuccuca aguugaaaga aaccauuucu cagaaggaag aauuuucacc cagcaguuaa 57060
   aucagaggga ucaacacaau uggguacuaa cuugugcaag gcagaagaga aagugggaag 57120
   cagucccagg cugcaggaug gcuucauguc uggaagcaca gcacagaagg ccgauggcua 57180
   agcuggaggc ugagacgugu ccuaugcaaa gaaggacaga uuuggaggcc acaguggcuu 57240
   aaggaaaagu uguuucuuau agggugcuug gccauugagc uuucuuauau ucaugacucu 57300
   uagcuauuuc ugaagucugu uuuuagggag aggaugcagg aguggucugu gaaugauuga 57360
   gauuuauccc uuguacagaa auauugucau uuuuuuuuuc uguggaagca uggguucccu 57420
   gucaagcaca ccugccuugg cuuccucccu gaggcucuau cauguuuccu aagguagaca 57480
   cucccuucau aguguugggu uuucucucac acucccaccu guauccuccu cuuggguuug 57540
   agugaugcuc uccagagggg uaagacacug ggggcugugg ccccauaauc cuuccugacu 57600
   ucugaccauu caucuucaug uagcugcauu auccugcaua cucuauacuc agcuaugcuc 57660
   uaccgaucau gagagauggc cuggccaccc caccugguuu ucuuucccug cuccacagga 57720
   agaauuggaa agaagccugg ggaacugucc agcagugcca agagcugccg ugcaggauua 57780
   ggaacaggac aggcuucaga auauuacugc uguguauuug gagccacuca agcccaacuc 57840
   cucucacagu ggguuuucac ggggggcauc ucuggaaaga aggagaggcu ugcuucagca 57900
   gcuaagugga agcagaccuc cgauguaagc aguggaggua auacuucccc uucccuggca 57960
   cuugaaugga aaagauugaa gcaaaaugaa aggccaaucu guaugcaauu aaaugcuucu 58020
   acuggcaauu gcuuuuccuu ccccugcuaa aaaagcagac auccaaauuu cccaccuucu 58080
   uagugugucc ccccuccugu gcuaaauugc cacaguuggg aguagcgucu ucaggcaguu 58140
   acuggcaaga auagaugcau caaaggaaug aagauuuuug uuuuugcuuu uaacuuaaaa 58200
   aaaaggcacu ucucuagaga uguaguuacu aagcuuuuua gcuuaaucuu uagaaacuau 58260
   acauauauau uuagaaacua uauauucuau acuauauaua guuucuaaau auauagcuuu 58320
   auauguaaau auaaagcuua aauauauaua guauagcuua auacacauau uaagcuauau 58380
   auuuaggaac uauauugcug cuccugugug uagauucuuc auuaaagauu auucaucuga 58440
   caccuucuug gguuuuaaau uagcuuuaag auuuuaaaau uucaaugaaa ccugacaaua 58500
   aaaucacaau uauucaguaa uuugcaauau gucucaaauu cuuagaaauu uuagagauuu 58560
   uaaaguccuc uuuuaaacug cauucaucag auauugcaau uugagaagag caauuuaugg 58620
   acauuuauua uucgucuucu gaacacuacu guucuuugca uucuuagacu auggggcuuc 58680
   augcauaguu uuaauuccau gaucacucaa ccaaacagaa ggaaagauuc auauuuucaa 58740
   uuuuauucau ucgaauauuu acuaaauuca uacugugugg caggaauaca gcagugaaaa 58800
   acagagguga aaucuaugac cuugugaagc uuucauuuag acaggaaagc cagaaagacu 58860
   acacgccaau aagcaaaugu gugugugugc uguggugaaa ggguuaugaa gaaccagagg 58920
   ggguaauaag gagagaaaaa ugggaguuua uuuuauacug uguauguuag aagaagagaa 58980
   gaauacacuu ugacaaaggc aaaagcuuau augcucauca uuuuauauua gcauauacaa 59040
   auaucuuaac acuauacaua uguagaaaau uugaggaaau aaacaagaga gauagaaauu 59100
   uccugaaaau uauauagaga aucaccuaaa aacauaagcc ugauauaaag auguuucaaa 59160
   auauaaccug uuauuuguau acaaccucgu auuuguacau uuguauauaa ccuuauauuu 59220
   guagcaaagg uaaaacuuug ccaaugauag guaguaauaa aaugugguac agcuuuguau 59280
   uauucuguuc uccuccugcu aaaaaagaca uaaucaagac uggguaauuu aaauuuauaa 59340
   agaaaggaga uuuaauugac ucacaguucc acguagcugg gaggccucac aaucauggug 59400
   gaaggcaaau gagcaaaaua acgucuuaca uaguggcaaa caagagcuug ugcaggggaa 59460
   cuucuauuua uaaaaccauc agauauugug agauuuaauc acuacaauga gaacaauaug 59520
   gaggaaacug ccuccaugau ucuauuaucu ccaccuggcc ccacccuuaa caugagggca 59580
   uuauuacagu ucaaggugag auuugggugg ggacacaucc aaaccauauc auuacacccu 59640
   gggacccucc caaaucucau guccucacau uucaaaacca aucaugccuu cccaacaguc 59700
   acccaaaguc uuaacucauu ucagcauuaa cucaaaaguc ucaucugaga caaggcaagu 59760
   cccuuccacc uaugagccug uaaauucgaa agaaaguuag uuacuuccca cguacaaugu 59820
   ggguacaggu auugggugaa uacgcccuuu ccaaauggaa gaaauuggcc aaaaugaagc 59880
   ggcuauaggu cccaugcaag ucugaugucc aauagggcag ucauuaaauc uucaaguucc 59940
   aaaaugaucu ccuuugacuc uauaucccac auccagguca ugcugaugca agaggugggc 60000
   ugccacaacc uugggcagau uggucccugu gguuuugcag gguucagccc cccucccagc 60060
   ugcuuucacu gauggcguug agugucugug gcuuuuccag gcacacggug caagcuguug 60120
   guggaucuac cacucugggu ucuggaggau gguuucccuc uuaucacagc uccacuaagc 60180
   agugccccag uggggaugug guguuggguc uccaauccca cauuucccuu cugcacugcc 60240
   cuagcagagg uucucuauga gggcuccgcc ccugaaacag accucugccu ggacauccag 60300
   guguuuccau acauccucug aaaucuagac aaagguuccc aaaccucaau ucuugauaug 60360
   ugugcaccug caggaccaac accaugugga agcugcaaag gcuuggggcu ugccuccucu 60420
   gaagccacag ccccagccuu acauuggccc cuuucagcua cagcuggagu ggcugggaca 60480
   cugggcacca agucccuagg cugcacacag caaggggucc cuggaccugg cccaggaaac 60540
   cauuuuuccc ucauagggaa aauguauuuu acucuguaaa auacaaagua guucaagcaa 60600
   gggauacaaa cuuuaauaua ucaugauguu gggacauauu ucaggaaugc aagaaggugg 60660
   uucaacauca gaaaucgacc acugcaagcu accagguuac cagguuaaaa ugagaaaaaa 60720
   aaaaugaaca ucuuucugga uucugcugaa agauucaaca aaauucauuu uuuucuauuu 60780
   auguuuuuua uuucauuauu cuauuauaaa acacucauuu auaauagaaa cucuuagcaa 60840
   acuauaaaug gagggacauu uuaaaacuga gauuuauucc caacugcuaa cauaggucaa 60900
   auuuaauagg gaaacuuugg cauuucuaug aaccagaaac aaaaaaugau ucucauauca 60960
   uucugacuau ucaacauuau acuagaaauu ccaaccacua ccaaaaaaac caaccagcaa 61020
   aacaaacaaa aacaagaaaa aaagaaguua auaguauaag aauugggaaa aaaagagcaa 61080
   aguuauacua acugauaaau gucauuacuc uacacaaaaa aauuucaagg guaacuauag 61140
   auaaacuuuu ggaacuaaug ggaaaguucu auaguguugu cagauacaag acacacaaaa 61200
   gccagugaca uugcuauagg ccuuaauuga cuaguaagug ugauaaaaag uauaaacaca 61260
   ugauaauaaa auuuacuaaa ugccuaugag uaucuauauc aaaaauauua caaaauguua 61320
   cuuaacaauc uauaacuuuu uuuuuuuuuu uuuuuuuuga gacagagucu ugcucuguug 61380
   ccaggcugga gugcaguggc gugaucucag cuuacugcaa cguccaccuc cuggguucaa 61440
   gcgauucucc ugccucagcc ucccaaguag cugagacuac aagcgcgugc caccgugccc 61500
   agcuaauuuu uguauuuuua guagagacgg gguuucacca cgcuggccag gauggucucc 61560
   aucccuugac cucgugaucc accuggcucg gccucccaaa gugcugggau uacaggccac 61620
   ugugcccggu caauaaucua uuauuaaaua gcaauauuua ucauauucga ggcagaaaau 61680
   guucaauauc auaauggugu uaaucuucuc caaauuauuc cauaaauuca acugaccaca 61740
   aaucaaaauc ccaaugaaau uaucugugga acuugacaaa cugauuccaa aauucauaug 61800
   aaagaguaaa gcuaagcucu auucuuaacu uggaaaagaa ugaggaggag ggguuuuuac 61860
   uaacaggugu cauaacuuac uacugaguuu gaguaaauaa gacuguguga uauuggcaga 61920
   gggauagaaa aauagaucag ugagacagaa uaaaaagcac auaaaaaaga cuuuucaagg 61980
   cacacuugaa gguuuuuccu uugcagccug aggucuucua ggauauuuac auugugacac 62040
   uuucuccgag gaaagacugg gauggaacaa augucaguau ugcuccuaca gcauguacuu 62100
   gaaagcuuag gucauuucag ccuaagaaau aauuuauaaa aaugcaacaa gagaaggaca 62160
   uaaagacaga agauauccua cagaaauuuu auauuuuacu cuggacaaaa augguuuauu 62220
   cauagcccaa guaaaaaaau aaaaguugua acuaaaugua aucaauguaa ccauuuaguu 62280
   guaaaagagu aagcagauuu uucuucuuuu auuaagcagu cacccuguug ggguaaaaca 62340
   cugcaacucu ccuuccaaaa guggggggcu uggaucuacu ugagcauugg gagaaaaaga 62400
   aggacaggga ggagagguag gaguaagagg aagaggaaga ggagaaaaag aaaaggagga 62460
   ggaagagaaa uaagagaaau gaucuuauua aauaaauauu aaaucuagug uagccuaaau 62520
   guaacgccuu uauuacuauu uuguuaguaa auaaauaaaa gagaacaaau aucaacaaca 62580
   aaaaauaucc auuugguugu ucuuagugca uuaaauauuc auuucaccau ucauuagaau 62640
   gaaccaauac auugaucagg gcacauacag aaguuuggaa uaaaauaaaa gaagcacuag 62700
   aauucaaugg aaaaaaagau auaugcaaua uuuaguaaug acaaucuggu uccccauaua 62760
   aaaaaaauca gauagauugc uauauuuuau cguacaauga auaaacucua aauagacuaa 62820
   agaccuaaau acaaagaaca aaauuuuaua auuuuagaau aaaauacuug agagugucuc 62880
   cuugauguca ggggagagaa uuauuuuuaa aaggaacaca aaaagcagaa aucacaaaau 62940
   gauagauaaa uuugacuaca ugaaaauuaa aaucuuugua ugacaaagaa cacgaaugaa 63000
   agauaaguua ugaaaaggag aaagaaauua gaaaauugua ugauagucaa aaaauuagua 63060
   cucagaaucu aaguacuaac aguaggauuc cuacaaauug gaaaaaaaaa aaagaugacc 63120
   caacagaaaa augguaaaag uguaugaaga agcaguccau agcagaggaa acuugagugg 63180
   aacugcaaug caugaaaagg uuuuaaaaac aaacaagcaa aaccaaauac auaauuauag 63240
   uaauuuagug gcaguuacug uuauaaauac aucuauagau agagccauac uuagaauuua 63300
   gggcaaaaua uaauguaaag auaacaacuu uaaagucauu aauuuucaag uaguucaaac 63360
   ucucagugug cacuagaaag ggauguguga gauaaacaca gggcccaggg agacucccca 63420
   gggaagaaga gcauuuugaa uggguaguua aggaggaaga gaugucaaua aaggaagaca 63480
   gccuuagucg ugggaguaga gaaagaccaa gaaaucaagg guaaaagaag gaggagguag 63540
   ggucaucagu gccaaauaca gcugaaauau gcauugauau aaagaaacaa auccuugcag 63600
   aucuuuuaca uucagcaacg aggaugcuca auauccgucu agggugugcu uaucuaaauc 63660
   ccuaaugugg ugcuauagac ugaauguugu guuaccacaa aaauucuuau guugaaaucc 63720
   uaacccccaa uguuauggca uuaggaggug gggugccugg gaggugauua ggucaugagg 63780
   guggagcacu caugaguggg auuagugcuc uuauaaaaga gaccccucgc aucuuccaca 63840
   auaugaggac acaaugagau gauggccauc uaugaggaau ugagcuguua cuaggcacug 63900
   aaccugccaa cgcuucaauc uaggguucuc cagccucuag aacuguacaa uguaaauuuc 63960
   uguuguuuau aagccagcca ucuuguggcu uauauggcac uuuguuauag cagccugaau 64020
   ggacuaaaac agaggacuga gcugccuggg acaaccugga gccucaucca uccacaucuc 64080
   ccacuuggac accaacauac ugaacucuga cuagaguaag gggacagucc ugcaguauga 64140
   caccacacca gacaacaguc cucuugcucc auggccuaac aacccaacuc cacagaagca 64200
   gcuucaaaau gucaggagaa augauuauau cucuuuugcu ccugaacaaa acuuauuuau 64260
   ccauuuauag uucuguauug ugcugaguga uuaaguuuca aaacucucuc uaaaaauaaa 64320
   ugcuacugcu gaucugagag aaacauaaau gaccacugag aacagaguuu caacgagccc 64380
   agagaacuga agcugucaga ugcagaccuc acauuccuuu cucuccacca aguuauuuuc 64440
   aaggacaaug cacuauguuc auggucuuug acuaacuuga ccucuggcug ccauccuugg 64500
   cucucauaau aucuuaacac uaaacauacu aagaauuuuu uuaaauggga agugauuaga 64560
   uaauaaauua ucuacagagg guggucuaaa ugagcucuau cuagaauuug gcucaauuuu 64620
   acuuugcugg auucaaucua agcacgcuuu uccuaaaugc caauuguaac cagacauaaa 64680
   ugucuuguua aaacaaguuu aucaucacua aacuuaacug uagucucuga uuagcucuuu 64740
   agagccuaaa uaaggcauau uuaugguugc uuuuuuuccu cuugauggua ugucagcuac 64800
   auaugggaaa aacuguaugg cucagauccc uuaaaaugcu uuagaauuuc uucuaugaac 64860
   uuugaaagaa caagugucau guuuaauuuu auccuauaua ugcaguuaaa gaucaauuaa 64920
   aaguauuuac cugcuuuaug aaaggauuaa gauucucuuu aaguuucuaa uauaaagcaa 64980
   guauccaacu ucaugagaga cuaucauuua cauuucaauu aauguuggcu uuucacauuu 65040
   gaguguuaua auggcuuuag ucugaauugg gcaagugcuc uagacuuguc acuaaaguau 65100
   uagggaucuu gcugucauag auuaaugagu gacacaauau uucagccaua uacuauguaa 65160
   uucuaucuaa uucuggauau uugucugcug gacacgaggg aggggacuaa aaacacaaag 65220
   ucuggaugcu ucacugcuua cuuauaagug aaguagcaga agaugccaac agcuaacaag 65280
   aggggucuug ccuggagaaa gggugacgac acagcugaug gcuaauuuau cccauuucuc 65340
   uugauaaggu uugucacuaa gguuccauca uaauauauga uguggauggc cacagcauuu 65400
   uguugacagg aggcugaaac cugacacagg ccagucacau uacccucugc uccuaaugaa 65460
   gaguaccucu gacuaaaugg ggcauacugc aucacacauu cuggccuccu auugccuccc 65520
   gaacucaguu cuuguuacug uucuuuucca acuuguucua gugaauugcu ccucacuguu 65580
   cuucaaacag acgaggcaca cucccaccua gaagcuugac acuaccuguc uagacuguuc 65640
   ugaucacaaa uaucuacacg gcucacuccc ucaccucaug caaaucuuua uuccaacauu 65700
   accuuccuga ugagguuuac cuagggcauc uuauuucaaa cuguaaacug uuucccugcu 65760
   acccugaucu ucccaaucuc ucuuaacaug aucuaguuuu ucuuuuucuu uuuguucugu 65820
   auagcucuca ucaccuuuca aaauacuaua uaauuugucu cauuuuucau guuucuuauu 65880
   guccgucuuc cuucguuaga gcccagaaaa gcagaaaucu uucuuuuguu gacugacaua 65940
   ucccaaagcu uucaaaacaa uuucuggcac auaguaagug uacaauaagu auuugacaga 66000
   ugaauaagag ugaaugaagg aaccugauag aauaugguac aaugauaaga uacaagaacc 66060
   augaaaggca gagaccaucu gaauuuuguu uacccugaca uccucgacau cuaggacagu 66120
   gcccaggaau ucuacuuuuu gaaugaguaa aaaagcaaau aaauguuauu gggaaggagg 66180
   aggguuccua uaaccuaucu aaguauuagu ggaaaaauaa auuauuuuga ccaggcugga 66240
   gguacuccug ugucuaaaau ccugcccaga guucuuuagc uuccugcaga ucauaaaauu 66300
   acccaggaug ccacaagaca uauaguuuuu uuaguuacuu gagaacuuca aaggacuaca 66360
   acuuaacaga aaguccuaac acaagugaca gugccucuua ugcaaagcaa aagcacauga 66420
   agaugauuuu uguccuguaa cagaaaugcu ugggauucca uugcagucac uguccagugu 66480
   uauuuucuug agugguaaua ggagaugccc agcuuuagag ucagaccugc aucagauggc 66540
   cuucauguuc uauacugcgu gacucucaga aagucacuua cauucuccag gccccuauuu 66600
   cucauauuuu aaaguaauau ccacucuucc aggauacugu gaagcugaga agcauguguc 66660
   aaguaaaacg cuuaguagac auucaaaaaa agucacucug cucugcccuc ugugaccuuu 66720
   cuaaacauga uucuaauggu uccauucuaa uauugguucu uaaaaauucu aucgcauauu 66780
   auaauugcau aauaauguau aauuucauau uauaauucua aauaagauuu uguuuuucuc 66840
   auucaagguc uaauuaucag accacuuucu ucucuuaauu ucuucuugca uuuugcauug 66900
   gugcuacaua gaagaaaaau gccauaagaa auggaguuaa aguaccggcc auuucauuuc 66960
   uuuccucagu ucaaaaccug cuggugauag ccaagagugg uuauucaugc ugaguuccuu 67020
   aaaaauuaua gcauucauca gaauauuuac uaaaagggca gagcaaugau ucacaaauca 67080
   cccacuauuu acauuggcaa gaagcuaaug guguauaucc cugauaaguc augacuugau 67140
   uacuaagcaa aaguaaccau uaaaauauga aacuguaguu uacaagagua gcccagcuca 67200
   uuacuuggaa ggauuuccua cugauaugug auuaacacau agcuucugga auuaaaggau 67260
   aagcaauuca gcaaccaaug aauuugcuaa auuuuuuguu gccuacuuag gucuuuaaca 67320
   auaccauuug caggauguaa aucuuacuga gaaaggagca cuucuguaau cugcauacuc 67380
   ccagaagucu agguuuucag ccucaaggcu acaaaacagg caugcugaaa gaaaaggaaa 67440
   ugagagcaug aaagcauuaa gaacuuuguc agguucacac agcuucuacg uggcagaguu 67500
   gugauaugaa cuucuugaag agaagccagu uaaggccacg ggauuuauaa ucagaaagau 67560
   gugcauuuaa gauugucugg agagcuucuc cucuaucggg uucagagaaa caauacuuuu 67620
   aucccuuuua uguauguguc uuucauggaa gauggagauc cacugaaaaa aagaaagcua 67680
   ccuaugccaa agaauaauuu ucaaaguuua aaaacaugac ccuuauauaa auagagaaaa 67740
   cugguguaaa aauacucuuu aacaccuuaa ugagggagcc aaccagauga auaugcugau 67800
   ucaaaagugc auaagaagaa uauauaggca gagaagaaaa aaauuuggaa uaagauuucu 67860
   gggugagaua caaaauugga caaaaaucug cauauuaaca uguaacauca cauaaucuag 67920
   gcuuucaguu aauaguaauu ggugauuaaa acaaaguuac guucccuaga uaagcguaca 67980
   gcccuuaggu ggauuuguua accaguguuc caccuauaug auauugaaau gacaugcugc 68040
   ccucuguuug ccuaauuucc aaguuuugga uaacuuuucu uaacuccugg uaagcauuuu 68100
   ucuggaaaaa gaaaauacac uaacaaauac aauaauguuu uauuuacaga uggcucuaua 68160
   guucaacauu uuauaguuua cacaauugcc uagaugguaa aaagagcacc uggauguuuc 68220
   ugaugcaacu ucuuaugacu guuauauucu aagaguagaa aucagaucuu cccuauauaa 68280
   aauuguuacu uaaauaugug uuucaaauga aaaugcuuau uuuugucaaa guagucaaua 68340
   ucaucacgaa aauuuuagaa uguccaugcc cuuccucuua aacaguacug aaaagccagu 68400
   gcaagaaagg gaaagugaug gacugaaggu gauauuccca accuggauag aaagacaaac 68460
   aagcauccuu ccaguguuac aaccucucuu agcuugaaua ugggucuccg ugcaucacac 68520
   agaaaaacgg caaagccuug ccccaucagc uaauaacaag guugagggua aaucuggaaa 68580
   gacuugucuu gugugacuau ugcuuuagag aggagagucu uguuccaugu uugcugaaau 68640
   uaaaucauca gggaagauac uggagcuguu cugccucagc aaagcugagu aaauaacgca 68700
   cucugggacu gcucuaaucu cacuguuugu guccccauaa aauucauaug uugaaauuuc 68760
   acuaaucuaa uaccaucacu aaucggugau gguauuagaa aauggugccu uuggaaagug 68820
   auuagguuau gagggcucug accucaugaa ugagacgagu gcccuuauca aagagacccc 68880
   agagagcuuu ccuguccuuc caccauguga ggacacagca agaaggugcc aucucucagc 68940
   cagagacuga gcccuacugg gaccuugauc auggacuccc caccuuccag aacuauaaga 69000
   aguagauuuc uguuguuuau aaguguauua guccgcuuuc auucugcuga uaaagacaua 69060
   cccgagacug acgcaauuua caaaagaaag agguuuaauc aauucacagu uccacauggc 69120
   uggggaggcu ucacaaucau gguggaaggu gaaagccaug ucucauauga gggcagacaa 69180
   gagaagagaa cuugugcagg aaaacugccc uuuauaaaac caucagauau caugaggcuu 69240
   auucacuauc augagaauag caugggaaag acccgccccu augguuaaau uaccucccac 69300
   cggguccuuu ccacaacaug ugggaauugu aggagcuaca auuuaagaug agauuugggu 69360
   ggggacacag ccaaaucaua ucaaaaagcc accuaguuua ugguauuuug uuauaggcag 69420
   cccaaaagga cuaagacaaa ggcucucuuu gaauuucugu cucaaucauc ccgaccugca 69480
   gaaugaagcc cucugagauc ucuuauugcc uugguuccuc ucugucuuug gauaaagaga 69540
   guuucuacaa acucaauguu guuugucacc aaagugucug caucagcuuu ccuucuucua 69600
   cugauagcca cuugcaaaac cuccuuaaac uagcuguuuu cgagaugguu aacucaaaug 69660
   ccuuguauua agaagguuca guugaaccug agaugaaauc aagauguuuu uagucacauu 69720
   ucugguaauu auuccacagu cuguuaccuu ugaauauaua acuucacacg gaggucugac 69780
   aaacugguau uuguuaagga acaaaucacu cccuugcuug auuuauuccc uagugagugu 69840
   cauuucuagg auuuguaaaa acaagcuuua aacagaugua acaaaauaau auuucaugau 69900
   auuacaaggc ugcuauucgc aaagauuacu ugcuucuaua ucuuuuuucu cugcuacuga 69960
   ggcagcauaa auagcuuccu ccauguacug cccucaggga aagggugaua caaggagagc 70020
   uucaaaacaa gcucucgaaa gucuuaguag aaacaagacu cuuuaaagcc agaauaaucu 70080
   aaauucauua gaaagguccg ggacuuccca gauguguagg uuccauuaac cagagucuaa 70140
   auaaaguauu uagaguagag aguuuauagc acaaaugcug gagaucagga ggaagcaguu 70200
   aagaccagug gcugcuuucu uacccugaag gccagcgaug ggaaggacuu cccauccuug 70260
   gaagagggcu ugaggggugu ggacagagca agggccaugg gcaaugggag ggcauggugg 70320
   uuuuacaaag gggcaaguca caaccugggg cugccauggg ugcagauagg ugucuauugu 70380
   cuucauuccg aaagaaaaga ugcuguccau guccccuucu cauauccugc cccuucccuc 70440
   cccuccacua ucccuccaga auuuacacca cuuucugaaa aggggaauuu ucucuccaug 70500
   cuccaugaga gcagccugga guucuccaac aauguuuccc agagugcguu ucacagaaca 70560
   cagucugugg aaaacagacc ucuuggcuca cccugcacau uagccaauua aagcuuuuau 70620
   aagaguucug ugcuuaacag ggucaugucc uaguguuucc ccacagcacu ggaccauaga 70680
   aauccauuaa acacagacac cucuuucccg ggaaucauac ugacgcccug ugaggcuagc 70740
   uagcuguugu gugaauggca cuuuggagac caccaggcca gguccucggc uaugcgguca 70800
   gugugcccug gguuugccuc cuguuccagg ucuuccccaa auagggagaa uaccacuuag 70860
   uaucacugga gguaucagga gagacugugc cauguaaaau gcagaaacug gcaugucgua 70920
   agaucugaaa aaugccuugu cuacucccuu ucccaccucu ucucagauac cugagacaaa 70980
   acaaucgcaa auucccuuca caagcuacuu auuccaucau gcaaggggug agguuauagg 71040
   caguuugaga agacugcugc agucccaaga gucacaauga uagcagcagc aguucaugag 71100
   ugaugccagu uaaccuugua agugcuguau uuucaucaaa ggcauggaug auaaggacgc 71160
   cauuggguuu cacauuuugu ccucagauuc agagaaagaa cuagacucca ggacugaggc 71220
   cuugcugcuu gagacucucc agauguaaua auuggaggaa acuagacucu aaggguaaaa 71280
   agaacaaggg gagggaaucu gcccucgggu gggcaauggg cagcuccagg gagaggauuu 71340
   ggaguugagc cugagugcug ggacugggug cugcccuggc auccccacuc cccuccacca 71400
   ucuucucucc cuuugggcac agucaauagg gagcaaaacu ggccucaagg ucaacaugug 71460
   acuaauaaac auaaaucaga aaagguucaa ccucacuaau aaugcaagau auguguaaua 71520
   auuaaaauaa gcugcacuuu gggaacagug gugggcccca gucugguggc gccugagguu 71580
   uguacaacuu gagggucucc ucuaaaguug gagaagaaag agauugaagu cuuaccugau 71640
   uguguuaaaa augucuaacc uuugcaaaau uuuccgugug ugugugugug ugugugugug 71700
   ugugugugug uguguuuuag auggagucuc acucugucgc ccaggcuaga guauaguggc 71760
   augaucucgg cucacugcaa ccucugccuc ccagguucca gugauucucc agccuuggcc 71820
   uccugaguag cugggauuac aggcgugugc caccaugccc aguuaauuuu uguauuuuua 71880
   guagagaugg gguuuuacca ugugggccag gcuggucucg aacuccugac cucaagugau 71940
   cugcccgccu uggccuccca aagugcuggg guuacaggug ugagccacca cgcccagcca 72000
   ccuugcuaag uuuucaaaaa ccuaugugaa uacauuucca agaccuugga aggacccagg 72060
   uuuggggaag ggaugagguu ggggagcuga aguuuaaguc ucaguggcuu cagaauaaau 72120
   gcagcuuugc uggggagagu uugaaacagg gggagaucug ggcaaaaaac gugcuaaaug 72180
   aguuuuucuu ugcucauuug ucaucacaac auguaauucu cuggguacgu cucaagaugu 72240
   aaaugauucg gauuucaguu uuauuuuaaa uuacccaaau uauuauacug uacuagagaa 72300
   gacagaaaac cugaaggcaa ucacuucuca ggaucuuuac aaaggaaaua uccaaguguc 72360
   acaccuuauu auuuuaauaa caaaauggug auauauuaaa agcaaaucuu gauguucaac 72420
   uuuucaaagu uaugauccca cugcagaaug auaauuaaua uucuuuacuu ucaggagcac 72480
   uuuuaggaaa auacagacug agugaggaca ggcaaacuua uaugccgggu ggugaauuuu 72540
   aaagcaaaga aagccaaugc uauuaagaug aaaauaauug cguaugauuu ugaaucuuaa 72600
   agcauuacca uauguuacuu ggaacuccca acauuagaaa aaauguagga auggaaaaaa 72660
   agucuguuuc auuuuuaacc auuuauuucu ugagaugaua uuggaccaug cuuuguuugu 72720
   uuuuauaauu ucaacuuuua uuuugcaugc agaggguaca uauagaacau gguuuuaaaa 72780
   ugcacaucaa uagcagcuuc agcuuuucau aauuauaaga agacuuauuu aaaaauucua 72840
   uaguauuuuu gcaguuacaa auuuccaggg acuaacggca aacaaacaac aaauaaaaac 72900
   aaaagaagcu guaaccaaug agcugcaaaa accaccacca ccaccaccac aaagacaauu 72960
   acuguagaag acaaggauaa aaaugaagac uuaagaugac ucugaagguu gaaggguuaa 73020
   uucaggguca cuauuaaaua agaauaugaa aauccauugu cacuuaauua ucugguuuau 73080
   gcaaccugag aacucaagcu auauuaaaaa ccuaauaaug cggggcagug accugggcuu 73140
   caguagauca ugaaucuuuc accauucaaa uuaagggugc guuacuaaca cugaucuuuu 73200
   ugcaugcuuc acuucagcua uuuaauuaaa aaugccaaua gccuaaaggc cagauaacuu 73260
   uucagaaauc aaaaaauguu agcauuuguu gcccugucuu caaucuguuu cucagaucca 73320
   cugaaguugu uuccuguagg uugacaaaug aucuggcaaa cacuauguau gaauuauauc 73380
   cagacuggcc auauucugga uaucaacuau aucuaagauu ucuaccaaau ucuguucagc 73440
   cuuuccaacg uaacacggca agucagggag agacuaaacc uaaaaguccu uccuuggcac 73500
   ugaaggucgg cuggaucccc agaacuuuaa aucuuugggu cucccagagu uccugauuca 73560
   uuuucaggug ccuuucaccu uguuaccuga gccuacagug cagagucaag aagccaucuc 73620
   agaagguguu caauuaauag agaaggcucc cagcuggagg ggauuuauau guucuauuca 73680
   guuaaugaaa acaacccuuu aaaagcuaua uuugaaaaau ucaacuaggg uucauucauu 73740
   cauucaacuu aagauugcca gauuaaauac aagacacuca gugaaaccgg aauuucaugu 73800
   aauaaauuau uucuuaguac aagugugucc caaauaaugc augagauaua uuuauacuaa 73860
   agaaauacua ugucauuuuc uuaaaaaucg aauuuaucug gguguuauau aucuuuauuu 73920
   gcuaagucug gcaauucuaa cucaaucuua uacuaauaau agccagcaac aauaccuaca 73980
   uagcacugac uacacuuggc acuuaaaaug uuugaugugu uuuuaauucu ugcaacaacc 74040
   ucauaagaua gguauuauuu ccauuuuuca gguaugaugu gaaacuggaa gcacagagga 74100
   aucaaacaaa cauugucuua ugaaauucag uaucuuccac cugaaugccu aaagccugga 74160
   ugguugccua ucuugcagag augggcaggg ccaagguuuu ccacagaaaa caguucugag 74220
   cugcuugaac auaaggacaa augcuugauc aagguccuua acaauuuuug gcauggagug 74280
   uuguuguaua auuguagccu ggaaaauauu cacagcaggu guuaccaauu gcccucauaa 74340
   ggauauugaa uuuuaagaaa uuaaauucug ggugagucuu ccaagauaaa cuggcugcaa 74400
   cgcaugccuc uuaguagcag ccaugguuug aauguuugcc ucuucccaga cucacaguga 74460
   gaucugauug ccauuauaau gguauaggga gaggagaccu uugaguggug auugggcugu 74520
   gagggcucug uccuccagaa uggauuaaug ucguuaucau ggaguggauu cauugguaag 74580
   uucagacccc uuucucucuu ucucucacuc ugcugccuuu cgccauauga ugacauauca 74640
   agaaggcccu caccagauac ugguaucuug auauugaacu ucccagacua aagaacuuug 74700
   aaccaauaaa uuuguuuaug auaaauuacu cauucucaaa cauucuguua uagaagaaca 74760
   aagucaacua aaacaauagc ugaugauuug accugcuauu uguuugacau acgcuauuca 74820
   auugagcucc aaugacccca aaaaugaaga uuucuguuuu aacauaauuu cauuuauuau 74880
   gcacuuccca ugugucagau cuaggaaaac caaagauacc uaaggcagag aucgaaaaaa 74940
   aaccaaacca aaaaaacaaa acagacaaaa aaaaccacau ugagagaauu uaaauguaau 75000
   auauugaaug uuaggauaga gauacaggca gaaucuacac aaaggcagag cagagcaggg 75060
   ugaugcaucu ccaaauuaag gggcuauaag gaugucuuug uggaggaaau gauggcugaa 75120
   cuaaaccgua aggaaugaaa aaguguucau ggggcaggaa gugcggaagg ugauuguagg 75180
   ugaagaaaac agaaaaaugg agagaaccuu gucucucauc aucauuaggu agcuagaucc 75240
   aucauccuuu guguaaauuu cucguggaaa aaaaaaaaag ccacagguac agcccagagg 75300
   agaaugucug guucugugca augucaggag acuacgagaa ggagcacagu uuuacucugu 75360
   uauuucaaga cucuccucau uuuaacauua uuuuugauag uacugaaaua gccuaggcau 75420
   acgacuaggu cucaggguau guugcaugga gcaaagacaa agucacagaa aauguuggug 75480
   cuggccaggu gcgguggauc augcguguaa ucccagcacu uagggagacu gaggugggca 75540
   gaucacuuga ggacaggagu uugggaccag ccuggccaac auggugaaac cccgucuuua 75600
   cugaaaauac aaaaauuagc ugggcauagu ggcaggugcc uguaauccca gcuacccaag 75660
   aggcugaggc acaagaauca cuagaacccg ggagguggag guugcaguga gcagagauca 75720
   cgacacugca cuccagccug ggugacagag ugagacuuug ucucaacaaa auaaaaaaag 75780
   aaaauguugc ugcuaucuuu ccucauucug uacuuaaaag auggaauucu acacccuaua 75840
   gacgcuauuu ucucgaagua caaaaguuuu uaaagcuaaa uacuuucucu gagaguuucu 75900
   caacuuuaga cauuuagcau uagauaggga gucuuuaaaa uguuuucaaa ugugaacucu 75960
   cucuaguauu uucauguuaa gcugucggua aggagugaaa auaaagacuu cgaaaagaac 76020
   uggagcugau guugguuaga caucccaaaa uaucacaaaa acguuggcuu caauguauuu 76080
   aauuuugccc cuuaagagaa gucuuauucu aggccuggug ccuguuaccu agcgaagaaa 76140
   uguuggcugu guccucaaac uuccaacucc caucucauaa uuuuaaguuc acaaaaaaau 76200
   cuccuuaagg aagcuuauag gauauacuuc aggcacaggg caaugagcgc uagcccggua 76260
   aucaaugaaa cccggguuug cuccugguuu uguuucuuac uugggcaaau caaaccguug 76320
   aguuucuccu uuucuuccuc ucuaaaauaa gacaacugaa guugacacuu agucuaaaga 76380
   auucuaaugc uauaacuuga gcuguuagau gguauuacuu uuaccugguu gcauuucaaa 76440
   ucuuuggcaa uuuagaaaaa ccauaugaga acagggauag uauccaggga acagaugaca 76500
   augccuucca cccauccuaa uguauaggaa uuuagaaauu aagaaacacu uaaaauaaaa 76560
   uuuccauagc uuuauauauu acaacagaag ugagaauaau uaguauuucu gauacaaaca 76620
   ugacaggucu acuuuauuca aacgcacagg ugaguucuuu cuguuggcua gaagcuggug 76680
   aaaaccucua acaauucuaa auaagcaaau uacaaaguug cucuacuaau gaguuguaag 76740
   aucuauuuga uauuuuacaa caauguauau gguguuuuaa ugaauugaga ccuuauauuc 76800
   cauauaaagu uuuuuuuuuc uuccaaauaa agaugguuuu acuguuuuac uggaccaagu 76860
   ucagauaaua uuaacauucc uggacaaauu ccaaacuuuc uuugguuuuu aguaaucaua 76920
   uaggaauaag agugacuaca acaguucuau uaauacauuu acuucuguca gaagccguau    76980
   uagcaagcau aacaaggaaa cauaagaaaa uaaacacuga aaauuggacu uuuaaggcau    77040
   ccgucauagc uuauggauuu uauuaacuaa augccaaaau aauuaaauua cugugaaauu    77100
   uuuacuaaau guuuauuauu uacuuuggcu uaaaauaaaa aacccauuga guuuauaaua    77160
   ucucauguuc caaaauuaau auauuugauu uacgaaaaua aaaacauauc gcaaugaauu    77220
   agguucacau cugaaauuca cagacuggcu uugacacugc uugugcauuu uggaaacucu    77280
   ucuagcuucu cagaaucuug guuucaaaac ugaaaagauu ggccugacaa uuuuaaaacu    77340
   gucuucaagc uaugaaaguu uaugauucag gaaacagcuu ucaaaaacua cugagaggac    77400
   ccugaaacuc uucugcuaga ucgauuccaa auaucuagug uaaaaaacau ccaguguuuu    77460
   ccaacaugac ucagugggug agacuggguu uuuuaagacc aagcaaaacc gauaaaggaa    77520
   gcuuagaagg uuugucuucc augaaaaaga aaauugcuau ugucuuaaau aaacagaagg    77580
   uauguuuucc uuugaauugc acuuagcuuu uuaauagcua auuucagcua cagaaaaccu    77640
   uuuuuuuuuc uauuuuaauu aaguuuucaa ucuggaacuu ucuugagaaa ggcacuuuag    77700
   caacucugaa gaacaguaga gggcgccacg gucacaagca aagcaaacaa acaaaaacgu    77760
   uggcaucaca gacugggccg ggaagugccg aggaguacac cugagaaaau aagccacagu    77820
   cagguaggaa caacacauuu ucaaaaugca uuugggaaac ugccagugaa aauggucuac    77880
   guuuuucucu uuuuuuuuuu uuuuugagac ggagucuugc ucuguugccc aggcuggaau    77940
   gcaguggcgu gaucucugcc cacugcaacc uccuccuccc ggguucaagc aauucuccug    78000
   c                                                                    78001
<210> 2
   <211> 22605
   <212> RNA
   <213> Homo sapiens
<400> 2
   auggcucugu guccccaccc aaaucucauc uuguagcucc cacaguuccc acauguugug      60
   ggagggaucc aaugggagau aauugaauca ugggggcggg ucuuuccugu gcuguucuug     120
   ugauagugga uaagucucac gagaucugau ggguuuuuuu uuugguuugu uuuuuuuuuu     180
   uuuuuuuuga gacggagucu ugcuguugcc caggcuggaa ugcaguggcg cgaucuaggc     240
   ucacugcagg cuccgccccc cgggguucac gcgauaucug augcuuuuaa aaauaagagu     300
   uugccugcac aagcucucuc uuugccugcu gccauccacg ugacuuucuc cuccgugccu     360
   ucugucauga uugugagguc uccccagcca cauggaacug uaaguccacu aaaccucuuc     420
   uuuuguaaau ugcccagucu cagguauguc uuuaucagca gcgugaaaac agacucauac     480
   aacuaacaag ccugcuuuag cuuuucacug uucugcccaa gcaacuuucc ccuucagcca     540
   ggccaguaga uuucucauac agcaggguga cccuguucag ggccuuugua cuguuuauau 600
   uuuuuacuuu gugcauacua ugaugucuug acguuuugga aaccucucug gcuggggaga 660
   gacuguccuu cccagagcug gccaguucuu agaaccagaa gggcccagcc aggagcacag 720
   cucugauaug caaaccaacc aacccagagc uaugccgucu uuaucuagcc uguccacccc 780
   aggaagcagu auuucuucac cuauaucaug ccagagccug auaacaagca acuagaaacc 840
   augccuguag cuuacagccu gccaaaauua uucaaacuag ccaauucuaa acuguucaca 900
   ugcacucccc ugcccugucc cugaaagcuc aagaaagacc auggcccagg cucuccccuc 960
   acuccugccc cuucaggcuc cuacaggggg gaccaagacu gaugcuuccc cauguggccc 1020
   ugcagggcau gacaugcuuc ccgucucuaa gaccugugag uauaauaacc uuggccuucc 1080
   ugagcaccuc cucugccucc uccuguggcc acaucucacu gaccaucaca aaaaacaaca 1140
   uagaauaagc caggcauggu ggcuuaugcc uguaauccca gcacuuuggg aggccgaggc 1200
   aggcggauca cuugacguca ggaguucgag accagccugg ccaacauggu acaacccugu 1260
   cucuacuaaa aacacaaaaa uuagccaggu gugguggugg gugccuguaa ucccagcuac 1320
   ucaggaggcu gaagcaggau aaucacuuga acccgggagg cagagguugc agugagccga 1380
   gauugugcca cugcacucca gccugggcaa cagagcaaga cuccgucuca aaaaauaaaa 1440
   uaaaacaggu uuucauuuua auuuuuaaca uauaaaccuc cacaggaaca uucugacaca 1500
   gacuucagcc ccaggccuac aucacccuau gauccaaauu auuuuauagu aaaugguaug 1560
   aaguuuggag aggaagggca auuuggccau caugcuguga uuuuacugca gguaaaguag 1620
   aaucaccaug aaaguggugg agccuugcuu uuucuuuaau uuuuauaaug ccaccacccu 1680
   acauucuuag gagaaaaggc aaggaguuaa auaaauaagc acagauuuaa guucacauuu 1740
   cugguaauua uccgcaagcu aauaacugag auugcuuaag cucuguaaac aaaacacaca 1800
   aagcccuugu ggcucugcgc cagaaucaaa ggucugaucc aaaacucccc cauccccuug 1860
   uuggcuuuga acaugaaacc aauuuucuac accaaaucag gccuacuucu uuguaaccau 1920
   ccuuaccauc ucucuuauag gaaaauguuu aggacucuug gcggugacuc uagaacuagg 1980
   aaaucaacuc ucucagcagc acucuaauga gcaaaaacaa cugugccuca aaauacaggc 2040
   ugcagugggc agaaaucucu guacugcacu ccagccuggg ugacagagua aguuucuguc 2100
   ucaaaaaaaa aaaaaaauca aguaacaaag aacagcuguc uaaaacuguu aacacugauu 2160
   caaaauauaa uaaccuuuau gccuguuauu cagaguuaaa guggguacgc uaauuauaaa 2220
   ucauacacuu uucuaauugu gcaauacucu gucaauggga uagauuuuag gaaaauucca 2280
   gcugaaccag uccauucuuu gaaucauuag gucaauuaca aacaugccaa uccuuagccu 2340
   gaauagcugc auauuuagaa agaccuaaaa cauucuucaa aaagcugcca uacauggucu 2400
   guuaaagggc uuacuuggua ggaaauaaau caagagggaa uacuccauag ugaaucucag 2460
   ggcacuuuuu aaaacaaauc ucagggcuga gcacaauggu ucaugccuau aaucucacug 2520
   cuuugagggg ccaaggcagg aggauugcuc aagcccagga guuugagacc agccugggca 2580
   acauggcaag accuaguccc uacaaaaaaa uuguuuuauu agcuaggcau gguggaacac 2640
   gccugugguc ccagcuauuc cagaggcuga agugggagga ucacuuaagc ccaggaaguc 2700
   aaggcugcag ugagccauga ucucaccacu gcacucgagc cugggagaca guacaggacc 2760
   caucuccaaa aaaaaaaaaa aaaaaaguga acgaagcugc aauaagcugu gaucauacca 2820
   cugcacacua gccuaggcga cagagugaga cccugccuca aaaaaaaaaa aagaaaaaaa 2880
   gaaaaaacca auugauaaaa gaguaaccuc ccaaucccca gcuguggaac ccaagagcag 2940
   agaaggcagc cacccgccuc ucuuucccuc aaagacacca gagugcaaua ggcucuuccu 3000
   cacucauucc caugggaagu agccuucaac cccucucaaa ccacagcaua uagagaugcc 3060
   uuuauuaaua aaaaaggaua caacccacgg gccagccgca guggcucacg ccuguaaucc 3120
   caacacuuug ggaagccaag gcaggcagau caccugaggc caggaguccg agaccagccu 3180
   ggccaacaug gugaaacucu gucucuacua aaagcacaaa cauuaaccga gugugguggc 3240
   acacgccugu agucccagcu acuuaggagg cugaggcagg agaaucgcuu gaauccggga 3300
   ggcaaaaguu ggaguuagcc gaagucgcac cacugcacuc cagacugggc gacagagcga 3360
   gacucagucu caaaaauaaa uaaauacaau aaauaaauaa acaaaguaua caacccacaa 3420
   cucacuuuuu uuucuaacca ggaaucucaa caacaggacu auagggaaac aagaauuauu 3480
   aaccucauua auucaaauua acucuuaagc acccaccacu aacaggcacu cuuauagguu 3540
   cuagaccuag gaucauuuuu acagaguuca cauuggggaa gggagagggg agagggaaag 3600
   gcaaucacac uaaaaagagg gggucagagu aacaguaaga uccacacaga aggccaggca 3660
   caguggcuca caccuauaau ccaagcacuu ugggaggcca aggagggugg aucaccugag 3720
   uucaggaguu cgagaccagc cuggccaaca uggugaaacc uugucucuac caaaaauaca 3780
   aaaauuagcc gggcauggug gcgggcgccu guaaucucag uuauuuggga gccugaggca 3840
   ggaggaucgc cugaacccag gagguggagg uugcagugag cugggaccau gccauugcac 3900
   uccagccuag gcaacaagag ugagacuucu caaaaaaaaa aaaaaaaucc acaaagaaag 3960
   uagggagcca ugguagugaa ugggggcguu uacucuagau ggguggucaa ggcagaguuu 4020
   cccaggagga caaagccccc agugacaaga gcagggucaa caaguuccaa gcaaagagaa 4080
   cagcaagugc ugaagccuaa ggcuaaugca cuuacagaaa aauccuuaau gacuaagugu 4140
   guugccucug uuugcgcaga gaucacaaaa cgugucccgc agaagggaga gaaaaaggga 4200
   ccccaggaga ggguggcggg gcuucagguc cuggagaaag gcagcaagca uggcuaacac 4260
   auuccuuggu ccacccugca ggguaauacu gcaccuucaa uugacuuaug aagaaacuag 4320
   cucagauugg aacaacagcc aggacaguca ccaccaaggg gacuugcccc acuggagaag 4380
   gcaagucuga cccccuaccc caccgcgguc aacccaggga cggaggagag aacgccgcau 4440
   gccauuguuc uuauguauaa agcacagguu acagguuauu ucugaagaug ucugcucaag 4500
   uggagaugcu accagguuuu uaccaaucau uacuuuggau aaaaguuuag caaauauuua 4560
   caucacugug cccacaguuu gugcuuaugc cccauggcuu uagagggccc guauaccuua 4620
   uguauacuuc auauuaugua uaauaugaac uucagcucca uucaugguca uaucaucuaa 4680
   ccugggauac ucuuaggagu gaaaggggga gcuuaauagu ggcaguguga uaagggauau 4740
   aaaccaggau ucuccugggc caacugguac aggugaaaag gaugacugaa acggcagagg 4800
   uccuccauuc uucuagaugg gugaucaagg cagagguucc caggaggacc aagcccagac 4860
   agagaggcaa ggggcggugg cucacgccua uaaucccaac acauugggag gcugaggugg 4920
   guggauugca ugagccaggg aguuugaaag cagccugugc aacaugguaa aacccugccu 4980
   cuacaaaaca aaaacaaaaa uuagccaggc augguggugc augccuauag ucccagcuac 5040
   ucaggaagcu gaggugggag gaucaccuga gcccgagcac agggagguug aggcugcagu 5100
   gagcuaugau cgcaccacuu auacuccagc cugagagaca gagcaagacc cugucucgga 5160
   aaaaaaaaaa aaaaaaaaaa aaaaaacaca gacugacaac aacucaauga acucagauaa 5220
   uuagaaauuc uauuagucua auuucauaug aaaccaaggu auuuuuuuuu uccuuccuca 5280
   gagaccggag ugggagagaa acuguuacag ccaaucacuu ccuuccagga aaaaaaaaga 5340
   cgcaaauaac aacacaaaaa aacgugaucu aauagacaau ccagauauaa agaacacaaa 5400
   cccuuuuccc uacaauuuaa ccaaaugcua aaaugcacca ggaaacacag gauacuacag 5460
   agauaaagau uuggccagca gauuaucaac cccaggaaaa gaaauccauu ucuguagcau 5520
   gcuaugcugu uggaggcaca cgucacacuu cuuaauucaa ggacagaaaa gcacaaacua 5580
   aaaugcacac uggaguauga cucccccuuc ucuaucugcu aaagcgcuuc gcaugugacu 5640
   uucaaucucu aagcugggaa ucaaucaggc agcccagccu ccuauauuuu cuaguuguau 5700
   ugcuuugagc aucaaauuua accucugugc accucaauuu cuucagcugg agacaaagaa 5760
   aguggcugca cuguggcaga guguuugcaa auguuaauca ugguuauggu agcauucuac 5820
   cuugucuuac agaugguugc cuaaaaaggu aggaaacugu ucacgaaaaa gucucaaccg 5880
   guccuagagu gaucagaaug acagcgcuga aauaaggagg gcaguaccuc uuauuccaga 5940
   gaagugggga gcagagagga agauggagug gaaaggggcg aggcaaggcc cuccugaaau 6000
   accucaaccc aaaucuucaa gaaaucccca aguccucaca gugcuuuuug uggauuuuug 6060
   uggaaaccgg uaaaaggggc ugauuugcug accccagugg guagaaaaca gagacuguca 6120
   agagaacaga agagaaggca gaaaggggau ggggaagugg gguucgccau guucacgagc 6180
   uccuggagcc acagggcccc ccaggaacaa cagagcugag acuggguggc cuuguuucug 6240
   gcccaauucc cugggaccac cacccuggaa ggacacuuga gcccuacuca aggacccacc 6300
   acaacaucuc agcauugucg guccuuaaau gccaagguuu gugcucacca uuuccauccu 6360
   uaagauucuc acagggaaga acccacacaa cagcuuuguc ccugcgacuc ugggugaguu 6420
   ucugacacug gccugggcuc ccguuuugcc cgucaccaac uucucacagu auaacuaucc 6480
   aagagaagua gcauuugaug ucagggaauc aaaagggcua aggcucggga ggccaaggca 6540
   ggugaaucac uuguaaucag gaguucgaga ccacccugac caacauagug aaaccccauc 6600
   ucuacuaaca auacaaaaaa uuaaccaggu gugguggcag gugccuguaa ucccagcuac 6660
   ucaggaggcu gagguaggag gaucacuuga acccaggagg cagagguugu gguacgccaa 6720
   gaucaugaca cugcacucca gccuggguga cagagugaga cuccaucuca aaaaaaaaaa 6780
   aaaaaaaggc cuaaggcuua cucaggguca ucuaugguuu gccaagcaga guucaaucca 6840
   aucaacaaaa cccucacugc ccacuuuucc aaaaccugau auagucaguc acuaguagcu 6900
   gccauuugcu guuuugccuu cuauuaaaaa aaaaaaaaaa aaaaaaacca uccaauuccc 6960
   agaagucaaa ggcaauuccu ucccuuuucu gugucuguga gacggcucuc ugugggauuc 7020
   cuuccugcug caccucaucc uuccuggaag ggacucucag aaaaugugcc uccuccuuac 7080
   aggaaaggaa uuccucccga ggaacugugg acaugaggca gcuuugugga auuucccuuc 7140
   cacagacuuc agccuauuuu cugggcaaac uuagcagaaa ugaccaaaau ccacucgaga 7200
   gagacagcaa caaggagagg ggaagagauc cuauuuuggc uucuucaaau ggaccaauuu 7260
   ucucuuuaaa auauuuauuu uaaggaaaaa caaggacuac auuauauuuu aaagggggag 7320
   aaggaagagg auuuugaguu gcuaagagaa cagagccauc ucuuuucuuu uaauacugga 7380
   ugcuaagaac uugcacaggg uuuucccaaa agccagauag uucagguaau uugcaugugg 7440
   cuguaaaaga gaaaguucag gccaggcacu guggcaacau ggugaaaccc caucucuacu 7500
   aaaaauacaa aaauuagcug ggugguggug ugcaagccug uaaucccagc uacuugggag 7560
   gcugaggcag gggaaucacu ugaaucuggg agguggacgu ugcagugagc caagaucaca 7620
   ccacugcacu ccugggcgac agagacucuc ucaaaaaaaa aaaaaaaaaa aaaaaaagag 7680
   agagagaaag uugagagcau aagucugagu gauucugccu gaaagaacag aacagcuuac 7740
   acuggacagg cuuuugccca agacacaccu caucuggcau ugagaaacga acaccaacaa 7800
   uaaugaguga cagaauguua agaacaauaa uuauacagac uuggaccucc cagacugcuu 7860
   gucuguagua auucagaagg aagcaaagua uucugaagug uuuaugaaga uugguuccac 7920
   aucugaagaa uuuuaguaau cuuacccaaa cucaacccaa gaaauaugag gaaaagaggg 7980
   aguaguucua uaauaagcag guggaauaga gggucaaacu ccuuggcgag uucagucuaa 8040
   cagaggacac auuacugccc uacaaagcag cucuggaaaa ugacaaccag uggcacuaac 8100
   uccaggcaau aucuuuguga ggcuuguggg cuguaauaua gucugcucag gcugccauaa 8160
   aaaaaaaauc acaaucaggc caggcacuau ggcucacacc uguaauccca gcacuuuggg 8220
   aggccaagga gggcagauca ccugagguua ggaguucgag accaggcugg ccaacauggc 8280
   aaaaccccgu cucuacuaaa aauacaaaaa uuacccggau gugguagcac gcaccgguag 8340
   ucccagcuac ucgucaggcu gaggcaggag aauugcuuga accugggagg gguggagguu 8400
   gcagugggcu gagaucacac cacuguacuc cagccuggac aacagagcca cugcacucca 8460
   gccugggcaa cagagagaga cuccaucuua aaaaaaaaaa aaaaaaaucc ccgucuucaa 8520
   auacaaucac auucugaagu acuagagauc aggacuccaa caugcgaacu uuggggggac 8580
   auaauucagc ccguaauaug ggugauaaag gagcaggucc agacuuagag aaacaaggac 8640
   ucccuggguc aagcucugga agugaaaggc uucuucaaag acaggaaagu cccacugaag 8700
   guggcuuugg aagugcaccc uugcagcaca gcaaccuucu ugauuugcuc aagagcaggg 8760
   guucugacag gucucaaagc acuuuguuua augaaacaga aucgaaaaua ccaguguguc      8820
   acauguuucc uaagacagga cacuguuucu ugaaacuuuu acuaaaguau guuuuucuuu 8880
   uauuuuuuua uuuuuuauuu uuuuugagaa ggagucuugc ucugucgccc aggcuggagu 8940
   gcaguggcac aaucucgacu cacugcaacc ucugccuccc ggguucaaac aauucuccug 9000
   ccucagccuc cugaguagcu gggauuacag gugcacgcca ucaugccugg cuaauuuuug 9060
   uauuuuuagu agagacaggg uuucaccaug uugaccaguc ugcucucaaa cuccugaccu 9120
   ugugaucugc ccaccuuggc cucccaaagu gcugggauua caggugugag ccaccguguc 9180
   cagccuaaag uauguuaaau auguuaugug ugagagagag aaagagaaua uaucucuugg 9240
   ucauuacaua uguaaaacuu guagaacgug aucaaaaugu uuaaaaacug gccaggcaca 9300
   guggcucaag ccuguauccc agcacuuugg gaucccaagg cagguggauc acaaggucag 9360
   gaaucaagac cauccugguc aacacaguga aaccccaucu cuacuaaaaa uacaaaaaau 9420
   uagccuggcg cgguggcacg caccuguagu cccagcuacu ugggaggcug gggcaggaga 9480
   aucgcuugaa ccugggaggc ggaggcugca gugagcugag auagcgccac ugcacuccug 9540
   ccugggcaac agagcaagac uccaacucaa aaaaaaaaaa aaaaguuuaa aaacuggucc 9600
   uguggcagug ggucucaaac aucagcaugc aucagaauca cuuggaggac ugauuaaaac 9660
   acaggcugcu gcugaugcug cuaauccuag agccaccuug aaguuuuucu gugguuuuuu 9720
   uuuuuuuuuu uuuuuuuuuu uugagacagg gucuugcugu cucccaggcu ggagugcagu 9780
   ggugcaauga uaccucacug cagccuugaa cuccugggcu caagcaaugc ccccaucuca 9840
   gccucccaag caacuaggac uguagcuagc uccuaacuca uucccuccau uccugcccuc 9900
   cccuaacuuu auauugcagc cagaaggaua uuuuuucuga agagcaucuc augcugagug 9960
   gcagcagaau gcagagauua agagaggggg ccaggcacag uggcucacac cucuaauccc 10020
   agcacuuugg gaggccgagg uggguggauc acccgagguc aggaguucaa gaccagccua 10080
   gccaacaugg agaaaccccg ucucuauggg cgugguggug cauaccugua aucccagcua 10140
   cuagggaggc ugaggcagga gaaucacuug aacccaggag gcagagguug ccgugagccg 10200
   agaucucguc auugcacucc agccugggca acaagagcga aacuccgucu caaaaaaaaa 10260
   aaaagagugg cgccaggugc aguggcucac gccuguaauc ccagcacuuu gggaggccga 10320
   ggcaggcaaa ucaucugagg ucgggaguuc gagaccagcc ugaccaacau agagaaaccc 10380
   cgucucuacu aaaaauacaa aaaaauuagc cgagcguggu ggcauguguc guaugccugu 10440
   aaucccagcu acuggggagg cugaggcagg agaauuacuu gaaccuggga ggcagguuuu 10500
   uuguuuuuug uuuugguuuu guuugagauu uugguuguug uuuuuugggg uuuuuuugag 10560
   acagggucuc acuguguugu ccaggcugga gugcaggggu gcgaucaugu gucacugcag 10620
   ccacaaccuc cugggcucag gugauccucc caccucagcc uuccugagua acuguuacua 10680
   caggugugca ccaccaugac uggcuaauuu uuguauuuuu uguagagaca agaguuucac 10740
   cauguugcac aggcuguuca caaacuccua ggcucaagcu auccaccugc cucagccucc 10800
   caaagugcug ggauuauagg caugagccac cuugcccagc caacugagcc acuuugugcc 10860
   aggacuaggu cagguacuug ggacauauac aggcaaggac acugcccaca gggaacugac 10920
   agugucuagc agcuacacag augacacggc cuuuucggca gacaccccug uccccugugc 10980
   uuggcaaaca gcagagacuc aaaaauguau augguuugac ucccacugga acauaagccc 11040
   cuuagggcag ggaccagggu cacuguauuc ccagcaccua uaaucaagag guacaugacg 11100
   cugcagcuga gagcagggac ucugugcuaa guuaccuacc ugguuugaac cuaggcucug 11160
   ccacucaaag gcugugugac cgugggaaau aauaguaccc accccaaagg uuuguuauua 11220
   guauuuaaua auauuuauug guaagguacu uagaauagcc ccccugccua caggaagcag 11280
   uacauaauua uaaugucuaa auauacagua guuccaggcc aggcauggug gcucaugucu 11340
   guauuccuag cagaaugaga ggccgaggaa ggagaaucau uugaguccag gaguuugaga 11400
   ccagccugga uaacauagug agaucucguc ucuacaaaaa auaaacaaaa guaagccaga 11460
   caugguggca caugccugca aucccagcua cucaaaaggc ugugauuaga gaaucacuug 11520
   agcccaggag guagaggcug caaugagcug agaucacacc acuguacucc agccugggca 11580
   acagagugag acuccgucuc uuaaauaaau aaauaaauag gggccaggcg ugguagcuua 11640
   caccuguaau cccagcacau ugggaggcug aggcgggagg aucacuugag gacaggaguu 11700
   caagaccaac cugaccaaca uggugaaacc ccaucucuac uaaaacacaa caaucagcug 11760
   ggcauggugg cgggugccug uaaucccagc uauuuugggg gcugaggcag gagaaucacu 11820
   ugaacccagg aggcagaggu ugcagugagc caagaucaca cuacuacacu ccagccuggg 11880
   caacagagcg ggacuccauc ucaaauaaau aaauaaauag caguuccaua uccucugaau 11940
   aauggacuag aaguugguua auggaugggu uuucagggag aauuuuagca gccgagcuga 12000
   gagguaggua cucuagccag gguauauaac augagcuaag ggaacaggac agcaaauaua 12060
   aggugugauc aggggaaagg caucauauau ugacugucca ggaacaaugg auaucugaga 12120
   auguaggaaa gcuaaguuag gaccaggaaa aggagaguau uaaaugccaa agaagaugcc 12180
   accaaagguu cuggaacaga aagcagcaga gcuaugccuu guaaagauaa agccugcagu 12240
   guauugacug gguuggaauc uggaaucauu gcgugccucc agucaagacg uggcaacuga 12300
   ccaacuaccu uuuaucacug accagacucu ucccuacgug cagauaacug aggacgcaag 12360
   ccuuaguuac uggguguggu ggugcuauua acggaaaaag ggaagucagc cagcaaaggc 12420
   ugacuggggc aauuucagcc uuguugcucu gcugugaaac ugcuuuccag aaaaacuuuu 12480
   uuuuuuuuuu gagacggagu cucacucugu cacccaggcu ggagugcagu ggcacaauuu 12540
   cggcucacug caaccuccgc cucccagguu caagcgauuc uccugucuca gccuccccag 12600
   aagcugggau uacaggcgcc caccaccaca cucagcuaau uuuuguauuu uuaguaaagu 12660
   caggguucca cuauguuggc caggcugauc uccaacuccu gaccucaggu gauccgccug 12720
   ccucggccuc ccaaagugcu gagauuacag gcaugagcca cagugcccag cccaggaaaa 12780
   cuucuuaaag auguaauaau aacgcaccuu auuauuuucc aaguacuucc auagauagug 12840
   gcccacuucu gaguaugaau uaaauuuccc uuuguggccu uuguuuuuuu aauagaucuu 12900
   cauuucuggg gggacuuaca aacaggugac uucuccauuc accagagaug ggacauuaua 12960
   cacugaccua cuuccauauu cacuuucccu gcuugauugg aggugaggga gaaaaauuag 13020
   uguauaaggc cgggcgcgga ggcucacgcc uguaauccca ggacuuuaga aggcugaggc 13080
   agguggauca ccugagguca ggaguucgag accagccugg ccaacauggu gaaaccccuu 13140
   gucuacuaaa aauacaaaaa uuagccaggu gugguggcac acgccuguaa ucccaguuac 13200
   ucaggaggcu gaggcaggag aauugcuuga accccgaggc agagguugca gugagcugag 13260
   uccaugccau ugcacucuag ccugggcaac aagagugaaa cucugucuca aaaaaaaaaa 13320
   aaaaagaaaa gaaaagaaaa auaaguguau agaacuuuau uguuucauug accgggcacg 13380
   auggcucacg ccuguaacuc uagcacuuug ggaggccaag gugggcagau uggcugagcu 13440
   cagcaguuug aaaccagccu gggcaacaug gugaaaccua ucucuaauaa aauacaaaaa 13500
   auuagcccgg uaugguagca caugccuaua guuucagcua ccugggaggc ugaggcauga 13560
   gaaucacuug aacccagaag guugcaguga gccaagauug ugccacugca cuccagccug 13620
   ggcaacagag caaaacucug ucuccaaaaa aagagaacug gccagggaug guggcucacg 13680
   ccucuaaucc cagcacuuug ggaggcuaag gugggcagau cacgagguca ggaguucaag 13740
   accagccugg ccaacauggu gaaaccccgu cucuacuaaa aauacaaaaa uuagcugggc 13800
   augguggcag gcaccuguaa ucccagcuac ucaggaggcu gagacagcag aaucguuuga 13860
   aucugggagg uagaaguugc agugagccaa gaucacgcca uugcacucca gccugggugg 13920
   aagggcauga cuucgucuca aaaaaaaaaa aacuuuuucc uuaaaauaca ccucuuggag 13980
   ccagucccag auacuuggga ggaucaggaa ggaggaccuc uugaggccau aaauuugagg 14040
   auguagugug ccugugaaua gccacuacac ucuagccugg gcaacacaga aagaccucau 14100
   aaauaaauaa auaggccaga ugagguggcu cacaccugua aucucagcac uuugggaggc 14160
   caaggcuggc agaucacgaa guuaggagau caagaccauc cuggcuaaca cagugaaacc 14220
   ccgucucuac uaaaacuaca aaaaauuagc caggcauggu ggcaugcacc uauaguccca 14280
   gcuacucggg aggcugaggc acgagaauca cuugaacccg ggagacggag guugcaguga 14340
   gccgaaaucg caccacugca cuccagccug ggcaacggag ugagacucug ccucacacaa 14400
   auaauaaaua aauaaauaaa uauuuuauaa uguauuuuua aaauauaguu cuuaaaacau 14460
   gccaugagua gguuuaauuu uucuaaauca uagaauagga uacugaagcc cagagugguc 14520
   augugucuug ccuaagguca cacacagcua auauauacaa gaucaugggu cuucuggcuc 14580
   cagucugagg cugucacaau aacuagcuuc ucacuugguc cuaccuaaga cacagguguc 14640
   agcaacucua aauucaagaa auugagauua agacuaaagu aauagcuuau aaacauuaau 14700
   uaagcauauu aauugcaaga uguuaaacca gucacugaga gauuaagaca uugaagauac 14760
   ugguuucugc cuuaauagag cuaacaauuu aguaaaaaag ucgcaaauga gucaagacuu 14820
   aaauaagacu uaaagacuua agacaauaaa aaaggcauga uaaaagggua aauuuaggcu 14880
   ggacgcagug gcucaugccu guaaucccag cacuuuggga ggcugaggug ggcggaucac 14940
   cugaggucag gaguucaaga ccagccuggc caacauggca aagucccauc ucuacuaaaa 15000
   auacaaaaau uagccaggug ucauggcaca agccuguaau uccagcuacu cgggaggcug 15060
   aggcaggaga acugcuugaa cccagaaggu ggagguugcu caagagcaag acucugacuc 15120
   aaaaaaaaaa ggauggggga uaaauuuagc aagaacccuc aucaucccau uuaccuacag 15180
   ccuaauaugu acuaggaacu uacauauaca auaauaggua aaaucuauua agaauguauu 15240
   auguuuuaua guugaacaaa uugugucgcu uaaucaacuu agaauucaug uaaaugccac 15300
   auuugagcuc uguuacauug agagcucucc aggcccagcc uaccauguua agccauugau 15360
   aaaccucccu gcuacuauuu aaaacaagau ggauaccuuu cuacugucuu uaaagaggaa 15420
   gaguacacac uguuggacug uaugcacucu ccgucucuca agacaguaau cugagggauu 15480
   acuucaauag cagaaguuaa aagaagcggu caguuggguu uauuacauaa ucucaaucac 15540
   auccugacca auaaaaugca ccaauaaaga ucagcaaaua aaagcaagug auuagaagag 15600
   caauuuuaug cucacgagcu cugaauucac aagugugaga uuuccuguca aguacucuca 15660
   ugagcucaau uaaucucuga cccaggguga gaaaauaaac auagugacgc agaggcugca     15720
   gcucaugcuu uggaauuugu cagacuugac ucaguuccca acuccaucau guccugccug 15780
   ggugacccuc auuaugcuac cuggacucuc ucaguuuccu ucaccuguau guaacacaag 15840
   agcagagcca cccucaacaa gggguuacua uaaaaaucga agaacccggc aucucuaaca 15900
   acaucccugg cacggagcaa ucccccuucu cccucagcca cuggcugagc acccacagag 15960
   gggaguaagu ccuauugugg uaaugguucu caugcuuuga gaucagacaa guccuuugag 16020
   aggaaccuga aaaaggggca gcgaaccuuu ggccagaaaa auggaaauau gcacagaaau 16080
   auuuuugacu uuggcaagca guugaucagu gguucuuggg cuucagugcc caucagaaac 16140
   guagagaacc uuuuuaaaau auugaugugc ggggcgcggu ggcucacacc uguaauccca 16200
   gcauuuuggg aggccaaggc ugacggauca ccugaggucg agaguucgag accagccugg 16260
   ccaacaugga gaaaccccau cucuacuaaa aauacaaaau uagccaggca ugguagcgca 16320
   ugccuauaau cccagcuacu caggaggcua aggcaggaga acugcuugaa cccgggaggc 16380
   agagguugcg gugagccaag aucacaccau uccacuccag ccugggcaac aagagcaaaa 16440
   cucugucuca aaaaaaauag auagauagau agauagauag auagauagau agauagauag 16500
   augauagaua gauagaugcg uggaucccac ccccagaaau uauuuuucca auuggccuau 16560
   aaggggaaca gacauccaua uuuuuauggc uguccaggug auccaaucau ggugagggcu 16620
   aagaaacugc uauguaaucu aaaaagaauu uagaaaaaau aaagcuaaua agguuaaaag 16680
   aaauaaugca caucaacaau cauuugaagg gagcaauuug agaguauauu caugaaaagc 16740
   agcaccaaga gccagcuugu guuaagagga aucauggccg gguguggugu cucacgccug     16800
   uaaucccagc acuuugggag gccaagguag guggaucacc ugaggucagg cguucgagac 16860
   uagcauggcc aacaugguga aacccuaucu cuacuaaaaa uacaaaaauc agcuaggcgu     16920
   agugccacau gccuauaauc ccagcuacug gggaggcuga ggcaggagaa ucacuugaac 16980
   cugggaggca gagguugcag uuagccaaga ucgugccauu gcacuccagc cugggcaaca 17040
   agagcaaaac ucugucucaa aaaaaaaaaa aaaaaaaaaa aaaagaggaa aucgugaggc 17100
   caggcacagu agcaucgcgc cauugcacuc cagccugggc aaaaagagca aaacuccauc 17160
   ucaaaaaaau aaauaaaaau aaaaaauaca aauacaaaaa uuagccgggu gugguggcaa 17220
   gcaccuguaa ucccagcuac ucgggaggcu gagacaggag aaccgcuuga accccgggag 17280
   gcagagguug uacugagcug agguugcgcc acugcacuuc ggccugggcg acagagcaag 17340
   acccugucuc aaaaaaaaaa aaagaggaaa ccaugccaaa ugacuuuaac ccuuuucuug 17400
   guaggguuuc ugaaugacug cccaggcugg gcaugacggu ucacaccugu caucccagca 17460
   uuuugggagg ccaaggcagg uggaucacuu gagcucaaga guucgagacu agucugggca 17520
   acauggcaaa accccaccuc uacaaaaaau uagaaaaauu agccagcugu gguggcccgc 17580
   accuguaguc ccagcuacuu ggggggcuga agcaguagga ucacuugagc cugggaaguc 17640
   aaggcuccaa uaagcuauga augcaccacu gcacuccaag gcuggcuggc aaggcaagau 17700
   ccugucucaa aaaaaaaaaa aagacagaag ugcccaggug auccaauacu ccaauaccuc 17760
   uugacuucca uaaagcagac cuccauauga cacgaccucu cuugacacuu guggccaagu 17820
   uaaucuagca accacagugu ugauuagugg aucugccaca accuagagga cauuccuagg 17880
   acccauccuu ucuagcauuc agacuacgaa aguguuccaa gaacuuucaa cagcaacaau 17940
   gagcugcuaa acguuagcau cuacaaaaug aauuugcaua gggauagaag agcaguuaca 18000
   uggggacaag cccugcggac ugugagguca auuggccaug uguccaauau agcucaacug 18060
   cauguaucac ugaugaugcc caaauugaca uaaacuuccu gaccacauug gccaacauga 18120
   ugaaacucca ucucuacuaa aauacaagua uuagcugggu gugguggcgc gugccuguaa 18180
   ucccagcuac ucgggaggcu gaggcacgag aauugcuuga accagaaggu ggagguugca 18240
   gugagccaag aucauaccac ugcacgccag ccugggcaac acagugaaac uccauuuaaa 18300
   gaaauaauaa uaauaagaua aacuucucca ugagccccaa acucaugacc aauugaaugc 18360
   cuauucaaca ucuccacuug aauguguagc aggcaucuca aacuuaacag ggucaaaaau 18420
   aaacuccugu ccaggcacca uggcgcaugc cuguaauccc agcacuuuug gaggcuaggg 18480
   cgggaggauc acuugagccc aggaguucaa uaccagccag ggcaacaugg caaaacuuca 18540
   ucucuacaaa aaauauaaaa aauuagcugg gcaugcuggc acacaccugu ggucccagcu 18600
   acucaggggg cugaggugga aggauggcuu gagcccagga ggugaaaguu gcagugaacu 18660
   gagaucgcac cacugcaauc cagccugggu gacagaguga cacccugucu caaacaaaga 18720
   aacaaagauu uaauguggcc augauagcug ucuuugaaua cuaugaggcg agcauaugaa 18780
   gggagguagc caacuugcuc ugcguguuga caaaaaaaca aaggagagcg gcucugaacc 18840
   uauuuugguu cuaggagcug cuugauucac agaucauucu uugcuucauu aaauucugua 18900
   augcuuuucu uagaaguaag uaaacacaag uagaaauugc auggaaagaa aacuguggcu 18960
   caauacaaca uaauucuggc auucgaaucu aaagucauga gagucacaua augcauuucc 19020
   ccacugccca guaucuggaa gggauuccug uacugagugu gggcuagaag aucucuuccu 19080
   uucaacccac uaauucuaug acacuaagga aaaggggaag auaggagcua gcgcagagaa 19140
   aagagcagac agggacuagg ccugaaacag accagcacgg aaguggucau ggaacccaga 19200
   aauggaacaa ggagcuacgu cgcaggccac aaaagggcug uaaguacuca ugaguaaggg 19260
   gcuggaagug ggagaagcaa agucugaaca ucugcuggga ugaaagaggg gagggcaacc 19320
   uuggguguaa caaacgcuac cggcuggcag ggacgacaua gcgacugagg gguggucauu     19380
   cgccacacgu cucaggcaac acuuucucca acugcaguuc ugugaccugg acaucucauc 19440
   cccugccagg aacgauuucc caccuucucc auccaagcuc caacaggaag guaaaccccu 19500
   ccccagagau cuccaucuug gccaacaugg caaaacccug ucucuaacaa caacaaaaaa 19560
   auuagccagg ccugguggug cacaucugua aucccagcua cuugggaggc ugcggcagga 19620
   gaaucgcuug agcccgggag gcggagguug uagugagccg agaucaugcc acugcacucc 19680
   agccugugcg acagagacuc caucucaaaa aacaaaaaca aaaacaaaau ccccacccuc 19740
   acacagcuuu uccauacuuc uacuccagca cacccaacac ggguucagga ugaacuccug 19800
   cagcagcgac ugcuggacac acggacuaug uuuuuuugag auggagucuc gcucuguugc 19860
   ccaggcugga gugcaguggc gugaucuugg cucacugcaa cuucugccuc ccggguucaa 19920
   gcaauucucc ugccucagcc ucucgaguag cugggacuac aggugcaugc caccacgccc 19980
   agcuaauuuu uguauuuuua guagagacgg gguuucauua cguuggccag gcuggucuug 20040
   aacuccugac cuugugaucc accugccucg gccucccaaa gugcugggau uacaggcaua 20100
   agccaccgca cccagccugg acuacguuuu cuuuugcuuu uuuuuuuuuu ugagacagag 20160
   ucucauucug uagcccaggc uggagugcag uggugcaauc uuggcucacu gcagccucca 20220
   ccuccugggu uaaagcgauu cuccugcucg gucucccaag uagcugggau uacaggcaug 20280
   ugccaccaug aaagaaggca gggugccuuc uuucuugcca ggugcaaauc cauuacaaua 20340
   aaguaccauu ccuguucuca aagagcucac acaaugaggg aggcagccac uguaucgugc 20400
   aaauaaugcu agcuauuaaa caagcccuga uucucaaugg cuuaauucaa aaucaccagc 20460
   cgaggcagug gaucacauga agucaggagu uggagaccag ccuggccaac acagcaaaac 20520
   cccuucucua cuaaaaauac aaaauaaaua aauaaauaaa aaauuauccg ggcgugguuu 20580
   ugagcuccug uaaucccaga uacuugggag gaggcuaagg caagagaauc gcuugaaccc 20640
   gggaggcaga gguugcagug agccgagauc acgccacugc acucuagccu gcaagacaga 20700
   gauagacucc gucucaaaaa agaaaacaag aaaccaagac caugccuauu gucaugcuau 20760
   aguccccuuu ugggccaucu agggugguga acaacaccau acauuuacuc cuagcuggac 20820
   uuucaccauc uugcggcucg gccuccucuc aguccuccuc cucuccagga ggccucuuua 20880
   uucagcugga gaggcguugc auggggcugg uuuuuguguu ccagucgugg aagcgagaca 20940
   ccuuuugcaa ccacuuccua uuggccaggu cucaguuuca uggucaccuc ugaccacaag 21000
   guugggguag aaaacauagu ccaggcuggg cauggcggcu cacgccugua aucccagcac 21060
   uuugggaggc ccaggugggu ggaucacccg aggucaggag uucaagacca gccuggacaa 21120
   cauguugaaa ccccaucucu ccuaaaagua caaaaaauua gcugagcaug guggcgcaca 21180
   ucuguagucc cagcuauuug ggaggcugag acaggagaau cgcuugaacu ccggaggcag 21240
   agguugcaau gagccgagau cacgccacug cacuccagcu ugggcaacaa gagugaaacu 21300
   ccaucucaaa aauuaaaaaa uuaaaaacaa aaaauaaaau aaaaauaagg cacucaaaug 21360
   uuugcuaaau gaauaaucua aaccgcagag gaucaaggca guaaccuugg ccucccagac 21420
   accgaucaag uagaauugga caccaaagac ugagaaagac cugagaaacg uggcucccaa 21480
   aucucagcau cugaaucaac caggaaauaa ugcuuuaaaa aaugcagauu ucaggaugcu 21540
   caaccccaag agacugugau ucugcagaca cagaaauuua cuauuuggcc ggccacagug 21600
   acucacaccu gcaaugucag cacuuuggga ggccaaggcg ggagaaucac uugaagccaa 21660
   gaguuuaaga ccaacauggg caacacagcg agaccugguc ucuaccaaaa auguauuaau 21720
   aaaaauuacu ggccaggcac aguggcucgc ccccguaauc ccagcacuuu gggaggccga 21780
   gguggguaga ucacaagguc agaagaucaa gaccauccug gucaacauga ugaaauccca 21840
   ucucuacuga aaauacaaaa auuagcugga ugcaguggcg ugcgcuugua gucccagcua 21900
   cucaggaggc ugagacagga gaaucacuug aacccuggag gcagagauug uagugaccca 21960
   agaucacgcc accgcacucc agacugggca acagagcaag acuccguccc ccgccccuca 22020
   aaaaauuagc uaggugucca ggugcggugg cucaugcuug uaaucccagc acuuugggag 22080
   gccaaggagg gcggaucaca aggucaggag uucaagacca gccuggccaa cauacugaaa 22140
   ccccauuucu acuaaaaaca cacaaaaaaa uuaguugggc auaguggcag gcaccuguag 22200
   ucccagcuac uugggaggcu gaggcaggag aaucacuuga acccaggagg cagagguugc 22260
   agugagcuga gaucacacca cugcacucca gccuggguga cagugagacu ccgucaaaaa 22320
   aaaaaaaaaa aaacacuuua gcuaggugug guggcgugca ccuguagcuc agcuacuugg 22380
   gaggcugagc uggaaggauc acuuaagccu aggaauuuga ggcuacaaug agccaugauu 22440
   gcaucacugc acucccaccu gggcaacaga gcaagacccu gucucaaaac acacacacac 22500
   acacacacac acacacacac acacacacac acacacaaaa acacaaaauu aaauggccag 22560
   gaauaguggc ucauuccugu aaucccaacg cuugggaaaa ccaag 22605
<210> 3
   <211> 38666
   <212> RNA
   <213> Homo sapiens
<400> 3
   cccccauuaa ugcagccaac cuucucucug ggccacuaga cagucuuaaa cauacugaac 60
   uacuugucuu uccuacccga agccaaauuc ucucaguuuu ugcauauguu gcucccucuc 120
   caaaucggcc ucuuccuaaa aauguucaag ggucguugac uacaugcuau uuuguuuuag 180
   gcacaaacau uaaaauauug uuccugcaua aaauaggacu ucacuauaaa caaauacaua 240
   uuuuaccuca agauuguauc cucagaacuu uaagucuuuu ucuucccuuu ccacugaucc 300
   acaccuccau gccucugaac ugggacucau aguuugauuu gacucuucuu ugcaccuuuu 360
   ugaugauuuc uauauuauau acugguguuu ugcauccgag cuaauuuguc cacaaugaca 420
   aaaugggcga ucccacauuc cuaauauuuc cucugcauuc cacugcuugg uuccuuauau 480
   gcaguaaauu aucacauuuu uuuuagaugg gggucuagcu cugucaccca gguuggagug 540
   caguggcaca auccuggcuc acugcaaccu ccgccuccca ggcucaagag aucuucccau 600
   cucagccucc cgagcagcug ggaccauagg cgugcaccac caugcccagc caacuucuuu 660
   uuuuuuuuua auuuguggag augggggucc cgcuauguug cccaggcugg ucucaaaccc 720
   cugaccucca gcaauccucc ugccuuaacu cccacacuau ugggauuaca ggugugagcc 780
   acagugccua gcccucauac uuuugagaaa aaaaaauucc uaucaugggg cuuuacaacu 840
   guuauuauuu uuuaauuucc aaaaauaaau aauguauuac uaguuauggu acugcucaug 900
   uucacgaucu uaauucugaa gagaguuaaa ugcuucaaga agaguggagc ucccuucucc 960
   uuagcaacau gcuuauuuuc uaauagcagc cuuuggcaga cagugcuccc accuuuucaa 1020
   ugacuguugu gggaauuuca aguuuggcag gaggacagaa caauauccug gcagaugaau 1080
   ucugcauccu ucagaucgua ugauguccua aauaagacaa cugacuucuc uacccacuca 1140
   cuuucucggg gacauuuacu gagggccuau aaacugugcc aggcauuguu uagaugcugu 1200
   aaaacagauc aguaugacuu ggucccuguc cucaaaggaa ucuaaaaaca gucguaaaag 1260
   acaccuaugu aaacaauuaa aaugaacagg augaaugcua uaacaaagac auuauugaaa 1320
   uuuuguagga guauugaaaa agaacaggua acaagagggc ucaagaaagc uucacaaagg 1380
   gggaacaagc uggucuuaaa agguaaagag aaguuuguca ggaguagaag ggauggaaaa 1440
   gcauuucaaa uaaaaggagc agcuuguaca aagcagaaaa gugugugcca aggaaugaua 1500
   ugaacucaug aacuucagug ugaguggacu auagggaaca guguuauaaa augaagcugg 1560
   agaagaaggu uggccugaga uugcaagggg accaaaaauu cuguuaagaa gcuaagcauu 1620
   uugaccagca gcagacagcc ccaaacauuu ucaagcaggg acaucacaug agaaccaguu 1680
   uuuggacagu aacaauguaa guuauuaaug ccaucaagaa aaggaauaaa ggcauagaaa 1740
   guuaaccucu gaguauccuc uaaguaucug cagccuaguu auaucauuug auuucacaag 1800
   aaugcuauag gagaagcuuu uaaggucuuu auuuuaauag uuaaagaaau ggaggccggg 1860
   cgugguggcu uaugccugua aucccagcac uuugggaggc cggggugggu gggucacgag 1920
   gucaggagau ugagaccauc cuggccaaca uggugaaacc ccgucucuau uaaaaauaca 1980
   aaaauuagcu ggguguggug gcaggcaccu guagucccag cuacucagga ggcuaaggca 2040
   ggagaaucac uugaaccugg gagguggagg uugcagugag ccgagauugu gccacuguac 2100
   uccagccugg gcgacagagu gagggaggaa gggaaggaag gagggaaagg ugagaaggag 2160
   ggagggaggg agagagggag ggaaggaggg agggagagag ggaaaaggcu cacaaaaaug 2220
   cuaaccuucu gaaauucaua ccugcaaagg gcaggcauuu cuauuuuggg gggccaccac 2280
   cagucagaaa acuggcucaa ugaacaacgg guccagaucu aucaaagcau ugcuuauauu 2340
   cuaaugaauu auucauucuc auuuuguaua ugauuaaacu aaaauuagaa aacugaugug 2400
   uuccaacaca gaaaggcucu ucaugacuuu augacugaau uugugaaaaa cauuucauua 2460
   cauuuguaag auaguaauau uuuauacaua cauuuguauc uaaugaacua aauuuaccua 2520
   agcugcuauc cauaauuuaa acaggaacgc auaaacacau gaugaauccg cuaucaccuu 2580
   cuaaaucaaa agacuuuuuc auuugcauca ggaagcuuuu gaaacuauuu aucuaagacu 2640
   aagaguacau ucugagauga uuuuguuguu guuauugucu uuaaacuaug guauaccauu 2700
   guguagggca gaagaauuug aguggcuggg aauuuuguuu uuauuuuuaa auuuuccaaa 2760
   auaguacaaa auaaguuaac uuugccacaa cguaauaacu gaauaaaugc cuguuuauag 2820
   caccuggagc aagauaaaua agaacucagu aaauauuuuc aaaauugagc uaagaauuau 2880
   gauguaauuc aacugugacu ucacaacagg aaaacauucu uuuuauaauu uuguuuuaua 2940
   auguuauaua auguauaaug uuuuauauug uaguuuugcu cauauaaaau ucauuuauau 3000
   uuucuagaaa aagcauaaug uuuuaaacac uaaccaugug acucauuauu auaauugcua 3060
   auauuggccu acuuugauua uucucacaaa uuucauuaga cuuggagaaa gcacuccauc 3120
   gcccucuugu gguaauuuug cuguacuaua uucgccaaga guucaaaaaa augaacuuuu 3180
   ucuuuccacu uaaaaggcag agcaguacuu uaaccauaaa cuucugaacu uccaaaggcc 3240
   guauauaaac uuucuguccu ccacugucca uauacuuugu gccuucucuu cgguuucuac 3300
   cucagaaauu uagucuuuuc guugcuuuuu acaaaacauu cuccuguuag cuuccaauua 3360
   ccauaccaau agcauuuuuc ugaaaauuac uuucuguauu caaaugcaca aaguaauuac 3420
   agaaaaucuu caucaucaua cauaggcuuu aaauaaauag aaauaacuca aaaauguaug 3480
   gggcugagcu uagcauuuuu uccccaaaag aauagaucau gguaagucag uuaauagucc 3540
   agaaauuuca gagacuaaua auauuagacu aaguguuuuu uaguagucca gaaauuucag 3600
   agacuaauaa cauuagacua aauguuuuaa guuuuuagaa guaaugaaag ucuuuccucu 3660
   aaaauagucu aacucauaug ccauggccug uccucuuucc aacacaauag aaauacuucu 3720
   agcuuuuuuu uuuuuuuuuu uuuuuuuuuu ugagacagag ucucacucug ccacccaggc 3780
   uggaguguag uggcaccauc uuggcucacu gcaaccuucg ccuccugggu ucaagugauu 3840
   cuccugccuc agccuccuga guagcuagga cuauaggugc ccaccaccac gccuggcuaa 3900
   cuuuucuuuu auuuuuagua gagacagggu uuuacccauc aggugaucuu gaucuccagg 3960
   auggucucga ucuccugacc ucgugauccg cccgccucag ccucccaaag ugcugggauu 4020
   auaggcguga gucacugugc ccagccacuu cuagcuucuu agcaaaauua gugacauguu 4080
   aacacaugcc acuauaaaau agacuguaua aauaacguuu ucuaaaaaua auuuauaaau 4140
   aauuuauuua guaucuaaua uauuuaaaca cugugaaaaa uacaaaaaca aagauggauu 4200
   ccuacuuaau uaacuaaaaa auucacucuu acaaaucuca aaaauauucu uaaaagaaua 4260
   augagugacc auuuuuuuuu uggagacaag guaucucacu ccuguugccc aggcuggagu 4320
   guaguggugc aaucacagcu cacuguagcc ucaacuuccu gagcucagau gaugcuccca 4380
   ccucagccuc ccaaguaucu gggacuacag gcucaugugc caccaugucu ggcuaauauu 4440
   uuauauuuuu aguagagaug ggguuuugcc augcugccca ggcugaucuc aaacuccugg 4500
   gcucaaguga uccucaggag ccaccgcacu cagcuaugau caauucuuaa agcuaucaca 4560
   uugugaaaag uggccccuug gguacucaau cagcauucau cucaaaacug augaagaacg 4620
   acuagaaauu agagggcuag gugaagaagu acauucugua cagaguggua agaacuuuca 4680
   aaagcauggu aguaugccau gucuuggaaa cugaagaugg uuuagugcaa ccaagcacag 4740
   auuauaaauu gggugaagca ggagaugagg cugagaaguu aguaggggcc aaaguauaaa 4800
   agguccuaaa ugauuuguua aggaagggug ggauagucaa gcauccacag cagaggagua 4860
   aagcagcaaa ucagaguuug agaaagauca cuugcugcug gccaagcaug agagcucaca 4920
   ccuguaaucc caaugcuuug ggaagacaag gugggaggau cgcuuaagcu caggaguucg 4980
   agggcagccu gggcaauaua gugauauucc accucuacca gaaaaaaaau aauaauaaua 5040
   auucccagcu acuagggaag uugaaguggc augaucacuu gaaccuugga gguugaggcu 5100
   acagugagcu augauugugc uacugcacuu cagccuggga gacugggcaa cagagugaga 5160
   uccugucucu cuuuuuuuuu uugagaugga gucucgcucu gucgcccagg cuggagugca 5220
   guggcgugau cuuggcucac ugcaagcucc accuaccggg uucaugccau ucuccugccu 5280
   cagucuccca aguagcuggg acuacaggcg cccgccacca ugcccggaua auuuuuugug 5340
   uuuuuaguag agacaggguu ucacuguguu acccaggaug gucuagaucu ccugaccucg 5400
   ugauccgccg gccucggccu cccaaagugc ugggauuaca ggcgugagcc accguggaaa 5460
   aaaaaaaaag aaaaaaaaga aagaucacuu gcuguaguau ggagguaugg gggacagacu 5520
   gaaggagggg aaaggagaca gggaaauuag aagucauuug uaaugaagag gucaugaauc 5580
   aaagcaaugg cucugaugac acagagaaga aauuugagaa auaauaaaaa guaaaaccau 5640
   uagauguuag uuacugugag aauguggggc guggggucua aaagucuaca uucuagcuuu 5700
   ggugacugag uggaugugca auucccagag auaggaaaug guuauuuuca gacauacugg 5760
   auuugaggug ccaccaggag auggcaaaua gaucacucag uuggagagaa agaucuguaa 5820
   auuaucgaua acuaaguaau gaaaggaacc uggaugagag aucacccagg gagagcauaa 5880
   agagugagaa gagaugaagg cugagucuga gauuagcaac auuuaaguac agucccauuu 5940
   uccuguuaaa agggguacuu uuuuuuacau aaucauaaac uuuguucaug uugggagaaa 6000
   uaggaaauga uuuagucuuu cuucauuuug cagaucagaa aacugagggu gucaaagagg 6060
   uuaugugacu uuauuuaaag cuguuaaucu guagaaguca augagaacuc agguuuccaa 6120
   acugaggcca ggcaguucca uccugcugcu caaaugcucu uucuaguacu guguccuucu 6180
   uccccuaugc uacauaaacu guaagaacaa gaauuggaag augcuuucuu caguaauugu 6240
   uugauggcaa uaaauguuuc ugugguuguu auuuguacau aucaucaaaa cuaaaauguu 6300
   auauuuccaa ggauaaccuu ccaacaauau aguuguuauu aaauaagacc caaauuuaca 6360
   auuuaacaca ugcuuccuau ucugggaaac aaggaaaugc ccaauccuac aauuaugaau 6420
   agcaaaugcg acuguaucaa auacacacaa agaauguuua ugagaugucu cagagagaaa 6480
   aauagcuaua guuuaauccu gauaguccuu ucuaaaucuu agguuauuga gaauggguuu 6540
   uaccagugaa uucuaccaua uaucccagaa cacauuuuca uaaugaaacu cauuacgaaa 6600
   agcaagacgc aagagauuuc ucugggcuac cuccuugguu uggccguugc cagcuuggug 6660
   acuugaugac gccaguggau ccuacaggcu ugaaggcagc ugcagcugug aggcugguaa 6720
   ccccuggucu gccagagguu gggcugucca ggcucucggg caugcuccgu guggaagcua 6780
   ccgaggaagc caacugcgga gaaggcuccu gagaguuacu guaaaggaaa uaaagaaaca 6840
   ucuuugguga agugguagca acguaacuca gcaauaaucc uuucuuuuca aaauccauua 6900
   aucaaucaaa auccauugau uaaucaauuc uuuagaaaag aaaaucuuug aagucaugac 6960
   ugagggauug ccucucuuua aaacugaaaa aaagaaaaag gagucagauu gcauuuguuu 7020
   cauacuuuga aaauucuuuc uaaaaggucu accaugagaa auuaggcuac ugaaaauuua 7080
   cacaguuaau cauuuuaaau gauaaaaguc ucccugacac cucagcccuu accaguuacu 7140
   cuuuuucuuc uucuacuuuu uuuuuuuuuu uuuuuuugua gacacagggg ucucacuaug 7200
   cuccucaggc uggucuugaa cuccuggccu caagcaauca ucccacuuca guuuccgaaa 7260
   gcacugggau uacaggugug agccaucauu cccagcccag uuacuccuuc uacucacaua 7320
   cucacucauu cauaaauauu uaagcaccua cuauaugcca aacguugguu gugcuaugca 7380
   cuggccacaa aacaauaaac aagaaacaac ccuugccuaa cagaacaccc aguguuaugc 7440
   auucauaaua aagggaaaaa agagacaaga cugccauuau cuucucucaa ccaagaguua 7500
   ccuguuuaaa cagcaggucu uaucuaugcu aguuccaguu ugguuuugaa gaucauaugg 7560
   ccuuuauuac caagucauuu gugauuuaua acuacauaua acaucauuuc caaaaaaaaa 7620
   auaauaauuc uagguagccc aaauuauaaa auauaugcuu ugguaugaua uaauacuaua 7680
   gcagugguua uuucuuucaa guaaaguaua auaacaauuc uuaaaaguaa uacuuucagg 7740
   cugggugcag uggcacaugc cuguaauccc agcacuuugg gaggccaagg cuggcagauc 7800
   accugagguc aggaguucga gacucauuuc cuccagacuu ggucccaugc accuuuuucc 7860
   uuugcuaauc uugcugugua uucuuuugcu auaauaaauc uuagucagga gugcaacuau 7920
   auacuaaucc uguaaguccu ucuaucgaaa caauaaaccu aggagugguc uuggggcauc 7980
   ucugacacua aacaguuuug aaaaacagua agaaugauuu ucuugaaaua uugcucccua 8040
   uucuuguucu cucaaaugca agugucauag agccaucagc uuuuauuauu uuugaucagu 8100
   uuaacuuaaa aaacuaaaac cuguuugaug auaaaacacg cuauacaaua aaaaacuuua 8160
   agcacuuuaa uaaucuuuau cauaaauaaa aagucaaaga cugccucccu caugggaaua 8220
   uugaacacaa uuaucaguaa aggcaacaca acaaaaagca uuaauggaau caggguuuga 8280
   aauuacaaua uagugguuac gagcaucuuu uaaaagcacu gugguggaca aauuauuuua 8340
   caucucuaag ccucaguuac cuguacuuua aguguaccga guaacauuuu gcucauaaga 8400
   uuucuacgag gcuuaaauaa gauaauuaug uauaaagugc uuagcauacu cuaaggcaca 8460
   gaauauaacc uuaacauauu aaaaggauaa uuacauaauu accauauuca aauaauaaaa 8520
   guaagccuua uuuucaacau uuuuacucau uacaaauaaa ugauuuuuga aauacuuaua 8580
   uagaggucau uuucauucug ggauuaaauu acccuugcgg auuugauuuu uaaaacuaau 8640
   uaauaaugua uauaaucacg guucuaagaa aaaaaauuau gaccaagcaa ugcuaggaau 8700
   uauagaugaa acaaaaauau auagucugga aauaacaaaa cccaauccau caccauuuaa 8760
   uguguaguca ucugaaagaa ucaaaauuua auuccaggag agcugccaga caaacugacu 8820
   uaaaagagag gaauucagug ucaagcuuaa uauaguuugg gugaaggaac ugguaauagu 8880
   agcccagguu augaaaaaac cuagagaagc augacagcca uccucaaaug ucugaccugc 8940
   agauucacuc uauguaacua cauuguuugc cuaauuucaa ggagugaaau cuacagugag 9000
   auggauuugc uucaauaaaa ggugauuccu aauauaaaaa aauccuccaa aggaaaaaaa 9060
   ucuguuccua augaggcuuu cugugagagc ccugucacca gaagccacuu aagcagagga 9120
   uggaaugccg cacaacgaau uuagccauuu agccugugga aggcucaguu auauuucagc 9180
   augacauaca aagccuucua agauaaagcu acaaucuacc agucuaaucu uaucuccuau 9240
   cacuaugcua uacaaacuau auucuggcca ccccuacugu cguccacaau ucuaccuuuu 9300
   uuuuccagau ucuauauacg agguaccucu uccuacucuc ucucugcacc cauccacauc 9360
   uuauccauaa uuaauaauaa uagaaguaaa uauaauaacg caucaugcac uguguucaaa 9420
   uuaacauaca uuauuuuguu uaauuauuga agccuccagc aguagagaca cucuuuuuaa 9480
   uucaggacau ucguuaacug cauggcauau aauuuuaugc ugauuuguaa uagcuguuuu 9540
   aaaauuacua uuaauaguca cuucuuuaug caucuugucu ccuccacuuc auuauaaacu 9600
   accugagguu ggggacuuua cugugacugu ggguaaauua uuuaaacuug guaaauuucc 9660
   ucaccuguaa aaugguauaa aaauacuacc uuuaucccaa gauuacuguu aagaauuaaa 9720
   uaagauauga gauaauacau gcaaauuacu uagcacagug cccagcagag aguauaugcu 9780
   caggaaauaa aaaggauucu uacuucuaca uccuguaaua ucuggucugg ugauaauaaa 9840
   auuagcaaua aguacauuua cuaaauaagc auccuucauc ugcacuaaga auuuugcuuc 9900
   uaaauguacu auggaucacu ugggugcagg guauugaccc cagacaaaau cuugcuuccu 9960
   caauucaguu uuaaaaacac acaauacuuc uuaaaguucc cacagaauaa guaucauaua 10020
   agcuaaagaa auuucaauuu uugauuguac cuuuuguaca cagguuugua uacguucucu 10080
   cucagaggcc uaagauguuu aaaucuuaaa caguacucuu uagcuacaaa gggcuuggag 10140
   uagacuauaa agaauucaua gaaaucuaag uuauuaccaa ggcaguuuuu ccauuuaugc 10200
   uuaaauccag aauugaucug aaguucaauu uucucugagg auaaaauuua auugaaccau 10260
   uuaauguugu aauggaagaa uauuaggguu uaucuagaca aucauauauc ugaauauaug 10320
   augcccaauc cccuaaaaau cuaacauuuu uucccuaauu uuaacuauua gcuacuauuu 10380
   aaagagcaua cauaaacauu ugguauuggc ugggcacagu aguucaugcc uguaauccca 10440
   gcacuuuagg agaccaagac agaaggacug cuugagccca ggaguucaag accagccugg 10500
   gcaacacggc aagaccuugu cucuguaaaa aaacaaacau uguacuaaag augaaaggau 10560
   uggcugggcg cgguggcuca ugccuguaau cccagcaccu ugggaggccg aggugggcgg 10620
   aucacgaggu caagagaugg agaccauccu ggacaaugug gugaaacccc gucucuacua 10680
   aaaauacaaa aauuagcugg gugugguggu gcaugccuga agucccagcu acucgggagg 10740
   cugaggcagg agaaucacuu gaacccgcga ggcggagguu gcagugaacu gagaucgcac 10800
   cacugcacuc cagccuggug aaagagcaag acuccgucuc aaaaaaaaaa aaaaaaaaaa 10860
   aggauuaaaa gucacuggcc auauuauuuu auauguagga auaaaauauu aucaauaauc 10920
   acuagcaaga aacuucuaau gauuaaugua uuaauauagu auagcauugu uacuuaaauu 10980
   agauugcuau uuuauuucug aacuaaugau ucaaauauau uuucuaaauu uaaauuauuu 11040
   aagguguaac uaaauccuaa auaaaauuua aauguuuucu aaauaauucu aaugaggaaa 11100
   uugguguaaa uccauaaaua cccuuggcug auaccacaga uacugauauu aaaauauggu 11160
   uauuuucacu aaucuaccaa auaaacaggg acugaaauga aauaauagau ugauaugacu 11220
   ucuaucuuaa gaauaccaag guuuuuguuu cauuuuuuca aaaacugaaa auagagacaa 11280
   guacacuaau agagcaaaag uuuauuuuca agauggagcu aaugaggaaa aacgaaaagg 11340
   aagaaaguua accucagugu cagcaaccug auuuaucuug aguuuacauu ccuuucccac 11400
   auuuguuuaa cuguugcaac aaauaaagcu cagauacaga uuaagaauac uugggaauuu 11460
   uaaguccauu uucaaaggaa aaacucugcu ugaagaauac uuuucagagu aacuuauucu 11520
   aauuacaugu uuaaauuuuu uaaaagacaa cuguacauca cagguuuaau uaucaauguu 11580
   uaaguuaaac aguuuuuggc uaaagucuua ucaaaaaaug guauacuaau auacagauuc 11640
   ugaaauggca ggaaaggaau auuucuggaa aggccuuagu uacaagcaau uguuuauuuu 11700
   auagaauucc ucugucagaa cgccuuuaaa agaccuggac auguagagag gguaaugagu 11760
   gucuuuccuu ccagaauucu auauuuagaa gaaaauuuuu aauauuuucc uaagcaaaau 11820
   ucuuuaacuu agaguuugua aaccaaaauu uaaaguauaa uauacuaaua uauauuauaa 11880
   uggaaauaua uuuuaaugga aaaauauauu uuaauggaaa aaaauaaaua aaaacuauua 11940
   uacuauauuu auacuuuuaa agauuacuau cacacaaaag uaaaaaucaa gacaaaaugu 12000
   cucugacaca uuuuaugauu acugucauac aagauaaaau auaucugguc uuuuggguac 12060
   uccucucacu aaguuagcaa gggucuccuc aggaaacccc agaaugccaa cuagacuauc 12120
   uuuuuagaaa ccauuucccu cucucaauau cacacuauau uuugucugug uccuauuguu 12180
   aauaagaagc aauggugaug acuacuaaua cucgcucuag agguggggcc auagauagca 12240
   cagucagucc auaaaugcuu gcacacuuuu gcauguauau gcacgcaggc acuuuucugg 12300
   agagaggauc uauagcucuu accggguccu aaaagugugg ucuaagugua cucaaguacu 12360
   caaaguguac ucuaagcaug gucaguaacc ugccucugua caaucucagc ccugacaaug 12420
   aaagacuucu gcucaaugaa cacucacaau auucuaauau aauuucaggu auugucuucu 12480
   cuuagagaaa aauguacuau gaucuuucuc ccuuguuuaa acaccucccu uaaaaguuac 12540
   ccagaauaau agguaaauau auauauauuu uuuguuuguu uguuugcuuu uguuuuuguu 12600
   uuuugagaca gagucuugcc cugucgccca ggcuggagug caauggcgca auuucggcuc 12660
   acugcaacuu cugccucccg gguucaagug auucuccugc cucagccucc ugaguagcug 12720
   ggauuacagg cacccacugc cacacccggc uaauuuuuuu guaucuuuag uagagauggg 12780
   guuucaccau guuggcuagg cuggucucga acuccugacc ucaugaucca ccagccuugg 12840
   ccucccaaaa ugcugggauu acagguguga gccacugcac cuggcccagg uaaauauauu 12900
   gaaaaaucuc uauaagaaug guuagaauac aaacaucuaa ucaauagaca gccuaguuau 12960
   aucagguuuu cacuuugccu gauauauguu guacacuuug ccacugauuu uacguucugu 13020
   caaaaaucau uguauacagu uauuucauug agauuauauc uuuaguaaac acuugugaua 13080
   guuuuauuuu uauauuaugc cuaaaaauuu cuggcuuaau ccuuaagcuu aggagaaaaa 13140
   ucuauuuugg uguuuagcau uucccaaaua cagugaaaau uaaguugcuu uuccaagcaa 13200
   aaccuucaua uuuaacaacu auaauaugua gccaaucaaa auugaucauu aucauuuuca 13260
   uuuuucuaaa agcuaugucu uaccaaacau uugaggauuc uaauauagcu auauccaugc 13320
   aaugaaagac agaaauucua cugcaauuua aauacuugca caauuacuca gaauugcaca 13380
   ccaaacauca aguaugcuaa ggaaagaugu cacaaaaggu uaaaagguga guuaauaauc 13440
   auaggacaua cugugcuugu gcauuagcau gccacugaca uuacaguuca uucaaaauug 13500
   ggcacaaauc ccuagaacuc uagcauguuc ucaccuggcc uguuaauaca gugguggagu 13560
   guuagagaaa gcaguaugcu uaacagcgaa ccaccacuug ccauaucugc ucccacagua 13620
   ccuuguuuuc uuaacaugga uagagccagu ugauuuccuu gaggacugca gauaggaaaa 13680
   agcuaaaggc uuggagacag ggcugaugca aagugcaucc aggagugagu gauaucuuuc 13740
   aggaguguag agccugguaa gcauuuagaa agaguuuuaa gucaaaaauc acacuaccaa 13800
   uagcagacag uaguucuuaa ggucugugau uugaaacauu uccuuuacga gagagagggu 13860
   ggggagagag aggaagaagg aggggaagca caaagcagca aaggaggaug aaagaagaua 13920
   aaaagaagca ccaagaguuu auucuauccc uuuacucauu aauauaacau ugauuagauu 13980
   uucaccaaaa uggucaaagc caucauuuag aaagcauuug aagcucuaaa gacuauagaa 14040
   auuaucuaaa auccccaaac cacgaaaaca aaaccaauuu auucuuuuug gggaaggaca 14100
   caauuuuauu uuuuuagggg agaguagucu ucagaucacc cuuuaaaggu cgugggagaa 14160
   gauaaaauca auagaaacuu aaaauauuca uuaauacuuu auuguuucaa auauaaaacc 14220
   ugaaaaaugu aaaaacccaa gugacaauaa gcacacaaca uguaauaauu cacgcaacug 14280
   agaaauacca uacauaaaug gcagcagugg agagauuaaa aggugauuuu uuaagaaaaa 14340
   accaguaaca aauacuguga gauuuaucca caaccuagau aaaauaugca cuacacaauu 14400
   aagagcucac aauagagcua ugauugcuuc aaaggggcau guuuucuuug aggugacuug 14460
   uaagacagug aagagcgcug agaucacauc agacagacgc uguacgugua guucuaccug 14520
   guuuuuguag caauaacagc agacacagua gcagcugcgc ugcucagaac ugcagcacua 14580
   uaucuagcag gagaagagaa gguagaaaag uuucgaaaac cuucgaaaga aucuuccaca 14640
   uuuccugugg agcugcuuaa acugacaggu agggaaagaa aaacaggaga ggaacaaaaa 14700
   ggaaagugag aaaggguagc auaauaaaua caagagagca accauuucau auuaauaucu 14760
   aagcauuaua aaagugugaa guguaagaag uguaauagga guucaggcca uuuagucugu 14820
   gcuccuacgg uuucucucag aagcuaaaag cugauguaua auccuccaua aagcugauuu 14880
   ggacauacga auacucuuca auguauuuaa auucacugac uguauuccua uuuaaaauua 14940
   ccucguauca cacauuugaa gacugacuug ccaacaacuu aguagaaaau aaaucuaaac 15000
   gugaagucug aauuucuaua agcauagaaa gaaggaaaau uguuuuuucc caaaaagucu 15060
   cuauacaaug agcuuuaccu uagagacgau auauacugac agcgugacau uuuaauacga 15120
   cuuauguuuu caggagaagc aggcacaaca gacugcuuau caagaaggag caauauauau 15180
   uaacaugaaa agagauaauu guuuaaaaua uugagaguaa agcuauguaa ggagggaaag 15240
   uaccuuugcg aaaauaaacc aauguuacag ugaauaugau caaaacauca ccuuuucugc 15300
   ugaguuccau auuucuauuu ucaguuaguu cuguaagcau uuuuaaaugc agguauagca 15360
   uuuuaucuuc agaacuaaua aguauuuauu aauuaaaaua auaauuuucc cuuggccuuu 15420
   uagaaaagau caaaugccug gacaggaaug aacauucuau aauuaugacu guagugauuu 15480
   ucaaauuggu uauuaaguaa aauaaaaaga guguauaaac guauagugug aaacuuguag 15540
   ucucucuucu ucauuuaugg gugcaacuua ucaaugagca ucaucacuac aguaugacca 15600
   cuuuuuucaa uggccaccuc caacacaaag uaauuacgug gcaaacucga ccucugguua 15660
   augugagcuu agaugucuga aacugugagg guugacauaa gucucuuuuu ccugucaaaa 15720
   gaauuugaua agucauucuu ccaauuucca caggcuauaa cuuuacaaaa acgauuagaa 15780
   aaaaaauuaa ggaaauaguu gcaagcuaug uuuucaaaau gauacagaau uaaguaauuu 15840
   uucuuuuggu cuaucuaagu cuauccauua aauuaaacug uauggcuaag uuuuaacaac 15900
   uguuaaugaa ugguuagaaa agaaaacguc agggucugga gagagaaaag aaagauguua 15960
   ucaauggcag agcaggguag guaacaugaa ugucaaaggg aauaauaaag uuaucaugug 16020
   auuccacuuc ugaguuucug cauuacucug caucucugag acaaugguua cuauuuggag 16080
   acauaauggu gaauaauaag aacuagaaac uuaguauauu gaaaaaucac uuaaaaauaa 16140
   aucagcuaau uuuuucuucu ccaggaagaa aaggaaacgg uaaugaauuu cuuucaucau 16200
   auugcaucac aaauaauaga aaaggauuua guaucagcag aaucuaguua uaucucauaa 16260
   gcaguuacuu aucugaguau cuuuagaaua ugucugggca ccugguaaca uauuguguau 16320
   gaccuuuacc ugucuuugag gcaaaugcuc uaauucagaa aaggagacuu augacaaagc 16380
   cagcaaguua acuuuccaaa ucacaaagug aauaaaugaa agagauuucu ugauucuaua 16440
   uuagaaguua uaaacuaaac aaaaauaaaa auauaaaagc agugagaagc gcuguuuuga 16500
   ggcugagugc caagcuuucu ugucuauagg uuaaugaugu ccguucugua aucaacaauc 16560
   auuuuuucuu uuucaaacag uuuaguauau uuaaauucac uaccagcuuu gaaauuuaga 16620
   aacaccaaga aggaagaguc uaccuagaau aaguaguugc uacuuuagaa aguguggcca 16680
   cuuggcuacc aauaguuaac accugaggag uugcaggagg gugaggucca cucuuaggua 16740
   ggucuuccag agcacuguca aaccuaaguu uucagaagua aaauauauaa ggcaaaggaa 16800
   auauuaauga aaaguacaaa agacauuuau caucuauacc aucuuuuaaa ugucuacuag 16860
   uucaauuuug ggaacacuau ugguuuucuu uuacuucaca ucuacaaaug guuaaaacaa 16920
   aacccaacac uucugccacc acaaaauaug ggcuguuuuc ccccaaaauu cuuuaauaug 16980
   gcuacuucua uguaauaaaa uauuacgcuu acaggggaaa aggaggugga aauucccauu 17040
   cacugacuac guguuuugaa ugcuuuaaaa aaacuuaggc uccaagaagu uaauuaaguu 17100
   gcccaauacc auacaucuag uaagggccag guggaauuug gaccaggucu gccuguauua 17160
   uuuccacuau auaauguuuu ccagaacauu gugagacagc uaccuaaaag uguccuacaa 17220
   gaaagcaacc aaaaguuuca cgucagaaug uucugucugu agaucugaag uuauauggau 17280
   guuguguauc uugauguaaa caaacuauuu uaucugcuca uacaacagau gguggcaugc 17340
   aaucguaggg uuuugaguau ucuuuacuuu aauuuucuga uaucuguauu auuuuuuaua 17400
   uagaaugcca gaaguaaguu cuagaagaac uauugccagg uaaagaaaaa augaagaacu 17460
   caauguuaag uucaaaaaaa agaaagcacc aaguauaaaa cuuugggaag aaaaauaaaa 17520
   caggaaaaau acaaaauuag ccauuaaaaa aaaaaagucu guggauaaaa aaauagaaug 17580
   gucagucaug uuacacugau uuucaaaugg auuaaaaaau aggaaaggag agguuauauu 17640
   ccuguuuuuu auuucacauu agucauaccu uuaccagagu guugaaaaaa cguagacagu 17700
   ugaagaaaaa ugucaugaua gaacuaaaac auuaagaggu agagcagaaa gguuaagaga 17760
   cuaagaaaua uaagaaccau aacauggguc agcaaaccac ggcaugcugg ccaaauucag 17820
   gcccugccug ucuuuguaug gccuaagaau aguuuucaua uuuuuaaaug guuaaugguu 17880
   aagaaaaucg aaauaacaau acaaugacac acaaaaauua uauaaaauuc aaauuucagc 17940
   auccauaaau aagguuuuau uggaacuuua uuggaacaua gccagguuca uucuucauau 18000
   gucuauggcu acuuucauau gauauaagaa cugacuaacu gugacagacc auauggccug 18060
   aaaaaucuag cacauuuccu aucuggaccu uuacagaaaa aaauuugcug accccugucc 18120
   uagaaaauaa cuucucuaca cagcuaccag agugaacucu gaaaaauaca aaugccauca 18180
   ugucuguugc uuagaccuuc cagcccugcu cuaggugauc uugggcuacc uguucuucag 18240
   cauuauguca ugaagcuugu ccacucucuu uggcuugcuc caguccacug ggccuuccau 18300
   cucuuuuuga aauaggcccu gcuucuaucc cccuccuggc cucugcacag ucuccuacuc 18360
   cucacugucc ucagcccucg uucuaccaca cccucacugg gauaacucca aggcauccuu 18420
   cuggccucag uuuaaauguc accuccuccc agaagccucc cugauucccc aagccaccuu 18480
   agcugccccu gucaugugcu gucauagcag ucuguacuuu ucacucauau aauuuguuau 18540
   uauuauuaua caauuugugu cguuauuugu uuaaauccag acagucucag aagacuagaa 18600
   gcucccugau accaauaguu acugauauac augaagugua acaaagugcu uaccauaaag 18660
   uuaacaaaua uuugugaaau aaaagcuuga aauuuauuuu uuccaaauaa cgaauucaca 18720
   auauuuguaa auugaaaagu auuuguaccu ucuagagacu gugucacaau ucugacuaaa 18780
   cagauuuuug ccuaagugga cacaugauaa cuugaaagaa augaauagug cucccaaaau 18840
   accuuuaaau aaauauuuua auauuaaaaa uuuauaaaua uuucaggaaa aaagaaaaua 18900
   ugguuauaaa uuauuaucaa uaucuacaua uuaccacagg acagauuaaa aaaaaaaaga 18960
   aaagggagug aguuggagaa guugagucuu ccaacaucuc cgugaaauau gcugauugga 19020
   aaaaagcuga gugccauuug agaauaaauu ucauauaaau caugaaauac cuuugaagau 19080
   gaucuccaag ucaucuucca agcaaacgca cuugggaagu uucccaugug uuacacguau 19140
   ugauuaucuc uucauugcuu agccaaacuc uaaaaaccug acuuaagaca aaaucuuaaa 19200
   cuaccucauu uacuauggaa caaacaucag uaucuguucu cauucugaca uuccccagug 19260
   uuuacuccuu uuagguugga ggaagcucug auuaauagca uaucugcagc agccaaauuu 19320
   ucugccuugu cuugcuuaaa ugaguuuuca uuucucacca gagaggguua ugaagacuuc 19380
   cucuuuccca gauaaagucu uucccuuuug gcuugcuuuu cucaggauac augaacaaag 19440
   agcuauucau gcauuguguc uauuucugca ccaucucugc acuuguucau uacggacccu 19500
   ugugaacaau cuguccagca aaucaggugu gaguugucca ucuacuaauu uaccauccuu 19560
   accauccugu aggaccagua agaagaauuc uuuuucuauc cccuaguccg gacaacugcc 19620
   caagagcaac auucuaaugc uguguacucc aucuccuccc uuaaucucag uggccucgau 19680
   uaauccaucu cuccuucucu acuggaugcu uuguguccuc uuuuaaaaug caguaguuuc 19740
   ucuuaaaaac aacagacuau cuuuacuuuc uauuuuccug uuccacuuac cagccagacu 19800
   cugaaauaac auuauacuca cuugacuguc agucuucagc caucucaacu agaccccaac 19860
   ccccaacacu cuaugaaacu gcaucuugcu aaggucacug augaccuucu uguggcuaaa 19920
   uucaauggac aauuugcauu ccucauuuua aaaacugugg uaaaaaauac auaacaugaa 19980
   acuuacuauc uuaacgauuu ucaagugcac agucaguagu gugaaguacg uucacauuau 20040
   uguggaagag aucuccagaa guuuuucauc uugcauauuc gaaacucuau acccauugaa 20100
   caacuacccc uucccccucc cccuagccug ugguaaccag caccauucua cuguuucuau 20160
   gaagaugacu acuuuaauuc uuuuuuugga gacggagccu cacucuguca cccaggcugg 20220
   aguacagugg cacaaucccg gcucacugca accuccaccu cccugguuca aacgauucuc 20280
   cugccucagc cuccugaaua gcugggacua cagguacgug ccaccacgcc uggcuaauuu 20340
   uuguguuuuu aguagagacg ggguuucucc auguugguca ggcuggucuc gaacucccga 20400
   ccuccaguga uccgccugcc uuggccuccc aaagugcugg gauuacaggc gugagccacc 20460
   acacccagcc cgaagaugac uacauuagau accuccuaag ccugacccga agaugacuac 20520
   auuagauacc uccuaucagu ggaaucauac aauauuuguc uuuuugugca cauuccucau 20580
   uuuacuuagu gucuggcuca auugacuacu gcuucucuuu uccugucauu ugugccucaa 20640
   cccuauuuac cugccuguuu uuaguaguau uucacuggcu auucuguugc ccgagaguuc 20700
   ucuguccuag gcuuuauuuc ucuguacucu gcuaucauua guacagugac auuacucugu 20760
   ccuguagcuu uaacaccacc uaauguccag auuuauaucu agcccagcug ucuccuauga 20820
   gcaguagccc auauuuugaa uugcccuuua guuauuaaua ccucuaauau cucucugucu 20880
   caaaucaucu uucaccaugu ccaaaugacu cuacuuccaa auuuauauuu uuaaucuauc 20940
   agugguguga ugggUccccu accagguuac uuaagggugu uauauucaau gcuugaaacu 21000
   ugagggccag gcaguuagcu aaggccaugg ugccuagccg agaagcaggg gucccugaga 21060
   acgcaaacau ccugaggcau ggcugggaau auaccaagga aaaaaguccc agcacacuca 21120
   cacacgcgcg cgugcgcaca cacauacacg gcaaagagcc agaaaauuag cuuaaaagca 21180
   gccuagagau gggaggcggu guggaucucu uggagccaua cugcugcugu ccagcagugc 21240
   ccuguaugua uguccuaaua aacucaucua cucaucaagc uggacuuguc ugagucauuc 21300
   ucuuggcaac gucccaguuu ucccaguuug cggaggguug ggggggcauu acagucccaa 21360
   guuuuucuca uuacgccauc caucuacccc auuucucugc uaccacccuc ucucauccag 21420
   acuaugauaa gcacuaacuu auacuaguga uuuaagaacu gucuaacugc ucucuugcuu 21480
   ccucucuugc caaauccugc cccuuccagc cccuguguuu ucuggcaugg uuuaguauag 21540
   cccaugugcc uuaagaaaaa acaacaaaac acauuuuauc aaaucauucu uuugauuaaa 21600
   accuugcaac aaauuccuac ugcaguuagg auaauaccca aagugcuuaa cauucucuac 21660
   aaaaucccca aaugaccugu uaugaucaac cucucaauua ucuuuccaca ccaauuuucc 21720
   cuuuauucac ugaacuccag ucacauuaga cuaccuguac aaugucuuga acaaacuaag 21780
   uucuuccagg auucaggcuc uuggucauua augauaauau uauuacuuuu acuucauuuu 21840
   uuaacuuaca uguaaaucau uuguuaauaa uaaaagcaaa uauuuuuuca ucgcuuaaua 21900
   uguaucauca uauuuaaucc uuccaauaau cuuucaaccc ugguacuaaa auuauccuug 21960
   guaauuuacu aaaguuagua cuuugguaua ugguggagcu acuguacugg cacccauggu 22020
   uagaauaacu ucaguggaau gaggagaaca aaaaccaggc ugcaggggcc ugguguggug 22080
   gcucacaccu gcaaucccag cacuuuggga ggccaaggca gguggaucac cugaggucag 22140
   gaguucaaga ccagccuggc ccacauggca aaacuccguc ucuacuaaaa aaaauauaaa 22200
   aauuagcugg gugugguggu gggugccugu aaucccagcu acucgggagg cugaggcagg 22260
   agaaucacuu gaaccuggga ggcggagguu gcagugagcc aagauccugc cauugcauuc 22320
   cagccuggau gagagagcga aacuccgucu caaaaagaaa ccaggcugca ggaugguaaa 22380
   guucucuauc aguaauaaaa uuucaaaaug uuaauuuggg ucauccagau uuugauggaa 22440
   uuauaugcaa acuauguucu aagaagaaau cugucacaaa ggcgaaccuu aaaaaaaucu 22500
   uaaaaauaau gcaaacauuu ucuuuuacaa cauaaaaaug gcaucuucaa uuaaaauuuu 22560
   aaaugcuucc uauuucugaa uauaaauaug auaaaaugau caaauagacu agugacuuau 22620
   uacauaccca cacgacgagu uuucaauaag aggaaaaauu cacaaaccuu gaaauauacg 22680
   uuuuauuaaa agguaaagga ggaaaaugac aauaaauugu ccuugguauu cuacuuccac 22740
   aaagcaaacu uuuauguaau aauauugcua agcagaaacu agaauauauc agagucauag 22800
   cuagauucug cccuuuuggg guucacuggc auagcaaagc uauuuauuuu agccaaggaa 22860
   acugcuaggc augugaaaaa aauaaguauu uucugcuggc uuccugacaa uaaauauguu 22920
   gaguuugcuc auuuguaacu augaucuccc uucuggaaaa cuaaaucaac uaaaccucag 22980
   gugggaauuu cugguacucu caaucauaag cuauccauuu auuaauuggu uuucaauagu 23040
   cuuuguuucu guagcagccu gaaaauguua cuugagccug cuaauucuac cuuuauuaca 23100
   uagauggcau cuugagaauu uaaaaauaag caaguagagu uauccuugcu uugcacaugg 23160
   cagacaagga ggacuggauc uuagaggcua aaagguuaug aaauaaauua ucaaacauga 23220
   aaauacuacc caaaaugagg uugauuuuuu guaagagcaa agaaggacua acucaaagaa 23280
   aucauuuuuc uaggagcuuc ccacuuacuu aagcaaggaa aagauaggca aacaaaaagg 23340
   gauauugcug aaggaauugg aacuuauuau uuguuguuau acagugaacg uuuguguccc 23400
   cccagcccca aaucauaugu caaaauccua acgcccuaug ugauaguauu aggaggugag 23460
   gccucuggga gaugauuagg ucacacaggc agagagcgcu caugaaaggg gucccuuaua 23520
   agagauaccu cagagagcuc ccucucuccu accauaugag gauacaguga gaaggcauca 23580
   ucuaugaacu aagaagcagu cccucaccag acacugagug uacagguacc ucgaucuuga 23640
   cuucccagcc uccagaacua ugagaaauaa cuguuuguug uuuaagccac ccaguccaug 23700
   auauuuuugu ucuagcagcu ugaucugacu aagacauuug uauuagggaa agaauuuccu 23760
   cuuugaugau auuuuccauu uucucacauc uuucaaacua uggcuuuaua acucuuacac 23820
   auuucucugc uguugccacu ccauaaugga uuuuaucauu auuaaguguu caauacuuaa 23880
   uauuuuguaa auucuuuagu uaucuagagu auucuuugau cucuauauag aaaauguucu 23940
   auagggucua aagccaggua aagaaugagc auuuuuuauc uauacaaaau gcaaauauaa 24000
   uaugaagauu uuaacgugua augacaacuu aaaagucacu uggagcaauc cccugauaaa 24060
   agcaugacuc ccucaguaac ugcagggcac uuaccacuaa ccuuuggccc ucucacuuuc 24120
   caucaucagu uucuucacug gaucauucug auccaugacu ucagagauuu auacuaguua 24180
   gaaauagcuu cuuauaugug acaaaaaucu gccauuuugu aguuuuaaca uaauccuaau 24240
   auuagaaaaa gaauuuauaa acaauaaagg uauuuuucag auucgggaua aagaggagaa 24300
   aggcauguau uauuuaauaa auaguguuaa gucaaguuac cuguuugaaa auauaaaaga 24360
   auacgucuga auguguccca cugaacagcc acccacagug aauccuuaaa gaggccacau 24420
   gucuugagaa agccagcaca guaccucaug cuccagugag aguguccugu uggcauuaca 24480
   gaacaauagc caaguggcua cacacucacu agccaaauau ucuggaucau augucccaaa 24540
   cgcacccauu caaucacuca uugcauucac ucauucauuc aauaacaaaa aacaauuacu 24600
   ugaauuccca cuuugugcca agcaccuuca cacauccuau ugucuacuuu aauuuccugc 24660
   ccucaugaag cuuacauucu aguauccauu uuccuguaau ggacucugag gauuaaucac 24720
   aucaugcuuc ccacuuaccc uuguuuaccu gccacaauca gaaguucaaa ucccuugggu 24780
   cauccagcau cuccuggacu cuuauuucuu cacugcuuug uguaucuccu aaucuaauca 24840
   cuucaccauc uaucacagcc cucacaaacc cauuacuguu uuacuauacc uucuuuacag 24900
   ucucuucaaa cagauguugc uuuuucuuuc auaugccuua uucuuauaaa ccuggagaug 24960
   gagcaagugu ccuccucauu acuguuuuaa cuacuaucag ucaucuuuga uccuccaaaa 25020
   ucccaagucc uuggaggcuc acuauaccau ucacuauagg gccuucccac uguuugucuc 25080
   uaccucaccc acuguuuuag cuccugacug ccuuccucuu caccccauuc caacaaucuc 25140
   ucaugauuau uucaauaauc augcacaauc acugaauaaa uaaccuuacu auugaauugu 25200
   uugauuucuu cacuuuaaug auacauuccc cuaaggcuaa gccuuacacc uagaauauau 25260
   cagcaugcac uccaaaauuc auaaccuuua auuuuucauc aacaaguaca acaugucuga 25320
   aaucuaaauu uuaaggaccc uuuuuuuucc ucacucaguc ugcuaucccu cgauucuauu 25380
   agcuuuuuuu acuguaccaa auugacucug accaaucuca uuagcucuua ucaacugaac 25440
   uaauucuuua caaauucacu gacuuaccaa uuuuccacuc uccauuaccc uccaccucaa 25500
   uauuucuaua caccuaucag cucuuugucu aaaauacaaa ccuauccaau gcucuccauc 25560
   uccacugcca cuacauugcu uaucugaacu auggggauac ucauaacagu cuccugcucc 25620
   cauuauuucu cccuuacaau uaauuuucca uaguauucag ggugaucugu uaaaaauaua 25680
   caucagaugu uacuccuaug acuacagucc uauauugggc cucucauuua auuagaaaaa 25740
   uaaucugaac uccuuaacac aaccuacaag gcagucaaua aucuggcccc ugaguacuuc 25800
   acguaaucuc uuaucauucu gucucuuauu uacuaugccc cauucauaau ggcucuuuuu 25860
   caguucccua aacacaccaa cugauuccug ucucagggac ucugcauuug cuguuccuuc 25920
   ugccuggaga ggacaucucc acauccucau gacugacucu uucagggcac ucacauuuca 25980
   gcucaaauua uuuucuuuuu cucuaacuag agucuaauga ccuuacucuu ccccuccccu 26040
   cauuauauuc auguuguacu auauuacuuu auagaauaua ucauaaccua aaacuguauu 26100
   uuuccuuauu uuccuuuuuc auugcauguc ugcucuacua gacuaaguuc auaauagcau 26160
   cuucauuuuc cuagaaacca gcacaaauaa gugccucaua aacauuuguu ggacguauuu 26220
   auuauauaua auauaugcua aacaugagca ggggccacug cuaauaagug aggccugaga 26280
   cuaaaggaga auguuuagag gaaaagaccc acuuggauuu aggucaaaga accagcccag 26340
   auuaucgucu aauaucaacu acucauauaa cugcuucuca cccaaagaca gcuuaauugu 26400
   cucagugagu cuucaguuga uuuucuuggg auuaucuaug uaauugucag agauuaacgu 26460
   caaggacuag cauaauaugc caccagguga gauuuacuag ugcagaacag aacuacugcu 26520
   uaucauuuua ggagccauac gacaugacua acucuuacug gguuuauuuu cuuuuaaaau 26580
   ucccacuucu ccuuugauau uuagguuugc caaccuuauu gguuauugaa cauguaugcu 26640
   auaucucccu gcuucauggu acucacaaau augggcagca aguuguggau cuccucaucc 26700
   aagucacuaa uuaauccuuu aaacaauauu cuuugguuaa acuacuucaa ccuuuuaugc 26760
   auuuaaaaac auacuuauca accucauuuc auuauuaaau ucaaaggaau uuuaaggguc 26820
   uuucuuugau caauuguuaa aauuaacauc ucucccaauc uauccaaaau guguuaaguu 26880
   ggaugugaug uguuucuuga gaacuuauuc ugggucuugg caauucugcc cuccucuucu 26940
   cagugcucac uagccaugcu uuucagaaug ccugcuugac uuuuuuccag augaucucaa 27000
   guucaaauua guuuaugguu agaaguuaac uuuuuuuucc uuuuuugaaa aucaguacua 27060
   uauuuuccuu uuaccacucu ucuauuuucu uucccaucca ggauuugaaa caaacgcaga 27120
   uaguacucag ugaaucucau cuguaagucc ucuacauacc cugagcggua agucauuaag 27180
   aaaaagaguc uagugagaac cauuuagaau cacuuucugu auggggggcu ucaaaucccu 27240
   ccaggauaua aauaaaccuc uggguuuugg uuauuuuuau uugcugucuu ucaacacaga 27300
   gcauuuauua guuucaccau cacccuugac gacaucacuu cuguuuuuua cuuaucccuu 27360
   gaauagaugu uaucugauug uuuuaccuuu cauauauaaa ggauuuuugg uuuuacuguu 27420
   ggugggugua cuuuuuguaa acauaccugu gaugcugcuc caacaucccu gcuaagagcu 27480
   uugcugauuu uaacaaguau gugauuccac ugcuguaagc caaggguaaa cuugauucca 27540
   ccaaauuugc uuaaguguua cuucucugua uuaaagcuuc aaguucacuc acugucuaac 27600
   caaugauaca cagcuaccug aagacauuaa aaucuuucag aaauuuauuc ucaauuuuuu 27660
   ucuucuuaua aauuacuagc auaucaggau aaagaauaaa gugugugaag aggcaaaaua 27720
   aguguguacc acuaacuuga aaauuccgca acagguguca ccuuaguuuu aacacauucu 27780
   ucuguucaaa gauauaaauu cagaaucuuu gggaguaguu aauuaggugc aucugugaga 27840
   ugaacaccua uuacagaguu ggacacagga guagcauuca aacaauaccc uuuaauagau 27900
   acaaaaauag aaaaacacaa aagagaauac auggacacau gagacuaauu uuuagauaaa 27960
   agcaauuugu agauaguucu uuaauuugac uaaaaggaaa gugaagaugu cuaaauaucc 28020
   auaccuguca uaugcuuuau acuauauaau auaauauauu gguucaauuu ucuuuuauaa 28080
   aaucauauca uucuaauaaa caaaccuuca cauaauauaa aaguaggaaa acuuggacuu 28140
   cugaaaccug caaugugucu gaaauacaua aacuagaaua gcagcaaaag auagaaauua 28200
   agccuuccau gauuuugaag ucugccuauc aucaaugcuc uuaauuaacu uuugaaguug 28260
   ugauuucuuu ugaagaaaaa aaaaaacuaa agugcaggag ucuucuucaa uacugaguuu 28320
   uaguaaacau aaguuucuuu aauuaucugg uuuaauucaa uaaaauaauu uuauugugua 28380
   auuuacuucu cuaaagguga aauggaaaac aacuuaucca aacagcauua uaagagaagg 28440
   acuaccgguc ugacaaaauu cugccucuaa ggaacguggu gaauauucug ggauuaugaa 28500
   agccaacuug gcauauuacg aacacugacu uuuauaaaua acagaucuuu auaagugcua 28560
   auaguacacg uuuaauuugg uaaacaacaa caacaacagg cauuuccagc auuaaacaga 28620
   auuaaacaau uuggauguga aaauacauag gaacuauguu ucuuggcuca auaauuuuua 28680
   aguacuaguc ucacaaggaa cugaagugua guaauuacuu auguucaaca gauggcgacc 28740
   uuuacauugu agucagcaaa acaguauuug cauaaaacag uauacucaac cagcagaaaa 28800
   ucaaagauca auuacaaaua uuccauauuu ugcaagagac aaaaauuacu ugaucauaua 28860
   caccauuuuu ugacaggugg uuuuuuuaca gguuggcaaa cauauuuugu gggaauguau 28920
   cuugcuguuc uauuuuuugg ggcaagucca auugcuaauu cuuaucucau gaaaauaugu 28980
   gcaaauauca ggcuagggaa uaauuccugc uuucuuugca uagcuuauua agaauagcag 29040
   cuuuagguaa uauacucaac uaacgugucu uaauaaggau augucacaag acaccaguau 29100
   guuugugaaa cacagcaaua augaagacau gggauggguc agcaaacuau uucuucauua     29160
   aucuauuuug ugugggucaa ugaauaucaa caaaguuuua aaaaaacuua caaaacagua 29220
   uguucagaaa uauguaauuu augcuuccua aauuuucaau gaauguacaa agguaauagg 29280
   uuucuccuua agaaguaaca uuaacucuau ucuuccagug cuaaaauucc auucugaugg 29340
   ccuucagcca gaaaucaugu gggcauuauu uggugcuaga agauagccua caagaagacg 29400
   guuucccggc ucaacauaac uguuuuaguu uguguauugu aauugguaac cugaguuuga 29460
   auuguuuuuc uacuaaaggg agaagcaguc acuccucucc uaaugagcau uuuaauucuu 29520
   ugacaauugg gaagccauau cauaccuuau aauucuuaac cauugauaug guuuggcucu 29580
   guguccccac ccaaaccaca ugucaaauug uaauccccac guguugaggg agggaccugg 29640
   caggagguac cuggaucauu ggggugguuu ucccaugcug uucuugugac agugagauug 29700
   uucucaugag aucugguugu uuaaaagugu guggcaccuc ccccuucucu cucucuccug 29760
   cuccaccauu gugaagaugu accagcuucc ccuucaccuu ccaccaugau uguaaguuuc 29820
   cugaggacuc ccacccauga uuccuguuaa gccuguggaa cuaugaguca aucaaaccgc 29880
   uuccucauaa auuacugucu cagguaguuc uuuuuagcag uaugagaaca gacuaauaca 29940
   accauaauaa auaugugaaa aaucagaacc caucauucuu uaagcaaggg agauaaagaa 30000
   aagacugaua aaaauguuuu gaucauuauu uuagauaucc uuuugcuaaa cuaaaauacc 30060
   acuuuauacu uauauauagc auuaaaaauu ugaaacuaca aagauugaua uuauuauaau 30120
   auaguagaaa auugagaaca uggugagauc aagaaaaaca uuuuuuaacu uaaauauacu 30180
   uugacaauau gauuuuuugg cauaaaugug ugugugugug ugcauguaua acauaauuau 30240
   cucuaaauaa aauuuucuau ggucucccau aauucucuau ucugguaggc cauauauuau 30300
   uaauaacuuu cuauuauuaa uaguuacuga uaagauggcu guugccucuu uuaaaucaau 30360
   guuguagauu agauucuuau auuuaaacau uuccuuauua augauaaaau uuucucuauu 30420
   aguauuucug uucagagcac uaacuaagca ucucauauac cuauuucuaa aauuguuauu 30480
   cucuccucau gggaaucacc augcucuuca uguugaaaca caccaaaaac acaaaggaag 30540
   acauuaaaaa acaaauguuc auuuaaugaa aaaaagucuu ucacuaauua acaaacagaa 30600
   aauaucaaga aaaaaguguc aacuuuuggc aauuagauua cuccaacaaa guacaauucc 30660
   ccuuucaagu gacuuaggua ugguuucacu uuacuuucaa uauagguauu aauguuuuga 30720
   uauuuuaaug ucuugauauu uauaucgaac uuauauucca uuagagauag ugugugccac 30780
   gcuguaaaca acuucccuaa aauuagagaa uuggaaaaau augcaauuau auauaauuag 30840
   uaacuuuuag uaccaaacau gguggggcau acuaaaauuc ucacauucaa agaacuuuua 30900
   cuguaguuga gaaaagagaa uauggagaca gauaccuguc auccaaaggu aaaaaggaag 30960
   caccaaaaga gaggugacaa caaugcuucc aauugggucc aagucaauga uucuacuuaa 31020
   gaauaauuuu uucuuaaaug acaaucuguc aaaaugagga uuaguauaca aagacaguua 31080
   guguauguua uggaccgaac uguaucccuc cauuauucau auguugaagc ccuaacccac 31140
   aaugugacua uuuggagaua gguccuuuaa agagguaauu aagguuaaau gaagucauaa 31200
   gggugggacc cugacccaau agaacugaug uccucaugag aagaagagac cccagagcuu 31260
   ucaugagaag aacagacccc agagcuuuca cucuuucucu gugcccccac agagaaaaag 31320
   ccaugugagg agacacagug agacaguggc cauacacaac cguuaggaaa ggccucaaca 31380
   gacaccaacu cugcuggcac cuugaccuag aacuuacagu cuccagaacu augagauaau 31440
   aaguuucuac ugucccaccu aaccagucug uguaucuugu ggcagccuaa acugacugaa 31500
   aacaguauau uucuaacuga agaaacaguu caccagagau gaauuuuaua aucuuaacaa 31560
   ccaccuucaa gauuagucau uucauugaau ugcuaggccu uuacuuacag uugcagcuac 31620
   aguugcaucc aggaagaaau acgaugacua cuagaaugcc uuuuccuucc aauuuauacu 31680
   auuacacuaa gacuuuuguu uuuaaagagc ucaaacuucg guaggaaacu uaagacaugu 31740
   agaccaauau ccauaagaca aauuagaaaa ucaaguguca aaugagaggu aaaaauugug 31800
   uagcauguaa guucagaggu gacaaaauua uuccuacugu gggguggggc aguggagagg 31860
   gcgcucugua ggugagaaug uaugauugug ggaaggaaua agaaaaucua ggccagacgc 31920
   aauggcucac accgacgauc cuaggacuuc gggaggcuga ggcaggcaga uugccaagag 31980
   uucgagacca gccugggcaa cauggcaaaa uccugccuuu acaaaaaauu agccaggugu 32040
   gguggugugu gccuguaguu ccagcuacuu gggaggcuga ggcgggagga ucacuugagc 32100
   cugggagguc gaggugcagu gagccaugau ggcgccacug cacucuagcc ugggugacaa 32160
   agcgagacuc ugucucaaaa aacaaaacaa aacaaaacaa aacuuggaaa agggaauauu 32220
   uacacucaau gcagucauuc ucaguagcac acaauuggca uauugggcug gaugauucuu 32280
   cagucugugg gauucuucag ugcauuacaa gauauuuagc auccuccuuc cuuuuaaugu 32340
   cuguacauua gccuacacau uuccaaaggc uacucagcaa cagaaucucc ccaggagaga 32400
   acuagaagua aacuuuuaag gaugugaaga aaacagaugu guucuaacaa aauuuauuaa 32460
   gugaaauuaa auggcuaaua cuuaagauuc caaaugcuau auauggaaga cuauaacaug 32520
   caaaaucaua auauuuuaag uacugcuuuc cuguuuuaag uguucauuac auuuacucag 32580
   uaaauccuaa uuucccauuu ugaagcuucu cauuucauug cuucauucac auaucucuga 32640
   uuauauuuga uuucuaaaua cauuugcuug cucauuacuc uuaggcugcc ugugaugaag 32700
   ucaacaaaua aacauccuua auuuaaggca aaaaaauaaa guaaaugcau guuccuuuug 32760
   acuuccacau gacuuucagu ccucccuuaa cugaucuuuu acuuugaaga cuucuaaaug 32820
   cuguguuugu gaucuggguu gaagaucuaa ggagaaaaga ugagaaguga ccaaggcaaa 32880
   gaaagugaug agaagaugaa uaaaagagaa ccugugcaac ugaggucuaa acuggggucu 32940
   acaaaaauau gauucuuuug cagaucaggu ucuccagaaa gcagagcuua gcgcuuagca 33000
   ugcaggaugc uuauuaagaa augucucuag ggucaacaac cgugaagggg aaaggacaga 33060
   aaaggaagca ggagugggua ggagaggugg aguugcaaac aaaacuuagg acagccucag 33120
   cugauaccac agcagcucag gagcuagaau ggucccucca aguuggucuu aauugggaug 33180
   aaauaaccag guuuuuuacu ucccucucuu cccacuuauc aauuaguuca cugcaugugg 33240
   gcagcaccac caagccuagg gucuugaaga gggacugucu gcagcugagg ugauuuguga 33300
   aggggcuggc acgugaaggc ugucugccua cgacacuccc aucagcagag guaucaaguc 33360
   cuccacugaa gugguaucug agugggcagc acagugucca ccacagauuc caacuccaua 33420
   uuuuaaagaa guggaaacuu ggggacagaa aguaaucagu gaguuacaaa ucucagucua 33480
   aaccccagaa uuuaucacuu gcugcuucuu acuacuucua accaagaaaa aaaugcuaug 33540
   aucccuauuu uggaaagaau gugcaaggaa auuauuuacu ugguuuaagg aauuuucugg 33600
   aaugggauga acaaaagcaa aguguccaga caauucuagc uaagauuuua cagagaagac 33660
   uaaauccuca aauucaucac uauugugaag agcaucauau gucaaaugua auacaccaaa 33720
   guguuuucgu auuuucugua uggcuuuaug uaugcucugc aggauauucu guuuguguuc 33780
   ccuacauuca uguuaccaac auauauuuau gccucucuuu ucuaggucuu cauugggucu    33840
   caacaggaaa aauauuaaau uguuaauaau cagggcuggu accaacuaga ugagcacacu 33900
   gcaauuagca gccugacuaa ccucccugcu ucuucauuuu cuaauuuuau uugcaagcag 33960
   uuagauauga uauaauaauu ucccauuuaa aaugcuaugg auaggaaugg gaauauaagu 34020
   guuuugucua uguacaggag uucuguuuuu cuagcacacu ucccugccua gccuggacuc 34080
   cagccacacu gaccuuucug uucuguauuc uugccaugcu ccuuuccaca cuugucuccc 34140
   cacccucaag ucaucacacg gcugacuccu uuuccacucc cagcugcugg cauuaagguc 34200
   gucuccucag agguccacau aaucuaaagu aauccuuccc ugacuaagcc aaacuuauuu 34260
   uauauuaacu uauuaucuug guaacacaau uaccucagau caucuugcuu auacauuuau 34320
   cuguuauuca uuacuguauc ccuaguacau ugauuagugc cuggcauuua cauauacagc 34380
   aauuaugcau uugaaagaau gaaugcuggc aaugaguaaa uauuuacaau uuauaugauu 34440
   uuaaauacac aucagaaaca caauaauuac aaaauuacau aucuggaggg aaaaucaauu 34500
   cauucuauau uuuugaauaa uuuugaaaaa aagaaggaac ugcuacucug uguccuaaua 34560
   ccacaauuug uauuaaauaa acucuaagau gucuaucuau cugagccuuc cuaaagggca 34620
   caaugaugca cgaaagaaug cuuuuguuuu ccacaaauac uguaauuuuu gagguaacaa 34680
   aaauacauug gaccaugugu gagacagucu ucugaauaaa acacuguaag aaaaaaaugu 34740
   ugaggccagg caugguggcu cacaucugua aucccagcac uuugggaggc cgaggcacag     34800
   gcggauaacu ggaggucagg aguucgagac cagccuggcc aacaugguga aacccugucu 34860
   cuacuaaaaa aacuauaaaa auuagcuggg cagguggugc acaccuguaa ucccagcuac 34920
   ucaggaggcu gaggcaggag aaucacucaa accugggagg uggagguugc aguaagccaa 34980
   uaccgcacca ucgcacucca gccuggguga cagagcacaa cucugucuca aauuuaaaaa 35040
   aaggaaagaa agaaggaaaa ggaaagaaaa gaaaagaaaa gaaaagaaag aaagaaagag 35100
   aaagagagaa agaaagguug aaaacauucu uaagagcaac uucccaauuu cacuuuaaaa 35160
   acaagccuau aaugaagaaa gucuguauaa gagagacaaa caaaaacaag ucauaaauua 35220
   ggucacuaga uuuacaauaa uauuuuugca agaagcuuuu auaauuuuug gcuagcauuu 35280
   aagcuaggaa uauaaaaugu cccaggagau ucacugaacu caggaauuca ucuucugaca 35340
   gggucaaaug gaauguccuu uaugauccug gcugaauauu uauccucaca cucaaguaug 35400
   gaucauucca uuuaucuaag ucuaugcuuu uaaugaaaca cuaaauacuu cauuuuuaau 35460
   uaagcaaggu aauaaauuuu uauuuuaacu acaucuguuu uaaguuguaa cucuauacau 35520
   guucucuugu cuguauuagg gaaaauuuuu cccauuuacc uaaaauucuu uugagucuuc 35580
   uuucugucaa cucuguaguu auucuagaaa uuggauucuu gguggaaaau accauguuua 35640
   cucuaccaca ccauuuauuu uuaugaggcu uuaauuccau cgucucucag acaucaucuu 35700
   uuaggauccu ggaagaauua cuggguauag gaaagccugg ucuaagaaag uagauuuucu 35760
   uacagaggcc agagacacag uuauaacugg gauugauguc agcaucauaa aaacacaagc 35820
   aggcaggacc ucugucuuag uaucugaaga acugauauga caaaagaaag augucuuaua 35880
   uaucaugagg uauacuagaa augagcauag auaaauccua gaauuauuuu uaaaaugcau 35940
   uuggcaggcc aaauuuuauc aaucacuauc uaaaauuaau guuaguuucu ccaaauuauc 36000
   aagcaguugu acuagacuau aaugucacua augugaaaga auaccuggaa aaauaaaaau 36060
   agacccuuga auguaggcaa gguccuucag cgguaauuuc acacaaacaa uaauucuaug 36120
   uugcacaaua gucaacugau uuuauguuac ucacauagga uacuacauau uguaauaauu 36180
   cagggggaaa aucaaacaug auccuuuacu gugucuuauu uauuuauuua uuuauuuauu 36240
   uauuuauuua uuuauuuaga gauggaguuu cgcucuuguu gcccaggcug gaaugcaaug 36300
   gugcaaucuc ggcucaccgc aaccuucgcc ucccggguuc aagcgauucu ccugccucag 36360
   ccucccgagu accugggauu acaggcaugc gccaccaugc ccggcuaauu uuguauguuu 36420
   aguagagaug ggguuucucc auguugguua gucuggucuu gaacucccaa ccucagguga 36480
   ucugcccgcc ucggccuccc aaaaugcugg gauuacaggc gugagccacc gcaccuggcc 36540
   ugccguguuu uauacccaua uuuuaguaac cucacaaagu uaaaaaugau uuccaaauga 36600
   aaugucagag aauuguuuau uuuacccagu uauacuguaa uaaguccugg auggcacugu 36660
   gucuagccuc aaggaaacag aacaaguaca aagcuaaguu aaaggcaacu uuauuacaau 36720
   acuuuuccaa acuucaaaau gcauuuauua cuaaauuuag cucuaauaua uaauacacua 36780
   uauuacaagg cagaaagcau uucuggucau auauuuagcu uccauguauu gcccaaaaca 36840
   cagcuuccua ggaauagcag cagugcacag uaguuuacgu cacuaaguaa aauauuucuc 36900
   ugaugucuca caaucauuca uauuguaauu uauuuuaugu ucuucugauu aaaaaaagca 36960
   aagcuaucuu uuccauaucu uauuaguuaa uaccuagaaa uugucuauau ucuaaaaaua 37020
   uccacuagag auauuuccuu ugauaggaaa uaggauuugc aaauccaacc aaaccaaaga 37080
   auacuguauu ucuccauugc aaauaggggu aaaauucucu ucuuuccuuu uucaggagcc 37140
   aaaucuggac cuccuccucu ucauagagcc auccugaaua guaccuacuu uaaaagauac 37200
   cuaaaucuac aaugcacgca cucuucuuua auaaagucuu uguuuauucu ugccuccuua 37260
   uauuuacuua uuuagaauaa uuuacaauca acuuugaaug uaacauaaua augaacuuaa 37320
   aaaguaaucu ugggagaugu uaauaauaua aagaaaguau uuuccaaauu agacaaggac 37380
   uuuauuugcu cauuauccuc ugcuucaacc aaccaacuaa ccaaauucaa cuuaugaaaa 37440
   uaauucguuu uccaagauua cuacccuaau cacaacaaaa acagauacaa auccacaaau 37500
   agaucuaguc ccuuaauuuu cuauuuuuuu uuuuuuuugg uuuuacauuu gugagcuacc 37560
   aacuagcaca aaacauguuc aucuuccuuu ucucacaaca auaggaaauu guuagaaaag 37620
   uauagaaaaa guucuauuuu uucauaaggc aaauuuaaua cagauuuugc uaugaaguug 37680
   aaaaaaaaaa aguaaaugaa aagguaugug cuggcugcuu ggggggcaag gcucauaaau 37740
   cacuguacgu uaagagcacg ugcugaaacu ucauggugcc agucacguaa uuuuguguau 37800
   uugggacuaa agacgugaag ggguaucuuu ucccugugga aauacaaaaa aaaaaaaaaa 37860
   aaaagaguga uagaagaguc auaagaaaua gaaggaaaaa gguucuagac uaauuccauc 37920
   augcagaaaa caugccuuau aaaagcuuaa uuucacauag cuguuuuauu aaguggcaaa 37980
   aauuuuaagg guagagcauu uuuagaaaua uagcaauuaa uccacagauc ccucuuggaa 38040
   acauacauuu uuauguucaa aauaaaguga cugcaagaca acacacgagc ucauucacag 38100
   acauauguau uguuagcaaa gcaccccgaa auauacccuu cugcccacuu cuuauuucaa 38160
   uacuagggaa aacaagccuu aaaauccaga caauaucuac uacuuuuaaa aucugcaagu 38220
   uguacauuua ggucacacaa aaggcaccaa aguuaaaugc uuagcuucua gcugcaaaga 38280
   cgaaaugcca uaccauuaua cuugacaauu ugaaaaacua aauugagacc auucgugaaa 38340
   ggaaaacauu caauugucaa aaagauagag aacuuacuuu gccuucuuug uauuaucggc 38400
   uucagauucu uucacugaaa uauaauaagc aauauccaau auuaguuugu uuuguuuaac 38460
   uucaaaauca cuuaagcaau uaccugcacc aaucaauauc aaauguucaa cuuccaugga 38520
   gccacuaaaa cgaauuccau aguagccugu uauuggccau aaacuauagu uaguacaacu 38580
   acuauuacau cuauacuuuu caauguuacu cuccugggcu gguguuuuau uuauuuauuu 38640
   auuuauuuau uuuugagaca gagccc 38666
<210> 4
   <211> 25
   <212> DNA
   <213> Artificial Sequence
<220>
   <223> primer
<400> 4
   gggtaatact gcaccttcaa ttgac 25
<210> 5
   <211> 20
   <212> DNA
   <213> Artificial Sequence
<220>
   <223> primer
<400> 5
   ataagaacaa tggcatgcgg 20
<210> 6
   <211> 22
   <212> DNA
   <213> Artificial Sequence
<220>
   <223> primer
<400> 6
   gaaaaagtct caaccggtcc ta 22
<210> 7
   <211> 22
   <212> DNA
   <213> Artificial Sequence
<220>
   <223> primer
<400> 7
   ggggatttct tgaagatttg gg 22
<210> 8
   <211> 20
   <212> DNA
   <213> Artificial Sequence
<220>
   <223> primer
<400> 8
   tgctggaaat gcctgttgtt 20
<210> 9
   <211> 21
   <212> DNA
   <213> Artificial Sequence
<220>
   <223> primer
<400> 9
   attctgcctc taaggaacgt g 21
<210> 10
   <211> 20
   <212> DNA
   <213> Artificial Sequence
<220>
   <223> primer
<400> 10
   ttttggtccc cttgcaatct 20
<210> 11
   <211> 21
   <212> DNA
   <213> Artificial Sequence
<220>
   <223> primer
<400> 11
   gaagggatgg aaaagcattt c 21
<210> 12
   <211> 21
   <212> DNA
   <213> Artificial Sequence
<220>
   <223> primer
<400> 12
   acggcctttg gaagttcaga a 21
<210> 13
   <211> 24
   <212> DNA
   <213> Artificial Sequence
<220>
   <223> primer
<400> 13
   ctcacaaatt tcattagact tgga 24
<210> 14
   <211> 24
   <212> DNA
   <213> Artificial Sequence
<220>
   <223> primer
<400> 14
   actttggccc ctactaactt ctca 24
<210> 15
   <211> 24
   <212> DNA
   <213> Artificial Sequence
<220>
   <223> primer
<400> 15
   gagggctagg tgaagaagta catt 24
<210> 16
   <211> 20
   <212> DNA
   <213> Artificial Sequence
<220>
   <223> primer
<400> 16
   gaatccaggc acaggttagc 20
<210> 17
   <211> 20
   <212> DNA
   <213> Artificial Sequence
<220>
   <223> primer
<400> 17
   aactgagttc acccccaacc 20
<210> 18
   <211> 20
   <212> DNA
   <213> Artificial Sequence
<220>
   <223> primer
<400> 18
   ctgtgcccca gtgtgaagta 20
<210> 19
   <211> 20
   <212> DNA
   <213> Artificial Sequence
<220>
   <223> primer
<400> 19
   gggcccagtt cactttctct 20
<210> 20
   <211> 20
   <212> DNA
   <213> Artificial Sequence
<220>
   <223> primer
<400> 20
   tttcttggtg ttggggtctg 20
<210> 21
   <211> 22
   <212> DNA
   <213> Artificial Sequence
<220>
   <223> primer
<400> 21
   tctgttgttt aatgccatca cc 22
<210> 22
   <211> 75
   <212> DNA
   <213> Artificial Sequence
<220>
   <223> probe
<400> 22
   cgaaagccat gacctccgat cactctccat aacatcttac caatttatac tcccatggca 60
ctgccgcagg cattg 75
<210> 23
   <211> 85
   <212> DNA
   <213> Artificial Sequence
<220>
   <223> probe
<400> 23
   tcagtaaacc ctaaccaaat gacagggata gagatcattc atcccacctc cataaaattg 60
gttttgcctt tcagcaattc aactt 85
<210> 24
   <211> 75
   <212> DNA
   <213> Artificial Sequence
<220>
   <223> probe
<400> 24
   cgaaagccat gacctccgat cactcaagct gttgtgtggg ttcttccctg tgagaatctt 60
aaggatggaa atggt 75
<210> 25
   <211> 85
   <212> DNA
   <213> Artificial Sequence
<220>
   <223> probe
<400> 25
   gagcacaaac cttggcattt aaggaccgac aatgctgaga tgttgtggtg cagataaggt 60
tgttattgtg gaggatgtta ctaca 85
<210> 26
   <211> 75
   <212> DNA
   <213> Artificial Sequence
<220>
   <223> probe
<400> 26
   cgaaagccat gacctccgat cactccacaa actaaaacag ttatgttgag ccgggaaacc 60
gtcttcttgt aggct 75
<210> 27
   <211> 85
   <212> DNA
   <213> Artificial Sequence
<220>
   <223> probe
<400> 27
   atcttctagc accaaataat gcccacatga tttctggctg aaggccatca cttccttcct 60
gtgttccagc tacaaactta gaaac 85
<210> 28
   <211> 14998
   <212> RNA
   <213> Homo sapiens
<400> 28
   guuugccugc acaagcucuc ucuuugccug cugccaucca cgugacuuuc uccuccgugc      60
   cuucugucau gauugugagg ucuccccagc cacauggaac uguaagucca cuaaaccucu     120
   ucuuuuguaa auugcccagu cucagguaug ucuuuaucag cagcgugaaa acagacucau     180
   acaacuaaca agccugcuuu agcuuuucac uguucugccc aagcaacuuu ccccuucagc     240
   caggccagua gauuucucau acagcagggu gacccuguuc agggccuuug uacuguuuau     300
   auuuuuuacu uugugcauac uaugaugucu ugacguuuug gaaaccucuc uggcugggga     360
   gagacugucc uucccagagc uggccaguuc uuagaaccag aagggcccag ccaggagcac     420
   agcucugaua ugcaaaccaa ccaacccaga gcuaugccgu cuuuaucuag ccuguccacc     480
   ccaggaagca guauuucuuc accuauauca ugccagagcc ugauaacaag caacuagaaa     540
   ccaugccugu agcuuacagc cugccaaaau uauucaaacu agccaauucu aaacuguuca     600
   caugcacucc ccugcccugu cccugaaagc ucaagaaaga ccauggccca ggcucucccc     660
   ucacuccugc cccuucaggc uccuacaggg gggaccaaga cugaugcuuc cccauguggc     720
   ccugcagggc augacaugcu ucccgucucu aagaccugug aguauaauaa ccuuggccuu     780
   ccugagcacc uccucugccu ccuccugugg ccacaucuca cugaccauca caaaaaacaa     840
   cauagaauaa gccaggcaug guggcuuaug ccuguaaucc cagcacuuug ggaggccgag     900
   gcaggcggau cacuugacgu caggaguucg agaccagccu ggccaacaug guacaacccu     960
   gucucuacua aaaacacaaa aauuagccag gugugguggu gggugccugu aaucccagcu    1020
   acucaggagg cugaagcagg auaaucacuu gaacccggga ggcagagguu gcagugagcc    1080
   gagauugugc cacugcacuc cagccugggc aacagagcaa gacuccgucu caaaaaauaa    1140
   aauaaaacag guuuucauuu uaauuuuuaa cauauaaacc uccacaggaa cauucugaca    1200
   cagacuucag ccccaggccu acaucacccu augauccaaa uuauuuuaua guaaauggua    1260
   ugaaguuugg agaggaaggg caauuuggcc aucaugcugu gauuuuacug cagguaaagu    1320
   agaaucacca ugaaaguggu ggagccuugc uuuuucuuua auuuuuauaa ugccaccacc    1380
   cuacauucuu aggagaaaag gcaaggaguu aaauaaauaa gcacagauuu aaguucacau    1440
   uucugguaau uauccgcaag cuaauaacug agauugcuua agcucuguaa acaaaacaca    1500
   caaagcccuu guggcucugc gccagaauca aaggucugau ccaaaacucc cccauccccu     1560
   uguuggcuuu gaacaugaaa ccaauuuucu acaccaaauc aggccuacuu cuuuguaacc    1620
   auccuuacca ucucucuuau aggaaaaugu uuaggacucu uggcggugac ucuagaacua 1680
   ggaaaucaac ucucucagca gcacucuaau gagcaaaaac aacugugccu caaaauacag 1740
   gcugcagugg gcagaaaucu cuguacugca cuccagccug ggugacagag uaaguuucug 1800
   ucucaaaaaa aaaaaaaaau caaguaacaa agaacagcug ucuaaaacug uuaacacuga 1860
   uucaaaauau aauaaccuuu augccuguua uucagaguua aaguggguac gcuaauuaua 1920
   aaucauacac uuuucuaauu gugcaauacu cugucaaugg gauagauuuu aggaaaauuc 1980
   cagcugaacc aguccauucu uugaaucauu aggucaauua caaacaugcc aauccuuagc 2040
   cugaauagcu gcauauuuag aaagaccuaa aacauucuuc aaaaagcugc cauacauggu 2100
   cuguuaaagg gcuuacuugg uaggaaauaa aucaagaggg aauacuccau agugaaucuc 2160
   agggcacuuu uuaaaacaaa ucucagggcu gagcacaaug guucaugccu auaaucucac 2220
   ugcuuugagg ggccaaggca ggaggauugc ucaagcccag gaguuugaga ccagccuggg 2280
   caacauggca agaccuaguc ccuacaaaaa aauuguuuua uuagcuaggc augguggaac 2340
   acgccugugg ucccagcuau uccagaggcu gaagugggag gaucacuuaa gcccaggaag 2400
   ucaaggcugc agugagccau gaucucacca cugcacucga gccugggaga caguacagga 2460
   cccaucucca aaaaaaaaaa aaaaaaaagu gaacgaagcu gcaauaagcu gugaucauac 2520
   cacugcacac uagccuaggc gacagaguga gacccugccu caaaaaaaaa aaaagaaaaa 2580
   aagaaaaaac caauugauaa aagaguaacc ucccaauccc cagcugugga acccaagagc 2640
   agagaaggca gccacccgcc ucucuuuccc ucaaagacac cagagugcaa uaggcucuuc 2700
   cucacucauu cccaugggaa guagccuuca accccucuca aaccacagca uauagagaug 2760
   ccuuuauuaa uaaaaaagga uacaacccac gggccagccg caguggcuca cgccuguaau 2820
   cccaacacuu ugggaagcca aggcaggcag aucaccugag gccaggaguc cgagaccagc 2880
   cuggccaaca uggugaaacu cugucucuac uaaaagcaca aacauuaacc gaguguggug 2940
   gcacacgccu guagucccag cuacuuagga ggcugaggca ggagaaucgc uugaauccgg 3000
   gaggcaaaag uuggaguuag ccgaagucgc accacugcac uccagacugg gcgacagagc 3060
   gagacucagu cucaaaaaua aauaaauaca auaaauaaau aaacaaagua uacaacccac 3120
   aacucacuuu uuuuucuaac caggaaucuc aacaacagga cuauagggaa acaagaauua 3180
   uuaaccucau uaauucaaau uaacucuuaa gcacccacca cuaacaggca cucuuauagg 3240
   uucuagaccu aggaucauuu uuacagaguu cacauugggg aagggagagg ggagagggaa 3300
   aggcaaucac acuaaaaaga gggggucaga guaacaguaa gauccacaca gaaggccagg 3360
   cacaguggcu cacaccuaua auccaagcac uuugggaggc caaggagggu ggaucaccug 3420
   aguucaggag uucgagacca gccuggccaa cauggugaaa ccuugucucu accaaaaaua 3480
   caaaaauuag ccgggcaugg uggcgggcgc cuguaaucuc aguuauuugg gagccugagg 3540
   caggaggauc gccugaaccc aggaggugga gguugcagug agcugggacc augccauugc 3600
   acuccagccu aggcaacaag agugagacuu cucaaaaaaa aaaaaaaaau ccacaaagaa 3660
   aguagggagc caugguagug aaugggggcg uuuacucuag augggugguc aaggcagagu 3720
   uucccaggag gacaaagccc ccagugacaa gagcaggguc aacaaguucc aagcaaagag 3780
   aacagcaagu gcugaagccu aaggcuaaug cacuuacaga aaaauccuua augacuaagu 3840
   guguugccuc uguuugcgca gagaucacaa aacguguccc gcagaaggga gagaaaaagg 3900
   gaccccagga gaggguggcg gggcuucagg uccuggagaa aggcagcaag cauggcuaac 3960
   acauuccuug guccacccug caggguaaua cugcaccuuc aauugacuua ugaagaaacu 4020
   agcucagauu ggaacaacag ccaggacagu caccaccaag gggacuugcc ccacuggaga 4080
   agagaccgga gugggagaga aacuguuaca gccaaucacu uccuuccagg aaaaaaaaag 4140
   acgcaaauaa caacacaaaa aaacgugauc uaauagacaa uccagauaua aagaacacaa 4200
   acccuuuucc cuacaauuua accaaaugcu aaaaugcacc aggaaacaca ggauacuaca 4260
   gagauaaaga uuuggccagc agauuaucaa ccccaggaaa agaaauccau uucuguagca 4320
   ugcuaugcug uuggaggcac acgucacacu ucuuaauuca aggacagaaa agcacaaacu 4380
   aaaaugcaca cuggaguaug acucccccuu cucuaucugc uaaagcgcuu cgcaugugac 4440
   uuucaaucuc uaagcuggga aucaaucagg cagcccagcc uccuauauuu ucuaguugua 4500
   uugcuuugag caucaaauuu aaccucugug caccucaauu ucuucagcug gagacaaaga 4560
   aaguggcugc acuguggcag aguguuugca aauguuaauc augguuaugg uagcauucua 4620
   ccuugucuua cagaugguug ccuaaaaagg uaggaaacug uucacgaaaa agucucaacc 4680
   gguccuagag ugaucagaau gacagcgcug aaauaaggag ggcaguaccu cuuauuccag 4740
   agaagugggg agcagagagg aagauggagu ggaaaggggc gaggcaaggc ccuccugaaa 4800
   uaccucaacc caaaucuuca agaaaucccc aaguccucac agugcuuuuu guggauuuuu 4860
   guggaaaccg guaaaagggg cugauuugcu gaccccagug gguagaaaac agagacuguc 4920
   aagagaacag aagagaaggc agaaagggga uggggaagug ggguucgcca uguucacgag 4980
   cuccuggagc cacagggccc cccaggaaca acagagcuga gacugggugg ccuuguuucu 5040
   ggcccaauuc ccugggacca ccacccugga aggacacuug agcccuacuc aaggacccac 5100
   cacaacaucu cagcauuguc gguccuuaaa ugccaagguu ugugcucacc auuuccaucc 5160
   uuaagauucu cacagggaag aacccacaca acagcuuugu cccugcgacu cugggugagu 5220
   uucugacacu ggccugggcu cccguuuugc ccgucaccaa cuucucacag uauaacuauc 5280
   caagagaagu agcauuugau gucagggaau caaaagggcu aaggcucggg aggccaaggc 5340
   aggugaauca cuuguaauca ggaguucgag accacccuga ccaacauagu gaaaccccau 5400
   cucuacuaac aauacaaaaa auuaaccagg ugugguggca ggugccugua aucccagcua 5460
   cucaggaggc ugagguagga ggaucacuug aacccaggag gcagagguug ugguacgcca 5520
   agaucaugac acugcacucc agccugggug acagagugag acuccaucuc aaaaaaaaaa 5580
   aaaaaaaagg ccuaaggcuu acucaggguc aucuaugguu ugccaagcag aguucaaucc 5640
   aaucaacaaa acccucacug cccacuuuuc caaaaccuga uauagucagu cacuaguagc 5700
   ugccauuugc uguuuugccu ucuauuaaaa aaaaaaaaaa aaaaaaaacc auccaauucc 5760
   cagaagucaa aggcaauucc uucccuuuuc ugugucugug agacggcucu cugugggauu 5820
   ccuuccugcu gcaccucauc cuuccuggaa gggacucuca gaaaaugugc cuccuccuua 5880
   caggaaagga auuccucccg aggaacugug gacaugaggc agcuuugugg aauuucccuu 5940
   ccacagacuu cagccuauuu ucugggcaaa cuuagcagaa augaccaaaa uccacucgag 6000
   agagacagca acaaggagag gggaagagau ccuauuuugg cuucuucaaa uggaccaauu 6060
   uucucuuuaa aauauuuauu uuaaggaaaa acaaggacua cauuauauuu uaaaggggga 6120
   gaaggaagag gauuuugagu ugcuaagaga acagagccau cucuuuucuu uuaauacugg 6180
   augcuaagaa cuugcacagg guuuucccaa aagccagaua guucagguaa uuugcaugug 6240
   gcuguaaaag agaaaguuca ggccaggcac uguggcaaca uggugaaacc ccaucucuac 6300
   uaaaaauaca aaaauuagcu ggguguggug gugcaagccu guaaucccag cuacuuggga 6360
   ggcugaggca ggggaaucac uugaaucugg gagguggacg uugcagugag ccaagaucac 6420
   accacugcac uccugggcga cagagacucu cucaaaaaaa aaaaaaaaaa aaaaaaaaga 6480
   gagagagaaa guugagagca uaagucugag ugauucugcc ugaaagaaca gaacagcuua 6540
   cacuggacag gcuuuugccc aagacacacc ucaucuggca uugagaaacg aacaccaaca 6600
   auaaugagug acagaauguu aagaacaaua auuauacaga cuuggaccuc ccagacugcu 6660
   ugucuguagu aauucagaag gaagcaaagu auucugaagu guuuaugaag auugguucca 6720
   caucugaaga auuuuaguaa ucuuacccaa acucaaccca agaaauauga ggaaaagagg 6780
   gaguaguucu auaauaagca gguggaauag agggucaaac uccuuggcga guucagucua 6840
   acagaggaca cauuacugcc cuacaaagca gcucuggaaa augacaacca guggcacuaa 6900
   cuccaggcaa uaucuuugug aggcuugugg gcuguaauau agucugcuca ggcugccaua 6960
   aaaaaaaaau cacaaucagg ccaggcacua uggcucacac cuguaauccc agcacuuugg 7020
   gaggccaagg agggcagauc accugagguu aggaguucga gaccaggcug gccaacaugg 7080
   caaaaccccg ucucuacuaa aaauacaaaa auuacccgga ugugguagca cgcaccggua 7140
   gucccagcua cucgucaggc ugaggcagga gaauugcuug aaccugggag ggguggaggu 7200
   ugcagugggc ugagaucaca ccacuguacu ccagccugga caacagagcc acugcacucc 7260
   agccugggca acagagagag acuccaucuu aaaaaaaaaa aaaaaaaauc cccgucuuca 7320
   aauacaauca cauucugaag uacuagagau caggacucca acaugcgaac uuugggggga 7380
   cauaauucag cccguaauau gggugauaaa ggagcagguc cagacuuaga gaaacaagga 7440
   cucccugggu caagcucugg aagugaaagg cuucuucaaa gacaggaaag ucccacugaa 7500
   gguggcuuug gaagugcacc cuugcagcac agcaaccuuc uugauuugcu caagagcagg 7560
   gguucugaca ggucucaaag cacuuuguuu aaugaaacag aaucgaaaau accagugugu 7620
   cacauguuuc cuaagacagg acacuguuuc uugaaacuuu uacuaaagua uguuuuucuu 7680
   uuauuuuuuu auuuuuuauu uuuuuugaga aggagucuug cucugucgcc caggcuggag 7740
   ugcaguggca caaucucgac ucacugcaac cucugccucc cggguucaaa caauucuccu 7800
   gccucagccu ccugaguagc ugggauuaca ggugcacgcc aucaugccug gcuaauuuuu 7860
   guauuuuuag uagagacagg guuucaccau guugaccagu cugcucucaa acuccugacc 7920
   uugugaucug cccaccuugg ccucccaaag ugcugggauu acaggaguga gccaccgugu 7980
   ccagccuaaa guauguuaaa uauguuaugu gugagagaga gaaagagaau auaucucuug 8040
   gucauuacau auguaaaacu uguagaacgu gaucaaaaug uuuaaaaacu ggccaggcac 8100
   aguggcucaa gccuguaucc cagcacuuug ggaucccaag gcagguggau cacaagguca 8160
   ggaaucaaga ccauccuggu caacacagug aaaccccauc ucuacuaaaa auacaaaaaa 8220
   uuagccuggc gcgguggcac gcaccuguag ucccagcuac uugggaggcu ggggcaggag 8280
   aaucgcuuga accugggagg cggaggcugc agugagcuga gauagcgcca cugcacuccu 8340
   gccugggcaa cagagcaaga cuccaacuca aaaaaaaaaa aaaaaguuua aaaacugguc 8400
   cuguggcagu gggucucaaa caucagcaug caucagaauc acuuggagga cugauuaaaa 8460
   cacaggcugc ugcugaugcu gcuaauccua gagccaccuu gaaguuuuuc ugugguuuuu 8520
   uuuuuuuuuu uuuuuuuuuu uuugagacag ggucuugcug ucucccaggc uggagugcag 8580
   uggugcaaug auaccucacu gcagccuuga acuccugggc ucaagcaaug cccccaucuc 8640
   agccucccaa gcaacuagga cuguagcuag cuccuaacuc auucccucca uuccugcccu 8700
   ccccuaacuu uauauugcag ccagaaggau auuuuuucug aagagcaucu caugcugagu 8760
   ggcagcagaa ugcagagauu aagagagggg gccaggcaca guggcucaca ccucuaaucc 8820
   cagcacuuug ggaggccgag guggguggau cacccgaggu caggaguuca agaccagccu 8880
   agccaacaug gagaaacccc gucucuaugg gcgugguggu gcauaccugu aaucccagcu 8940
   acuagggagg cugaggcagg agaaucacuu gaacccagga ggcagagguu gccgugagcc 9000
   gagaucucgu cauugcacuc cagccugggc aacaagagcg aaacuccguc ucaaaaaaaa 9060
   aaaaagagug gcgccaggug caguggcuca cgccuguaau cccagcacuu ugggaggccg 9120
   aggcaggcaa aucaucugag gucgggaguu cgagaccagc cugaccaaca uagagaaacc 9180
   ccgucucuac uaaaaauaca aaaaaauuag ccgagcgugg uggcaugugu cguaugccug 9240
   uaaucccagc uacuggggag gcugaggcag gagaauuacu ugaaccuggg aggcagguuu 9300
   uuuguuuuuu guuuugguuu uguuugagau uuugguuguu guuuuuuggg guuuuuuuga 9360
   gacagggucu cacuguguug uccaggcugg agugcagggg ugcgaucaug ugucacugca 9420
   gccacaaccu ccugggcuca ggugauccuc ccaccucagc cuuccugagu aacuguuacu 9480
   acaggugugc accaccauga cuggcuaauu uuuguauuuu uuguagagac aagaguuuca 9540
   ccauguugca caggcuguuc acaaacuccu aggcucaagc uauccaccug ccucagccuc 9600
   ccaaagugcu gggauuauag gcaugagcca ccuugcccag ccaacugagc cacuuugugc 9660
   caggacuagg ucagguacuu gggacauaua caggcaagga cacugcccac agggaacuga 9720
   cagugucuag cagcuacaca gaugacacgg ccuuuucggc agacaccccu guccccugug 9780
   cuuggcaaac agcagagacu caaaaaugua uaugguuuga cucccacugg aacauaagcc 9840
   ccuuagggca gggaccaggg ucacuguauu cccagcaccu auaaucaaga gguacaugac 9900
   gcugcagcug agagcaggga cucugugcua aguuaccuac cugguuugaa ccuaggcucu 9960
   gccacucaaa ggcuguguga ccgugggaaa uaauaguacc caccccaaag guuuguuauu 10020
   aguauuuaau aauauuuauu gguaagguac uuagaauagc cccccugccu acaggaagca 10080
   guacauaauu auaaugucua aauauacagu aguuccaggc caggcauggu ggcucauguc 10140
   uguauuccua gcagaaugag aggccgagga aggagaauca uuugagucca ggaguuugag 10200
   accagccugg auaacauagu gagaucucgu cucuacaaaa aauaaacaaa aguaagccag 10260
   acaugguggc acaugccugc aaucccagcu acucaaaagg cugugauuag agaaucacuu 10320
   gagcccagga gguagaggcu gcaaugagcu gagaucacac cacuguacuc cagccugggc 10380
   aacagaguga gacuccgucu cuuaaauaaa uaaauaaaua ggggccaggc gugguagcuu 10440
   acaccuguaa ucccagcaca uugggaggcu gaggcgggag gaucacuuga ggacaggagu 10500
   ucaagaccaa ccugaccaac auggugaaac cccaucucua cuaaaacaca acaaucagcu 10560
   gggcauggug gcgggugccu guaaucccag cuauuugggg ggcugaggca ggagaaucac 10620
   uugaacccag gaggcagagg uugcagugag ccaagaucac acuacuacac uccagccugg 10680
   gcaacagagc gggacuccau cucaaauaaa uaaauaaaua gcaguuccau auccucugaa 10740
   uaauggacua gaaguugguu aauggauggg uuuucaggga gaauuuuagc agccgagcug 10800
   agagguaggu acucuagcca ggguauauaa caugagcuaa gggaacagga cagcaaauau 10860
   aaggugugau caggggaaag gcaucauaua uugacugucc aggaacaaug gauaucugag 10920
   aauguaggaa agcuaaguua ggaccaggaa aaggagagua uuaaaugcca aagaagaugc 10980
   caccaaaggu ucuggaacag aaagcagcag agcuaugccu uguaaagaua aagccugcag 11040
   uguauugacu ggguuggaau cuggaaucau ugcgugccuc cagucaagac guggcaacug 11100
   accaacuacc uuuuaucacu gaccagacuc uucccuacgu gcagauaacu gaggacgcaa 11160
   gccuuaguua cugggugugg uggugcuauu aacggaaaaa gggaagucag ccagcaaagg 11220
   cugacugggg caauuucagc cuuguugcuc ugcugugaaa cugcuuucca gaaaaacuuu 11280
   uuuuuuuuuu ugagacggag ucucacucug ucacccaggc uggagugcag uggcacaauu 11340
   ucggcucacu gcaaccuccg ccucccaggu ucaagcgauu cuccugucuc agccucccca 11400
   gaagcuggga uuacaggcgc ccaccaccac acucagcuaa uuuuuguauu uuuaguaaag 11460
   ucaggguucc acuauguugg ccaggcugau cuccaacucc ugaccucagg ugauccgccu 11520
   gccucggccu cccaaagugc ugagauuaca ggcaugagcc acagugccca gcccaggaaa 11580
   acuucuuaaa gauguaauaa uaacgcaccu uauuauuuuc caaguacuuc cauagauagu 11640
   ggcccacuuc ugaguaugaa uuaaauuucc cuuuguggcc uuuguuuuuu uaauagaucu 11700
   ucauuucugg ggggacuuac aaacagguga cuucuccauu caccagagau gggacauuau 11760
   acacugaccu acuuccauau ucacuuuccc ugcuugauug gaggugaggg agaaaaauua 11820
   guguauaagg ccgggcgcgg aggcucacgc cuguaauccc aggacuuuag aaggcugagg 11880
   cagguggauc accugagguc aggaguucga gaccagccug gccaacaugg ugaaaccccu 11940
   ugucuacuaa aaauacaaaa auuagccagg ugugguggca cacgccugua aucccaguua 12000
   cucaggaggc ugaggcagga gaauugcuug aaccccgagg cagagguugc agugagcuga 12060
   guccaugcca uugcacucua gccugggcaa caagagugaa acucugucuc aaaaaaaaaa 12120
   aaaaaagaaa agaaaagaaa aauaagugua uagaacuuua uuguuucauu gaccgggcac 12180
   gauggcucac gccuguaacu cuagcacuuu gggaggccaa ggugggcaga uuggcugagc 12240
   ucagcaguuu gaaaccagcc ugggcaacau ggugaaaccu aucucuaaua aaauacaaaa 12300
   aauuagcccg guaugguagc acaugccuau aguuucagcu accugggagg cugaggcaug 12360
   agaaucacuu gaacccagaa gguugcagug agccaagauu gugccacugc acuccagccu 12420
   gggcaacaga gcaaaacucu gucuccaaaa aaagagaacu ggccagggau gguggcucac 12480
   gccucuaauc ccagcacuuu gggaggcuaa ggugggcaga ucacgagguc aggaguucaa 12540
   gaccagccug gccaacaugg ugaaaccccg ucucuacuaa aaauacaaaa auuagcuggg 12600
   cauggugcca ggcaccugua aucccagcua cucaggaggc ugagacagca gaaucguuug 12660
   aaucugggag guagaaguug cagugagcca agaucacgcc auugcacucc agccugggug 12720
   gaagggcaug acuucgucuc aaaaaaaaaa aaacuuuuuc cuuaaaauac accucuugga 12780
   gccaguccca gauacuuggg aggaucagga aggaggaccu cuugaggcca uaaauuugag 12840
   gauguagugu gccugugaau agccacuaca cucuagccug ggcaacacag aaagaccuca 12900
   uaaauaaaua aauaggccag augagguggc ucacaccugu aaucucagca cuuugggagg 12960
   ccaaggcugg cagaucacga aguuaggaga ucaagaccau ccuggcuaac acagugaaac 13020
   cccgucucua cuaaaacuac aaaaaauuag ccaggcaugg uggcaugcac cuauaguccc 13080
   agcuacucgg gaggcugagg cacgagaauc acuugaaccc gggagacgga gguugcagug 13140
   agccgaaauc gcaccacugc acuccagccu gggcaacgga gugagacucu gccucacaca 13200
   aauaauaaau aaauaaauaa auauuuuaua auguauuuuu aaaauauagu ucuuaaaaca 13260
   ugccaugagu agguuuaauu uuucuaaauc auagaauagg auacugaagc ccagaguggu 13320
   caugugucuu gccuaagguc acacacagcu aauauauaca agaucauggg ucuucuggcu 13380
   ccagucugag gcugucacaa uaacuagcuu cucacuuggu ccuaccuaag acacaggugu 13440
   cagcaacucu aaauucaaga aauugagauu aagacuaaag uaauagcuua uaaacauuaa 13500
   uuaagcauau uaauugcaag auguuaaacc agucacugag agauuaagac auugaagaua 13560
   cugguuucug ccuuaauaga gcuaacaauu uaguaaaaaa gucgcaaaug agucaagacu 13620
   uaaauaagac uuaaagacuu aagacaauaa aaaaggcaug auaaaagggu aaauuuaggc 13680
   uggacgcagu ggcucaugcc uguaauccca gcacuuuggg aggcugaggu gggcggauca 13740
   ccugagguca ggaguucaag accagccugg ccaacauggc aaagucccau cucuacuaaa 13800
   aauacaaaaa uuagccaggu gucauggcac aagccuguaa uuccagcuac ucgggaggcu 13860
   gaggcaggag aacugcuuga acccagaagg uggagguugc ucaagagcaa gacucugacu 13920
   caaaaaaaaa aggauggggg auaaauuuag caagaacccu caucauccca uuuaccuaca 13980
   gccuaauaug uacuaggaac uuacauauac aauaauaggu aaaaucuauu aagaauguau 14040
   uauguuuuau aguugaacaa auugugucgc uuaaucaacu uagaauucau guaaaugcca 14100
   cauuugagcu cuguuacauu gagagcucuc caggcccagc cuaccauguu aagccauuga 14160
   uaaaccuccc ugcuacuauu uaaaacaaga uggauaccuu ucuacugucu uuaaagagga 14220
   agaguacaca cuguuggacu guaugcacuc uccgucucuc aagacaguaa ucugagggau 14280
   uacuucaaua gcagaaguua aaagaagcgg ucaguugggu uuauuacaua aucucaauca 14340
   cauccugacc aauaaaaugc accaauaaag aucagcaaau aaaagcaagu gauuagaaga 14400
   gcaauuuuau gcucacgagc ucugaauuca caagugugag auuuccuguc aaguacucuc 14460
   augagcucaa uuaaucucug acccagggug agaaaauaaa cauagugacg cagaggcugc 14520
   agcucaugcu uuggaauuug ucagacuuga cucaguuccc aacuccauca uguccugccu 14580
   gggugacccu cauuaugcua ccuggacucu cucaguuucc uucaccugua uguaacacaa 14640
   gagcagagcc acccucaaca agggguuacu auaaaaaucg aagaacccgg caucucuaac 14700
   aacaucccug gcacggagca aucccccuuc ucccucagcc acuggcugag cacccacaga 14760
   ggggaguaag uccuauugug guaaugguuc ucaugcuuug agaucagaca aguccuuuga 14820
   gaggaaccug aaaaaggggc agcgaaccuu uggccagaaa aauggaaaua ugcacagaaa 14880
   uauuuuugac uuuggcaagc aguugaucag ugguucuugg gcuucagugc ccaucagaaa 14940
   cguagagaac cuuuuuaaaa uauugaugug cggggcgcgg uggcucacac cuguaauc 14998
<210> 29
   <211> 14985
   <212> RNA
   <213> Homo sapiens
<400> 29
<210> 30
   <211> 11120
   <212> RNA
   <213> Homo sapiens
<400> 30